(19) Europäisches Patentamt
European Patent Office
Office européen des brevets

(11) **EP 3 715 477 A1**

(12) **EUROPEAN PATENT APPLICATION**

(43) Date of publication:
**30.09.2020 Bulletin 2020/40**

(51) Int Cl.:
***C12Q 1/70*** *(2006.01)*     ***C12Q 1/6886*** *(2018.01)*

(21) Application number: **19305394.9**

(22) Date of filing: **28.03.2019**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Institut Pasteur**
**75015 Paris (FR)**

(72) Inventors:
• **ELOIT, Marc**
 **75006 PARIS (FR)**

• **PEROT, Philippe**
 **75014 PARIS (FR)**
• **BITON, Anne Emmanuelle Marie**
 **75015 PARIS (FR)**

(74) Representative: **Gevers & Orès**
**Immeuble le Palatin 2**
**3 Cours du Triangle**
**CS 80165**
**92939 Paris La Défense Cedex (FR)**

Remarks:
The complete document including Reference Tables and the Sequence Listing can be downloaded from the EPO website

(54) **COMPOSITION OF PRIMERS FOR DETECTING HIGH GRADE SQUAMOUS INTRAEPITHELIAL LESION**

(57) The present invention relates to a composition of primers for detecting HSIL comprising a first set of primers, called splice junctions set of primers which comprises at least 2 pairs of primers of each of a first subset of pairs of primers specific of HPV16, a second subset specific of HPV18, a third subset specific of HPV31, a fourth subset specific of HPV33, a fifth subset specific of HPV35, a sixth subset specific of HPV39, a seventh subset specific of HPV45, a eighth subset specific of HPV51, a ninth subset specific of HPV52, a tenth subset specific of HPV56, an eleventh subset specific of HPV58, a twelfth subset specific of HPV59 and a thirteenth subset specific of HPV66.

FIG. 1

**Description**

**FIELD OF THE INVENTION:**

The present invention relates to a composition of primers, a kit and a method for detecting high grade squamous intraepithelial lesion (HSIL) and/or for typing a Human Papillomavirus (HPV).

**BACKGROUND OF THE INVENTION:**

Human papillomaviruses (HPV) infections are associated with the development of cervical carcinoma, one of the most common cancers among women, and other cancers like anal cancer (Lin C et al. Human papillomavirus types from infection to cancer in the anus, according to sex and HIV status: a systematic review and meta-analysis. Lancet Infect Dis, 2018, 18:198-206) and head and neck cancer (Chaturvedi AK, et al. Human papillomavirus and rising oropharyngeal cancer incidence in the United States. J Clin Oncol Off J Am Soc Clin Oncol, 2011, 29:4294-301). HPV are the etiologic agents responsible for over 99% of all cervical cancers (Walboomers JM, et al. Human papillomavirus is a necessary cause of invasive cervical cancer worldwide. J Pathol, 1999, 189:12-9). HPV are small, non-enveloped DNA viruses commonly transmitted through sexual contact, which infect basal cells and replicate in the nucleus of squamous epithelial cells. HPV include more than 200 genotypes characterized by their oncogenic potential, with highly oncogenic HPV types (high-risk HPV) having a unique ability to drive cell proliferation (Schiffman M, et al. S. Carcinogenic human papillomavirus infection. Nat Rev Dis Primer, 2016, 2:16086).

The genomic organization of papillomaviruses is divided into functional early and late regions. The model of HPV infection, which is mainly derived from knowledge on HPV16, is that following the infection of basal cells in the cervical epithelium, the early HPV genes (E6, E7, E1, E2, E4 and E5) are expressed and the viral DNA replicates from the episomal form of the viral DNA. As the cells divide, in the upper layers of the epithelium the viral genome is replicated further, and the late genes (L1 and L2) and E4 are expressed. Viral shedding then further initiates new infections (Woodman CBJ, et al. The natural history of cervical HPV infection: unresolved issues. Nat Rev Cancer, 2007, 7:11-22).

HPV infection during the development of cervical cancer is associated with a shift from productive infection (which in most of the cases will be cleared by the immune system), towards non-productive persistent and transforming infection (in a minority of cases) characterized in particular by a high level of E6 and E7 mRNAs and low expression of E2 and late genes such as L1 (Doorbar J, et al. The biology and life-cycle of human papillomaviruses. Vaccine, 2012, 30 Suppl 5:F55-70, Shulzhenko N, et al. Ménage à trois: an evolutionary interplay between human papillomavirus, a tumor, and a woman. Trends Microbiol, 2014, 22:345-53). High-risk HPV infection may result in low-grade lesions, with highly productive infection and high rate of spontaneous regression. In contrast, high-risk persistent HPV infection is responsible for high-grade lesion, the true precancerous lesion.

Cervical cancer screening allows detection and treatment of precancerous lesions before the development of cervical cancer. Screening is based on different algorithms, some allowing detection of HPV, and others identifying abnormal cells. Despite the role of high-risk HPV in cervical cancer, screening tests of cancer or precancerous lesions remain in many countries mainly based on the Papanicolaou (Pap) cytology test and do not include molecular virology tests (Schiffman M, et al. 2016). This is largely due to the low Positive Predictive Value (PPV) of current molecular tests. Indeed, because most of the current molecular diagnostic methods rely on the detection of HPV genome (DNA) and do not address the patterns of viral expression (RNA), they remain weak predictors of the evolution from low-grade squamous intraepithelial lesion (LSIL) to high-grade squamous intraepithelial lesion (HSIL) of the cervix (Tornesello ML, et al. Viral and cellular biomarkers in the diagnosis of cervical intraepithelial neoplasia and cancer. BioMed Res Int, 2013, 2013:519619). In addition, DNA identification of high-risk HPV is not fully predictive of cancer since only persistence for years of high-risk HPV is associated with an increased risk of cancer development (Schiffman M, et al. 2016). Thus, the use of HPV DNA tests, as a screening assay, is currently increasing worldwide and shows high sensitivity (Ogilvie GS, et al. Effect of Screening With Primary Cervical HPV Testing vs Cytology Testing on High-grade Cervical Intraepithelial Neoplasia at 48 Months: The HPV FOCAL Randomized Clinical Trial. JAMA, 2018, 320:43-52) but low PPV for HSIL detection (Cuzick J, et al. Comparing the performance of six human papillomavirus tests in a screening population. Br J Cancer, 2013, 108:908-13).

HPV RNA tests and in particular expression of E6 and E7 mRNAs of high-risk HPV have been proposed as better molecular markers of cancer development, but E6 and E7 are also expressed during HPV transient infection so it remains difficult to define a threshold of expression associated with the persistence and evolution to high-grade lesions and cancer. There is no consensus that HPV RNA tests have a better diagnostic accuracy compared to HPV DNA tests and cytology for the detection of cervical precancerous lesions (Virtanen E, et al. Performance of mRNA- and DNA-based high-risk human papillomavirus assays in detection of high-grade cervical lesions. Acta Obstet Gynecol Scand, 2017, 96:61-8, Cook DA, et al. Aptima HPV Assay versus Hybrid Capture® 2 HPV test for primary cervical cancer screening in the HPV FOCAL trial. J Clin Virol Off Publ Pan Am Soc Clin Virol, 2017, 87:23-9, Ge Y et al. Aptima Human Papillomavirus E6/E7 mRNA Test Results Strongly Associated With Risk for High-Grade Cervical Lesions in Follow-Up Biopsies. J Low Genit Tract Dis, 2018, 22:195-200). There is therefore a need for a novel generation of molecular diagnostic tests that can not only detect HPV infection, but also have the ability to accurately predict precancerous stages to offer a better and cost saving medical benefit (de Thurah L, et al. Concordant testing results between various human papillomavirus assays in primary cervical cancer screening: systematic review. Clin Microbiol Infect Off Publ Eur Soc Clin Microbiol Infect Dis, 2018, 24:29-36, Hawkes D, et al. Not all HPV nucleic acid tests are equal: only those calibrated to detect high grade lesions matter for cervical screening. Clin Microbiol Infect Off Publ Eur Soc Clin Microbiol Infect Dis, 2018, 24:436-7, de Thurah L, et al. Not all HPV nucleic acid tests are equal: only those calibrated to detect high grade lesions matter for cervical screening: Response to "Concordant testing results between various human papillomavirus assays in primary cervical cancer screening: systematic review" Published 27 May, 2017. Clin Microbiol Infect Off Publ Eur Soc Clin Microbiol Infect Dis, 2018, 24:438-9).

## SUMMARY OF THE INVENTION:

Now, taking advantage of Next-Generation Sequencing (NGS) technologies, the inventors have developed a multiplexed amplification system targeting the virus splice junctions coupled with NGS analysis that allows to describe fine equilibrium among transcript species of 13 high-risk HPV (HPV16, 18, 31, 33, 35, 39, 45, 51, 52, 56, 58, 59, 66) plus 3 putative high-risk HPV (HPV68, 73, 82), in a single reaction. This molecular approach makes, in particular, possible to take a snapshot of the early vs late populations of HPV transcripts and to define a model based on a combination of reads that reflects the biology of the virus, which can then be correlated to the evolution of lesions. The ultimate goal is to replace the conventional methods of the triage of women at risk of transforming infection before colposcopy.

Based on a study conducted on 55 patients, starting from cervical smears conserved at room temperature, the inventors have showed that the method of the invention can be used as a marker of high-grade cytology, with encouraging diagnostic performances as a triage test.

A subject of the present invention is therefore a composition of primers for detecting high grade squamous intraepithelial lesion (HSIL) comprising a first set of primers, called splice junctions set of primers, which comprises:

- at least 2 pairs of primers of a first subset of HPV16 specific pairs of primers having the nucleic acid sequence selected from the group consisting of SEQ ID NO: 1-2 to SEQ ID NO: 27-28;
and

- at least 2 pairs of primers of a second subset of HPV18 specific pairs of primers having the nucleic acid sequence selected from the group consisting of SEQ ID NO: 29-30 to SEQ ID NO: 63-64;
and

- at least 2 pairs of primers of a third subset of HPV31 specific pairs of primers having the nucleic acid sequence selected from the group consisting of SEQ ID NO: 65-66 to SEQ ID NO: 91-92;
and

- at least 2 pairs of primers of a fourth subset of HPV33 specific pairs of primers having the nucleic acid sequence selected from the group consisting of SEQ ID NO: 93-94 to SEQ ID NO: 117-118;
and

- at least 2 pairs of primers of a fifth subset of HPV35 specific pairs of primers having the nucleic acid sequence selected from the group consisting of SEQ ID NO: 119-120 to SEQ ID NO: 145-146;
and

- at least 2 pairs of primers of a sixth subset of HPV39 specific pairs of primers having the nucleic acid sequence selected from the group consisting of SEQ ID NO: 147-148 to SEQ ID NO: 165-166;
and

- at least 2 pairs of primers of a seventh subset of HPV45 specific pairs of primers having the nucleic acid sequence selected from the group consisting of SEQ ID NO: 167-168 to SEQ ID NO: 193-194;
and

- at least 2 pairs of primers of a eighth subset of HPV51 specific pairs of primers having the nucleic acid sequence selected from the group consisting of SEQ ID NO: 195-196 to SEQ ID NO: 213-214;
and

- at least 2 pairs of primers of a ninth subset of HPV52 specific pairs of primers having the nucleic acid sequence selected from the group consisting of SEQ ID NO: 215-216 to SEQ ID NO: 245-246; and

- at least 2 pairs of primers of a tenth subset of HPV56 specific pairs of primers having the nucleic acid sequence selected from the group consisting of SEQ ID NO: 247-248 to SEQ ID NO: 277-278; and

- at least 2 pairs of primers of an eleventh subset of HPV58 specific pairs of primers having the nucleic acid sequence selected from the group consisting of SEQ ID NO: 279-280 to SEQ ID NO: 303-304; and

- at least 2 pairs of primers of a twelfth subset of HPV59 specific pairs of primers having the nucleic acid sequence selected from the group consisting of SEQ ID NO: 305-306 to SEQ ID NO: 331-332; and

- at least 2 pairs of primers of a thirteenth subset of HPV66 specific pairs of primers having the nucleic acid sequence selected from the group consisting of SEQ ID NO: 333-334 to SEQ ID NO: 361-362.

The aim of the invention is notably to lower the number of primers used in the multiplex system. This lowering of the numbers of primers may be done by lowering the number of the targeted splice junctions and by using redundant nucleic acid sequences.

Thus the 362 nucleic acid sequences of the primers of the splice junctions set primers are redundant and represent in fact 165 unique nucleic acid sequences.

The present invention also relates to a composition of primers for detecting HSIL comprising a first set of pairs of primers, called splice junctions set of primers, which comprises:

- at least 2 pairs of primers of a first subset of HPV16 specific pairs of primers able to produce the amplicons having the nucleic acid sequence selected from the group consisting of SEQ ID NO: 1501 to SEQ ID NO: 1514; and

- at least 2 pairs of primers of a second subset of HPV18 specific pairs of primers able to produce the amplicons having the nucleic acid sequence selected from the group consisting of SEQ ID NO: 1515 to SEQ ID NO: 1532; and

- at least 2 pairs of primers of a third subset of HPV31 specific pairs of primers able to produce the amplicons having the nucleic acid sequence selected from the group consisting of SEQ ID NO: 1533 to SEQ ID NO: 1546; and

- at least 2 pairs of primers of a fourth subset of HPV33 specific pairs of primers able to produce the amplicons having the nucleic acid sequence selected from the group consisting of SEQ ID NO:1547 to SEQ ID NO:1559; and

- at least 2 pairs of primers of a fifth subset of HPV35 specific pairs of primers able to produce the amplicons having the nucleic acid sequence selected from the group consisting of SEQ ID NO: 1560 to SEQ ID NO: 1573; and

- at least 2 pairs of primers of a sixth subset of HPV39 specific pairs of primers able to produce the amplicons having the nucleic acid sequence selected from the group consisting of SEQ ID NO: 1574 to SEQ ID NO: 1583; and

- at least 2 pairs of primers of a seventh subset of HPV45 specific pairs of primers able to produce the amplicons having the nucleic acid sequence selected from the group consisting of SEQ ID NO: 1584 to SEQ ID NO: 1597; and

- at least 2 pairs of primers of a eighth subset of HPV51 specific pairs of primers able to produce the amplicons having the nucleic acid sequence selected from the group consisting of SEQ ID NO: 1598 to SEQ ID NO: 1607; and

- at least 2 pairs of primers of a ninth subset of HPV52 specific pairs of primers able to produce the amplicons having the nucleic acid sequence selected from the group consisting of SEQ ID NO: 1608 to SEQ ID NO: 1623; and

- at least 2 pairs of primers of a tenth subset of HPV56 specific pairs of primers able to produce the amplicons having the nucleic acid sequence selected from the group consisting of SEQ ID NO: 1624 to SEQ ID NO: 1639; and

- at least 2 pairs of primers of an eleventh subset of HPV58 specific pairs of primers able to produce the amplicons having the nucleic acid sequence selected from the group consisting of SEQ ID NO:1640 to SEQ ID NO: 1652; and

- at least 2 pairs of primers of a twelfth subset of HPV59 specific pairs of primers able to produce the amplicons having the nucleic acid sequence selected from the group consisting of SEQ ID NO: 1653 to SEQ ID NO: 1666; and

- at least 2 pairs of primers of a thirteenth subset of HPV66 specific pairs of primers able to produce the amplicons having the nucleic acid sequence selected from the group consisting of SEQ ID NO: 1667 to SEQ ID NO: 1681.

## DETAILED DESCRIPTION OF THE INVENTION:

*Definitions*

High-risk HPV also called HR-HPV herein refer to the HPV of the following types: HPV16, HPV18, HPV31, HPV33, HPV35, HPV39, HPV45, HPV51, HPV52, HPV56, HPV58, HPV59 and HPV66.

Putative high risk HPV herein refer to the HPV of the following types: HPV68, HPV73 and HPV82.

HSIL refers to high grade squamous intraepithelial lesion. HSIL may be cervical, anogenital, head and neck HSIL. Preferably, HSIL is cervix HSIL.

LSIL refers to low grade squamous intraepithelial lesion. LSIL may be cervical, anogenital, head and neck LSIL. Preferably, LSIL is cervix LSIL.

Splice junctions set of primers refer herein to a set of primers which target high risk and optionally putative high risk HPV splice events involving a pair of splice donor (SD) and splice acceptor (SA) sites.

Unsplice junctions set of primers refer herein to a set of primers which target high risk and optionally putative high risk HPV genomic regions spanning either splice donor or splice acceptor sites in the absence of any splice event. In this context, the term "junction" refers to exon-intron interface (i.e. the position where a donor or acceptor site would be found in case of a splice event).

Genomic set of primers refer herein to a set of primers which target high risk and optionally of putative high risk HPV genomic regions away from any splice donor or splice acceptor sites.

Fusion set of primers refer herein to a set of primers which target high risk and optionally of putative high risk HPV fusion transcripts.

Human set of primers refer herein to a set of primers which target human sequences.

HPV RNA Seq refers herein to a multiplexed amplification system coupled with Next Generation Sequencing analysis.

The expression *"the nucleic acid sequence selected from the group consisting of SEQ ID NO: x-x+1 to SEQ ID NO: x+n-x+n+1"* means *"the nucleic acid sequence selected from the group consisting of SEQ ID NO: x-x+1, SEQ ID NO: x+2-x+3, SEQ ID NO: x+4-x+5.... and SEQ ID NO: x+n-x+n+1"*. For example, the nucleic acid sequence selected from the group consisting of SEQ ID NO: 1-2 to SEQ ID NO: 5-6 means the nucleic acid sequence selected from the group consisting of SEQ ID NO: 1-2 (pair of primers SEQ ID NO: 1 and SEQ ID NO: 2), SEQ ID NO: 3-4 (pair of primers SEQ ID NO: 3 and SEQ ID NO: 4) and SEQ ID NO: 5-6 (pair of primers SEQ ID NO: 5 and SEQ ID NO: 6).

The expression *"the nucleic acid sequence selected from the group consisting of SEQ ID NO: x to SEQ ID NO: x+n"* means *"the nucleic acid sequence selected from the group consisting of SEQ ID NO: x, SEQ ID NO: x+1, SEQ ID NO: x+2.... and SEQ ID NO: x+n"*. For example, the nucleic acid sequence selected from the group consisting of SEQ ID NO: 1 to SEQ ID NO: 6 means the nucleic acid sequence selected from the group consisting of SEQ ID NO: 1, SEQ ID NO: 2, SEQ ID NO: 3, SEQ ID NO: 4, SEQ ID NO: 5 and SEQ ID NO: 6.

Biological samples as referred herein include, without limitation, mammalian bodily fluids, especially oral fluids or scrapings, genital scrapings, in particular cervix scrapings.

Primers and amplicons encompassed by the invention are not limited to the sequences defined in the primers and amplicons depicted below. Primers and amplicons may encompass primers having at least 95% of identity with the primers and amplicons defined below. Primers can also comprise extra bases at the 5' end. Also, primers shall be understood as embracing shorter sequences of at least 12, 15, 20 or 25 consecutive bases of the primers featured below. In some embodiments, it shall be understood that the invention also contemplates generic probes which have the sequences of the primers depicted herein and which are directly or indirectly labeled. The probes and primers can be extended or swifted from 1 to 15 bases depending on the desired specificity of the PCR amplification step and/or on the specificity of the detection step using standard parameters such as the nucleic acid size and GC contents, stringent hybridization conditions and temperature reactions. For example, low stringency conditions are used when it is desired to obtain broad positive results on a range of homologous targets whereas high stringency conditions are preferred to obtain positive results only if the specific target nucleic is present in the sample.

As used herein, the term "stringent hybridization conditions" refers to conditions under which the primer or probe will hybridize only to that exactly complementary target(s). The hybridization conditions affect the stability of hybrids, e.g., temperature, salt concentration, pH, formamide concentration and the like. These conditions are optimized to maximize specific binding and minimize non-specific binding of primer or probe to its target nucleic acid sequence. Stringent conditions are sequence dependent and will be different in different circumstances. Longer sequences hybridize specifically at higher temperatures. Generally, stringent conditions are selected to be about 5°C lower than the thermal melting point (Tm) for the specific sequences at a defined ionic strength and pH. The Tm is the temperature (under defined ionic strength and pH) at which 50% of a complementary target sequence hybridizes to a perfectly matched probe or primer. Typically, stringent conditions will be those in which the salt concentration is less than about 1.0 M Na+, typically about 0.01 to 1.0 M Na+ concentration (or other salts) at pH 7.0 to 8.3 and the temperature is at least about 30°C for short probes or primers (e.g. 10 to 50 nucleotides) and at least about 60°C for long probes or primers (e.g. greater than 50 nucleotides). Stringent conditions may also be achieved with the addition of destabilizing agents such as formamide. Exemplary low stringent conditions include hybridization with a buffer solution of 20-30% formamide, 1 M NaCl, 1% SDS at 37°C and a wash in 2*SSC at 40°C. Exemplary high stringency conditions include hybridization in 40-50% formamide, 1 M NaCl, 1 % SDS at 37°C, and a wash in 0.1*SSC at 60°C. Determination of particular hybridization conditions relating to a specified nucleic acid is routine and is well known in the art, for instance, as described in J. Sambrook and D.W. Russell, Molecular Cloning: A Laboratory Manual, Cold Spring Harbor Laboratory Press; 3rd Ed., 2001; and F.M. Ausubel, Ed., Short Protocols in Molecular Biology, Current Protocols; 5th Ed., 2002.

Moreover, to improve the hybridization with the coupled oligonucleotide, it can be advantageous for the oligonucleotide to contain an "arm" and a "spacer" sequence of bases. The use of an arm makes it possible, in effect, to bind the primer at a chosen distance from the support, enabling its conditions of interaction with the DNA to be improved. The arm advantageously consists of a linear carbon chain, comprising 1 to 18 and preferably 6 or 12 ($CH_2$) groups, and an amine which permits binding to the column. The arm is linked to a phosphate of the oligonucleotide or of a "spacer" composed of bases which do not interfere with the hybridization. Thus, the "spacer" can comprise purine bases. As an example, the "spacer" can comprise the sequence GAGG. The arm is advantageously composed of a linear carbon chain comprising 6 or 12 carbon atoms.

For implementation of the present invention, different types of support may be used. These can be functionalized chromatographic supports, in bulk or prepacked in a column, functionalized plastic surfaces or functionalized latex beads, magnetic or otherwise. Chromatographic supports are preferably used. As an example, the chromatographic supports capable of being used are agarose, acrylamide or dextran as well as their derivatives (such as Sephadex, Sepharose, Superose, etc.), polymers such as poly(styrene/divinylbenzene), or grafted or ungrafted silica, for example. The chromatography columns can operate in the diffusion or perfusion mode.

As used herein, the term "sequencing" is used in a broad sense and refers to any technique known by the skilled person including but not limited to Sanger dideoxy termination sequencing, whole- genome sequencing, sequencing by hybridization, pyrosequencing, capillary electrophoresis, cycle sequencing, single-base extension sequencing, solid- phase sequencing, high-throughput sequencing, massively parallel signature sequencing (MPSS), sequencing by reversible dye terminator, paired-end sequencing, near-term sequencing, exonuclease sequencing, sequencing by ligation, short-read sequencing, single-molecule sequencing, sequencing-by-synthesis, real-time sequencing, reverse-terminator sequencing, nanopore sequencing, 454 sequencing, Solexa Genome Analyzer sequencing, SOLiD sequencing, MS-PET sequencing, mass spectrometry, and a combination thereof. In specific embodiments, the method and kit of the invention is adapted to run on ABI PRISM(R) 377 DNA Sequencer, an ABI PRISM(R) 310, 3100, 3100-Avant, 3730, or 3730x1 Genetic Analyzer, an ABI PRISM(R) 3700 DNA Analyzer, or an Applied Biosystems SOLiD(TM) System (all from Applied Biosystems), a Genome Sequencer 20 System (Roche Applied Science).

For all technologies described herein, although the said primers can be used in solution, in another embodiment the said primers are linked to a solid support.

To permit its covalent coupling to the support, the primer is generally functionalized. Thus, it may be modified by a thiol, amine or carboxyl terminal group at the 5' or 3' position. In particular, the addition of a thiol, amine or carboxyl group makes it possible, for example, to couple the oligonucleotide to a support bearing disulphide, maleimide, amine, carboxyl, ester, epoxide, cyanogen bromide or aldehyde functions. These couplings form by establishment of disulphide, thioether, ester, amide or amine links between the primer and the support. Any other method known to a person skilled in the art may be used, such as bifunctional coupling reagents, for example.

Moreover, to improve the hybridization with the coupled oligonucleotide, it can be advantageous for the oligonucleotide to contain an "arm" and a "spacer" sequence of bases. The use of an arm makes it possible, in effect, to bind the primer at a chosen distance from the support, enabling its conditions of interaction with the DNA to be improved. The arm advantageously consists of a linear carbon chain, comprising 1 to 18 and preferably 6 or 12 ($CH_2$) groups, and an amine which permits binding to the column. The arm is linked to a phosphate of the oligonucleotide or of a "spacer" composed of bases which do not interfere with the hybridization. Thus, the "spacer" can comprise purine bases. As an example, the "spacer" can comprise the sequence GAGG. The arm is advantageously composed of a linear carbon chain comprising 6 or 12 carbon atoms.

For implementation of the present invention, different types of support may be used. These can be functionalized chromatographic supports, in bulk or prepacked in a column, functionalized plastic surfaces or functionalized latex beads, magnetic or otherwise. Chromatographic supports are preferably used. As an example, the chromatographic supports capable of being used are agarose, acrylamide or dextran as well as their derivatives (such as Sephadex, Sepharose, Superose, etc.), polymers such as poly(styrene/divinylbenzene), or grafted or ungrafted silica, for example. The chromatography columns can operate in the diffusion or perfusion mode.

### *Composition of primers for detecting high grade squamous intraepithelial lesion*

The present invention relates to a composition of primers for detecting HSIL comprising a first set of primers, called splice junctions set of primers, which comprises:

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers of a first subset of HPV16 specific pairs of primers having the nucleic acid sequence selected from the group consisting of SEQ ID NO: 1-2, SEQ ID NO: 3-4, SEQ ID NO: 5-6, SEQ ID NO: 7-8, SEQ ID NO: 9-10, SEQ ID NO: 11-12, SEQ ID NO: 13-14, SEQ ID NO: 15-16, SEQ ID NO: 17-18, SEQ ID NO: 19-20, SEQ ID NO: 21-22, SEQ ID NO: 23-24, SEQ ID NO: 25-26 and SEQ ID NO: 27-28; and

- at least 2, at least 3, at least 4, at least 5, at least 6, at least 7, at least 8, at least 9, at least 10, at least 11, at least 12, at least 13, at least 14, at least 15, at least 16, at least 17 or at least 18, or 2, 3, 4, 5, 6, 7, 8, 9, 10, 11, 12, 13, 14, 15, 16, 17 or 18 pairs of primers of a second subset of HPV18 specific pairs of primers having the nucleic acid sequence selected from the group consisting of SEQ ID NO: 29-30, SEQ ID NO: 31-32, SEQ ID NO: 33-34, SEQ ID NO: 35-36, SEQ ID NO: 37-38, SEQ ID NO: 39-40, SEQ ID NO: 41-42, SEQ ID NO: 43-44, SEQ ID NO: 45-46, SEQ ID NO: 47-48, SEQ ID NO: 49-50, SEQ ID NO: 51-52, SEQ ID NO: 53-54, SEQ ID NO: 55-56, SEQ ID NO: 57-58, SEQ ID NO: 59-60, SEQ ID NO: 61-62 and SEQ ID NO: 63-64; and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers of a third subset of HPV31 specific pairs of primers having the nucleic acid sequence selected from the group consisting of SEQ ID NO: 65-66, SEQ ID NO: 67-68, SEQ ID NO: 69-70, SEQ ID NO: 71-72, SEQ ID NO: 73-74, SEQ ID NO: 75-76, SEQ ID NO: 77-78, SEQ ID NO: 79-80, SEQ ID NO: 81-82, SEQ ID NO: 83-84, SEQ ID NO: 85-86, SEQ ID NO: 87-88, SEQ ID NO: 89-90 and SEQ ID NO: 91-92; and

- at least 2, at least 3, at least 4, at least 5, at least 6, at least 7, at least 8, at least 9, at least 10, at least 11, at least 12 or at least 13, or 2, 3, 4, 5, 6, 7, 8, 9, 10, 11, 12 or 13 pairs of primers of a fourth subset of HPV33 specific pairs of primers having the nucleic acid sequence selected from the group consisting of SEQ ID NO: 93-94, SEQ ID NO: 95-96, SEQ ID NO: 97-98, SEQ ID NO: 99-100, SEQ ID NO: 101-102, SEQ ID NO: 103-104, SEQ ID NO: 105-106, SEQ ID NO: 107-108, SEQ ID NO: 109-110, SEQ ID NO: 111-112, SEQ ID NO: 113-114, SEQ ID NO: 115-116 and SEQ ID NO: 117-118; and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers of a fifth subset of HPV35 specific pairs of primers having the nucleic acid sequence selected from the group consisting of SEQ ID NO: 119-120, SEQ ID NO: 121-122, SEQ ID NO: 123-124, SEQ ID NO: 125-126, SEQ ID NO: 127-128, SEQ ID NO: 129-130, SEQ ID NO: 131-132, SEQ ID NO: 133-134, SEQ ID NO: 135-136, SEQ ID NO: 137-138, SEQ ID NO: 139-140, SEQ ID NO: 141-142, SEQ ID NO: 143-144 and SEQ ID NO: 145-146; and

- at least 2, at least 3, at least 4, at least 5, at least 6, at least 7, at least 8, at least 9 or at least 10, or 2, 3, 4, 5, 6, 7, 8, 9 or 10 pairs of primers of a sixth subset of HPV39 specific pairs of primers having the nucleic acid sequence selected from the group consisting of SEQ ID NO: 147-148, SEQ ID NO: 149-150, SEQ ID NO: 151-152, SEQ ID NO: 153-154, SEQ ID NO: 155-156, SEQ ID NO: 157-158, SEQ ID NO: 159-160, SEQ ID NO: 161-162, SEQ ID NO: 163-164 and SEQ ID NO: 165-166;
  and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13, or 14 pairs of primers of a seventh subset of HPV45 specific pairs of primers having the nucleic acid sequence selected from the group consisting of SEQ ID NO: 167-168, SEQ ID NO: 169-170, SEQ ID NO: 171-172, SEQ ID NO: 173-174, SEQ ID NO: 175-176, SEQ ID NO: 177-178, SEQ ID NO: 179-180, SEQ ID NO: 181-182, SEQ ID NO: 183-184, SEQ ID NO: 185-186, SEQ ID NO: 187-188, SEQ ID NO: 189-190, SEQ ID NO: 191-192 and SEQ ID NO: 193-194;
  and

- at least 2, at least 3, at least 4, at least 5, at least 6, at least 7, at least 8, at least 9 or at least 10, or 2, 3, 4, 5, 6, 7, 8, 9 or 10 pairs of primers of an eighth subset of HPV51 specific pairs of primers having the nucleic acid sequence selected from the group consisting of SEQ ID NO: 195-196, SEQ ID NO: 197-198, SEQ ID NO: 199-200; SEQ ID NO: 201-202, SEQ ID NO:203-204, SEQ ID NO: 205-206, SEQ ID NO: 207-208, SEQ ID NO: 209-210, SEQ ID NO: 211-212 and SEQ ID NO: 213-214;
  and

- at least 2, at least 3, at least 4, at least 5, at least 6, at least 7, at least 8, at least 9, at least 10, at least 11, at least 12, at least 13, at least 14, at least 15 or at least 16, or 2, 3, 4, 5, 6, 7, 8, 9, 10, 11, 12, 13, 14, 15 or 16 pairs of primers of a ninth subset of HPV52 specific pairs of primers having the nucleic acid sequence selected from the group consisting of SEQ ID NO: 215-216, SEQ ID NO: 217-218, SEQ ID NO: 219-220, SEQ ID NO: 221-222, SEQ ID NO: 223-224, SEQ ID NO: 225-226, SEQ ID NO: 227-228, SEQ ID NO: 229-230, SEQ ID NO: 231-232, SEQ ID NO: 233-234, SEQ ID NO: 235-236, SEQ ID NO: 237-238, SEQ ID NO: 239-240 SEQ ID NO: 241-242, SEQ ID NO: 243-244 and SEQ ID NO: 245-246;
  and

- at least 2, at least 3, at least 4, at least 5, at least 6, at least 7, at least 8, at least 9, at least 10, at least 11, at least 12, at least 13, at least 14, at least 15 or at least 16, or 2, 3, 4, 5, 6, 7, 8, 9, 10, 11, 12, 13, 14, 15 or 16 pairs of primers of a tenth subset of HPV56 specific pairs of primers having the nucleic acid sequence selected from the group consisting of SEQ ID NO: 247-248, SEQ ID NO: 249-250, SEQ ID NO: 251-252, SEQ ID NO: 253-254, SEQ ID NO: 255-256, SEQ ID NO: 257-258, SEQ ID NO: 259-260, SEQ ID NO: 261-262, SEQ ID NO: 263-264, SEQ ID NO: 265-266, SEQ ID NO: 267-268, SEQ ID NO: 269-270, SEQ ID NO: 271-272, SEQ ID NO: 273-274, SEQ ID NO: 275-276 and SEQ ID NO: 277-278;
  and

- at least 2, at least 3, at least 4, at least 5, at least 6, at least 7, at least 8, at least 9, at least 10, at least 11, at least 12 or at least 13, or 2, 3, 4, 5, 6, 7, 8, 9, 10, 11, 12 or 13 pairs of primers of an eleventh subset of HPV58 specific pairs of primers having the nucleic acid sequence selected from the group consisting of SEQ ID NO: 279-280, SEQ ID NO: 281-282, SEQ ID NO: 283-284, SEQ ID NO: 285-286, SEQ ID NO: 287-288, SEQ ID NO: 289-290, SEQ ID NO: 291-292, SEQ ID NO: 293-294, SEQ ID NO: 295-296, SEQ ID NO: 297-298, SEQ ID NO: 299-300, SEQ ID NO: 301-302 and SEQ ID NO: 303-304;
  and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers of a twelfth subset of HPV59 specific pairs of primers having the nucleic acid sequence selected from the group consisting of SEQ ID NO: 305-306, SEQ ID NO: 307-308, SEQ ID NO: 309-310, SEQ ID NO: 311-312, SEQ ID NO: 313-314, SEQ ID NO: 315-316, SEQ ID NO: 317-318, SEQ ID NO: 319-320, SEQ ID NO: 321-322, SEQ ID NO: 323-324, SEQ ID NO: 325-326, SEQ ID NO: 327-328, SEQ ID NO: 329-330 and SEQ ID NO: 331-332;
  and

- at least 2, at least 3, at least 4, at least 5, at least 6, at least 7, at least 8, at least 9, at least 10, at least 11, at least 12, at least 13, at least 14 or at least 15, or 2, 3, 4, 5, 6, 7, 8, 9, 10, 11, 12, 13, 14 or 15 pairs of primers of a thirteenth subset of HPV66 specific pairs of primers having the nucleic acid sequence selected from the group consisting of SEQ ID NO: 333-334, SEQ ID NO: 335-336, SEQ ID NO: 337-338, SEQ ID NO: 339-340, SEQ ID NO: 341-342, SEQ ID NO: 343-344, SEQ ID NO: 345-346, SEQ ID NO: 347-348, SEQ ID NO: 349-350, SEQ ID NO: 351-352, SEQ ID NO: 353-354, SEQ ID NO: 355-356, SEQ ID NO: 357-358, SEQ ID NO: 359-360 and SEQ ID NO: 361-362.

The splice junctions set of primers may further comprise:

- at least 2, at least 3, at least 4, at least 5, at least 6, at least 7, at least 8, at least 9 or at least 10, or 2, 3, 4, 5, 6, 7, 8, 9 or 10 pairs of primers of a fourteenth subset of HPV68 specific pairs of primers having the nucleic acid sequence selected from the group consisting of SEQ ID NO: 363-364, SEQ ID NO: 365-366, SEQ ID NO: 367-368, SEQ ID NO: 369-370, SEQ ID NO: 371-372, SEQ ID NO: 373-374, SEQ ID NO: 375-376, SEQ ID NO: 377-378, SEQ ID NO: 379-380 and SEQ ID NO: 381-382;
and/or

- at least 2, at least 3, at least 4, at least 5, at least 6, at least 7, at least 8, at least 9, at least 10, at least 11, at least 12 or at least 13, or 2, 3, 4, 5, 6, 7, 8, 9, 10, 11, 12 or 13 pairs of primers of a fifteenth subset of HPV73 specific pairs of primers having the nucleic acid sequence selected from the group consisting of SEQ ID NO: 383-384, SEQ ID NO: 385-386, SEQ ID NO: 387-388, SEQ ID NO: 389-390 SEQ ID NO: 391-392, SEQ ID NO: 393-394, SEQ ID NO: 395-396, SEQ ID NO: 397-398, SEQ ID NO: 399-400, SEQ ID NO: 401-402, SEQ ID NO: 403-404, SEQ ID NO: 405-406 and SEQ ID NO: 407-408;
and/or

- at least 2, at least 3, at least 4, at least 5, at least 6, at least 7, at least 8, at least 9, at least 10 or at least 11, or 2, 3, 4, 5, 6, 7, 8, 9, 10 or 11 pairs of primers of a sixteenth subset of HPV82 specific pairs of primers having the nucleic acid sequence selected from the group consisting of SEQ ID NO: 409-410, SEQ ID NO: 411-412, SEQ ID NO: 413-414, SEQ ID NO: 415-416, SEQ ID NO: 417-418, SEQ ID NO: 419-420, SEQ ID NO: 421-422, SEQ ID NO: 423-424, SEQ ID NO: 425-426, SEQ ID NO: 427-428 and SEQ ID NO: 429-430.

These additional subsets of pairs of primers correspond to the putative high risk HPV: HPV68, HPV73 and HPV82.

The composition of primers for detecting HSIL may comprises the splice junctions set comprising at least 10 pairs of primers of each of the first to the thirteenth subsets of pairs of primers of the splice junctions set of primers as defined above and optionally at least 10 pairs of primers of the fourteenth and/or the fifteenth and/or the sixteenth subsets of pairs of primers of the splice junctions set of primers as defined above.

The composition of primers for detecting HSIL may comprises the splice junctions set comprising 2, 3, 4, 5, 6, 7, 8, 9 or more preferably 10 pairs of primers of each of the first to the thirteenth subsets of pairs of primers of the splice junctions set of primers as defined above and optionally 2, 3, 4, 5, 6, 7, 8, 9 or more preferably 10 pairs of primers of the fourteenth and/or the fifteenth and/or the sixteenth subsets of pairs of primers of the splice junctions set of primers as defined above.

The composition of primers for detecting HSIL may comprises the splice junctions set consisting of 2, 3, 4, 5, 6, 7, 8, 9 or more preferably 10 pairs of primers of each of the first to the thirteenth subsets of pairs of primers of the splice junctions set of primers as defined above and optionally of 2, 3, 4, 5, 6, 7, 8, 9 or more preferably 10 pairs of primers of the fourteenth and/or the fifteenth and/or the sixteenth subsets of pairs of primers of the splice junctions set of primers as defined above.

In one embodiment, the composition of primers for detecting HSIL according to the invention comprises the pairs of primers having the nucleic acid sequence SEQ ID NO: 1-2 to SEQ ID NO: 361-362 and optionally of SEQ ID NO: 363-364 to SEQ ID NO: 381-382 and/or SEQ ID NO: 383-384 to SEQ ID NO: 385-386 and/or SEQ ID NO: 387-388 to SEQ ID NO: 429-430.

In a preferred embodiment, the composition of primers for detecting HSIL according to the invention comprises the pairs of primers having the nucleic acid sequence SEQ ID NO: 1-2 to SEQ ID NO: 429-430.

In one embodiment, the composition of primers for detecting HSIL consists of the pairs of primers having the nucleic acid sequence SEQ ID NO: 1-2 to SEQ ID NO: 361-362 and optionally of SEQ ID NO: 363-364 to SEQ ID NO: 381-382 and/or SEQ ID NO: 383-384 to SEQ ID NO: 385-386 and/or SEQ ID NO: 387-388 to SEQ ID NO: 429-430.

In a preferred embodiment, the composition of primers for detecting HSIL according to the invention consists of the pairs of primers having the nucleic acid sequence SEQ ID NO: 1-2 to SEQ ID NO: 429-430.

The splice junctions set of primers of the invention may be defined by the nucleic acid sequence of the pairs of primers that compose it as defined above or by the nucleic acid sequence of the amplicons which are produced by the pairs of primers that compose it as defined below.

The pairs of primers that compose splice junctions set of primers as defined above correspond to the amplicons which are produced by the pairs of primers that compose splice junctions set of primers as defined below. The correspondence between the pairs of primers and their corresponding amplicons is given in table 2Abis.

The present invention also relates to a composition of primers for detecting HSIL comprising a first set of pairs of primers, called splice junctions set of primers, which comprises:

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers a first subset of HPV16 specific pairs of primers able to produce the amplicons having the nucleic acid sequence selected from the group consisting of SEQ ID NO: 1501, SEQ ID NO: 1502, SEQ ID NO: 1503, SEQ ID NO: 1504, SEQ ID NO: 1505, SEQ ID NO: 1506, SEQ ID NO: 1507, SEQ ID NO: 1508, SEQ ID NO: 1509, SEQ ID NO: 1510, SEQ ID NO: 1511, SEQ ID NO: 1512, SEQ ID NO: 1513 and SEQ ID NO: 1514;
and

- at least 2, at least 3, at least 4, at least 5, at least 6, at least 7, at least 8, at least 9, at least 10, at least 11, at least 12, at least 13, at least 14, at least 15, at least 16, at least 17 or at least 18 or 2, 3, 4, 5, 6, 7, 8, 9, 10, 11, 12, 13, 14, 15, 16, 17 or 18 pairs of primers of a second subset of HPV18 specific pairs of primers able to produce the amplicons having the nucleic acid sequence selected from the group consisting of SEQ ID NO: 1515, SEQ ID NO: 1516, SEQ ID NO: 1517, SEQ ID NO: 1518, SEQ ID NO: 1519, SEQ ID NO: 1520, SEQ ID NO: 1521, SEQ ID NO: 1522, SEQ ID NO: 1523, SEQ ID NO: 1524, SEQ ID NO: 1525, SEQ ID NO: 1526, SEQ ID NO: 1527, SEQ ID NO: 1528, SEQ ID NO: 1529, SEQ ID NO: 1530, SEQ ID NO: 1531 and SEQ ID NO: 1532;
and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 of pairs primers of a third subset of HPV31 specific pairs of primers able to produce the amplicons having the nucleic acid sequence selected from the group consisting of SEQ ID NO: 1533, SEQ ID NO: 1534, SEQ ID NO: 1535, SEQ ID NO: 1536, SEQ ID NO: 1537, SEQ ID NO: 1538, SEQ ID NO: 1539, SEQ ID NO: 1540, SEQ ID NO: 1541, SEQ ID NO: 1542, SEQ ID NO: 1543, SEQ ID NO: 1544, SEQ ID NO: 154 and SEQ ID NO: 1546;
and

- at least 2, at least 3, at least 4, at least 5, at least 6, at least 7, at least 8, at least 9, at least 10, at least 11, at least 12 or at least 13, or 2, 3, 4, 5, 6, 7, 8, 9, 10, 11, 12 or 13 pairs of primers of a fourth subset of pairs of primers HPV33 specific able to produce the amplicons having the nucleic acid sequence selected from the group consisting of SEQ ID NO:1547, SEQ ID NO: 1548, SEQ ID NO: 1549, SEQ ID NO: 1550, SEQ ID NO: 1551, SEQ ID NO: 1552, SEQ ID NO: 1553, SEQ ID NO: 1554, SEQ ID NO: 1555, SEQ ID NO: 1556, SEQ ID NO: 1557, SEQ ID NO: 1558 and SEQ ID NO:1559;
and

- at least 2, at least 3, at least 4, at least 5, at least 6, at least 7, at least 8, at least 9, at least 10, at least 11, at least 12, at least 13, or at least 14 or 2, 3, 4, 5, 6, 7, 8, 9, 10, 11, 12, 13 or 14 pairs of primers of a fifth subset of HPV35 specific pairs of primers able to produce the amplicons having the nucleic acid sequence selected from the group consisting of SEQ ID NO: 1560, SEQ ID NO: 1561, SEQ ID NO: 1562, SEQ ID NO: 1563, SEQ ID NO: 1564, SEQ ID NO: 1565, SEQ ID NO: 1566, SEQ ID NO: 1567, SEQ ID NO: 1568, SEQ ID NO: 1569, SEQ ID NO: 1570, SEQ ID NO: 1571, SEQ ID NO: 1572 and SEQ ID NO: 1573;
and

- at least 2, at least 3, at least 4, at least 5, at least 6, at least 7, at least 8, at least 9 or at least 10, or 2, 3, 4, 5, 6, 7, 8, 9 or 10 pairs of primers of a sixth subset of HPV39 specific pairs of primers able to produce the amplicons having the nucleic acid sequence selected from the group consisting of SEQ ID NO: 1574, SEQ ID NO: 1575, SEQ ID NO: 1576, SEQ ID NO: 1577, SEQ ID NO: 1578, SEQ ID NO: 1579, SEQ ID NO: 1580, SEQ ID NO: 1581, SEQ ID NO: 1582 and SEQ ID NO: 1583;
and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers of a seventh subset of HPV45 specific pairs of primers able to produce the amplicons having the nucleic acid sequence selected from the group consisting of SEQ ID NO: 1584, SEQ ID NO: 1585, SEQ ID NO: 1586, SEQ ID NO: 1587, SEQ ID NO: 1588, SEQ ID NO: 1589, SEQ ID NO: 1590, SEQ ID NO: 1591, SEQ ID NO: 1592, SEQ ID NO: 1593, SEQ ID NO: 1594, SEQ ID NO: 1595, SEQ ID NO: 1596 and SEQ ID NO: 1597;
and

- at least 2, at least 3, at least 4, at least 5, at least 6, at least 7, at least 8, at least 9 or at least 10, or 2, 3, 4, 5, 6, 7, 8, 9 or 10 pairs of primers of an eighth subset of HPV51 specific pairs of primers able to produce the amplicons having the nucleic acid sequence selected from the group consisting of SEQ ID NO: 1598, SEQ ID NO: 1599, SEQ ID NO: 1600, SEQ ID NO: 1601, SEQ ID NO: 1602, SEQ ID NO: 1603, SEQ ID NO: 1604, SEQ ID NO: 1605, SEQ ID NO: 1606 and SEQ ID NO: 1607;
and

- at least 2, at least 3, at least 4, at least 5, at least 6, at least 7, at least 8, at least 9, at least 10, at least 11, at least 12, at least 13, at least 14, at least 15 or at least 16, or 2, 3, 4, 5, 6, 7, 8, 9, 10, 11, 12, 13, 14, 15 or 16 pairs of primers a ninth subset of HPV52 specific pairs of primers able to produce the amplicons having the nucleic acid sequence selected from the group consisting of SEQ ID NO: 1608, SEQ ID NO: 1609, SEQ ID NO: 1610, SEQ ID NO: 1611, SEQ ID NO: 1612, SEQ ID NO: 1613, SEQ ID NO: 1614, SEQ ID NO: 1615, SEQ ID NO: 1616, SEQ ID NO: 1617, SEQ ID NO: 1618, SEQ ID NO: 1619, SEQ ID NO: 1620, SEQ ID NO: 1621, SEQ ID NO: 1622 and SEQ ID NO: 1623;
and

- at least 2, at least 3, at least 4, at least 5, at least 6, at least 7, at least 8, at least 9, at least 10, at least 11, at least 12, at least 13, at least 14, at least 15 or at least 16, or 2, 3, 4, 5, 6, 7, 8, 9, 10, 11, 12, 13, 14, 15 or 16 pairs of primers of a tenth subset of HPV56 specific pairs of primers able to produce the amplicons having the nucleic acid sequence selected from the group consisting of SEQ ID NO: 1624, SEQ ID NO: 1625, SEQ ID NO: 1626, SEQ ID NO: 1627, SEQ ID NO: 1628, SEQ ID NO: 1629, SEQ ID NO: 1630, SEQ ID NO: 1631, SEQ ID NO: 1632, SEQ ID NO: 1633, SEQ ID NO: 1634, SEQ ID NO: 1635, SEQ ID NO: 1636, SEQ ID NO: 1637, SEQ ID NO: 1638 and SEQ ID NO: 1639;
and

- at least 2, at least 3, at least 4, at least 5, at least 6, at least 7, at least 8, at least 9, at least 10, at least 11, at least 12 or at least 13, or 2, 3, 4, 5, 6, 7, 8, 9, 10, 11, 12 or 13 pairs of primers of an eleventh subset of HPV58 specific pairs of primers able to produce the amplicons having the nucleic acid sequence selected from the group consisting of SEQ ID NO: 1640, SEQ ID NO: 1641, SEQ ID NO: 1642, SEQ ID NO: 1643, SEQ ID NO: 1644, SEQ ID NO: 1645, SEQ ID NO: 1646, SEQ ID NO: 1647, SEQ ID NO: 1648, SEQ ID NO: 1649, SEQ ID NO: 1650, SEQ ID NO: 1651 and SEQ ID NO: 1652;
and

- at least 2, at least 3, at least 4, at least 5, at least 6, at least 7, at least 8, at least 9, at least 10, at least 11, at least 12, at least 13 or at least 14, or 2, 3, 4, 5, 6, 7, 8, 9, 10, 11, 12, 13 or 14 pairs of primers of a twelfth subset of HPV59 specific pairs of primers able to produce the amplicons having the nucleic acid sequence selected from the group consisting of SEQ ID NO: 1653, SEQ ID NO: 1654, SEQ ID NO: 1655, SEQ ID NO: 1656, SEQ ID NO: 1657, SEQ ID NO: 1658, SEQ ID NO: 1659, SEQ ID NO: 1660, SEQ ID NO: 1661, SEQ ID NO: 1662, SEQ ID NO: 1663, SEQ ID NO: 1664, SEQ ID NO: 1665 and SEQ ID NO: 1666;
and

- at least 2, at least 3, at least 4, at least 5, at least 6, at least 7, at least 8, at least 9, at least 10, at least 11, at least 12, at least 13, at least 14 or at least 15, or 2, 3, 4, 5, 6, 7, 8, 9, 10, 11, 12, 13, 14 or 15 pairs of primers a thirteenth subset of HPV66 specific pairs of primers able to produce the amplicons having the nucleic acid sequence selected from the group consisting of SEQ ID NO: 1667, SEQ ID NO: 1668, SEQ ID NO: 1669, SEQ ID NO: 1670, SEQ ID NO: 1671, SEQ ID NO: 1672, SEQ ID NO: 1673, SEQ ID NO: 1674, SEQ ID NO: 1675, SEQ ID NO: 1676, SEQ ID NO: 1677, SEQ ID NO: 1678, SEQ ID NO: 1679, SEQ ID NO: 1680 and SEQ ID NO: 1681.

The splice junctions set of primers may further comprise:

- at least 2, at least 3, at least 4, at least 5, at least 6, at least 7, at least 8, at least 9 or at least 10, or 2, 3, 4, 5, 6, 7, 8, 9 or 10 pairs of primers of a fourteenth subset of HPV68 specific pairs of primers able to produce the amplicons having the nucleic acid sequence selected from the group consisting of SEQ ID NO: 1682, SEQ ID NO: 1683, SEQ ID NO: 1684, SEQ ID NO: 1685, SEQ ID NO: 1686, SEQ ID NO: 1687, SEQ ID NO: 1688, SEQ ID NO: 1689, SEQ ID NO: 1690 and SEQ ID NO: 1691;
and/or

- at least 2, at least 3, at least 4, at least 5, at least 6, at least 7, at least 8, at least 9, at least 10, at least 11, at least 12 or at least 13, at least 14, at least 15, at least 16, at least 17 or at least 18, or 2, 3, 4, 5, 6, 7, 8, 9, 10, 11, 12, 13, 14, 15, 16, 17 or 18 pairs of primers of a fifteenth subset of HPV73 specific pairs of primers able to produce the amplicons having the nucleic acid sequence selected from the group consisting of SEQ ID NO: 1692, SEQ ID NO: 1693, SEQ ID NO: 1694, SEQ ID NO: 1695, SEQ ID NO: 1696, SEQ ID NO: 1697, SEQ ID NO: 1698, SEQ ID NO: 1699, SEQ ID NO: 1700, SEQ ID NO: 1701, SEQ ID NO: 1702, SEQ ID NO: 1703 and SEQ ID NO: 1704;
and/or

- at least 2, at least 3, at least 4, at least 5, at least 6, at least 7, at least 8, at least 9, at least 10 or at least 11, or 2, 3, 4, 5, 6, 7, 8, 9, 10 or 11 pairs of primers of a sixteenth subset of HPV82 specific pairs of primers able to produce the amplicons having the nucleic acid sequence selected from the group consisting of SEQ ID NO: 1705, SEQ ID NO: 1706, SEQ ID NO: 1707, SEQ ID NO: 1708, SEQ ID NO: 1709, SEQ ID NO: 1710, SEQ ID NO: 1711, SEQ ID NO: 1712, SEQ ID NO: 1713, SEQ ID NO: 1714 and SEQ ID NO: 1715.

The composition of primers for detecting HSIL may comprises the splice junction set of primers comprising at least 10 pairs of primers of each of the first to the thirteenth subsets of pairs of primers of the splice junctions set of primers as defined above and optionally at least 10 pairs of primers of the fourteenth and/or the fifteenth and/or the sixteenth subsets of pairs of primers of the splice junctions set of primers as defined above.

The composition of primers for detecting HSIL may comprises the splice junction set of primers comprising 2, 3, 4, 5, 6, 7, 8, 9 or more preferably 10 pairs of primers of each of the first to the thirteenth subsets of pairs of primers of the splice junctions set of primers as defined above and optionally 2, 3, 4, 5, 6, 7, 8, 9 or more preferably 10 pairs of primers of the fourteenth and/or the fifteenth and/or the sixteenth subsets of pairs of primers of the splice junctions set of primers as defined above.

The composition of primers for detecting HSIL may comprises the splice junction set of primers consisting of 2, 3, 4, 5, 6, 7, 8, 9 or more preferably 10 pairs of primers of each of the first to the thirteenth subsets of pairs of primers of the splice junctions set of primers as defined above and of optionally 2, 3, 4, 5, 6, 7, 8, 9 or more preferably 10 pairs of primers of the fourteenth and/or the fifteenth and/or the sixteenth subsets of pairs of primers of the splice junctions set of primers as defined above.

In one embodiment, the composition for detecting HSIL of primers according to the invention comprises the splice junction set of primers comprising the pairs of primers able to produce the amplicons having the nucleic acid sequence SEQ ID NO: 1501 to SEQ ID NO: 1681 and optionally of SEQ ID NO: 1682 to SEQ ID NO: 1691 and/or SEQ ID NO: 1692 to SEQ ID NO: 1704 and/or SEQ ID NO: 1705 to SEQ ID NO: 1715.

In a preferred embodiment, the composition of primers for detecting HSIL according to the invention comprises the splice junction set of primers comprising the pairs of primers able to produce the amplicons having the nucleic acid sequence SEQ ID NO: 1501 to SEQ ID NO: 1715.

In one embodiment, the composition of primers consists of the splice junction set of primers consisting of the pairs of primers having the nucleic acid sequence SEQ ID NO: 1501 to SEQ ID NO: 1681 and optionally of SEQ ID NO: 1682 to SEQ ID NO: 1691 and/or SEQ ID NO: 1692 to SEQ ID NO: 1704 and/or SEQ ID NO: 1705 to SEQ ID NO: 1715.

In a preferred embodiment, the composition of primers for detecting HSIL according to the invention consists of the splice junction set of primers consisting of the pairs of primers able to produce the amplicons having the nucleic acid sequence SEQ ID NO: 1501 to SEQ ID NO: 1715.

In one embodiment, the composition of primers for detecting HSIL does not comprise an additional set of primers selected from the group consisting of a unsplice junctions set of primers, a genomic set of primers and a fusion set of primers. In particular, the composition of primers for detecting HSIL may not comprise an unsplice junctions set of primers, a genomic set of primers and a fusion set of primers.

In one embodiment, the composition of primers for detecting HSIL comprises a human set of primers. The primers of the human set of primers target human sequences.

The human set of primers may be used as an internal control.

The human set of primers may comprise at least 1, at least 2, at least 3, at least 4, at least 5, at least 6, at least 7, at least 8, at least 9, at least 10, at least 11, at least 12, at least 13, at least 14, at least 15 , at least 16, at least 17, at least 18, at least 19, at least 20, at least 21, at least 22, at least 23, at least 24, at least 25, at least 26, at least 27, at least 28, at least 29 or at least 30, or 1, 2, 3, 4, 5 ,6,7,8, 9, 10, 11, 12, 13, 14, 15 ,16, 17, 18, 19, 20, 21, 22, 23, 24, 25, 26, 27, 28, 29 or 30 pairs of primers having the nucleic acid sequence selected from the group consisting of SEQ ID NO: 1441-1442, SEQ ID NO: 1443-1444, SEQ ID NO: 1445-1446, SEQ ID NO: 1447-1448, SEQ ID NO: 1449-1450, SEQ ID NO: 1451-1452, SEQ ID NO: 1453-1454, SEQ ID NO: 1455-1456, SEQ ID NO: 1457-1458, SEQ ID NO: 1459-1460, SEQ ID NO: 1461-1462, SEQ ID NO: 1463-1464, SEQ ID NO: 1465-1466, SEQ ID NO: 1467-1468, SEQ ID NO: 1469-1470, SEQ ID NO: 1471-1472, SEQ ID NO: 1473-1474, SEQ ID NO: 1475-1476, SEQ ID NO: 1477-1478, SEQ ID NO: 1479-1480, SEQ ID NO: 1481-1482, SEQ ID NO: 1483-1484, SEQ ID NO: 1485-1486, SEQ ID NO: 1487-1488, SEQ ID NO: 1489-1490, SEQ ID NO: 1491-1492, SEQ ID NO: 1493-1494, SEQ ID NO: 1495-1496, SEQ ID NO: 1497-1498 and SEQ ID NO: 1499-1500.

In one preferred embodiment, the human set of primers comprises SEQ ID NO: 1441-1442 to SEQ ID NO: 1499-1500.

In one more preferred embodiment, the human set of primers consists of SEQ ID NO: 1441-1442 to SEQ ID NO: 1499-1500.

The human set of primers may comprise at least 2, at least 3, at least 4, at least 5 , at least 6, at least 7, at least 8, at least 9, at least 10, at least 11, at least 12, at least 13, at least 14, at least 15 , at least 16, at least 17, at least 18, at least 19, at least 20, at least 21, at least 22, at least 23, at least 24, at least 25, at least 26, at least 27, at least 28, at least 29 or at least 30, or 2, 3, 4, 5 ,6,7,8, 9, 10, 11, 12, 13, 14, 15 ,16, 17, 18, 19, 20, 21, 22, 23, 24, 25, 26, 27, 28, 29 or 30 pairs of primers able to produce the amplicons having the nucleic acid sequence selected from the group consisting of SEQ ID NO: 2221 to SEQ ID NO: 2250.

In one preferred embodiment, the human set of primers comprises pairs of primers able to produce the amplicons having the nucleic acid sequence SEQ ID NO: 2221 to SEQ ID NO: 2250.

In one more preferred embodiment, the human set of primers consists of pairs of primers able to produce the amplicons having the nucleic acid sequence SEQ ID NO: 2221 to SEQ ID NO: 2250.

In one embodiment, the composition of primers for detecting HSIL may also comprise a fusion set of primers. The primers of fusion set of primers target high risk and optionally of putative high risk HPV fusion transcripts.

The primers of fusion set of primers may comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a first subset of HPV16 specific pairs of primers having the nucleic acid sequence selected from the group consisting of SEQ ID NO: 865-866 to SEQ ID NO: 899-900;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a second subset of HPV18 specific pairs of primers having the nucleic acid sequence selected from the group consisting of SEQ ID NO: 901-902 to SEQ ID NO: 935-936;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a third subset of HPV31 specific pairs of primers having the nucleic acid sequence selected from the group consisting of SEQ ID NO: 937-938 to SEQ ID NO: 971-972;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourth subset of HPV33 specific pairs of primers having the nucleic acid sequence selected from the group consisting of SEQ ID NO: 973-974 to SEQ ID NO: 1007-1008;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifth subset of HPV35 specific pairs of primers having the nucleic acid sequence selected from the group consisting of SEQ ID NO: 1009-1010 to SEQ ID NO: 1043-1044;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixth subset of HPV39 specific pairs of primers having the nucleic acid sequence selected from the group consisting of SEQ ID NO: 1045-1046 to SEQ ID NO: 1079-1080;

- at least 1, at least 2, at least 3, at least 4, at least 5, at least 6, at least 7, at least 8, at least 9, at least 10, at least 11, at least 12, at least 13, at least 14, at least 15, at least 16, preferably at least 17, more preferably 18 or 1, 2, 3, 4, 5, 6, 7, 8, 9, 10, 11, 12, 13, 14, 15, 16, 17 or 18 pairs of primers of a seventh subset of HPV45 specific pairs of primers having the nucleic acid sequence selected from the group consisting of SEQ ID NO: 1081-1082 to SEQ ID NO: 1115-1116.

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a eighth subset of HPV51 specific pairs of primers having the nucleic acid sequence selected from the group consisting of SEQ ID NO: 1117-1118 to SEQ ID NO: 1151-1152;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a ninth subset of HPV52 specific pairs of primers having the nucleic acid sequence selected from the group consisting of SEQ ID NO: 1153-1154 to SEQ ID NO: 1187-1188;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a tenth subset of HPV56 specific pairs of primers having the nucleic acid sequence selected from the group consisting of SEQ ID NO: 1189-1190 to SEQ ID NO: 1223-1224;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of an eleventh subset of HPV58 specific pairs of primers having the nucleic acid sequence selected from the group consisting of SEQ ID NO: 1225-1226 to SEQ ID NO: 1259-1260;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a twelfth subset of HPV59 specific pairs of primers having the nucleic acid sequence selected from the group consisting of SEQ ID NO: 1261-1262 to SEQ ID NO: 1295-1296;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a thirteenth subset of HPV66 specific pairs of primers having the nucleic acid sequence selected from the group consisting of SEQ ID NO: 1297-1298 to SEQ ID NO: 1331-1332.

The fusion set of primers may further comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourteenth subset of HPV68 specific pairs of primers having the nucleic acid sequence selected from the group consisting of SEQ ID NO: 1333-1334 to SEQ ID NO: 1367-1368;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifteenth subset of HPV73 specific pairs of primers having the nucleic acid sequence selected from the group consisting of SEQ ID NO: 1369-1370 to SEQ ID NO: 1403-1404;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixteenth subset of HPV82 specific pairs of primers having the nucleic acid sequence selected from the group consisting of SEQ ID NO: 1405-1406 to SEQ ID NO: 1439-1440.

In one embodiment, the fusion set of primers comprises the pairs of primers having the nucleic acid sequence SEQ ID NO: 865-866 to SEQ ID NO: 1331-1332 and optionally of SEQ ID NO: 1333-1334 to SEQ ID NO: 1367-1368 and/or SEQ ID NO: 1369-1370 to SEQ ID NO: 1403-1404 and/or SEQ ID NO: 1405-1406 to SEQ ID NO: 1439-1440.

In a preferred embodiment, the fusion set of primers comprises the pairs of primers having the nucleic acid sequence SEQ ID NO: 865-866 to SEQ ID NO: 1439-1440.

In one embodiment, the fusion set of primers consists of the pairs of primers having the nucleic acid sequence SEQ ID NO: 865-866 to SEQ ID NO: 1331-1332 and optionally of SEQ ID NO: 1333-1334 to SEQ ID NO: 1367-1368 and/or SEQ ID NO: 1369-1370 to SEQ ID NO: 1403-1404 and/or SEQ ID NO: 1405-1406 to SEQ ID NO: 1439-1440.

In a preferred embodiment, the fusion set of primers consists of the pairs of primers having the nucleic acid sequence SEQ ID NO: 865-866 to SEQ ID NO: 1439-1440.

The primers of fusion set of primers may comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a first subset of HPV16 specific pairs of primers able to produce the amplicons having the nucleic acid sequence selected from the group consisting of SEQ ID NO: 1933 to 1 SEQ ID NO: 1950;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a second subset of HPV18 specific pairs of primers able to produce the amplicons having the nucleic acid sequence selected from the group consisting of SEQ ID NO: 1951 to SEQ ID NO: 1968;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a third subset of HPV31 specific pairs of primers able to produce the amplicons having the nucleic acid sequence selected from the group consisting of SEQ ID NO: 1969 to SEQ ID NO: 1986;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourth subset of HPV33 specific pairs of primers able to produce the amplicons having the nucleic acid sequence selected from the group consisting of SEQ ID NO: 1987 to SEQ ID NO: 2004;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifth subset of HPV35 specific pairs of primers able to produce the amplicons having the nucleic acid sequence selected from the group consisting of SEQ ID NO: 2005 to SEQ ID NO: 2022;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixth subset of HPV39 specific pairs of primers able to produce the amplicons having the nucleic acid sequence selected from the group consisting of SEQ ID NO: 2023 to SEQ ID NO: 2040;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a seventh subset of HPV45 specific pairs of primers selected from the group consisting of SEQ ID NO: 2041 to SEQ ID NO: 2058,

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a eighth subset of HPV51 specific pairs of primers able to produce the amplicons having the nucleic acid sequence selected from the group consisting of SEQ ID NO: 2059 to SEQ ID NO: 2076;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a ninth subset of HPV52 specific pairs of primers able to produce the amplicons having the nucleic acid sequence selected from the group consisting of SEQ ID NO: 2077 to SEQ ID NO: 2094;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a tenth subset of HPV56 specific pairs of primers able to produce the amplicons having the nucleic acid sequence selected from the group consisting of SEQ ID NO: 2095 to SEQ ID NO: 2112;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of an eleventh subset of HPV58 specific pairs of primers able to produce the amplicons having the nucleic acid sequence selected from the group consisting of SEQ ID NO: 2113 to SEQ ID NO: 2130;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a twelfth subset of HPV59 specific pairs of primers able to produce the amplicons having the nucleic acid sequence selected from the group consisting of SEQ ID NO: 2131 to SEQ ID NO: 2148;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a thirteenth subset of HPV66 specific pairs of primers able to produce the amplicons having the nucleic acid sequence selected from the group consisting of SEQ ID NO: 2149 to SEQ ID NO: 2166.

The fusion set may also comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourteenth subset of HPV68 specific pairs of primers able to produce the amplicons having the nucleic acid sequence selected from the group consisting of SEQ ID NO: 2167 to SEQ ID NO: 2184; and/or

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifteenth subset of HPV73 specific pairs of primers able to produce the amplicons having the nucleic acid sequence selected from the group consisting of SEQ ID NO:2185 to SEQ ID NO: 2202; and/or

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixteenth subset of HPV82 specific pairs of primers able to produce the amplicons having the nucleic acid sequence selected from the group consisting of SEQ ID NO: 2203 to SEQ ID NO: 2220.

In one embodiment, the fusion set of primers comprises the pairs of primers having the nucleic acid sequence SEQ ID NO: 1933 to SEQ ID NO: 2166 and optionally of SEQ ID NO: 2167 to SEQ ID NO: 2184 and/or SEQ ID NO: 2185 to SEQ ID NO: 2202 and/or SEQ ID NO: 2203 to SEQ ID NO: 2220.

In a preferred embodiment, the fusion set of primers comprises the pairs of primers able to produce the amplicons having the nucleic acid sequence SEQ ID NO: 1869 to SEQ ID NO: 2220.

In one embodiment, the fusion set of primers consists of the pairs of primers able to produce the amplicons having the nucleic acid sequence SEQ ID NO: 1933 to SEQ ID NO: 2166 and optionally of SEQ ID NO: 2167 to SEQ ID NO: 2184 and/or SEQ ID NO: 2185 to SEQ ID NO: 2202 and/or SEQ ID NO: 2203 to SEQ ID NO: 2220.

In a preferred embodiment, the fusion set of primers consists of the pairs of primers able to produce the amplicons having the nucleic acid sequence SEQ ID NO: 1869 to SEQ ID NO: 2220.

The present invention also relates to a kit for detecting HSIL comprising the composition of primers for detecting HSIL of the invention and optionally reagents for a cDNA amplification. Reagents available for this purpose are well-known in the art and include the DNA polymerases, buffers for the enzymes, detergents, enhancing agents. The kit of the invention may also comprise reagent for reverse transcription and/or for sequencing. In some preferred embodiments of the kit of the invention, the primers, and optional reagents are in lyophilised form to allow ambient storage. The components of the kits are packaged together into any of the various containers suitable for nucleic acid amplification such as plates, slides, wells, dishes, beads, particles, cups, strands, chips, strips and others. The kit optionally includes instructions for performing at least one specific embodiment of the method of the invention. In some advantageous embodiments, the kit comprises micro-well plates or microtubes, preferably in a dried format, i.e., wherein the wells of the plates or microtubes comprise a dried composition containing at least the primers, and preferably further comprising all the reagents for the reverse transcription, cDNA amplification or sequencing.

The present invention also relates to the use of the composition of primers for detecting HSIL of the invention or of the kit for detecting HSIL of the invention.

### An in vitro method for detecting HSIL in a biological sample

The present invention also relates to an *in vitro* method for detecting HSIL in a biological sample comprising the steps of:

- (a) extraction of RNA from the biological sample,
- (b) reverse transcription of the RNA so as to generate cDNA,
- (c) amplification of the cDNA generated at step (b) with the composition of primers of the invention so as to produce amplicons,
- (d) quantifying the expression level of each amplicon produced at step (c),
- (e) determining if the biological sample comprises HSIL based on the expression level of the amplicons quantified at step (d).

Preferably, the step (d) of quantifying the expression level of each amplicon is carried by sequencing.

The step (d) of quantifying the expression level of each amplicon may comprise the steps of:

- (d1) sequencing the amplicons so as to generate reads,
- (d2) aligning the reads to sequence of the corresponding amplicon,
- (d3) for each amplicon, quantifying the reads corresponding to the sequence of said amplicon.

The quantification of the expression level of each amplicons may be carried using a partial digestion of the amplicons. Then, the step (d) of quantifying the expression level of each amplicon may comprise the steps of:

- (d1) partially digesting the amplicon so as to generate fragments,
- (d2) sequencing the fragments produced at step (d1) so as to generate reads,
- (d3) aligning the reads to sequence of the corresponding amplicon,
- (d4) for each amplicon, quantifying the reads corresponding to the sequence of said amplicon.

In a preferred embodiment, the step of determining if the biological sample comprises HSIL comprises a step of determining if the biological sample comprises HSIL corresponding to one HPV type defined herein based on the expression level of the amplicons quantified in step (d) specific of the said HPV type. In this embodiment, the step of determining if the biological sample comprises HSIL is carried for each HPV type. Thus, for each HPV type, the expression level of the amplicons corresponding this HPV type is analyzed and it is determined if the biological sample comprises a HSIL corresponding to this HPV type. If it is determined that the biological sample comprises a HSIL corresponding to at least one HPV type, than the biological sample is classified as comprising HSIL.

Preferably, the step of determining if the biological sample comprises HSIL is carried out by using a logistic regression analysis wherein the variables depend on the quantified level of expression the amplicons.

Thus, the step of determining if the biological sample comprises HSIL may comprise:

- for each type of HPV selected from the group consisting of HPV16, HPV18, HPV31, HPV33, HPV35, HPV39, HPV45, HPV51, HPV52, HPV56, HPV58, HPV59, and HPV66 and optionally HPV68 and/or HPV73 and/or HPV82, a step calculating a probability $p_{HPV_j}$ that the biological sample comprises an HSIL of $HPV_j$ type wherein $j$ =16, 18, 31, 33, 35, 39, 45, 51, 52, 56, 58, 59, 66, 68, 73 and/or 82 using the following formula:

$$logit(\mathrm{p}_{HPVj}) = \boldsymbol{\beta_0} + \sum_{i=1}^{25}\left(\boldsymbol{\beta_i\, X_{ij}}\right)$$

with:

$\beta_0$ is the intercept,
$\beta_i$ is a coefficient corresponding to a given splice junction, called splice junction i,
$X_{ij}$ is a variable depending on the quantified level of expression of the amplicon corresponding to the splice junction i for the $HPV_j$

wherein if one $p_{HPVj}$ is higher than 0.5, it is indicative of the presence of a $HPV_j$ HSIL in the biological sample.

In a preferred embodiment, the amplicons corresponding to the splice junction i=1 are respectively:

the amplicons corresponding to the splice junction i=1 are respectively SEQ ID NO: 1501 for HPV16, SEQ ID NO: 1515 for HPV18, SEQ ID NO: 1533 for HPV31, SEQ ID NO: 1547 for HPV33, SEQ ID NO: 1560 for HPV35, SEQ ID NO: 1574 for HPV39, SEQ ID NO: 1584 for HPV45, SEQ ID NO: 1598 for HPV51, SEQ ID NO: 1608 for HPV52, SEQ ID NO: 1624 for HPV56, SEQ ID NO: 1640 for HPV58, SEQ ID NO: 1653 for HPV59, SEQ ID NO: 1667 for HPV66, SEQ ID NO: 1682 for HPV68, SEQ ID NO: 1692 for HPV73 and SEQ ID NO: 1705 for HPV82,
the amplicons corresponding to the splice junction i=2 are respectively SEQ ID NO: 1502 for HPV16, SEQ ID NO: 1516 for HPV18, SEQ ID NO: 1534 for HPV31, SEQ ID NO: 1548 for HPV33, SEQ ID NO: 1561 for HPV35, absent for HPV39, SEQ ID NO: 1585 for HPV45, absent for HPV51, SEQ ID NO: 1609 for HPV52, SEQ ID NO: 1625 for HPV56, SEQ ID NO: 1641 for HPV58, SEQ ID NO: 1654 for HPV59, SEQ ID NO: 1668 for HPV66, absent for HPV68, absent for HPV73 and absent for HPV82,
the amplicons corresponding to the splice junction i=3 are respectively SEQ ID NO: 1503 for HPV16, SEQ ID NO: 1517 for HPV18, SEQ ID NO: 1535 for HPV31, SEQ ID NO: 1549 for HPV33, SEQ ID NO: 1562 for HPV35, absent for HPV39, absent for HPV45, SEQ ID NO: 1599 for HPV51, SEQ ID NO: 1610 for HPV52, SEQ ID NO: 1626 for HPV56, SEQ ID NO: 1642 for HPV58, absent for HPV59, SEQ ID NO: 1669 for HPV66, SEQ ID NO: 1683 for HPV68, SEQ ID NO: 1693 for HPV73 and SEQ ID NO: 1706 for HPV82,
the amplicons corresponding to the splice junction i=4 are respectively SEQ ID NO: 1504 for HPV16, SEQ ID NO: 1518 for HPV18, SEQ ID NO: 1536 for HPV31, SEQ ID NO: 1550 for HPV33, SEQ ID NO: 1563 for HPV35, SEQ ID NO: 1576 for HPV39, SEQ ID NO: 1587 for HPV45, SEQ ID NO: 1600 for HPV51, SEQ ID NO: 1611for HPV52, SEQ ID NO: 1627 for HPV56, SEQ ID NO: 1643 for HPV58, SEQ ID NO: 1656 for HPV59, SEQ ID NO: 1671 for HPV66, SEQ ID NO: 1684 for HPV68, SEQ ID NO: 1694 for HPV7 and SEQ ID NO: 1707 for HPV82,
the amplicons corresponding to the splice junction i=5 are respectively SEQ ID NO: 1505 for HPV16, SEQ ID NO: 1519 for HPV18, SEQ ID NO: 1537 for HPV31, SEQ ID NO: 1551 for HPV33, SEQ ID NO: 1564 for HPV35, absent for HPV39, SEQ ID NO: 1588 for HPV45, absent for HPV51, SEQ ID NO: 1612 for HPV52, SEQ ID NO: 1628 for HPV56, SEQ ID NO: 1644 for HPV58, SEQ ID NO: 1657 for HPV59, SEQ ID NO: 1672 for HPV66, absent for HPV68, absent for HPV73 and absent for HPV82,
the amplicons corresponding to the splice junction i=6 are respectively SEQ ID NO: 1506 for HPV16, SEQ ID NO: 1520 for HPV18, SEQ ID NO: 1538 for HPV31, SEQ ID NO: 1552 for HPV33, SEQ ID NO: 1565 for HPV35, absent for HPV39, absent for HPV45, SEQ ID NO: 1601 for HPV51, SEQ ID NO: 1613 for HPV52, SEQ ID NO: 1629 for HPV56, SEQ ID NO: 1645 for HPV58, absent for HPV59, absent for HPV66, SEQ ID NO: 1686 for HPV68, SEQ ID NO: 1695 for HPV73 and SEQ ID NO: 1708 for HPV82,
the amplicons corresponding to the splice junction i=7 are respectively SEQ ID NO: 1507 for HPV16, SEQ ID NO: 1521 for HPV18, SEQ ID NO: 1539 for HPV31, SEQ ID NO: 1553 for HPV33, SEQ ID NO: 1566 for HPV35, SEQ ID NO: 1578 for HPV39, SEQ ID NO: 1590 for HPV45, SEQ ID NO: 1602 for HPV51, SEQ ID NO: 1614 for HPV52, SEQ ID NO: 1630 for HPV56, SEQ ID NO: 1646 for HPV58, absent for HPV59, SEQ ID NO: 1674 for HPV66, SEQ ID NO: 1685 for HPV68, SEQ ID NO: 1696 for HPV73 and SEQ ID NO: 1709 for HPV82,
the amplicons corresponding to the splice junction i=8 are respectively SEQ ID NO: 1508 for HPV16, absent for HPV18, SEQ ID NO: 1540 for HPV31, SEQ ID NO: 1554 for HPV33, absent for HPV35, absent for HPV39, absent for HPV45, absent for HPV51, SEQ ID NO: 1615 for HPV52, SEQ ID NO: 1631 for HPV56, SEQ ID NO: 1647 for HPV58, absent for HPV59, SEQ ID NO: 1675 for HPV66, absent for HPV68, SEQ ID NO: 1697 for HPV73 and SEQ ID NO: 1710 for HPV82,
the amplicons corresponding to the splice junction i=9 are respectively SEQ ID NO: 1509 for HPV16, SEQ ID NO: 1522 for HPV18, SEQ ID NO: 1541 for HPV31, absent for HPV33, absent for HPV35, SEQ ID NO: 1579 for HPV39, SEQ ID NO: 1591 for HPV45, SEQ ID NO: 1603 for HPV51, SEQ ID NO: 1616 for HPV52, SEQ ID NO: 1632 for HPV56, absent HPV58, SEQ ID NO: 1659 for HPV59, SEQ ID NO: 1676 for HPV66, SEQ ID NO: 1687 for HPV68, SEQ ID NO: 1698 for HPV73 and SEQ ID NO: 1711 for HPV82,

the amplicons corresponding to the splice junction i=10 are respectively absent for HPV16, SEQ ID NO: 1523 for HPV18, absent for HPV31, absent for HPV33, absent for HPV35, absent for HPV39, absent for HPV45, absent for HPV51, absent for HPV52, absent for HPV56, absent for HPV58, absent for HPV59, absent for HPV66, absent for HPV68, absent for HPV73 and absent for HPV82,

the amplicons corresponding to the splice junction i=11 are respectively SEQ ID NO: 1510 for HPV16, SEQ ID NO: 1524 for HPV18, SEQ ID NO: 1542 for HPV31, SEQ ID NO: 1555 for HPV33, SEQ ID NO: 1567 for HPV35, SEQ ID NO: 1580 for HPV39, SEQ ID NO: 1592 for HPV45, SEQ ID NO: 1604 for HPV51, SEQ ID NO: 1617 for HPV52, SEQ ID NO: 1633 for HPV56, SEQ ID NO: 1648 for HPV58, SEQ ID NO: 1660 for HPV59, SEQ ID NO: 1677 for HPV66, SEQ ID NO: 1688 for HPV68, SEQ ID NO: 1699 for HPV73 and SEQ ID NO: 1712 for HPV82,

the amplicons corresponding to the splice junction i=12 are respectively absent for HPV16, SEQ ID NO: 1525 for HPV18, absent for HPV31, absent for HPV33, SEQ ID NO: 1568 or HPV35, absent for HPV39, absent for HPV45, absent for HPV51, SEQ ID NO: 1618 for HPV52, SEQ ID NO: 1634 for HPV56, absent for HPV58, SEQ ID NO: 1661 for HPV59, absent for HPV66, absent for HPV68, SEQ ID NO: 1700 for HPV73, absent for HPV82,

the amplicons corresponding to the splice junction i=13 are respectively absent for HPV16, SEQ ID NO: 1526 for HPV18, absent for HPV31, absent for HPV33, absent for HPV35, absent for HPV39, SEQ ID NO: 1593 for HPV45, absent for HPV51, absent for HPV52, absent for HPV56, absent for HPV58, absent for HPV59, absent for HPV66, absent for HPV68, absent for HPV73 and absent for HPV82,

the amplicons corresponding to the splice junction i=14 are respectively absent for HPV16, SEQ ID NO: 1527 for HPV18, absent for HPV31, absent for HPV33, absent for HPV35, absent for HPV39, absent for HPV45, absent for HPV51, absent for HPV52, absent for HPV56, absent for HPV58, absent for HPV59, absent for HPV66, absent for HPV68, absent for HPV73 and absent for HPV82,

the amplicons corresponding to the splice junction i=15 are respectively SEQ ID NO: 1511 for HPV16, SEQ ID NO: 1528 for HPV18, SEQ ID NO: 1543 for HPV31, SEQ ID NO: 1556 for HPV33, SEQ ID NO: 1569 for HPV35, SEQ ID NO: 1581 for HPV39, SEQ ID NO: 1594 for HPV45, SEQ ID NO: 1605 for HPV51, SEQ ID NO: 1619 for HPV52, SEQ ID NO: 1635 for HPV56, SEQ ID NO: 1649 for HPV58, SEQ ID NO: 1662 for HPV59, SEQ ID NO: 1678 for HPV66, SEQ ID NO: 1689 for HPV68, SEQ ID NO: 1701 for HPV73 and SEQ ID NO: 1713 for HPV82,

the amplicons corresponding to the splice junction i=16 are respectively SEQ ID NO: 1512 for HPV16, SEQ ID NO: 1529 for HPV18, SEQ ID NO: 1544 for HPV31, SEQ ID NO: 1557 for HPV33, SEQ ID NO: 1570 for HPV35, absent for HPV39, SEQ ID NO: 1595 for HPV45, absent for HPV51, SEQ ID NO: 1620 for HPV52, SEQ ID NO: 1636 for HPV56, SEQ ID NO: 1650 for HPV58, SEQ ID NO: 1663 for HPV59, SEQ ID NO: 1679 for HPV66, absent for HPV68, absent for HPV73 and absent for HPV82,

the amplicons corresponding to the splice junction i=17 are respectively SEQ ID NO: 1513 for HPV16, absent for HPV18, SEQ ID NO: 1545 for HPV31, SEQ ID NO: 1558 for HPV33, SEQ ID NO: 1571 for HPV35, absent for HPV39, absent for HPV45, SEQ ID NO: 1606 for HPV51, SEQ ID NO: 1621 for HPV52, SEQ ID NO: 1637 for HPV56, SEQ ID NO: 1651 for HPV58, absent for HPV59, absent for HPV66, SEQ ID NO: 1690 for HPV68, SEQ ID NO: 1702 for HPV73 and SEQ ID NO: 1714 for HPV82,

the amplicons corresponding to the splice junction i=18 are respectively SEQ ID NO:1514 for HPV16, SEQ ID NO: 1531 for HPV18, SEQ ID NO: 1546 for HPV31, SEQ ID NO: 1559 for HPV33, SEQ ID NO: 1572 for HPV35, SEQ ID NO: 1583 for HPV39, SEQ ID NO: 1597 for HPV45, SEQ ID NO: 1607 for HPV51, SEQ ID NO: 1622 for HPV52, SEQ ID NO: 1638 for HPV56, SEQ ID NO: 1652 for HPV58, SEQ ID NO: 1665 for HPV59, SEQ ID NO: 1681 for HPV66, SEQ ID NO: 1691 for HPV68, SEQ ID NO: 1703 for HPV73 and SEQ ID NO: 1715 for HPV82,

the amplicons corresponding to the splice junction i=19 are respectively absent for HPV16, SEQ ID NO: 1532 for HPV18, absent for HPV31, absent for HPV33, SEQ ID NO: 1573 for HPV35, absent for HPV39, absent for HPV45, absent for HPV51, SEQ ID NO: 1623 for HPV52, SEQ ID NO: 1639 for HPV56, absent for HPV58, SEQ ID NO: 1666 for HPV59, absent for HPV66, absent for HPV68, SEQ ID NO: 1704 for HPV73 and absent for HPV82,

the amplicons corresponding to the splice junction i=20 are respectively absent for HPV16, absent for HPV18, absent for HPV31, absent for HPV33, absent for HPV35, SEQ ID NO: 1577 for HPV39, absent for HPV45, absent for HPV51, absent for HPV52, absent for HPV56, absent for HPV58, SEQ ID NO: 1658 for HPV59, absent for HPV66, absent for HPV68, absent for HPV73 and absent for HPV82,

the amplicons corresponding to the splice junction i=21 are respectively absent for HPV16, absent for HPV18, absent for HPV31, absent for HPV33, absent for HPV35, SEQ ID NO: 1582 for HPV39, absent for HPV45, absent for HPV51, absent for HPV52, absent for HPV56, absent for HPV58, SEQ ID NO: 1664 for HPV59, absent for HPV66, absent for HPV68, absent for HPV73 and absent for HPV82,

the amplicons corresponding to the splice junction i=22 are respectively absent for HPV16, absent for HPV18, absent for HPV31, absent for HPV33, absent for HPV35, SEQ ID NO: 1575 for HPV39, absent for HPV45, absent for HPV51, absent for HPV52, absent for HPV56, absent for HPV58, SEQ ID NO: 1655 for HPV59, absent for HPV66, absent for HPV68, absent for HPV73 and absent for HPV82,

the amplicons corresponding to the splice junction i=23 are respectively absent for HPV16, absent for HPV18, absent for HPV31, absent for HPV33, absent for HPV35, absent for HPV39, SEQ ID NO: 1586 for HPV45, absent for HPV51, absent for HPV52, absent for HPV56, absent for HPV58, absent for HPV59, SEQ ID NO: 1670 for HPV66, absent for HPV68, absent for HPV73 and absent for HPV82,

the amplicons corresponding to the splice junction i=24 are respectively absent for HPV16, absent for HPV18, absent for HPV31, absent for HPV33, absent for HPV35, absent for HPV39, SEQ ID NO: 1589 for HPV45, absent for HPV51, absent for HPV52, absent for HPV56, absent for HPV58, absent for HPV59, SEQ ID NO: 1673 for HPV66, absent for HPV68, absent for HPV73 and absent for HPV82,

the amplicons corresponding to the splice junction i=25 are respectively absent for HPV16, SEQ ID NO: 1530 for HPV18, absent for HPV31, absent for HPV33, absent for HPV35, absent for HPV39, SEQ ID NO: 1596 HPV45, absent for HPV51, absent for HPV52, absent for HPV56, absent for HPV58, absent for HPV59, SEQ ID NO: 1680 for HPV66, absent for HPV68, absent for HPV73 and absent for HPV82.

The *in vitro* method for detecting HSIL may comprise a step of treatment of the biological sample with a solution comprising 30-60 wt% of methanol and 40-70 wt% of water such as preservCyt.

## *Composition of primers for HPV typing*

The present invention relates to a composition of primers for typing HPV selected from the group consisting of:

- at least one pair of primers of each of the first to the thirteenth and optionally of the fourteenth and/or fifteenth and/or sixteenth subsets of the splice junctions set of pairs of primers as defined above,
- a second set of primers, called unsplice junctions set of primers,
- a third set of primers, called genomic set of primers, and
- a fourth set of primers, called fusion set of primers.

The present invention relates to a composition of primers for typing HPV comprising the set of primers selected from the group consisting of the splice junctions set of primers as defined above, a second set of primers, called unsplice junctions set of primers, a third set of primers, called genomic set of primers, and a fourth set of primers, called fusion set of primers.

The present invention also relates to a composition of primers for typing HPV comprising the splice junctions set of primers as defined above and an additional set of primers selected from the group consisting of a second set of primers, called unsplice junctions set of primers, a third set of primers, called genomic set of primers and a fourth set of primers, called fusion set of primers.

The method and composition of primers for typing HPV according the invention provides results as good as current gold standard test for HPV typing.

Moreover, the method and the composition of primers of the invention replace the current combination of cytology (Pap smear) and HPV molecular screening by a single molecular test for both the detection of high-risk or putative high-risk HPV and the triage of women at risk of transforming infection, before colposcopy. In particular, the splice junctions set of primers may be used for both detecting a HSIL lesion and typing HPV.

Preferably, the composition of primers for typing HPV of the invention comprises the splice junctions set of primers as defined above, an unsplice junctions set of primers, a genomic set of primers and a fusion set of primers.

Each of the unspliced junctions set, the genomic set of primers and the fusion set of primers may comprise a subset of pairs of primers specific of each high risk HPV and optionally a subset of primers specific of each putative high risk HPV.

The composition of primers for typing HPV of the invention may also comprises an additional fifth set of primers, called human set of primers. The human set of primers is as defined above.

The primers of unsplice junctions set of primers target high risk and optionally of putative high risk HPV genomic regions spanning either splice donor or splice acceptor sites in the absence of any splice event.

The unsplice junctions set of primers may comprise:

- at least 1, at least 2, at least 3, at least 4, at least 5, at least 6, at least 7, at least 8, at least 9, at least 10 or at least 11, or 1, 2, 3, 4, 5, 6, 7, 8, 9, 10 or 11 pairs of primers of a first subset of HPV16 specific pairs of primers having the nucleic acid sequence selected from the group consisting of SEQ ID NO: 431-432, to SEQ ID NO: 451-452;
- at least 1, at least 2, at least 3, at least 4, at least 5, at least 6, at least 7, at least 8, at least 9, at least 10, at least 11 or at least 12, or 1, 2, 3, 4, 5, 6, 7, 8, 9, 10, 11 or 12 pairs of primers of a second subset of HPV18 specific pairs of primers having the nucleic acid sequence selected from the group consisting of SEQ ID NO: 453-454 to SEQ ID NO: 475-476;

- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a third subset of HPV31 specific pairs of primers having the nucleic acid sequence selected from the group consisting of SEQ ID NO: 477-478 to SEQ ID NO: 497-498;
- at least 1, at least 2, at least 3, at least 4, at least 5, at least 6, at least 7, at least 8 or at least 9, or 1, 2, 3, 4, 5, 6, 7, 8 or 9 pairs of primers of a fourth subset of HPV33 specific pairs of primers having the nucleic acid sequence selected from the group consisting of SEQ ID NO: 499-500 to SEQ ID NO: 515-516;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a fifth subset of HPV35 specific pairs of primers having the nucleic acid sequence selected from the group consisting of SEQ ID NO: 517-518 to SEQ ID NO: 535-536;
- at least 1, at least 2, at least 3, at least 4, at least 5, at least 6, at least 7 or at least 8, or 1, 2, 3, 4, 5, 6, 7 or 8 pairs of primers of a sixth subset of HPV39 specific pairs of primers having the nucleic acid sequence selected from the group consisting of SEQ ID NO: 537-538 to SEQ ID NO: 551-552;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a seventh subset of HPV45 specific pairs of primers having the nucleic acid sequence selected from the group consisting of SEQ ID NO: 553-554 to SEQ ID NO: 571-572;
- at least 1, at least 2, at least 3, at least 4, at least 5, at least 6, at least 7, at least 8 or at least 9, or 1, 2, 3, 4, 5, 6, 7, 8 or 9 pairs of primers of a eighth subset of HPV51 specific pairs of primers having the nucleic acid sequence selected from the group consisting of SEQ ID NO: 573-574 to SEQ ID NO: 589-590;
- at least 1, at least 2, at least 3, at least 4, at least 5, at least 6, at least 7, at least 8, at least 9, at least 10 or at least 11, or 1, 2, 3, 4, 5, 6, 7, 8, 9, 10 or 11 pairs of primers of a ninth subset of HPV52 specific pairs of primers having the nucleic acid sequence selected from the group consisting of SEQ ID NO: 591-592 to SEQ ID NO: 611-612;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a tenth subset of HPV56 specific pairs of primers having the nucleic acid sequence selected from the group consisting of SEQ ID NO: 613-614 to SEQ ID NO: 631- 632;
- at least 1, at least 2, at least 3, at least 4, at least 5, at least 6, at least 7 or at least 8, or 1, 2, 3, 4, 5, 6, 7 or 8 pairs of primers of an eleventh subset of HPV58 specific pairs of primers having the nucleic acid sequence selected from the group consisting of SEQ ID NO: 633-634 to SEQ ID NO: 647-648;
- at least 1, at least 2, at least 3, at least 4, at least 5, at least 6, at least 7 or at least 8, or 1, 2, 3, 4, 5, 6, 7 or 8 pairs of primers of a twelfth subset of HPV59 specific pairs of primers having the nucleic acid sequence selected from the group consisting of SEQ ID NO: 649-650 to SEQ ID NO: 663-664;
- at least 1, at least 2, at least 3, at least 4, at least 5, at least 6, at least 7 or at least 8, or 1, 2, 3, 4, 5, 6, 7 or 8 pairs of primers of a thirteenth subset of HPV66 specific pairs of primers having the nucleic acid sequence selected from the group consisting of SEQ ID NO: 665-666 to SEQ ID NO: 679-680.

The unsplice junctions set of primers may further comprise:

- at least 1, at least 2, at least 3, at least 4, at least 5, at least 6, at least 7, at least 8 or at least 9, or 1, 2, 3, 4, 5, 6, 7, 8 or 9 pairs of primers of a fourteenth subset of HPV68 specific pairs of primers having the nucleic acid sequence selected from the group consisting of SEQ ID NO: 681-682 to SEQ ID NO: 697-698; and/or
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of the fifteenth subset of HPV73 specific pairs of primers having the nucleic acid sequence selected from the group consisting of SEQ ID NO: 699-700 to SEQ ID NO: 717-718; and/or
- at least 1, at least 2, at least 3, at least 4, at least 5, at least 6, at least 7, at least 8 or at least 9, or 1, 2, 3, 4, 5, 6, 7, 8 or 9 pairs of primers of the sixteenth subset of HPV82 specific pairs of primers having the nucleic acid sequence selected from the group consisting of SEQ ID NO: 719-720 to SEQ ID NO: 735-736.

In one embodiment, the unsplice junctions set of primers comprises the pairs of primers having the nucleic acid sequence SEQ ID NO: 431-432 to SEQ ID NO: 679-680 and optionally of SEQ ID NO: 681-682 to SEQ ID NO: 697-698 and/or SEQ ID NO: 699-700 to SEQ ID NO: 717-718 and/or SEQ ID NO: 719-720 to SEQ ID NO: 735-736.

In a preferred embodiment, the unsplice junctions set of primers comprises the pairs of primers having the nucleic acid sequence SEQ ID NO: 431-432 to SEQ ID NO: 735-736.

In one embodiment, the unsplice junctions set of primers consists of the pairs of primers having the nucleic acid sequence SEQ ID NO: 431-432 to SEQ ID NO: 679-680 and optionally of SEQ ID NO: 681-682 to SEQ ID NO: 697-698 and/or SEQ ID NO: 699-700 to SEQ ID NO: 717-718 and/or SEQ ID NO: 719-720 to SEQ ID NO: 735-736.

In a preferred embodiment, the unsplice junctions set of primers consists of the pairs of primers having the nucleic acid sequence SEQ ID NO: 431-432 to SEQ ID NO: 735-736.

The unsplice junctions set of primers may comprise:

- at least 1, at least 2, at least 3, at least 4, at least 5, at least 6, at least 7, at least 8, at least 9, at least 10 or at least 11, or 1, 2, 3, 4, 5, 6, 7, 8, 9, 10 or 11 pairs of primers a first subset of HPV16 specific pairs of primers able to produce the amplicons having the nucleic acid sequence selected from the group consisting of SEQ ID NO: 1716 to SEQ ID NO: 1726;
- at least 1, at least 2, at least 3, at least 4, at least 5, at least 6, at least 7, at least 8, at least 9, at least 10, at least 11 or at least 12, or 1, 2, 3, 4, 5, 6, 7, 8, 9, 10, 11 or 12 pairs of primers of a second subset of HPV18 specific pairs of primers able to produce the amplicons having the nucleic acid sequence selected from the group consisting of SEQ ID NO: 1727 to SEQ ID NO: 1738;
- at least 1, at least 2, at least 3, at least 4, at least 5, at least 6, at least 7, at least 8, at least 9, at least 10, at least 11 or at least 12, or 1, 2, 3, 4, 5, 6, 7, 8, 9, 10, 11 or 12 pairs of primers of a third subset of HPV31 specific pairs of primers able to produce the amplicons having the nucleic acid sequence selected from the group consisting of SEQ ID NO: 1739 to SEQ ID NO: 1749;
- at least 1, at least 2, at least 3, at least 4, at least 5, at least 6, at least 7, at least 8 or at least 9, or 1, 2, 3, 4, 5, 6, 7, 8 or 9 pairs of primers of a fourth subset of HPV33 specific pairs of primers able to produce the amplicons having the nucleic acid sequence selected from the group consisting of SEQ ID NO: 1750 to SEQ ID NO: 1758;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a fifth subset of HPV35 specific pairs of primers able to produce the amplicons having the nucleic acid sequence selected from the group consisting of SEQ ID NO: 1759 to SEQ ID NO: 1768;
- at least 1, at least 2, at least 3, at least 4, at least 5, at least 6, at least 7 or at least 8, or 1, 2, 3, 4, 5, 6, 7 or 8 pairs of primers of a sixth subset of HPV39 specific pairs of primers able to produce the amplicons having the nucleic acid sequence selected from the group consisting of SEQ ID NO: 1769 to SEQ ID NO: 1776;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a seventh subset of HPV45 specific pairs of primers able to produce the amplicons having the nucleic acid sequence selected from the group consisting of SEQ ID NO: 1777 to SEQ ID NO: 1786;
- at least 1, at least 2, at least 3, at least 4, at least 5, at least 6, at least 7, at least 8 or at least 9, or 1, 2, 3, 4, 5, 6, 7, 8 or 9 pairs of primers of a eighth subset of HPV51 specific pairs of primers able to produce the amplicons having the nucleic acid sequence selected from the group consisting of SEQ ID NO: 1787 to SEQ ID NO: 1795;
- at least 1, at least 2, at least 3, at least 4, at least 5, at least 6, at least 7, at least 8, at least 9, at least 10 or at least 11, or 1, 2, 3, 4, 5, 6, 7, 8, 9, 10 or 11 pairs of primers of a ninth subset of HPV52 specific pairs of primers able to produce the amplicons having the nucleic acid sequence selected from the group consisting of SEQ ID NO: 1796 to SEQ ID NO: 1806;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a tenth subset of HPV56 specific pairs of primers able to produce the amplicons having the nucleic acid sequence selected from the group consisting of SEQ ID NO: 1807 to SEQ ID NO: 1816;
- at least 1, at least 2, at least 3, at least 4, at least 5, at least 6, at least 7 or at least 8, or 1, 2, 3, 4, 5, 6, 7 or 8 pairs of primers of an eleventh subset of HPV58 specific pairs of primers able to produce the amplicons having the nucleic acid sequence selected from the group consisting of SEQ ID NO: 1817 to SEQ ID NO: 1824;
- at least 1, at least 2, at least 3, at least 4, at least 5, at least 6, at least 7 or at least 8, or 1, 2, 3, 4, 5, 6, 7 or 8 pairs of primers of a twelfth subset of HPV59 specific pairs of primers able to produce the amplicons having the nucleic acid sequence selected from the group consisting of SEQ ID NO: 1825 to SEQ ID NO: 1832;
- at least 1, at least 2, at least 3, at least 4, at least 5, at least 6 or at least 7, or 1, 2, 3, 4, 5, 6 or 7 pairs of primers of a thirteenth subset of HPV66 specific pairs of primers able to produce the amplicons having the nucleic acid sequence selected from the group consisting of SEQ ID NO: 1833 to SEQ ID NO: 1840.

The unsplice junctions set of primers may further comprise:

- at least 1, at least 2, at least 3, at least 4, at least 5, at least 6, at least 7, at least 8 or at least 9, or 1, 2, 3, 4, 5, 6, 7, 8 or 9 pairs of primers of a fourteenth subset of HPV68 specific pairs of primers able to produce the amplicons having the nucleic acid sequence selected from the group consisting of SEQ ID NO: 1841 to SEQ ID NO: 1849;
- at least 1, at least 2, at least 3, at least 4, at least 5, at least 6, at least 7, at least 8, at least 9 or at least 10, or 1, 2, 3, 4, 5, 6, 7, 8, 9 or 10 pairs of primers of a fifteenth subset of HPV73 specific pairs of primers able to produce the amplicons having the nucleic acid sequence selected from the group consisting of SEQ ID NO: 1850 to SEQ ID NO: 1859,
- at least 1, at least 2, at least 3, at least 4, at least 5, at least 6, at least 7, at least 8 or at least 9, or 1, 2, 3, 4, 5, 6, 7, 8 or 9 pairs of primers of a sixteenth subset of HPV82 specific pairs of primers able to produce the amplicons having the nucleic acid sequence selected from the group consisting of SEQ ID NO: 1860 to SEQ ID NO: 1868.

In one embodiment, the unsplice junctions set of primers comprises the pairs of primers able to produce the amplicons having the nucleic acid sequence SEQ ID NO: 1716 to SEQ ID NO: 1840 and optionally of SEQ ID NO: 1841 to SEQ ID NO: 1849 and/or SEQ ID NO: 1850 to SEQ ID NO: 1859 and/or SEQ ID NO: 1860 to SEQ ID NO: 1868.
In a preferred embodiment, the unsplice junctions set of primers comprises the pairs of primers able to produce the amplicons having the nucleic acid sequence SEQ ID NO: 1716 to SEQ ID NO: 1726 to SEQ ID NO: 1860 to SEQ ID NO: 1868.
In one embodiment, the unsplice junctions set of primers consists of the pairs of primers able to produce the amplicons having the nucleic acid sequence SEQ ID NO: 1716 to SEQ ID NO: 1840 and optionally of SEQ ID NO: 1841 to SEQ ID NO: 1849 and/or SEQ ID NO: 1850 to SEQ ID NO: 1859 and/or SEQ ID NO: 1860 to SEQ ID NO: 1868.
In a preferred embodiment, the unsplice junctions set of primers consists of the pairs of primers having the nucleic acid sequence SEQ ID NO: 1716 to SEQ ID NO: 1726 to SEQ ID NO: 1868.
The primers of the genomic set of primers target high risk and optionally of putative high risk HPV genomic regions away from any splice donor or splice acceptor sites,
**[0111]** The genomic set of primers may comprise:

- at least 1, at least 2, at least 3 or at least 4, or 1, 2, 3 or 4 pairs of primers of a first subset of HPV16 specific pairs of primers having the nucleic acid sequence selected from the group consisting of SEQ ID NO: 737-738, SEQ ID NO: 739-740, SEQ ID NO: 741-742 and SEQ ID NO: 743-744;
- at least 1, at least 2, at least 3 or at least 4 or 1, 2, 3 or 4 pairs of primers of a second subset of HPV18 specific pairs of primers having the nucleic acid sequence selected from the group consisting of SEQ ID NO: 745-746, SEQ ID NO: 747-748, SEQ ID NO: 749-750 and SEQ ID NO: 751-752;
- at least 1, at least 2, at least 3 or at least 4, or 1, 2, 3 or 4 pairs of primers of a third subset of HPV31 specific pairs of primershaving the nucleic acid sequence selected from the group consisting of SEQ ID NO: 753-754, SEQ ID NO: 755-756, SEQ ID NO: 757-758 and SEQ ID NO: 759-760;
- at least 1, at least 2, at least 3 or at least 4, or 1, 2, 3 or 4 pairs of primers of a fourth subset HPV33 of specific pairs of primers having the nucleic acid sequence selected from the group consisting of SEQ ID NO: 761-762, SEQ ID NO: 763-764, SEQ ID NO: 765-766 and SEQ ID NO: 767-768;
- at least 1, at least 2, at least 3 or at least 4, or 1, 2, 3 or 4 pairs of primers of a fifth subset of HPV35 specific pairs of primers having the nucleic acid sequence selected from the group consisting of SEQ ID NO: 769-770, SEQ ID NO: 771-772, SEQ ID NO: 773-774 and SEQ ID NO: 775-776;
- at least 1, at least 2, at least 3 or at least 4, or 1, 2, 3 or 4 pairs of primers of a sixth subset of HPV39 specific pairs of primers having the nucleic acid sequence selected from the group consisting of SEQ ID NO: 777-778, SEQ ID NO: 779-780, SEQ ID NO: 781-782 and SEQ ID NO: 783-784;
- at least 1, at least 2, at least 3 or at least 4, or 1, 2, 3 or 4 pairs of primers of a seventh subset of HPV45 specific pairs of primers having the nucleic acid sequence selected from the group consisting of SEQ ID NO: 785-786, SEQ ID NO: 787-788, SEQ ID NO: 789-790 and SEQ ID NO: 791-792;
- at least 1, at least 2, at least 3 or at least 4, or 1, 2, 3 or 4 pairs of primers of a eighth subset of HPV51 specific pairs of primers having the nucleic acid sequence selected from the group consisting of SEQ ID NO: 793-794, SEQ ID NO: 795-796, SEQ ID NO: 797-798 and SEQ ID NO: 799-800;
- at least 1, at least 2, at least 3 or at least 4, or 1, 2, 3 or 4 pairs of primers of a ninth subset of HPV52 specific pairs of primers having the nucleic acid sequence selected from the group consisting of SEQ ID NO: 801-802, SEQ ID NO: 803-804, SEQ ID NO: 805-806 and SEQ ID NO: 807-808;
- at least 1, at least 2, at least 3 or at least 4, or 1, 2, 3 or 4 pairs of primers a tenth subset of HPV56 specific pairs of primers having the nucleic acid sequence selected from the group consisting of SEQ ID NO: 809-810, SEQ ID NO: 811-812, SEQ ID NO: 813-814 and SEQ ID NO: 815-816;
- at least 1, at least 2, at least 3 or at least 4, or 1, 2, 3 or 4 pairs of primers of an eleventh subset of HPV58 specific pairs of primers having the nucleic acid sequence selected from the group consisting of SEQ ID NO: 817-818, SEQ ID NO: 819-820, SEQ ID NO: 821-822 and SEQ ID NO: 823-824;
- at least 1, at least 2, at least 3 or at least 4, or 1, 2, 3 or 4 pairs of primers of a twelfth subset of HPV59 specific pairs of primers having the nucleic acid sequence selected from the group consisting of SEQ ID NO: 825-826, SEQ ID NO: 827-828, SEQ ID NO: 829-830 and SEQ ID NO: 831-832;
- at least 1, at least 2, at least 3 or at least 4, or 1, 2, 3 or 4 pairs of primers of a thirteenth subset of HPV66 specific pairs of primers having the nucleic acid sequence selected from the group consisting of SEQ ID NO: 833-834, SEQ ID NO: 835-836, SEQ ID NO: 837-838 and SEQ ID NO: 839-840.

The genomic set of primers may further comprise:

- at least 1, at least 2, at least 3 or at least 4, or 1, 2, 3 or 4 pairs of primers of a fourteenth subset of HPV68 specific pairs of primers having the nucleic acid sequence selected from the group consisting of SEQ ID NO: 841-842, SEQ ID NO: 843-844, SEQ ID NO: 845-846 and SEQ ID NO: 847-848;
- at least 1, at least 2, at least 3 or at least 4, or 1, 2, 3 or 4 pairs of primers of a fifteenth subset of HPV73 specific pairs of primers having the nucleic acid sequence selected from the group consisting of SEQ ID NO: 849-850, SEQ ID NO: 851-852, SEQ ID NO: 853-854 and SEQ ID NO: 855-856;
- at least 1, at least 2, at least 3 or at least 4, or 1, 2, 3 or 4 pairs of primers of a sixteenth subset of HPV82 specific pairs of primers having the nucleic acid sequence selected from the group consisting of SEQ ID NO: 857-858, SEQ ID NO: 859-860, SEQ ID NO: 861-862 and SEQ ID NO: 863-864.

In one embodiment, the genomic set of primers comprises the pairs of primers having the nucleic acid sequence SEQ ID NO: 737-738 to SEQ ID NO: 839-840 and optionally of SEQ ID NO: 841-842 to SEQ ID NO: 847-848 and/or SEQ ID NO: 849-850 to SEQ ID NO: 853-854 and SEQ ID NO: 855-856 and/or SEQ ID NO: 857-858 to SEQ ID NO: 863-864. In a preferred embodiment, the genomic set of primers comprises the pairs of primers having the nucleic acid sequence SE SEQ ID NO: 737-738 to SEQ ID NO: 863-864.

In one embodiment, the genomic set of primers consists of the pairs of primers having the nucleic acid sequence SEQ ID NO: 737-738 to SEQ ID NO: 839-840 and optionally of SEQ ID NO: 841-842 to SEQ ID NO: 847-848 and/or SEQ ID NO: 849-850 to SEQ ID NO: 853-854 and SEQ ID NO: 855-856 and/or SEQ ID NO: 857-858 to SEQ ID NO: 863-864. In a preferred embodiment, the genomic set of primers consists of the pairs of primers having the nucleic acid sequence SEQ ID NO: 737-738 to SEQ ID NO: 863-864.

The genomic set of primers may comprise:

- at least 1, at least 2, at least 3 or at least 4, or 1, 2, 3 or 4 pairs of primers of first subset of HPV16 specific pairs of primers able to produce the amplicons having the nucleic acid sequence selected from the group consisting of SEQ ID NO: 1869 to SEQ ID NO: 1872;
- at least 1, at least 2, at least 3 or at least 4, or 1,2, 3 or 4 pairs of primers the second subset of HPV18 specific pairs of primers able to produce the amplicons having the nucleic acid sequence selected from the group consisting of SEQ ID NO: 1873 to SEQ ID NO: 1876;
- at least 1, at least 2, at least 3 or at least 4, or 1, 2, 3 or 4 pairs of primers of a third subset of HPV31 specific pairs of primers able to produce the amplicons having the nucleic acid sequence selected from the group consisting of SEQ ID NO: 1877 to SEQ ID NO: 1880;
- at least 1, at least 2, at least 3 or at least 4, or 1, 2, 3 or 4 pairs of primers of a fourth subset of HPV33 specific pairs of primers able to produce the amplicons having the nucleic acid sequence selected from the group consisting of SEQ ID NO: 1881 to SEQ ID NO: 1884;
- at least 1, at least 2, at least 3 or at least 4, or 1, 2, 3 or 4 pairs of primers of a fifth subset of HPV35 specific pairs of primers able to produce the amplicons having the nucleic acid sequence selected from the group consisting of SEQ ID NO: 1885 to SEQ ID NO: 1888;
- at least 1, at least 2, at least 3 or at least 4, or 1, 2, 3 or 4 pairs of primers of a sixth subset of HPV39 specific pairs of primers able to produce the amplicons having the nucleic acid sequence selected from the group consisting of SEQ ID NO: 1889 to SEQ ID NO: 1892;
- at least 1, at least 2, at least 3 or at least 4, or 1, 2, 3 or 4 pairs of primers of a seventh subset of HPV45 specific pairs of primers able to produce the amplicons having the nucleic acid sequence selected from the group consisting of SEQ ID NO: 1893 to SEQ ID NO: 1896;
- at least 1, at least 2, at least 3 or at least 4, or 1, 2, 3 or 4 pairs of primers of a eighth subset of HPV51 specific pairs of primers able to produce the amplicons having the nucleic acid sequence selected from the group consisting of SEQ ID NO: 1897 to SEQ ID NO: 1900;
- at least 1, at least 2, at least 3 or at least 4, or 1, 2, 3 or 4 pairs of primers of a ninth subset of HPV52 specific pairs of primers able to produce the amplicons having the nucleic acid sequence selected from the group consisting of SEQ ID NO: 1901 to SEQ ID NO: 1904;
- at least 1, at least 2, at least 3 or at least 4, or 1, 2, 3 or 4 pairs of primers of a tenth subset of HPV56 specific pairs of primers able to produce the amplicons having the nucleic acid sequence selected from the group consisting of SEQ ID NO: 1905 to SEQ ID NO: 1908;
- at least 1, at least 2, at least 3 or at least 4, or 1, 2, 3 or 4 pairs of primers of an eleventh subset of HPV58 specific pairs of primers specific able to produce the amplicons having the nucleic acid sequence selected from the group consisting of SEQ ID NO: 1909 to SEQ ID NO: 1912;
- at least 1, at least 2, at least 3 or at least 4, or 1, 2, 3 or 4 pairs of primers of a twelfth subset of HPV59 specific pairs of primers able to produce the amplicons having the nucleic acid sequence selected from the group consisting of SEQ ID NO: 1913 to SEQ ID NO: 1916;

- at least 1, at least 2, at least 3 or at least 4, or 1, 2, 3 or 4 pairs of primers of a thirteenth subset of HPV66 specific pairs of primers able to produce the amplicons having the nucleic acid sequence selected from the group consisting of SEQ ID NO: 1917 to SEQ ID NO: 1920.

The genomic set of primers may further comprise:

- at least 1, at least 2, at least 3 or at least 4, or 1, 2, 3 or 4 pairs of primers of a fourteenth subset of HPV68 specific pairs of primers able to produce the amplicons having the nucleic acid sequence selected from the group consisting of SEQ ID NO: 1921 to SEQ ID NO: 1924; and/or
- at least 1, at least 2, at least 3 or at least 4, or 1, 2, 3 or 4 pairs of primers of a fifteenth subset of HPV73 specific pairs of primers able to produce the amplicons having the nucleic acid sequence selected from the group consisting of SEQ ID NO: 1925 to SEQ ID NO: 1928; and/or
- at least 1, at least 2, at least 3 or at least 4, or 1, 2, 3 or 4 pairs of primers of a sixteenth subset of HPV82 specific pairs of primers able to produce the amplicons having the nucleic acid sequence selected from the group consisting of SEQ ID NO: 1929 to SEQ ID NO: 1932.

In one embodiment, the genomic set of primers comprises the pairs of primers able to produce the amplicons having the nucleic acid sequence SEQ ID NO: 1869 to SEQ ID NO: 1920 and optionally of SEQ ID NO: 1921 to SEQ ID NO: 1924 and/or SEQ ID NO: 1925 to SEQ ID NO: 1928 and/or SEQ ID NO: 1929 to SEQ ID NO: 1932.

In a preferred embodiment, the genomic set of primers comprises the pairs of primers able to produce the amplicons having the nucleic acid sequence SEQ ID NO: 1869 to SEQ ID NO: 1932.

In one embodiment, the genomic set of primers consists of the pairs of primers able to produce the amplicons having the nucleic acid sequence SEQ ID NO: 1869 to SEQ ID NO: 1833 to 1840 and optionally of SEQ ID NO: 1921 to SEQ ID NO: 1924 and/or SEQ ID NO: 1925 to SEQ ID NO: 1928 and/or SEQ ID NO: 1929 to SEQ ID NO: 1932.

In a preferred embodiment, the genomic set of primers consists of the pairs of primers having the nucleic acid sequence SEQ ID NO: 1869 to SEQ ID NO: 1932.

The primers of fusion set of primers target high risk and optionally of putative high risk HPV fusion transcripts.

The primers of fusion set of primers may comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a first subset of HPV16 specific pairs of primers having the nucleic acid sequence selected from the group consisting of SEQ ID NO: 865-866 to SEQ ID NO: 899-900;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a second subset of HPV18 specific pairs of primers having the nucleic acid sequence selected from the group consisting of SEQ ID NO: 901-902 to SEQ ID NO: 935-936;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a third subset of HPV31 specific pairs of primers having the nucleic acid sequence selected from the group consisting of SEQ ID NO: 937-938 to SEQ ID NO: 971-972;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourth subset of HPV33 specific pairs of primers having the nucleic acid sequence selected from the group consisting of SEQ ID NO: 973-974 to SEQ ID NO: 1007-1008;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifth subset of HPV35 specific pairs of primers having the nucleic acid sequence selected from the group consisting of SEQ ID NO: 1009-1010 to SEQ ID NO: 1043-1044;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixth subset of HPV39 specific pairs of primers having the nucleic acid sequence selected from the group consisting of SEQ ID NO: 1045-1046 to SEQ ID NO: 1079-1080;
- at least 1, at least 2, at least 3, at least 4, at least 5, at least 6, at least 7, at least 8, at least 9, at least 10, at least 11, at least 12, at least 13, at least 14, at least 15, at least 16, preferably at least 17, more preferably 18 or 1, 2, 3, 4, 5, 6, 7, 8, 9, 10, 11, 12, 13, 14, 15, 16, 17 or 18 pairs of primers of a seventh subset of HPV45 specific pairs of primers having the nucleic acid sequence selected from the group consisting of SEQ ID NO: 1081-1082 to SEQ ID NO: 1115-1116.

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a eighth subset of HPV51 specific pairs of primers having the nucleic acid sequence selected from the group consisting of SEQ ID NO: 1117-1118 to SEQ ID NO: 1151-1152;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a ninth subset of HPV52 specific pairs of primers having the nucleic acid sequence selected from the group consisting of SEQ ID NO: 1153-1154 to SEQ ID NO: 1187-1188;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a tenth subset of HPV56 specific pairs of primers having the nucleic acid sequence selected from the group consisting of SEQ ID NO: 1189-1190 to SEQ ID NO: 1223-1224;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of an eleventh subset of HPV58 specific pairs of primers having the nucleic acid sequence selected from the group consisting of SEQ ID NO: 1225-1226 to SEQ ID NO: 1259-1260;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a twelfth subset of HPV59 specific pairs of primers having the nucleic acid sequence selected from the group consisting of SEQ ID NO: 1261-1262 to SEQ ID NO: 1295-1296;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a thirteenth subset of HPV66 specific pairs of primers having the nucleic acid sequence selected from the group consisting of SEQ ID NO: 1297-1298 to SEQ ID NO: 1331-1332.

The fusion set of primers may further comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourteenth subset of HPV68 specific pairs of primers having the nucleic acid sequence selected from the group consisting of SEQ ID NO: 1333-1334 to SEQ ID NO: 1367-1368;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifteenth subset of HPV73 specific pairs of primers having the nucleic acid sequence selected from the group consisting of SEQ ID NO: 1369-1370 to SEQ ID NO: 1403-1404;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixteenth subset of HPV82 specific pairs of primers having the nucleic acid sequence selected from the group consisting of SEQ ID NO: 1405-1406 to SEQ ID NO: 1439-1440.

In one embodiment, the fusion set of primers comprises the pairs of primers having the nucleic acid sequence SEQ ID NO: 865-866 to SEQ ID NO: 1331-1332 and optionally of SEQ ID NO: 1333-1334 to SEQ ID NO: 1367-1368 and/or SEQ ID NO: 1369-1370 to SEQ ID NO: 1403-1404 and/or SEQ ID NO: 1405-1406 to SEQ ID NO: 1439-1440.

In a preferred embodiment, the fusion set of primers comprises the pairs of primers having the nucleic acid sequence SEQ ID NO: 865-866 to SEQ ID NO: 1439-1440.

In one embodiment, the fusion set of primers consists of the pairs of primers having the nucleic acid sequence SEQ ID NO: 865-866 to SEQ ID NO: 1331-1332 and optionally of SEQ ID NO: 1333-1334 to SEQ ID NO: 1367-1368 and/or SEQ ID NO: 1369-1370 to SEQ ID NO: 1403-1404 and/or SEQ ID NO: 1405-1406 to SEQ ID NO: 1439-1440.

In a preferred embodiment, the fusion set of primers consists of the pairs of primers having the nucleic acid sequence SEQ ID NO: 865-866 to SEQ ID NO: 1439-1440.

The primers of fusion set of primers may comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a first subset of HPV16 specific pairs of primers able to produce the amplicons having the nucleic acid sequence selected from the group consisting of SEQ ID NO: 1933 to 1 SEQ ID NO: 1950;
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a second subset of HPV18 specific pairs of primers able to produce the amplicons having the nucleic acid sequence selected from the group consisting of SEQ ID NO: 1951 to SEQ ID NO: 1968;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a third subset of HPV31 specific pairs of primers able to produce the amplicons having the nucleic acid sequence selected from the group consisting of SEQ ID NO: 1969 to SEQ ID NO: 1986;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourth subset of HPV33 specific pairs of primers able to produce the amplicons having the nucleic acid sequence selected from the group consisting of SEQ ID NO: 1987 to SEQ ID NO: 2004;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifth subset of HPV35 specific pairs of primers able to produce the amplicons having the nucleic acid sequence selected from the group consisting of SEQ ID NO: 2005 to SEQ ID NO: 2022;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixth subset of HPV39 specific pairs of primers able to produce the amplicons having the nucleic acid sequence selected from the group consisting of SEQ ID NO: 2023 to SEQ ID NO: 2040;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a seventh subset of HPV45 specific pairs of primers selected from the group consisting of SEQ ID NO: 2041 to SEQ ID NO: 2058,

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a eighth subset of HPV51 specific pairs of primers able to produce the amplicons having the nucleic acid sequence selected from the group consisting of SEQ ID NO: 2059 to SEQ ID NO: 2076;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a ninth subset of HPV52 specific pairs of primers able to produce the amplicons having the nucleic acid sequence selected from the group consisting of SEQ ID NO: 2077 to SEQ ID NO: 2094;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a tenth subset of HPV56 specific pairs of primers able to produce the amplicons having the nucleic acid sequence selected from the group consisting of SEQ ID NO: 2095 to SEQ ID NO: 2112;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of an eleventh subset of HPV58 specific pairs of primers able to produce the amplicons having the nucleic acid sequence selected from the group consisting of SEQ ID NO: 2113 to SEQ ID NO: 2130;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a twelfth subset of HPV59 specific pairs of primers able to produce the amplicons having the nucleic acid sequence selected from the group consisting of SEQ ID NO: 2131 to SEQ ID NO: 2148;

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a thirteenth subset of HPV66 specific pairs of primers able to produce the amplicons having the nucleic acid sequence selected from the group consisting of SEQ ID NO: 2149 to SEQ ID NO: 2166.

The fusion set may also comprise:

- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fourteenth subset of HPV68 specific pairs of primers able to produce the amplicons having the nucleic acid sequence selected from the group consisting of SEQ ID NO: 2167 to SEQ ID NO: 2184; and/or
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a fifteenth subset of HPV73 specific pairs of primers able to produce the amplicons having the nucleic acid sequence selected from the group consisting of SEQ ID NO:2185 to SEQ ID NO: 2202;and/or
- at least 1, at least 2, at least 3, at least 4, at least 5, at least 6, at least 7, at least 8, at least 9, at least 10, at least 11, at least 12, at least 13, at least 14, at least 15, at least 16, at least 17 or at least 18, or 1, 2, 3, 4, 5, 6, 7, 8, 9, 10, 11, 12, 13, 14, 15, 16, 17 or 18 pairs of primers of a sixteenth subset of HPV82 specific pairs of primers able to produce the amplicons having the nucleic acid sequence selected from the group consisting of SEQ ID NO: 2203 to SEQ ID NO: 2220.

In one embodiment, the fusion set of primers comprises the pairs of primers having the nucleic acid sequence SEQ ID NO: 1933 to SEQ ID NO: 2166 and optionally of SEQ ID NO: 2167 to SEQ ID NO: 2184 and/or SEQ ID NO: 2185 to SEQ ID NO: 2202 and/or SEQ ID NO: 2203 to SEQ ID NO: 2220.

In a preferred embodiment, the fusion set of primers comprises the pairs of primers able to produce the amplicons having the nucleic acid sequence SEQ ID NO: 1869 to SEQ ID NO: 2220.

In one embodiment, the fusion set of primers consists of the pairs of primers able to produce the amplicons having the nucleic acid sequence SEQ ID NO: 1933 to SEQ ID NO: 2166 and optionally of SEQ ID NO: 2167 to SEQ ID NO: 2184 and/or SEQ ID NO: 2185 to SEQ ID NO: 2202 and/or SEQ ID NO: 2203 to SEQ ID NO: 2220.

In a preferred embodiment, the fusion set of primers consists of the pairs of primers able to produce the amplicons having the nucleic acid sequence SEQ ID NO: 1869 to SEQ ID NO: 2220.

In one embodiment, the composition of primers for HPV typing comprises the pairs of primers having the nucleic acid sequence SEQ ID NO: 1-2 to SEQ ID NO: 1439-1440.

In one embodiment, the composition of primers for HPV typing consists of the pairs of primers able to produce amplicons having the nucleic acid sequence SEQ ID NO: 1-2 to SEQ ID NO: 1439-1440.

In one embodiment, the composition of primers for HPV typing comprises the pairs of primers able to produce amplicons having the nucleic acid sequence SEQ ID NO: 1501 to SEQ ID NO: 2220.

In one embodiment, the composition of primers for HPV typing consists of the pairs of primers having the nucleic acid sequence SEQ ID NO: 1501 to SEQ ID NO: 2220.

The primers of the human set of primers target human sequences.

The human set of primers may comprise at least 2, at least 3, at least 4, at least 5 , at least 6, at least 7, at least 8, at least 9, at least 10, at least 11, at least 12, at least 13, at least 14, at least 15 , at least 16, at least 17, at least 18, at least 19, at least 20, at least 21, at least 22, at least 23, at least 24, at least 25, at least 26, at least 27, at least 28, at least 29 or at least 30, or 2, 3, 4, 5 ,6,7,8, 9, 10, 11, 12, 13, 14, 15 ,16, 17, 18, 19, 20, 21, 22, 23, 24, 25, 26, 27, 28, 29 or 30 pairs of primers having the nucleic acid sequence selected from the group consisting of SEQ ID NO: 1441-1442, SEQ ID NO: 1443-1444, SEQ ID NO: 1445-1446, SEQ ID NO: 1447-1448, SEQ ID NO: 1449-1450, SEQ ID NO: 1451-1452, SEQ ID NO: 1453-1454, SEQ ID NO: 1455-1456, SEQ ID NO: 1457-1458, SEQ ID NO: 1459-1460, SEQ ID NO: 1461-1462, SEQ ID NO: 1463-1464, SEQ ID NO: 1465-1466, SEQ ID NO: 1467-1468, SEQ ID NO: 1469-1470, SEQ ID NO: 1471-1472, SEQ ID NO: 1473-1474, SEQ ID NO: 1475-1476, SEQ ID NO: 1477-1478, SEQ ID NO: 1479-1480, SEQ ID NO: 1481-1482, SEQ ID NO: 1483-1484, SEQ ID NO: 1485-1486, SEQ ID NO: 1487-1488, SEQ ID NO: 1489-1490, SEQ ID NO: 1491-1492, SEQ ID NO: 1493-1494, SEQ ID NO: 1495-1496, SEQ ID NO: 1497-1498 and SEQ ID NO: 1499-1500.

In one preferred embodiment, the human set of primers comprises SEQ ID NO: 1441-1442 to SEQ ID NO: 1499-1500.

In one more preferred embodiment, the human set of primers consists SEQ ID NO: 1441-1442 to SEQ ID NO: 1499-1500.

The human set of primers may comprise at least 2, at least 3, at least 4, at least 5 , at least 6, at least 7, at least 8, at least 9, at least 10, at least 11, at least 12, at least 13, at least 14, at least 15 , at least 16, at least 17, at least 18, at least 19, at least 20, at least 21, at least 22, at least 23, at least 24, at least 25, at least 26, at least 27, at least 28, at least 29 or at least 30, or 2, 3, 4, 5 ,6,7,8, 9, 10, 11, 12, 13, 14, 15 ,16, 17, 18, 19, 20, 21, 22, 23, 24, 25, 26, 27, 28, 29 or 30 pairs of primers able to produce the amplicons having the nucleic acid sequence selected from the group consisting of SEQ ID NO: 2221 to SEQ ID NO: 2250.

In one preferred embodiment, the human set of primers comprises pairs of primers able to produce the amplicons having the nucleic acid sequence SEQ ID NO: 2221 to SEQ ID NO: 2250.

In one more preferred embodiment, the human set of primers consists of pairs of primers able to produce the amplicons having the nucleic acid sequence SEQ ID NO: 2221 to SEQ ID NO: 2250.

In one embodiment, the composition of primers for HPV typing comprises the pairs of primers having the nucleic acid sequence SEQ ID NO: 1-2 to SEQ ID NO: 1499-1500.

Due to the redondancy between the pairs of primers the nucleic acid sequence SEQ ID NO: 1-2 to SEQ ID NO: 1499-1500 represent only 525 unique pairs of primers.

In one embodiment, the composition of primers for HPV typing consists of the pairs of primers able to produce amplicons having the nucleic acid sequence SEQ ID NO: 1-2 to SEQ ID NO: 1499-1500.

In one embodiment, the composition of primers for HPV typing comprises the pairs of primers able to produce amplicons having the nucleic acid sequence SEQ ID NO: 1501 to SEQ ID NO: 2250.

In one embodiment, the composition of primers for HPV typing consists of the pairs of primers having the nucleic acid sequence SEQ ID NO: 1501 to SEQ ID NO: 2250.

The present invention also relates to a kit for HPV typing comprising the composition of primers for HPV typing of the invention and optionally reagents for cDNA amplification.

The reagents for the kit for HPV typing may be the same as those for detecting HSIL.

The present invention also relates to the use of the composition of primers for HPV typing as defined above or of the kit for HPV typing as defined above for HPV typing.

### *An in vitro method for HPV typing in a biological sample*

The present invention also relates to an *in vitro* method for HPV typing in a biological sample comprising the steps of:

- (a) extraction of RNA from the biological sample,
- (b) reverse transcription of the RNA so as to generate cDNA,
- (c) amplification of the cDNA generated at step (b) with the composition of primers for HPV typing so as to produce amplicons,
- (d) quantification of the expression level of each amplicon.

The quantification of the expression level of each amplicon as well as the step (a) to (c) may be carried by the same methods as those disclosed for the in vitro method for detecting HSIL.

Typically, the *in vitro* method for HPV typing in a biological sample further comprises the step (e) of for each HPV type, comparing the expression level of all amplicons specific of said HPV type with a reference value, wherein if the expression level of all the amplicons specific of said HPV type is higher than the reference value, it is indicative of the presence of said HPV type in the biological sample.

Indeed, the number of reads mapping to HPV-specific amplicons (i.e. the sum of categories "splice junction", "unsplice junction" and "genomic") was used to detect the presence of a given HPV genotype. According the results of the inventors, the reference value is preferably between of 100-200 reads, more preferably 150 reads.

The practice of the present invention will employ, unless otherwise indicated, conventional techniques, which are within the skill of the art. Such techniques are explained fully in the literature.

For a better understanding of the invention and to show how the same may be carried into effect, there will now be described by way of example a specific mode contemplated by the Inventors with reference to the accompanying drawings in which:

Figure 1 shows an alignment of the genomes of HPV wherein are located the known and predictive splice donor and splice acceptor sites.

Figure 2 shows the location on the genomes of HPV and predictive splice donor (SD) and splice acceptor (SA) sites.

Figure 3 shows a Receiver Operating Characteristic (ROC) curve. HPV DNA (PapilloCheck) was used as a reference to evaluate the performances of HPV RNA-Seq for HPV genotyping applications. AUC means Area Under Curve.

Figure 4 shows the number of HPV genotypes identified by HPV RNA-Seq (left bars) at threshold value of 150 reads, vs HPV DNA -PapilloCheck (right bars).

The examples and figures should not be interpreted in any way as limiting the scope of the present invention.

**EXAMPLES:**

**Material and Methods:**

Evaluation of transport medium for RNA conservation

HPV16-positive cervical squamous cell carcinoma SiHa cells were cultivated and inoculated at a final concentration of $7x10^4$ cells/mL in four transport medium: PreservCyt Solution (Hologic, USA), NovaPrep HQ+ Solution (Novaprep, France), RNA Protect Cell Reagent (Qiagen, Germany) and NucliSens Lysis Buffer (BioMérieux, France). The mixtures were aliquoted in 1mL tubes and kept at room temperature for 2 hours (D0), 48 hours (D2), 168 hours (D7), 336 hours (D14) and 504 hours (D21). In parallel, $7x10^4$ cells pellets without transport medium were kept frozen -80°C for 2 hours, 48 hours, 168 hours, 336 hours and 504 hours as a control. At D0, D2, D7, D14 and D21, room temperature aliquots were centrifuged, the medium removed, and the pellets were frozen -80°C for a short time (<1h) before proceeding with RNA extraction. In the particular case of the NucliSens Lysis Buffer since the cells were lysed, the entire 1mL aliquot was frozen -80°C for a short time without prior centrifugation. For each sample, RNA was extracted using the PicoPure RNA Isolation kit (Thermo Fisher Scientific, USA), together with the corresponding (time match) frozen control, so that all samples have undergone one freezing cycle. RT-qPCR was performed to quantify the expression of the two human genes G6PD (forward primer: TGCAGATGCTGTGTCTGG (SEQ ID NO: 2251); reverse primer: CGTACTGGCCCAGGACC (SEQ ID NO: 2252) and GAPDH (forward primer: GAAGGTGAAGGTCGGAGTC; reverse primer: GAAGATGGTGATGGGATTTC (SEQ ID NO: 2253)) and the expression of the two viral genes HPV16 E6 (forward primer: ATGCACCAAAAGAGAACTGC (SEQ ID NO: 2254); reverse primer: TTACAGCTGGGTTTCTCTAC (SEQ ID NO: 2255)) and E7 (forward primer: GTAACCTTTTGTTGCAAGTGTGACT (SEQ ID NO: 2256); reverse primer: GATTATGGTTTCTGAGAACAGATGG (SEQ ID NO:2257)). RNA integrity was assessed on a Bioanalyzer instrument (Agilent, USA).

HPV selection and splice sites analysis

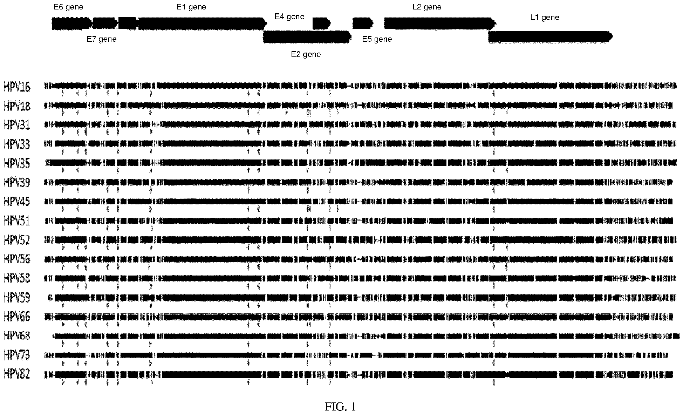

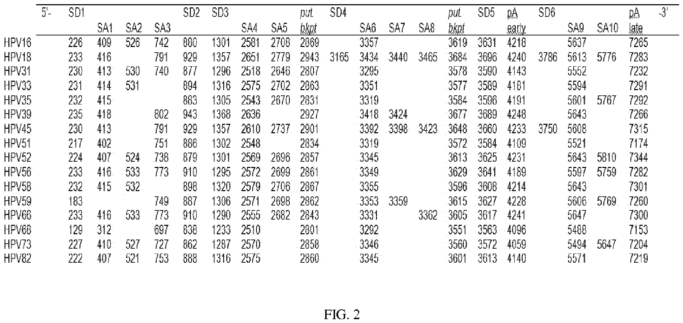

HPV reference clones made available by the International Human Papillomavirus Reference Center (Karolinska University, Stockholm, Sweden) served as reference genomes, except for HPV68 which was retrieved from Chen et al. (Evolution and Taxonomic Classification of Alphapapillomavirus 7 Complete Genomes: HPV18, HPV39, HPV45, HPV59, HPV68 and HPV70. PLOS ONE, 2013, 8:e72565). Accession numbers used in this study were: K02718 (HPV16), X05015 (HPV18), J04353 (HPV31), M12732 (HPV33), X74477 (HPV35), M62849 (HPV39), X74479 (HPV45), M62877 (HPV51), X74481 (HPV52), X74483 (HPV56), D90400 (HPV58), X77858 (HPV59), U31794 (HPV66), KC470267 (HPV68), X94165 (HPV73) and AB027021 (HPV82). Multiple alignments of HPV genomes was done with ClustalW v2.1 using Geneious v10 (Kearse M. et al. Geneious Basic: an integrated and extendable desktop software platform for the organization and analysis of sequence data. Bioinforma Oxf Engl, 2012, 28:1647-9). Previously known splice donor (SD) and splice acceptor (SA) sites for HPV16 (Zheng Z-M, et al. Papillomavirus genome structure, expression, and post-transcriptional regulation. Front Biosci J Virtual Libr, 2006, 11:2286-302) and HPV18 (Wang X, et al. Construction of a full transcription map of human papillomavirus type 18 during productive viral infection. J Virol, 2011, 85:8080-92) were reported on the alignment, and predictions of unknown SD and SA sites were done manually for the other genotypes by sequence analogy (Figures 1 and 2).

HPV RNA-Seq AmpliSeq custom panel

A custom AmpliSeq panel was designed to be used on both PGM and Ion Proton instruments (Thermo Fisher Scientific). Five categories of target sequences were defined as follow:
HPV splice junctions (sp): a set of target sequences which are specific HPV splice events, involving a pair of splice donor (SD) and splice acceptor (SA) sites. The nomenclature includes a "sp" tag. For example, "31_sp_1296_3295_J43-46" stands for HPV31 (31), splice junction (sp), SD at position 1296 on HPV31 genome, SA at position 3295 on HPV31 genome, and junction (J) at position 43-46 on amplicon. The junction coordinates are given in a 4-bases interval, where the first 2 bases correspond to the donor part (or left part) and the last 2 bases to the acceptor part (or right part) of the sequence. Primers and amplicons corresponding to splice junctions set are given at Table 2A and 2Abis.
HPV unsplice junctions (unsp): a set of target sequences which are specific HPV genomic regions spanning either SD or SA sites, in the absence of any splice event. The nomenclature includes an "unsp" tag. For example, "31_unsp_1296_1297_J43-46" stands for HPV31 (31), unspliced (unsp), last base of the left part of the amplicon at position 1296 on HPV31 genome, first base of the right part of the amplicon at position 1297 on HPV31 genome, junction (J) at position 43-46 on amplicon. Primers and amplicons corresponding to unsplice junctions set are given at Table 2B and 2Bbis. In this context, the term 'junction' refers to the exon-intron interface (i.e. the position where a donor or acceptor site would be found in case of a splice event), and the associated junction coordinates are used to characterize unspliced sequences bioinformatically as described in section "Sequencing data processing".

HPV genome away from splice junctions (gen): a set of target sequences which are specific HPV genomic regions, away from any SD or SA sites. The nomenclature includes a "gen" tag. For example, "45_gen_1664_1794_NoJ" stands for HPV45 (45), HPV genomic region (gen), amplicon coordinates from position 1664 to position 1794 on HPV45 genome. Primers and amplicons corresponding to the genomic set are given at Table 2C and 2Cbis.

HPV-human fusion sequences (fus): a set of hypothesis-driven viral-cellular fusion transcripts, based on previous descriptions (Wentzensen N, et al. Characterization of viral-cellular fusion transcripts in a large series of HPV16 and 18 positive anogenital lesions. Oncogene, 2002, 21:419-2622-26, Tang K-W, et al. The landscape of viral expression and host gene fusion and adaptation in human cancer. Nat Commun, 2013, 4:2513, Peter M, et al. MYC activation associated with the integration of HPV DNA at the MYC locus in genital tumors. Oncogene, 2006, 25:5985-93, Lu X, et al. Multiple-integrations of HPV16 genome and altered transcription of viral oncogenes and cellular genes are associated with the development of cervical cancer. PloS One, 2014, 9:e97588, Kraus I, et al. The majority of viral-cellular fusion transcripts in cervical carcinomas cotranscribe cellular sequences of known or predicted genes. Cancer Res, 2008, 68:2514-22). For each HPV, 18 fusion sequence candidates involving SA2 or putative breakpoint 1 or 2 (put. bkpt, see Figure 2) for the viral part, and specific exons from MYC or PVT1 oncogenes for the cellular part, were added to the design. For example, "18_fus_929_MYC_001_exon3_J37-40" stands for HPV18 (18), candidate fusion transcript (fus), break/fusion at position 929 on HPV18 genome, fused with MYC mRNA isoform 001 exon 3, junction (J) at position 37-40 on amplicon. Primers and amplicons corresponding to the fusion set are given at Table 2D and 2Dbis.

Human sequences (hg): a set of 30 human sequences used as internal controls retrieved from publically available AmpliSeq projects and representing housekeeping genes (ACTB, B2M, GAPDH, GUSB, RPLP0), epithelial markers (KRT10, KRT14, KRT17), oncogenes, tumor supressor genes canonical cancer pathways and direct or indirect downstream effectors of HPV oncoproteins (AKT1, BCL2, BRAF, CDH1, CDKN2A, CDKN2B, ERBB2, FOS, HRAS, KRAS, MET, MKI67, MYC, NOTCH1, PCNA, PTEN, RB1, STAT1, TERT, TOP2A, TP53, WNT1). The nomenclature for these sequences includes an "hg" tag. For example, "hg_TOP2A_E21E22" stands for human topoisomerase 2A mRNA exon (Wang X, et al. 2011, Wentzensen N, et al., 2002). Primers and amplicons corresponding to the human set are given at Table 2E and 2Ebis.

In total, 750 target sequences were included into the panel (Table 1) and can be amplified with a pool of 525 unique primers (Table 2A-2E). The average amplicon size of the panel (primers included) is 141bp (range: 81-204bp). A detailed table including the nucleic acid sequences of the primers along with their corresponding amplicons and amplicon sequences is given in Table 2Abis-2Ebis.

Table 1 below shows the HPV RNA-Seq AmpliSeq custom panel contents. The number of target amplicons is indicated for each category (sp, unsp, gen, fus, hg) and for each viral and cellular origin. Putative high-risk HPV are indicated by a star (*).

Table 1

|  | Sp | unsp | gen | fus | Hg | |
|---|---|---|---|---|---|---|
| HPV16 | 14 | 11 | 4 | 18 | 0 | |
| HPV18 | 18 | 12 | 4 | 18 | 0 | |
| HPV31 | 14 | 11 | 4 | 18 | 0 | |
| HPV33 | 13 | 9 | 4 | 18 | 0 | |
| HPV35 | 14 | 10 | 4 | 18 | 0 | |
| HPV39 | 10 | 8 | 4 | 18 | 0 | |
| HPV45 | 14 | 10 | 4 | 18 | 0 | |
| HPV51 | 10 | 9 | 4 | 18 | 0 | |
| HPV52 | 16 | 11 | 4 | 18 | 0 | |
| HPV56 | 16 | 10 | 4 | 18 | 0 | |
| HPV58 | 13 | 8 | 4 | 18 | 0 | |
| HPV59 | 14 | 8 | 4 | 18 | 0 | |
| HPV66 | 15 | 8 | 4 | 18 | 0 | |
| HPV68* | 10 | 9 | 4 | 18 | 0 | |

(continued)

|         | Sp  | unsp | gen | fus | Hg |     |
|---------|-----|------|-----|-----|-----|-----|
| HPV73*  | 13  | 10   | 4   | 18  | 0   |     |
| HPV82*  | 11  | 9    | 4   | 18  | 0   |     |
| human   | 0   | 0    | 0   | 0   | 30  |     |
| TOTAL   | 215 | 153  | 64  | 288 | 30  | 750 |

Table 2A

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV16 | SD3-SA4 i=1 | GCGGGTATGGCAATACTGAAGT | 1 | GTTTTCGTCAAATGGAAACTCATTAGGA | 2 |
| HPV16 | SD3-SA5 i=2 | GCGGGTATGGCAATACTGAAGT | 3 | TGACACACATTTAAACGTTGGCAAAG | 4 |
| HPV16 | SD3-SA6 i=3 | GCGGGTATGGCAATACTGAAGT | 5 | AAGGCGACGGCTTTGGTAT | 6 |
| HPV16 | SD1-SA4 i=4 | CACAGAGCTGCAAACAACTATACAT | 7 | GTTTTCGTCAAATGGAAACTCATTAGGA | 8 |
| HPV16 | SD1-SA5 i=5 | CACAGAGCTGCAAACAACTATACAT | 9 | TGACACACATTTAAACGTTGGCAAAG | 10 |
| HPV16 | SD1-SA6 i=6 | CACAGAGCTGCAAACAACTATACAT | 11 | AAGGCGACGGCTTTGGTAT | 12 |
| HPV16 | SD1-SA11 i=7 | CACAGAGCTGCAAACAACTATACAT | 13 | TGTCCAGATGTCTTTGCTTTTCTTCA | 14 |
| HPV16 | SD1-SA2 i=8 | CACAGAGCTGCAAACAACTATACAT | 15 | TCAGTTGTCTCTGGTTGCAAATCT | 16 |
| HPV16 | SD1-SA3 i=9 | CACAGAGCTGCAAACAACTATACAT | 17 | CCATTAACAGGTCTTCCAAAGTACGA | 18 |
| HPV16 | SD5-SA9 i=11 | GCTCACACAAAGGACGGATTAAC | 19 | ATCCGTGCTTACAACCTTAGATACTG | 20 |
| HPV16 | SD2-SA4 i=15 | GGAATTGTGTGCCCCATCTGT | 21 | GTTTTCGTCAAATGGAAACTCATTAGGA | 22 |
| HPV16 | SD2-SA5 i=16 | GGAATTGTGTGCCCCATCTGT | 23 | TGACACACATTTAAACGTTGGCAAAG | 24 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV16 | SD2-SA6 i=17 | GGAATTGTGTGCCCCATCTGT | 25 | AAGGCGACGGCTTTGGTAT | 26 |
| HPV16 | SD2-SA9 i=18 | GGAATTGTGTGCCCCATCTGT | 27 | ATCCGTGCTTACAACCTTAGATACTG | 28 |
| HPV18 | SD3-SA4 i=1 | TCAGATAGTGGCTATGGCTGTTCT | 29 | GTCATTTATTTCATATACTGGATTGCCA | 30 |
| HPV18 | SD3-SA5 i=2 | TCAGATAGTGGCTATGGCTGTTCT | 31 | GGTTTCCTTCGGTGTCTGCAT | 32 |
| HPV18 | SD3-SA6 i=3 | TCAGATAGTGGCTATGGCTGTTCT | 33 | ACGTCTGGCCGTAGGTCT | 34 |
| HPV18 | SD1-SA4 i=4 | TTCACTGCAAGACATAGAAATAACCTGT | 35 | GTCATTTATTTCATATACTGGATTGCCA | 36 |
| HPV18 | SD1-SA5 i=5 | TTCACTGCAAGACATAGAAATAACCTGT | 37 | GGTTTCCTTCGGTGTCTGCAT | 38 |
| HPV18 | SD1-SA6 i=6 | TTCACTGCAAGACATAGAAATAACCTGT | 39 | ACGTCTGGCCGTAGGTCT | 40 |
| HPV18 | SD1-SA1 i=7 | TTCACTGCAAGACATAGAAATAACCTGT | 41 | CCCAGCTATGTTGTGAAATCGT | 42 |
| HPV18 | SD1-SA3 i=9 | TTCACTGCAAGACATAGAAATAACCTGT | 43 | AGAAACAGCTGCTGGAATGCT | 44 |
| HPV18 | SD4-SA6 i=10 | GGATTGGACACTGCAAGACACA | 45 | ACGTCTGGCCGTAGGTCT | 46 |

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV18 | SD5-SA9 i=11 | CAGCTACACCTACAGGCAACAA | 47 | GTATTTACAACTCTTGCCACAGAAGGA | 48 |
| HPV18 | SD5-SA10 i=12 | CAGCTACACCTACAGGCAACAA | 49 | TCAGGTAACTGCACCCTAAATACTCTAT | 50 |
| HPV18 | SD6-SA9 i=13 | CGAAAACATAGCGACCACTATAGAGAT | 51 | GTATTTACAACTCTTGCCACAGAAGGA | 52 |
| HPV18 | SD6-SA10 i=14 | CGAAAACATAGCGACCACTATAGAGAT | 53 | TCAGGTAACTGCACCCTAAATACTCTAT | 54 |
| HPV18 | SD2-SA4 i=15 | TGCATCCCAGCAGTAAGCAA | 55 | GTCATTTATTTCATATACTGGATTGCCA | 56 |
| HPV18 | SD2-SA5 i=16 | TGCATCCCAGCAGTAAGCAA | 57 | GGTTTCCTTCGGTGTCTGCAT | 58 |
| HPV18 | SD2-SA8 i=25 | TGCATCCCAGCAGTAAGCAA | 59 | ACGTCTGGCCGTAGGTCT | 60 |
| HPV18 | SD2-SA9 i=18 | TGCATCCCAGCAGTAAGCAA | 61 | GTATTTACAACTCTTGCCACAGAAGGA | 62 |
| HPV18 | SD2-SA10 i=19 | TGCATCCCAGCAGTAAGCAA | 63 | TCAGGTAACTGCACCCTAAATACTCTAT | 64 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV31 | SD3-SA4 i=1 | GCGGGTATGGCAATACTGAAGT | 65 | AATGTAAAAACCACCAGTCTGCTATGTA | 66 |
| HPV31 | SD3-SA5 i=2 | GCGGGTATGGCAATACTGAAGT | 67 | CGTTGAGAAAGAGTCTCCATCGTTTT | 68 |
| HPV31 | SD3-SA6 i=3 | GCGGGTATGGCAATACTGAAGT | 69 | GAATTCGATGTGGTGGTGTTGTTG | 70 |
| HPV31 | SD1-SA4 i=4 | CGGCATTGGAAATACCCTACGAT | 71 | AATGTAAAAACCACCAGTCTGCTATGTA | 72 |
| HPV31 | SD1-SA5 i=5 | CGGCATTGGAAATACCCTACGAT | 73 | CGTTGAGAAAGAGTCTCCATCGTTTT | 74 |
| HPV31 | SD1-SA6 i=6 | CGGCATTGGAAATACCCTACGAT | 75 | GAATTCGATGTGGTGGTGTTGTTG | 76 |
| HPV31 | SD1-SA1 i=7 | CGGCATTGGAAATACCCTACGAT | 77 | TTTTCTTCTGGACACAACGGTCTT | 78 |
| HPV31 | SD1-SA2 i=8 | CGGCATTGGAAATACCCTACGAT | 79 | ACATAGTCTTGCAACGTAGGTGTTT | 80 |
| HPV31 | SD1-SA3 i=9 | CGGCATTGGAAATACCCTACGAT | 81 | CATTAACAGCTCTTGCAATATGCGAATA | 82 |
| HPV31 | SD5-SA9 i=11 | CAGCTGCATGCACAAACCA | 83 | TTTAGACACTGGGACAGGTGGTA | 84 |
| HPV31 | SD2-SA4 i=15 | AATCGTGTGCCCCAACTGT | 85 | AATGTAAAAACCACCAGTCTGCTATGTA | 86 |
| HPV31 | SD2-SA5 i=16 | AATCGTGTGCCCCAACTGT | 87 | CGTTGAGAAAGAGTCTCCATCGTTTT | 88 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV31 | SD2-SA6 i=17 | AATCGTGTGCCCCAACTGT | 89 | GAATTCGATGTGGTGGTGTTGTTG | 90 |
| HPV31 | SD2-SA9 i=18 | AATCGTGTGCCCCAACTGT | 91 | TTTAGACACTGGGACAGGTGGTA | 92 |
| HPV33 | SD3-SA4 i=1 | GATGAGCTAGAAGACAGCGGATATG | 93 | CATACACTGGGTTACCATTTTCATCAAA | 94 |
| HPV33 | SD3-SA5 i=2 | GATGAGCTAGAAGACAGCGGATATG | 95 | TGATATTTCCTCCATGGTTTTCCTTGTC | 96 |
| HPV33 | SD3-SA6 i=3 | GATGAGCTAGAAGACAGCGGATATG | 97 | GTGGTGGTCGGTTATCGTTGT | 98 |
| HPV33 | SD1-SA4 i=4 | AGCATTGGAGACAACTATACACAACATT | 99 | CATACACTGGGTTACCATTTTCATCAAA | 100 |
| HPV33 | SD1-SA5 i=5 | AGCATTGGAGACAACTATACACAACATT | 101 | TGATATTTCCTCCATGGTTTTCCTTGTC | 102 |
| HPV33 | SD1-SA6 i=6 | AGCATTGGAGACAACTATACACAACATT | 103 | GTGGTGGTCGGTTATCGTTGT | 104 |
| HPV33 | SD1-SA1 i=7 | AGCATTGGAGACAACTATACACAACATT | 105 | TCGTTTGTTTAAATCCACATGTCGTTTT | 106 |
| HPV33 | SD1-SA2 i=8 | AGCATTGGAGACAACTATACACAACATT | 107 | CATATTCCTTTAACGTTGGCTTGTGT | 108 |
| HPV33 | SD5-SA9 i=11 | ACGTACTGCAACTAACTGCACAA | 109 | ATCAGTGCTGACAACTTTAGATACAGG | 110 |

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV33 | SD2-SA4 i=15 | GTGCCCTACCTGTGCACAA | 111 | CATACACTGGGTTACCATTTTCATCAAA | 112 |
| HPV33 | SD2-SA5 i=16 | GTGCCCTACCTGTGCACAA | 113 | TGATATTTCCTCCATGGTTTTCCTTGTC | 114 |
| HPV33 | SD2-SA6 i=17 | GTGCCCTACCTGTGCACAA | 115 | GTGGTGGTCGGTTATCGTTGT | 116 |
| HPV33 | SD2-SA9 i=18 | GTGCCCTACCTGTGCACAA | 117 | ATCAGTGCTGACAACTTTAGATACAGG | 118 |
| HPV35 | SD3-SA4 i=1 | ATTATTTGAACTACCAGACAGCGGTT | 119 | TCATTGTGAAATGTAAAGACCACTACCC | 120 |
| HPV35 | SD3-SA5 i=2 | ATTATTTGAACTACCAGACAGCGGTT | 121 | GGAAAGCGTCTCCATCATTTTCTTTG | 122 |
| HPV35 | SD3-SA6 i=3 | ATTATTTGAACTACCAGACAGCGGTT | 123 | GCTTTGGTATGGGTCTCGGT | 124 |
| HPV35 | SD1-SA4 i=4 | CGAGGTAGAAGAAAGCATCCATGAAAT | 125 | TCATTGTGAAATGTAAAGACCACTACCC | 126 |
| HPV35 | SD1-SA5 i=5 | CGAGGTAGAAGAAAGCATCCATGAAAT | 127 | GGAAAGCGTCTCCATCATTTTCTTTG | 128 |
| HPV35 | SD1-SA6 i=6 | CGAGGTAGAAGAAAGCATCCATGAAAT | 129 | GCTTTGGTATGGGTCTCGGT | 130 |
| HPV35 | SD1-SA1 i=7 | CGAGGTAGAAGAAAGCATCCATGAAAT | 131 | TCCACCGATGTTATGGAATCGTTTT | 132 |

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV35 | SD5-SA9 i=11 | TCTACATCTGACTGCACAAACAAAGA | 133 | CATCAGTGCTAACAACCTTAGACACT | 134 |
| HPV35 | SD5-SA10 i=12 | TCTACATCTGACTGCACAAACAAAGA | 135 | ACTCTGTATTGCAAACCAGATACCTTG | 136 |
| HPV35 | SD2-SA4 i=15 | CGGCTGTTCACAGAGAGCATAAT | 137 | TCATTGTGAAATGTAAAGACCACTACCC | 138 |
| HPV35 | SD2-SA5 i=16 | CGGCTGTTCACAGAGAGCATAAT | 139 | GGAAAGCGTCTCCATCATTTTCTTTG | 140 |
| HPV35 | SD2-SA6 i=17 | CGGCTGTTCACAGAGAGCATAAT | 141 | GCTTTGGTATGGGTCTCGGT | 142 |
| HPV35 | SD2-SA9 i=18 | CGGCTGTTCACAGAGAGCATAAT | 143 | CATCAGTGCTAACAACCTTAGACACT | 144 |
| HPV35 | SD2-SA10 i=19 | CGGCTGTTCACAGAGAGCATAAT | 145 | ACTCTGTATTGCAAACCAGATACCTTG | 146 |
| HPV39 | SD3-SA4 i=1 | GGTGTATTCCGTGCCAGACA | 147 | CTGTTTTGGTCAAATGGAAATGCATTAG | 148 |
| HPV39 | SD3-SA7 i=22 | GGTGTATTCCGTGCCAGACA | 149 | GGTCGCGGTGGTGTTTGATAA | 150 |
| HPV39 | SD1-SA4 i=4 | CACCACCTTGCAGGACATTACAATA | 151 | CTGTTTTGGTCAAATGGAAATGCATTAG | 152 |

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV39 | SD1-SA7 i=20 | CACCACCTTGCAGGACATTACAATA | 153 | GGTCGCGGTGGTGTTTGATAA | 154 |
| HPV39 | SD1-SA1 i=7 | CACCACCTTGCAGGACATTACAATA | 155 | CTGTCCTGTATAGCTTCCTGCTATTTT | 156 |
| HPV39 | SD1-SA3 i=9 | CACCACCTTGCAGGACATTACAATA | 157 | TGCTGTAGTTGTCGCAGAGTATC | 158 |
| HPV39 | SD5-SA9 i=11 | CACAGTAACAGTACAGGCCACA | 159 | AGTATTGACAACCTTCGCCACA | 160 |
| HPV39 | SD2-SA4 i=15 | CGTGGTGTGCAACTGCAA | 161 | CTGTTTTGGTCAAATGGAAATGCATT AG | 162 |
| HPV39 | SD2-SA7 i=21 | CGTGGTGTGCAACTGCAA | 163 | GGTCGCGGTGGTGTTTGATAA | 164 |
| HPV39 | SD2-SA9 i=18 | CGTGGTGTGCAACTGCAA | 165 | AGTATTGACAACCTTCGCCACA | 166 |
| HPV45 | SD3-SA4 i=1 | TCAGATAGTGGCTATGGCTGTTCT | 167 | GAAATGCATGTGGAAATGTAAATACC GT | 168 |
| HPV45 | SD3-SA5 | TCAGATAGTGGCTATGGCTGTTCT | 169 | GGATTCCTTCGGTGTCTGCAT | 170 |
|  | i=2 |  |  |  |  |
| HPV45 | SD3-SA8 i=23 | TCAGATAGTGGCTATGGCTGTTCT | 171 | CCCACGGATGCGGTTTTG | 172 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV45 | SD1-SA4 i=4 | CTACAAGACGTATCTATTGCCTGTGT | 173 | GAAATGCATGTGGAAATGTAAATACCGT | 174 |
| HPV45 | SD1-SA5 i=5 | CTACAAGACGTATCTATTGCCTGTGT | 175 | GGATTCCTTCGGTGTCTGCAT | 176 |
| HPV45 | SD1-SA8 i=24 | CTACAAGACGTATCTATTGCCTGTGT | 177 | CCCACGGATGCGGTTTTG | 178 |
| HPV45 | SD1-SA1 i=7 | CTACAAGACGTATCTATTGCCTGTGT | 179 | CGTTTGTCCTTAAGGTGTCTACGTTTT | 180 |
| HPV45 | SD1-SA3 i=9 | CTACAAGACGTATCTATTGCCTGTGT | 181 | TCAAAAACAGCTGCTGTAGTGTTCT | 182 |
| HPV45 | SD5-SA9 i=11 | TCCTGTGTTCAAGTACAAGTAACAACAA | 183 | GCTGACAACTCTGGCCACA | 184 |
| HPV45 | SD6-SA9 i=13 | CGCAAATATGCAGACCATTACTCAGAA | 185 | GCTGACAACTCTGGCCACA | 186 |
| HPV45 | SD2-SA4 i=15 | AGCACCTTGTCCTTTGTGTGT | 187 | GAAATGCATGTGGAAATGTAAATACCGT | 188 |
| HPV45 | SD2-SA5 i=16 | AGCACCTTGTCCTTTGTGTGT | 189 | GGATTCCTTCGGTGTCTGCAT | 190 |
| HPV45 | SD2-SA8 i=25 | AGCACCTTGTCCTTTGTGTGT | 191 | CCCACGGATGCGGTTTTG | 192 |
| HPV45 | SD2-SA9 i=18 | AGCACCTTGTCCTTTGTGTGT | 193 | GCTGACAACTCTGGCCACA | 194 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV51 | SD3-SA4 i=1 | CGGACAGCGGATATGGCAATA | 195 | TCATTCAATGTATACACAGCATTCCCAT | 196 |
| HPV51 | SD3-SA6 i=3 | CGGACAGCGGATATGGCAATA | 197 | CCACGCAGGTGGTAAGGG | 198 |
| HPV51 | SD1-SA4 i=4 | CTGCATGAATTATGTGAAGCTTTGAAC | 199 | TCATTCAATGTATACACAGCATTCCCAT | 200 |
| HPV51 | SD1-SA6 i=6 | CTGCATGAATTATGTGAAGCTTTGAAC | 201 | CCACGCAGGTGGTAAGGG | 202 |
| HPV51 | SD1-SA1 i=7 | CTGCATGAATTATGTGAAGCTTTGAAC | 203 | TCCCGCTATTTCATGGAACCTTTT | 204 |
| HPV51 | SD1-SA3 I=9 | CTGCATGAATTATGTGAAGCTTTGAAC | 205 | CATCTGCTGTACAACGCGAAG | 206 |
| HPV51 | SD5-SA9 i=11 | CTAACACTGGAGGGCACCAAA | 207 | CAATTCGAGACACAGGTGCAG | 208 |
| HPV51 | SD2-SA4 i=15 | GGGCGAACTAAGCCTGGTTT | 209 | TCATTCAATGTATACACAGCATTCCCAT | 210 |
| HPV51 | SD2-SA6 i=17 | GGGCGAACTAAGCCTGGTTT | 211 | CCACGCAGGTGGTAAGGG | 212 |
| HPV51 | SD2-SA9 i=18 | GGGCGAACTAAGCCTGGTTT | 213 | CAATTCGAGACACAGGTGCAG | 214 |
| HPV52 | SD3-SA4 i=1 | CAAACCATGTCACGTAGAAGACAG | 215 | GGGTTTTTGAAATGAAACACAACCAATC | 216 |

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV52 | SD3-SA5 i=2 | CAAACCATGTCACGTAGAAGACAG | 217 | CGGTATCGACTCCATCGTTTTCC | 218 |
| HPV52 | SD3-SA6 i=3 | CAAACCATGTCACGTAGAAGACAG | 219 | GCGGAGGTCTTGGAGGTTT | 220 |
| HPV52 | SD1-SA4 i=4 | AGAATCGGTGCATGAAATAAGGCT | 221 | GGGTTTTTGAAATGAAACACAACCAATC | 222 |
| HPV52 | SD1-SA5 i=5 | AGAATCGGTGCATGAAATAAGGCT | 223 | CGGTATCGACTCCATCGTTTTCC | 224 |
| HPV52 | SD1-SA6 i=6 | AGAATCGGTGCATGAAATAAGGCT | 225 | GCGGAGGTCTTGGAGGTTT | 226 |
| HPV52 | SD1-SA1 i=7 | AGAATCGGTGCATGAAATAAGGCT | 227 | CGCTTGTTTGCATTAACATGTCTTTCT | 228 |
| HPV52 | SD1-SA2 i=8 | AGAATCGGTGCATGAAATAAGGCT | 229 | TCAGTTGTTTCAGGTTGCAGATCTAATA | 230 |
| HPV52 | SD1-SA3 i=9 | AGAATCGGTGCATGAAATAAGGCT | 231 | GCATTGCTGTAGAGTACGAAGGT | 232 |
| HPV52 | SD5-SA9 i=11 | TCACTGCAACTGAGTGCACAA | 233 | TGCTTACAACCTTAGAGACAGGTACA | 234 |
| HPV52 | SD5-SA10 i=12 | TCACTGCAACTGAGTGCACAA | 235 | CCTGTATTGCAGGCCAGACA | 236 |
| HPV52 | SD2-SA4 i=15 | GCTGTTGGGCACATTACAAGTT | 237 | GGGTTTTTGAAATGAAACACAACCAATC | 238 |

42

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV52 | SD2-SA5 i=16 | GCTGTTGGGCACATTACAAGTT | 239 | CGGTATCGACTCCATCGTTTTCC | 240 |
| HPV52 | SD2-SA6 i=17 | GCTGTTGGGCACATTACAAGTT | 241 | GCGGAGGTCTTGGAGGTTT | 242 |
| HPV52 | SD2-SA9 i=18 | GCTGTTGGGCACATTACAAGTT | 243 | TGCTTACAACCTTAGAGACAGGTACA | 244 |
| HPV52 | SD2-SA10 i=19 | GCTGTTGGGCACATTACAAGTT | 245 | CCTGTATTGCAGGCCAGACA | 246 |
| HPV56 | SD3-SA4 i=1 | CAAGACAGCGGGTATGGCAATA | 247 | TGAAACTGAAACACTAACATTCTACTGTGT | 248 |
| HPV56 | SD3-SA5 i=2 | CAAGACAGCGGGTATGGCAATA | 249 | TTTTCTTTGTCCTCGTCGTTATCCAA | 250 |
| HPV56 | SD3-SA6 i=3 | CAAGACAGCGGGTATGGCAATA | 251 | GGTGGTGGTGGTGGTCTT | 252 |
| HPV56 | SD1-SA4 i=4 | GCACCACTTGAGTGAGGTATTAGAA | 253 | TGAAACTGAAACACTAACATTCTACTGTGT | 254 |
| HPV56 | SD1-SA5 i=5 | GCACCACTTGAGTGAGGTATTAGAA | 255 | TTTTCTTTGTCCTCGTCGTTATCCAA | 256 |
| HPV56 | SD1-SA6 i=6 | GCACCACTTGAGTGAGGTATTAGAA | 257 | GGTGGTGGTGGTGGTCTT | 258 |
| HPV56 | SD1-SA1 i=7 | GCACCACTTGAGTGAGGTATTAGAA | 259 | CAATTGCTTTTCCTCCGGAGTTAA | 260 |
| HPV56 | SD1-SA2 i=8 | GCACCACTTGAGTGAGGTATTAGAA | 261 | ACGTCTTGCAGCGTTGGTA | 262 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV56 | SD1-SA3 i=9 | GCACCACTTGAGTGAGGTATTAGAA | 263 | TGTACAACACGCAGGTCCTC | 264 |
| HPV56 | SD5-SA9 i=11 | ACAACAACCACCCTGGTGATAAG | 265 | ACAACCTTTGAAACAGGTGTTGGA | 266 |
| HPV56 | SD5-SA10 i=12 | ACAACAACCACCCTGGTGATAAG | 267 | CAACCGTACCCTAAATACCCTATATTGA | 268 |
| HPV56 | SD2-SA4 i=15 | GTTAACAGTAACGTGCCCACTCT | 269 | TGAAACTGAAACACTAACATTCTACTGTGT | 270 |
| HPV56 | SD2-SA5 i=16 | GTTAACAGTAACGTGCCCACTCT | 271 | TTTTCTTTGTCCTCGTCGTTATCCAA | 272 |
| HPV56 | SD2-SA6 i=17 | GTTAACAGTAACGTGCCCACTCT | 273 | GGTGGTGGTGGTGGTCTT | 274 |
| HPV56 | SD2-SA9 i=18 | GTTAACAGTAACGTGCCCACTCT | 275 | ACAACCTTTGAAACAGGTGTTGGA | 276 |
| HPV56 | SD2-SA10 i=19 | GTTAACAGTAACGTGCCCACTCT | 277 | CAACCGTACCCTAAATACCCTATATTGA | 278 |
| HPV58 | SD3-SA4 i=1 | AAAATTATTGAGCTAGAAGACAGCGGAT | 279 | TGCATCAAATGGAAATGGATTGTTAAATTCA | 280 |
| HPV58 | SD3-SA5 i=2 | AAAATTATTGAGCTAGAAGACAGCGGAT | 281 | TGATATTTCCTCCATCGTTTTCCTTGTC | 282 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV58 | SD3-SA6 i=3 | AAAATTATTGAGCTAGAAGACAGCGGAT | 283 | CCCTGTGTACTTTCGTTGTTGGT | 284 |
| HPV58 | SD1-SA4 i=4 | GTCAGGCGTTGGAGACATCT | 285 | TGCATCAAATGGAAATGGATTGTTAAATTCA | 286 |
| HPV58 | SD1-SA5 i=5 | GTCAGGCGTTGGAGACATCT | 287 | TGATATTTCCTCCATCGTTTTCCTTGTC | 288 |
| HPV58 | SD1-SA6 i=6 | GTCAGGCGTTGGAGACATCT | 289 | CCCTGTGTACTTTCGTTGTTGGT | 290 |
| HPV58 | SD1-SA1 i=7 | GTCAGGCGTTGGAGACATCT | 291 | CGACCCGAAATATTATGAAACCTTTTGT | 292 |
| HPV58 | SD1-SA2 i=8 | GTCAGGCGTTGGAGACATCT | 293 | GCGTTGGGTTGTTTCCTCTCA | 294 |
| HPV58 | SD5-SA9 i=11 | GAGGAGGACTACACAGTACAACTAACT | 295 | GCTTACAACCTTAGACACAGGCA | 296 |
| HPV58 | SD2-SA4 i=15 | TGCTTATGGGCACATGTACCATT | 297 | TGCATCAAATGGAAATGGATTGTTAAATTCA | 298 |
| HPV58 | SD2-SA5 i=16 | TGCTTATGGGCACATGTACCATT | 299 | TGATATTTCCTCCATCGTTTTCCTTGTC | 300 |
| HPV58 | SD2-SA6 i=17 | TGCTTATGGGCACATGTACCATT | 301 | CCCTGTGTACTTTCGTTGTTGGT | 302 |
| HPV58 | SD2-SA9 i=18 | TGCTTATGGGCACATGTACCATT | 303 | GCTTACAACCTTAGACACAGGCA | 304 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV59 | SD3-SA4 i=1 | AAAGAAGGTTAATAACAGTGCCAGACA | 305 | TCTATTTTTGTCAAATGGCAATTTGTTTGGA | 306 |
| HPV59 | SD3-SA5 i=2 | AAAGAAGGTTAATAACAGTGCCAGACA | 307 | GGTGTCCATCACTGTCTGCAT | 308 |
| HPV59 | SD3-SA7 i=22 | AAAGAAGGTTAATAACAGTGCCAGACA | 309 | CCCAAGTACGTGGCTTCGG | 310 |
| HPV59 | SD1-SA4 i=4 | GCATCAATTGTGTGTTTTGCAAAGG | 311 | TCTATTTTTGTCAAATGGCAATTTGTTTGGA | 312 |
| HPV59 | SD1-SA5 i=5 | GCATCAATTGTGTGTTTTGCAAAGG | 313 | GGTGTCCATCACTGTCTGCAT | 314 |
| HPV59 | SD1-SA7 i=20 | GCATCAATTGTGTGTTTTGCAAAGG | 315 | CCCAAGTACGTGGCTTCGG | 316 |
| HPV59 | SD1-SA3 i=9 | GCATCAATTGTGTGTTTTGCAAAGG | 317 | TGTAAGGCTCGCAATCCGT | 318 |
| HPV59 | SD5-SA9 i=11 | TCCGTTTGCATCCAGGCAA | 319 | TGACATACTCATCAGTGCTGACAAC | 320 |
| HPV59 | SD5-SA10 i=12 | TCCGTTTGCATCCAGGCAA | 321 | GCCAAATTTATTGGGATCAGGTAACTT | 322 |
| HPV59 | SD2-SA4 i=15 | ACTATCCTTTGTGTGTCCTTTGTGT | 323 | TCTATTTTTGTCAAATGGCAATTTGTTTGGA | 324 |
| HPV59 | SD2-SA5 i=16 | ACTATCCTTTGTGTGTCCTTTGTGT | 325 | GGTGTCCATCACTGTCTGCAT | 326 |

(continued)

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV59 | SD2-SA7 i=21 | ACTATCCTTTGTGTGTCCTTTGTGT | 327 | CCCAAGTACGTGGCTTCGG | 328 |
| HPV59 | SD2-SA9 i=18 | ACTATCCTTTGTGTGTCCTTTGTGT | 329 | TGACATACTCATCAGTGCTGACAAC | 330 |
| HPV59 | SD2-SA10 i=19 | ACTATCCTTTGTGTGTCCTTTGTGT | 331 | GCCAAATTTATTGGGATCAGGTAACTT | 332 |
| HPV66 | SD3-SA4 i=1 | GAAGACAGCGGGTATGGCAATA | 333 | CATTACTTAATTCATACACAGGATTACCATT | 334 |
| HPV66 | SD3-SA5 i=2 | GAAGACAGCGGGTATGGCAATA | 335 | TTTTCTTTGTCCTCGTCGTTATCCAA | 336 |
| HPV66 | SD3-SA6 i=3 | GACAGGGAGACAGCTCAACAATTATT | 337 | CTCTCGGTACACAGTTTGCTGATTA | 338 |
| HPV66 | SD3-SA8 i=23 | GAAGACAGCGGGTATGGCAATA | 339 | GGTGGTGGTGGTCCTGTG | 340 |
| HPV66 | SD1-SA4 i=4 | CACCATCTGAGCGAGGTATTACA | 341 | CATTACTTAATTCATACACAGGATTACCATT | 342 |
| HPV66 | SD1-SA5 i=5 | CACCATCTGAGCGAGGTATTACA | 343 | TTTTCTTTGTCCTCGTCGTTATCCAA | 344 |
| HPV66 | SD1-SA8 i=24 | CACCATCTGAGCGAGGTATTACA | 345 | GGTGGTGGTGGTCCTGTG | 346 |
| HPV66 | SD1-SA1 i=7 | CACCATCTGAGCGAGGTATTACA | 347 | GAAATCGTCTTTTATGTTCACAGTGCAA | 348 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV66 | SD1-SA2 i=8 | CACCATCTGAGCGAGGTATTACA | 349 | AACCTCTTGCAACGTTGGTACT | 350 |
| HPV66 | SD1-SA3 i=9 | CACCATCTGAGCGAGGTATTACA | 351 | TGTACCACACGTAGCTCCTCT | 352 |
| HPV66 | SD5-SA9 i=11 | GTATCAACACACAAAGCCACTGT | 353 | ACAACCTTTGAAACAGGTGTTGGA | 354 |
| HPV66 | SD2-SA4 i=15 | GTTAACAGTAACGTGCCCACTCT | 355 | CATTACTTAATTCATACACAGGATTACCATT | 356 |
| HPV66 | SD2-SA5 i=16 | GTTAACAGTAACGTGCCCACTCT | 357 | TTTTCTTTGTCCTCGTCGTTATCCAA | 358 |
| HPV66 | SD2-SA8 i=25 | GTTAACAGTAACGTGCCCACTCT | 359 | GGTGGTGGTGGTCCTGTG | 360 |
| HPV66 | SD2-SA9 i=18 | GTTAACAGTAACGTGCCCACTCT | 361 | ACAACCTTTGAAACAGGTGTTGGA | 362 |
| HPV68 | SD3-SA4 i=1 | AGACAACCGGCGTATACAGTG | 363 | CTGTTTTGGTCAAATGGAAATGCATTAG | 364 |
| HPV68 | SD3-SA6 i=3 | AGACAACCGGCGTATACAGTG | 365 | TCGCGGTGGTGTTCTGTAG | 366 |
| HPV68 | SD1-SA4 i=4 | GACATTGGACACTACATTGCATGAC | 367 | CTGTTTTGGTCAAATGGAAATGCATTAG | 368 |
| HPV68 | SD1-SA1 i=7 | GACATTGGACACTACATTGCATGAC | 369 | CTTCGTTTTGTTGTTAGGTGCCTTAG | 370 |

EP 3 715 477 A1

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV68 | SD1-SA6 i=6 | GACATTGGACACTACATTGCATGAC | 371 | TCGCGGTGGTGTTCTGTAG | 372 |
| HPV68 | SD1-SA3 i=9 | GACATTGGACACTACATTGCATGAC | 373 | CTGTTGTAGTGTCCGCAGGTT | 374 |
| HPV68 | SD5-SA9 i=11 | AGTAGAAGTGCAGGCCAAAACAA | 375 | ATTGACAACCTTCGCCACTGA | 376 |
| HPV68 | SD2-SA4 i=15 | TCCGTGGTGTGCAACTGAA | 377 | CTGTTTTGGTCAAATGGAAATGCATTAG | 378 |
| HPV68 | SD2-SA6 i=17 | TCCGTGGTGTGCAACTGAA | 379 | TCGCGGTGGTGTTCTGTAG | 380 |
| HPV68 | SD2-SA9 i=18 | TCCGTGGTGTGCAACTGAA | 381 | ATTGACAACCTTCGCCACTGA | 382 |
| HPV73 | SD3-SA4 i=1 | AAACGAAGACTGTTTGAGGAGCA | 383 | GGGTTCCCATTACTGTCAAATGGA | 384 |
| HPV73 | SD3-SA6 i=3 | AAACGAAGACTGTTTGAGGAGCA | 385 | TGGTGTTGGTGGTTGTGGT | 386 |
| HPV73 | SD1-SA4 i=4 | AGCGTTATGTGACGAAGTGAATATTTCT | 387 | GGGTTCCCATTACTGTCAAATGGA | 388 |
| HPV73 | SD1-SA6 i=6 | AGCGTTATGTGACGAAGTGAATATTTCT | 389 | TGGTGTTGGTGGTTGTGGT | 390 |
| HPV73 | SD1-SA1 i=7 | AGCGTTATGTGACGAAGTGAATATTTCT | 391 | CTGTTCTGCTATTTGATGAAACCGTTT | 392 |

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV73 | SD1-SA2 i=8 | AGCGTTATGTGACGAAGTGAATATTTCT | 393 | TTCGGTTGTTGGTTTCAGGTCTAA | 394 |
| HPV73 | SD1-SA3 i=9 | AGCGTTATGTGACGAAGTGAATATTTCT | 395 | CCTAGTGTACCCATAAGCAACTCTTCTA | 396 |
| HPV73 | SD5-SA9 i=11 | ACCTACATCCCACCACAGAGT | 397 | GCTTACAACCTTAGACACAGACACA | 398 |
| HPV73 | SD5-SA10 i=12 | ACCTACATCCCACCACAGAGT | 399 | ACGAAGCCTAAACACCCTGTATTG | 400 |
| HPV73 | SD2-SA4 i=15 | TGCTTATGGGTACACTAGGTATTGTGT | 401 | GGGTTCCCATTACTGTCAAATGGA | 402 |
| HPV73 | SD2-SA6 i=17 | TGCTTATGGGTACACTAGGTATTGTGT | 403 | TGGTGTTGGTGGTTGTGGT | 404 |
| HPV73 | SD2-SA9 i=18 | TGCTTATGGGTACACTAGGTATTGTGT | 405 | GCTTACAACCTTAGACACAGACACA | 406 |
| HPV73 | SD2-SA10 i=19 | TGCTTATGGGTACACTAGGTATTGTGT | 407 | ACGAAGCCTAAACACCCTGTATTG | 408 |
| HPV82 | SD3-SA4 i=1 | CCGGACAGTGGATATGGCAATA | 409 | CATCATTTAGTGCATATACAGGATTCCC | 410 |
| HPV82 | SD3-SA6 i=3 | CCGGACAGTGGATATGGCAATA | 411 | GGGTGTTCGATAGCTGTTCAA | 412 |

50

| HPV type | Splice junction i | Forward primer nucleic acid sequence | Forward primer SEQ ID NO | Reverse primer nucleic acid sequence | Reverse primer SEQ ID NO |
|---|---|---|---|---|---|
| HPV82 | SD1-SA4 i=4 | CCTGCAATACGTCTATGCACAAT | 413 | CATCATTTAGTGCATATACAGGATTCCC | 414 |
| HPV82 | SD1-SA6 i=6 | CCTGCAATACGTCTATGCACAAT | 415 | GGGTGTTCGATAGCTGTTCAA | 416 |
| HPV82 | SD1-SA1 i=7 | CCTGCAATACGTCTATGCACAAT | 417 | TTTTTTGTCGTCCACCACCTTTTG | 418 |
| HPV82 | SD1-SA2 i=8 | CCTGCAATACGTCTATGCACAAT | 419 | TCCAACACTATGTCCTTAATTGTGGT | 420 |
| HPV82 | SD1-SA3 i=9 | CCTGCAATACGTCTATGCACAAT | 421 | CCAGTAACATTTGCTGAAATATGCGAA | 422 |
| HPV82 | SD5-SA9 i=11 | TGCGACCACCAAATACACTGT | 423 | GTGTTGACAATGCGTGACACT | 424 |
| HPV82 | SD2-SA4 i=15 | CGTGGTGTGCGACCAACTAA | 425 | CATCATTTAGTGCATATACAGGATTCCC | 426 |
| HPV82 | SD2-SA6 i=17 | CGTGGTGTGCGACCAACTAA | 427 | GGGTGTTCGATAGCTGTTCAA | 428 |
| HPV82 | SD2-SA9 i=18 | CGTGGTGTGCGACCAACTAA | 429 | GTGTTGACAATGCGTGACACT | 430 |

EP 3 715 477 A1

51

Table 2Abis

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV16 | SD3-SA4 i=1 | 1 | 2 | GGAAACTCAGCAGATGTTACAGATTCTAGGTGGCCTTATTTACATA ATAGATTGGTGGTGTTTACATT | 1501 |
| HPV16 | SD3-SA5 i=2 | 3 | 4 | GGAAACTCAGCAGATGTTACAGGACGTGGTCCAGATTAAGTTTGCA CGAGGACGAGGACAAGGAAAACGATGGAGACT | 1502 |
| HPV16 | SD3-SA6 i=3 | 5 | 6 | GGAAACTCAGCAGATGTTACAGCAGCAACGAAGTATCCTCTCCTGA AATTATTAGGCAGCACTTGGCCAACCACCCCGCCGCGACCC | 1503 |
| HPV16 | SD1-SA4 i=4 | 7 | 8 | GATATAATATTAGAATGTGTGTACTGCAAGCAACAGTTACTGCGAC GTGAGATTCTAGGTGGCCTTATTTACATAATAGATTGGTGGTGTTTA CATT | 1504 |
| HPV16 | SD1-SA5 i=5 | 9 | 10 | GATATAATATTAGAATGTGTGTACTGCAAGCAACAGTTACTGCGAC GTGAGGACGTGGTCCAGATTAAGTTTGCACGAGGACGAGGACAAG GAAAACGATGGAGACT | 1505 |
| HPV16 | SD1-SA6 i=6 | 11 | 12 | GATATAATATTAGAATGTGTGTACTGCAAGCAACAGTTACTGCGAC GTGAGCAGCAACGAAGTATCCTCTCCTGAAATTATTAGGCAGCACT TGGCCAACCACCCCGCCGCGACCC | 1506 |
| HPV16 | SD1-SA1 i=7 | 13 | 14 | GATATAATATTAGAATGTGTGTACTGCAAGCAACAGTTACTGCGAC GTGAGGTGTATTAACTGTCAAAAGCCACTGTGTCC | 1507 |
| HPV16 | SD1-SA2 i=8 | 15 | 16 | GATATAATATTAGAATGTGTGTACTGCAAGCAACAGTTACTGCGAC GTGAGATCATCAAGAACACGTAGAGAAACCCAGCTGTAATCATGC ATGGAGATACACCTACATTGCATGAATATATGTT | 1508 |
| HPV16 | SD1-SA3 i=9 | 17 | 18 | GATATAATATTAGAATGTGTGTACTGCAAGCAACAGTTACTGCGAC GTGAGTGTGACTCTACGCTTCGGTTGTGCGTACAAAGCACACACGT AGACAT | 1509 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV16 | SD5-SA9 i=11 | 19 | 20 | TGTAATAGTAACACTACACCCATAGTACATTTAAAAGATGTCTCTTT GGCTGCCTAGTGAGGCCACTGTCTACTTGCCTCCTGTCC | 1510 |
| HPV16 | SD2-SA4 i=15 | 21 | 22 | TCTCAGAAACCATAATCTACCATGGCTGATCCTGCAGATTCTAGGT GGCCTTATTTACATAATAGATTGGTGGTGTTTACATT | 1511 |
| HPV16 | SD2-SA5 i=16 | 23 | 24 | TCTCAGAAACCATAATCTACCATGGCTGATCCTGCAGGACGTGGTC CAGATTAAGTTTGCACGAGGACGAGGACAAGGAAAACGATGGAGA CT | 1512 |
| HPV16 | SD2-SA6 i=17 | 25 | 26 | TCTCAGAAACCATAATCTACCATGGCTGATCCTGCAGCAGCAACGA AGTATCCTCTCCTGAAATTATTAGGCAGCACTTGGCCAACCACCCC GCCGCGACCC | 1513 |
| HPV16 | SD2-SA9 i=18 | 27 | 28 | TCTCAGAAACCATAATCTACCATGGCTGATCCTGCAGATGTCTCTTT GGCTGCCTAGTGAGGCCACTGTCTACTTGCCTCCTGTCC | 1514 |
| HPV18 | SD3-SA4 i=1 | 29 | 30 | GAAGTGGAAGCAACACAGATTCAGGATAATAGATGGCCATATTTA GAAAGTAGAATAACAGTATTTGAATTTCCAAATGCATTTCCATTTG ATAAAAA | 1515 |
| HPV18 | SD3-SA5 i=2 | 31 | 32 | GAAGTGGAAGCAACACAGATTCAGGACATGGTCCAGATTAGATTTG CACGAGGAAGAGGAAG | 1516 |
| HPV18 | SD3-SA6 i=3 | 33 | 34 | GAAGTGGAAGCAACACAGATTCAGCTTGTTAAACAGCTACAGCAC ACCCCCTCACCGTATTCCAGCACCGTGTCCGTGGGCACCGCAA | 1517 |
| HPV18 | SD1-SA4 i=4 | 35 | 36 | GTATATTGCAAGACAGTATTGGAACTTACAGAGGATAATAGATGGC CATATTTAGAAAGTAGAATAACAGTATTTGAATTTCCAAATGCATT TCCATTTGATAAAAA | 1518 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV18 | SD1-SA5 i=5 | 37 | 38 | GTATATTGCAAGACAGTATTGGAACTTACAGAGGACATGGTCCAGA TTAGATTTGCACGAGGAAGAGGAAG | 1519 |
| HPV18 | SD1-SA6 i=6 | 39 | 40 | GTATATTGCAAGACAGTATTGGAACTTACAGAGCTTGTTAAACAGC TACAGCACACCCCCTCACCGTATTCCAGCACCGTGTCCGTGGGCAC CGCAA | 1520 |
| HPV18 | SD1-SA1 i=7 | 41 | 42 | GTATATTGCAAGACAGTATTGGAACTTACAGAGGTGCCTGCGGTGC CAGAAACCGTTGAATCCAGCAGAAAAACTTAGACACCTTAATGAA AAACG | 1521 |
| HPV18 | SD1-SA3 i=9 | 43 | 44 | GTATATTGCAAGACAGTATTGGAACTTACAGAGTGTGAAGCCAGAA TTGAGCTAGTAGTAGAAAGCTCAGCAGACGACCTTCG | 1522 |
| HPV18 | SD4-SA6 i=10 | 45 | 46 | TGCGAGGAACTATGGAATACAGAACCTACTCACTGCTTTAAAAAAG CTTGTTAAACAGCTACAGCACACCCCCTCACCGTATTCCAGCACCG TGTCCGTGGGCACCGCAA | 1523 |
| HPV18 | SD5-SA9 i=11 | 47 | 48 | CAAAAGACGGAAACTCTGTAGTGGTAACACTACGCCTATAATACAT TTAAAAGATGGCTTTGTGGCGGCCTAGTGACAATACCGTATATCTT CCACC | 1524 |
| HPV18 | SD5-SA10 i=12 | 49 | 50 | CAAAAGACGGAAACTCTGTAGTGGTAACACTACGCCTATAATACAT TTAAAAGGTGGTGGCAATAAGCAGGATATTCCTAAGGTTTCTGCAT ACCAAT | 1525 |
| HPV18 | SD6-SA9 i=13 | 51 | 52 | ATATCATCCACCTGGCATTGGACAGATGGCTTTGTGGCGGCCTAGT GACAATACCGTATATCTTCCACC | 1526 |
| HPV18 | SD6-SA10 i=14 | 53 | 54 | ATATCATCCACCTGGCATTGGACAGGTGGTGGCAATAAGCAGGATA TTCCTAAGGTTTCTGCATACCAAT | 1527 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV18 | SD2-SA4 i=15 | 55 | 56 | CAATGGCTGATCCAGAAGGATAATAGATGGCCATATTTAGAAAGTA GAATAACAGTATTTGAATTTCCAAATGCATTTCCATTTGATAAAAA | 1528 |
| HPV18 | SD2-SA5 i=16 | 57 | 58 | CAATGGCTGATCCAGAAGGACATGGTCCAGATTAGATTTGCACGAG GAAGAGGAAG | 1529 |
| HPV18 | SD2-SA8 i=25 | 59 | 60 | CAATGGCTGATCCAGAAGCTTGTTAAACAGCTACAGCACACCCCCT CACCGTATTCCAGCACCGTGTCCGTGGGCACCGCAA | 1530 |
| HPV18 | SD2-SA9 i=18 | 61 | 62 | CAATGGCTGATCCAGAAGATGGCTTTGTGGCGGCCTAGTGACAATA CCGTATATCTTCCACC | 1531 |
| HPV18 | SD2-SA10 i=19 | 63 | 64 | CAATGGCTGATCCAGAAGGTGGTGGCAATAAGCAGGATATTCCTAA GGTTTCTGCATACCAAT | 1532 |
| HPV31 | SD3-SA4 i=1 | 65 | 66 | GGAAACGCAGCAGATGGTACAGGATGACAGATGGCCATACC | 1533 |
| HPV31 | SD3-SA5 i=2 | 67 | 68 | GGAAACGCAGCAGATGGTACAGGACGTGGTGCAGATTAAATTTGC ACGAGGAAGAGGACAAAG | 1534 |
| HPV31 | SD3-SA6 i=3 | 69 | 70 | GGAAACGCAGCAGATGGTACAGCAGTGACGAAATATCCTTTGCTGG GATTGTTACAAAGCTACCAACAGC | 1535 |
| HPV31 | SD1-SA4 i=4 | 71 | 72 | GAACTAAGATTGAATTGTGTCTACTGCAAAGGTCAGTTAACAGAAA CAGAGGATGACAGATGGCCATACC | 1536 |
| HPV31 | SD1-SA5 i=5 | 73 | 74 | GAACTAAGATTGAATTGTGTCTACTGCAAAGGTCAGTTAACAGAAA CAGAGGACGTGGTGCAGATTAAATTTGCACGAGGAAGAGGACAAA G | 1537 |

EP 3 715 477 A1

55

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV31 | SD1-SA6 i=6 | 75 | 76 | GAACTAAGATTGAATTGTGTCTACTGCAAAGGTCAGTTAACAGAAACAGAGCAGTGACGAAATATCCTTTGCTGGGATTGTTACAAAGCTACCAACAGC | 1538 |
| HPV31 | SD1-SA1 i=7 | 77 | 78 | GAACTAAGATTGAATTGTGTCTACTGCAAAGGTCAGTTAACAGAAACAGAGGTGTATAACGTGTCA | 1539 |
| HPV31 | SD1-SA2 i=8 | 79 | 80 | GAACTAAGATTGAATTGTGTCTACTGCAAAGGTCAGTTAACAGAAACAGAGAAGACCTCGTACTGAAACCCAAGTGTAAACATGCGTGGAG | 1540 |
| HPV31 | SD1-SA3 i=9 | 81 | 82 | GAACTAAGATTGAATTGTGTCTACTGCAAAGGTCAGTTAACAGAAACAGAGTGTAAGTCTACACTTCGTTTGTGTGTACAGAGCACACAAGTAGA | 1541 |
| HPV31 | SD5-SA9 i=11 | 83 | 84 | AACAAGGGCTGTCAGTTGTCCTGCAACTACACCTATAATACACTTAAAAGATGTCTCTGTGGCGGCCTAGCGAGGCTACTGTCTACT | 1542 |
| HPV31 | SD2-SA4 i=15 | 85 | 86 | TCTACTAGACTGTAACTACAATGGCTGATCCAGCAGGATGACAGATGGCCATACC | 1543 |
| HPV31 | SD2-SA5 i=16 | 87 | 88 | TCTACTAGACTGTAACTACAATGGCTGATCCAGCAGGACGTGGTGCAGATTAAATTTGCACGAGGAAGAGGACAAAG | 1544 |
| HPV31 | SD2-SA6 i=17 | 89 | 90 | TCTACTAGACTGTAACTACAATGGCTGATCCAGCAGCAGTGACGAAATATCCTTTGCTGGGATTGTTACAAAGCTACCAACAGC | 1545 |
| HPV31 | SD2-SA9 i=18 | 91 | 92 | TCTACTAGACTGTAACTACAATGGCTGATCCAGCAGATGTCTCTGTGGCGGCCTAGCGAGGCTACTGTCTACT | 1546 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV33 | SD3-SA4 i=1 | 93 | 94 | GCAATACTGAAGTGGAAACTCAGCAGATGGTACAACAGACTCTAGATGGCCATATTTACATAGTAGATTAACAGTATTTGAATTTAAAAATCCATTCCCA | 1547 |
| HPV33 | SD3-SA5 i=2 | 95 | 96 | GCAATACTGAAGTGGAAACTCAGCAGATGGTACAACAGGACGTGGTGCAAATTAGATTTAATAGAGGAAGAG | 1548 |
| HPV33 | SD3-SA6 i=3 | 97 | 98 | GCAATACTGAAGTGGAAACTCAGCAGATGGTACAACAGCAACCAAATATCCACTACTGAAACTGCTGACATACAGACAG | 1549 |
| HPV33 | SD1-SA4 i=4 | 99 | 100 | GAACTACAGTGCGTGGAATGCAAAAAACCTTTGCAACGATCTGAGACTCTAGATGGCCATATTTACATAGTAGATTAACAGTATTTGAATTTAAAAATCCATTCCCA | 1550 |
| HPV33 | SD1-SA5 i=5 | 101 | 102 | GAACTACAGTGCGTGGAATGCAAAAAACCTTTGCAACGATCTGAGGACGTGGTGCAAATTAGATTTAATAGAGGAAGAG | 1551 |
| HPV33 | SD1-SA6 i=6 | 103 | 104 | GAACTACAGTGCGTGGAATGCAAAAAACCTTTGCAACGATCTGAGCAACCAAATATCCACTACTGAAACTGCTGACATACAGACAG | 1552 |
| HPV33 | SD1-SA1 i=7 | 105 | 106 | GAACTACAGTGCGTGGAATGCAAAAAACCTTTGCAACGATCTGAGGTGTATTATATGTCAAAGACCTTTGTGTCCTCAAGAAAA | 1553 |
| HPV33 | SD1-SA2 i=8 | 107 | 108 | GAACTACAGTGCGTGGAATGCAAAAAACCTTTGCAACGATCTGAGGTCCCGACGTAGAGAAACTGCACTGTGACGTGTAAAAACGCCATGAGAGG | 1554 |
| HPV33 | SD5-SA9 i=11 | 109 | 110 | ACAAGCAGCGGACTGTGTGTAGTTCTAACGTTGCACCTATAGTGCATTTAAAAGATGTCCGTGTGGCGGCCTAGTGAGGCCACAGTGTACCTGCCTCCTGTA | 1555 |

EP 3 715 477 A1

57

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV33 | SD2-SA4 i=15 | 111 | 112 | CAATAAACATCATCTACAATGGCCGATCCTGAAGACTCTAGATGGCCATATTTACATAGTAGATTAACAGTATTTGAATTTAAAAATCCATTCCCA | 1556 |
| HPV33 | SD2-SA5 i=16 | 113 | 114 | CAATAAACATCATCTACAATGGCCGATCCTGAAGGACGTGGTGCAAATTAGATTTAATAGAGGAAGAG | 1557 |
| HPV33 | SD2-SA6 i=17 | 115 | 116 | CAATAAACATCATCTACAATGGCCGATCCTGAAGCAACCAAATATCCACTACTGAAACTGCTGACATACAGACAG | 1558 |
| HPV33 | SD2-SA9 i=18 | 117 | 118 | CAATAAACATCATCTACAATGGCCGATCCTGAAGATGTCCGTGTGGCGGCCTAGTGAGGCCACAGTGTACCTGCCTCCTGTA | 1559 |
| HPV35 | SD3-SA4 i=1 | 119 | 120 | ATGGCAATTCTGAAGTGGAAATACAGCAGATACAACAGATGACAGGTGGCCATACTTACATAGCA | 1560 |
| HPV35 | SD3-SA5 i=2 | 121 | 122 | ATGGCAATTCTGAAGTGGAAATACAGCAGATACAACAGGACGTGGTGCAGATTAAATTTGCACGAGGAAGAGGA | 1561 |
| HPV35 | SD3-SA6 i=3 | 123 | 124 | ATGGCAATTCTGAAGTGGAAATACAGCAGATACAACAGCAGCACAGAACTATCCACTGCTGAAATTGCTACACAGCTACACGCCTACAACACC | 1562 |
| HPV35 | SD1-SA4 i=4 | 125 | 126 | TTGTTTGAATTGTGTATACTGCAAACAAGAATTACAGCGGAGTGAGATGACAGGTGGCCATACTTACATAGCA | 1563 |
| HPV35 | SD1-SA5 i=5 | 127 | 128 | TTGTTTGAATTGTGTATACTGCAAACAAGAATTACAGCGGAGTGAGGACGTGGTGCAGATTAAATTTGCACGAGGAAGAGGA | 1564 |
| HPV35 | SD1-SA6 i=6 | 129 | 130 | TTGTTTGAATTGTGTATACTGCAAACAAGAATTACAGCGGAGTGAGCAGCACAGAACTATCCACTGCTGAAATTGCTACACAGCTACACGCCTACAACACC | 1565 |

EP 3 715 477 A1

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV35 | SD1-SA1 i=7 | 131 | 132 | TTGTTTGAATTGTGTATACTGCAAACAAGAATTACAGCGGAGTGAG GTGTATTACATGTCAAAAACCGCTGTGTCCAGTTGAAAAGCAAAGA CATTTAGAAGAAAA | 1566 |
| HPV35 | SD5-SA9 i=11 | 133 | 134 | CCGGTGTGGTAGTTGTAGTACAACTACACCTATAGTACATTTAAAA GATGTCTCTGTGGCGGTCTAACGAAGCCACTGTCTACCTGCCTCCA GTGTC | 1567 |
| HPV35 | SD5-SA10 i=12 | 135 | 136 | CCGGTGTGGTAGTTGTAGTACAACTACACCTATAGTACATTTAAAA GATTCTAATAAAATAGCAGTACC | 1568 |
| HPV35 | SD2-SA4 i=15 | 137 | 138 | CTACAATGGCTGATCCTGCAGATGACAGGTGGCCATACTTACATAG CA | 1569 |
| HPV35 | SD2-SA5 i=16 | 139 | 140 | CTACAATGGCTGATCCTGCAGGACGTGGTGCAGATTAAATTTGCAC GAGGAAGAGGA | 1570 |
| HPV35 | SD2-SA6 i=17 | 141 | 142 | CTACAATGGCTGATCCTGCAGCAGCACAGAACTATCCACTGCTGAA ATTGCTACACAGCTACACGCCTACAACACC | 1571 |
| HPV35 | SD2-SA9 i=18 | 143 | 144 | CTACAATGGCTGATCCTGCAGATGTCTCTGTGGCGGTCTAACGAAG CCACTGTCTACCTGCCTCCAGTGTC | 1572 |
| HPV35 | SD2-SA10 i=19 | 145 | 146 | CTACAATGGCTGATCCTGCAGATTCTAATAAAATAGCAGTACC | 1573 |
| HPV39 | SD3-SA4 i=1 | 147 | 148 | GCGGATATGGCAATATGGAAGTGGAAACAGCTGAAGTGGAGGAGA CGATAGGTGGCCATATTTACGTAGTAGGCTAACAGTGTTTAAATTT C | 1574 |

EP 3 715 477 A1

(continued)

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV39 | SD3-SA7 i=22 | 149 | 150 | GCGGATATGGCAATATGGAAGTGGAAACAGCTGAAGTGGAGGAGTGACGGATCGGTACCCACTACTGAACTTACTACCGAA | 1575 |
| HPV39 | SD1-SA4 i=4 | 151 | 152 | GCCTGTGTCTATTGCAGACGACCACTACAGCAAACCGAGACGATAGGTGGCCATATTTACGTAGTAGGCTAACAGTGTTTAAATTTC | 1576 |
| HPV39 | SD1-SA7 i=20 | 153 | 154 | GCCTGTGTCTATTGCAGACGACCACTACAGCAAACCGAGTGACGGATCGGTACCCACTACTGAACTTACTACCGAA | 1577 |
| HPV39 | SD1-SA1 i=7 | 155 | 156 | GCCTGTGTCTATTGCAGACGACCACTACAGCAAACCGAGGTGCATGTGTTGTCTGAAACCGCTGTGTCCAGCAGAAAAATTAAGACACCTAAATAGCAAACGAAGATTTCAT | 1578 |
| HPV39 | SD1-SA3 i=9 | 157 | 158 | GCCTGTGTCTATTGCAGACGACCACTACAGCAAACCGAGTGTAACAACACACTGCAGCTGGTAGTAGAAGCCTCACGG | 1579 |
| HPV39 | SD5-SA9 i=11 | 159 | 160 | ACACAAGACGGTACCTCAGTTGTGGTAACACTACGCCTATAATACATTTAAAAGATGGCTATGTGGCGGTCTAGTGACAGCATGGTGTATTTGCCTCCACCTTC | 1580 |
| HPV39 | SD2-SA4 i=15 | 161 | 162 | ACCAGTAACCTGCTATGGCCAATCGTGAAGACGATAGGTGGCCATATTTACGTAGTAGGCTAACAGTGTTTAAATTTC | 1581 |
| HPV39 | SD2-SA7 i=21 | 163 | 164 | ACCAGTAACCTGCTATGGCCAATCGTGAAGTGACGGATCGGTACCCACTACTGAACTTACTACCGAA | 1582 |
| HPV39 | SD2-SA9 i=18 | 165 | 166 | ACCAGTAACCTGCTATGGCCAATCGTGAAGATGGCTATGTGGCGGTCTAGTGACAGCATGGTGTATTTGCCTCCACCTTC | 1583 |
| HPV45 | SD3-SA4 i=1 | 167 | 168 | GAAGTGGAAGCTGCAGAGACTCAGATAATAAATGGCCATATTTAGAAAGTAGGGTG | 1584 |

EP 3 715 477 A1

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV45 | SD3-SA5 i=2 | 169 | 170 | GAAGTGGAAGCTGCAGAGACTCAGGACATGGTCCAGATTAGATTTG CACGAGGACGATGAAG | 1585 |
| HPV45 | SD3-SA8 i=23 | 171 | 172 | GAAGTGGAAGCTGCAGAGACTCAGATTGTTAGACAGCTACAACAC GCCTCCACGTCGACCCC | 1586 |
| HPV45 | SD1-SA4 i=4 | 173 | 174 | ATATTGCAAAGCAACATTGGAACGCACAGAGATAATAAATGGCCA TATTTAGAAAGTAGGGTG | 1587 |
| HPV45 | SD1-SA5 i=5 | 175 | 176 | ATATTGCAAAGCAACATTGGAACGCACAGAGGACATGGTCCAGATT AGATTTGCACGAGGACGATGAAG | 1588 |
| HPV45 | SD1-SA8 i=24 | 177 | 178 | ATATTGCAAAGCAACATTGGAACGCACAGAGATTGTTAGACAGCTA CAACACGCCTCCACGTCGACCCC | 1589 |
| HPV45 | SD1-SA1 i=7 | 179 | 180 | ATATTGCAAAGCAACATTGGAACGCACAGAGGTGCCTGCGGTGCCA GAAACCATTGAACCCAGCAGA | 1590 |
| HPV45 | SD1-SA3 i=9 | 181 | 182 | ATATTGCAAAGCAACATTGGAACGCACAGAGTGTGACGGCAGAAT TGAGCTTACAGTAGAGAGCTCGGCAGAGGACCTT | 1591 |
| HPV45 | SD5-SA9 i=11 | 183 | 184 | AAGAAGGAAAGTGTGTAGTGGTAACACTACGCCTATAATACACTTA AAAGATGGCTTTGTGGCGGCCTAGTGACAGTACGGTATATCTTCCA CCACCTTC | 1592 |
| HPV45 | SD6-SA9 i=13 | 185 | 186 | ATATCCTCCACCTGGCATTGGACAGATGGCTTTGTGGCGGCCTAGT GACAGTACGGTATATCTTCCACCACCTTC | 1593 |
| HPV45 | SD2-SA4 i=15 | 187 | 188 | CCGTGGTGTGCAACTAACCAATAATCTACAATGGCGGATCCAGAAG ATAATAAATGGCCATATTTAGAAAGTAGGGTG | 1594 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV45 | SD2-SA5 i=16 | 189 | 190 | CCGTGGTGTGCAACTAACCAATAATCTACAATGGCGGATCCAGAAG GACATGGTCCAGATTAGATTTGCACGAGGACGATGAAG | 1595 |
| HPV45 | SD2-SA8 i=25 | 191 | 192 | CCGTGGTGTGCAACTAACCAATAATCTACAATGGCGGATCCAGAAG ATTGTTAGACAGCTACAACACGCCTCCACGTCGACCCC | 1596 |
| HPV45 | SD2-SA9 i=18 | 193 | 194 | CCGTGGTGTGCAACTAACCAATAATCTACAATGGCGGATCCAGAAG ATGGCTTTGTGGCGGCCTAGTGACAGTACGGTATATCTTCCACCAC CTTC | 1597 |
| HPV51 | SD3-SA4 i=1 | 195 | 196 | CACAAGTGGAAACTGTGGAAGCAACGTTGCAGGATGCAAACCTAA TGTATTTACATACAAGGGTAACAGTATTAAAGTTTTTAAATACATTT CCATTTGATAACA | 1598 |
| HPV51 | SD3-SA6 i=3 | 197 | 198 | CACAAGTGGAAACTGTGGAAGCAACGTTGCAGTACCTGCAGCGAC GCGTTATCCACTACTACAACTGTTGAACAACTATCAAACACCCCAA CGACCAATC | 1599 |
| HPV51 | SD1-SA4 i=4 | 199 | 200 | GTTTCTATGCACAATATACAGGTAGTGTGTGTGTATTGTAAAAAGG AATTATGTAGAGCAGGATGCAAACCTAATGTATTTACATACAAGGG TAACAGTATTAAAGTTTTTAAATACATTTCCATTTGATAACA | 1600 |
| HPV51 | SD1-SA6 i=6 | 201 | 202 | GTTTCTATGCACAATATACAGGTAGTGTGTGTGTATTGTAAAAAGG AATTATGTAGAGCAGTACCTGCAGCGACGCGTTATCCACTACTACA ACTGTTGAACAACTATCAAACACCCCAACGACCAATC | 1601 |
| HPV51 | SD1-SA1 i=7 | 203 | 204 | GTTTCTATGCACAATATACAGGTAGTGTGTGTGTATTGTAAAAAGG AATTATGTAGAGCAGGTGTCATAGATGTCAAAGACCACTTGGGCCT GAAGAAAAGCAAAAATTGGTGGACGAAAAA | 1602 |

EP 3 715 477 A1

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV51 | SD1-SA3 i=9 | 205 | 206 | GTTTCTATGCACAATATACAGGTAGTGTGTGTGTATTGTAAAAAGG AATTATGTAGAGCAGGTGTTCAAGTGTAGTACAACTGGCAGTGGAA AGCAGTGGAGACACC | 1603 |
| HPV51 | SD5-SA9 i=11 | 207 | 208 | GTGCAACTCAGACTGCGTTTATAGTGCATTTAAAAGATGGCATTGT GGCGCACTAATGACAGCAAGGTGTATTTGCCAC | 1604 |
| HPV51 | SD2-SA4 i=15 | 209 | 210 | GCCCGTGTTGTGCGAACAACTAGCAACGGCGATGGACTGTGAAGG ATGCAAACCTAATGTATTTACATACAAGGGTAACAGTATTAAAGTT TTTAAATACATTTCCATTTGATAACA | 1605 |
| HPV51 | SD2-SA6 i=17 | 211 | 212 | GCCCGTGTTGTGCGAACAACTAGCAACGGCGATGGACTGTGAAGTA CCTGCAGCGACGCGTTATCCACTACTACAACTGTTGAACAACTATC AAACACCCCAACGACCAATC | 1606 |
| HPV51 | SD2-SA9 i=18 | 213 | 214 | GCCCGTGTTGTGCGAACAACTAGCAACGGCGATGGACTGTGAAGAT GGCATTGTGGCGCACTAATGACAGCAAGGTGTATTTGCCAC | 1607 |
| HPV52 | SD3-SA4 i=1 | 215 | 216 | CGGCTATGGCAATAGTGAAGTGGAAGCGCAGCAGATGGCAGACCA GATCCTAGGTGGCCATATTTACATAGTA | 1608 |
| HPV52 | SD3-SA5 i=2 | 217 | 218 | CGGCTATGGCAATAGTGAAGTGGAAGCGCAGCAGATGGCAGACCA GGACGTGGTGCAAATTAGATTTAATACAGGAAGAGGACAA | 1609 |
| HPV52 | SD3-SA6 i=3 | 219 | 220 | CGGCTATGGCAATAGTGAAGTGGAAGCGCAGCAGATGGCAGACCA GTAACGAAGTATCCACTACTGAAACTGCTGTCCACCTATGCACCG | 1610 |
| HPV52 | SD1-SA4 i=4 | 221 | 222 | GCAGTGTGTGCAGTGCAAAAAAGAGCTACAACGAAGAGAGATCCT AGGTGGCCATATTTACATAGTA | 1611 |
| HPV52 | SD1-SA5 i=5 | 223 | 224 | GCAGTGTGTGCAGTGCAAAAAAGAGCTACAACGAAGAGAGGACGT GGTGCAAATTAGATTTAATACAGGAAGAGGACAA | 1612 |

EP 3 715 477 A1

63

(continued)

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV52 | SD1-SA6 i=6 | 225 | 226 | GCAGTGTGTGCAGTGCAAAAAAGAGCTACAACGAAGAGAGTAACGAAGTATCCACTACTGAAACTGCTGTCCACCTATGCACCG | 1613 |
| HPV52 | SD1-SA1 i=7 | 227 | 228 | GCAGTGTGTGCAGTGCAAAAAAGAGCTACAACGAAGAGAGATGTATAATTTGTCAAACGCCATTATGTCCTGAAGAAAA | 1614 |
| HPV52 | SD1-SA2 i=8 | 229 | 230 | GCAGTGTGTGCAGTGCAAAAAAGAGCTACAACGAAGAGAGACCCCGACCTGTGACCCAAGTGTAACGTCATGCGTGGAGACAAAGCAACTATAAAAGATTATA | 1615 |
| HPV52 | SD1-SA3 i=9 | 231 | 232 | GCAGTGTGTGCAGTGCAAAAAAGAGCTACAACGAAGAGAGTTGTGATAGCACACTACGGCTATGCATTCATAGCACTGCGACGG | 1616 |
| HPV52 | SD5-SA9 i=11 | 233 | 234 | ACAAAGGACGGGGTTGCACATACAACTTGTACTGCACCTATAATACACCTAAAAGATGTCCGTGGGCGCCTAGTGAGGCCACTGTGTACCTGCCTCC | 1617 |
| HPV52 | SD5-SA10 i=12 | 235 | 236 | ACAAAGGACGGGGTTGCACATACAACTTGTACTGCACCTATAATACACCTAAAAGTAGTGGTAATGGTAAAAAAGTTTTAGTTCCCAAGG | 1618 |
| HPV52 | SD2-SA4 i=15 | 237 | 238 | GTGTGCCCCGGCTGTGCACGGCTATAAACAACCCTGCAATGGAGGACCCTGAAGATCCTAGGTGGCCATATTTACATAGTA | 1619 |
| HPV52 | SD2-SA5 i=16 | 239 | 240 | GTGTGCCCCGGCTGTGCACGGCTATAAACAACCCTGCAATGGAGGACCCTGAAGGACGTGGTGCAAATTAGATTTAATACAGGAAGAGGACAA | 1620 |
| HPV52 | SD2-SA6 i=17 | 241 | 242 | GTGTGCCCCGGCTGTGCACGGCTATAAACAACCCTGCAATGGAGGACCCTGAAGTAACGAAGTATCCACTACTGAAACTGCTGTCCACCTATGCACCG | 1621 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV52 | SD2-SA9 i=18 | 243 | 244 | GTGTGCCCCGGCTGTGCACGGCTATAAACAACCCTGCAATGGAGGA CCCTGAAGATGTCCGTGTGGCGGCCTAGTGAGGCCACTGTGTACCT GCCTCC | 1622 |
| HPV52 | SD2-SA10 i=19 | 245 | 246 | GTGTGCCCCGGCTGTGCACGGCTATAAACAACCCTGCAATGGAGGA CCCTGAAGTAGTGGTAATGGTAAAAAGTTTTAGTTCCCAAGG | 1623 |
| HPV56 | SD3-SA4 i=1 | 247 | 248 | CATTGGAAACTCTGGAAACACCAGAACAGATGCTAAATTACGATAT TT | 1624 |
| HPV56 | SD3-SA5 i=2 | 249 | 250 | CATTGGAAACTCTGGAAACACCAGAACAGGACGTGGTCCAGATTA AAT | 1625 |
| HPV56 | SD3-SA6 i=3 | 251 | 252 | CATTGGAAACTCTGGAAACACCAGAACAGTACCTGTAGATACAACG TATCCCCTGTTGAAACTGTTAACGAATACAACACCCAC | 1626 |
| HPV56 | SD1-SA4 i=4 | 253 | 254 | ATACCTTTAATTGATCTTAGATTATCATGTGTATATTGCAAAAAAGA ACTAACACGTGCTGAGATGCTAAATTACGATATTT | 1627 |
| HPV56 | SD1-SA5 i=5 | 255 | 256 | ATACCTTTAATTGATCTTAGATTATCATGTGTATATTGCAAAAAAGA ACTAACACGTGCTGAGGACGTGGTCCAGATTAAAT | 1628 |
| HPV56 | SD1-SA6 i=6 | 257 | 258 | ATACCTTTAATTGATCTTAGATTATCATGTGTATATTGCAAAAAAGA ACTAACACGTGCTGAGTACCTGTAGATACAACGTATCCCCTGTTGA AACTGTTAACGAATACAACACCCAC | 1629 |
| HPV56 | SD1-SA1 i=7 | 259 | 260 | ATACCTTTAATTGATCTTAGATTATCATGTGTATATTGCAAAAAAGA ACTAACACGTGCTGAGGTGCTACAGATGTCAAAGTCCG | 1630 |
| HPV56 | SD1-SA2 i=8 | 261 | 262 | ATACCTTTAATTGATCTTAGATTATCATGTGTATATTGCAAAAAAGA ACTAACACGTGCTGAGACAAACATCTAGAGAACCTAGAGAATCTAC AGTATAATCATGCATGGTAAAG | 1631 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV56 | SD1-SA3 i=9 | 263 | 264 | ATACCTTTAATTGATCTTAGATTATCATGTGTATATTGCAAAAAAGAACTAACACGTGCTGAGTGTAAGTTTGTGGTGCAGTTGGACATTCAGAGTACCAAA | 1632 |
| HPV56 | SD5-SA9 i=11 | 265 | 266 | ACTACGCCTGTAGTACATTTAAAAGATGGCGACGTGGCGGCCTAGTGAAAATAAGGTGTATCTACC | 1633 |
| HPV56 | SD5-SA10 i=12 | 267 | 268 | ACTACGCCTGTAGTACATTTAAAAGGACAATACCAAAACAAACATTCCCAAAGTTAGTGCATA | 1634 |
| HPV56 | SD2-SA4 i=15 | 269 | 270 | GCGCATCAAGTAACTAACTGCAATGGCGTCACCTGAAGATGCTAAATTACGATATTT | 1635 |
| HPV56 | SD2-SA5 i=16 | 271 | 272 | GCGCATCAAGTAACTAACTGCAATGGCGTCACCTGAAGGACGTGGTCCAGATTAAAT | 1636 |
| HPV56 | SD2-SA6 i=17 | 273 | 274 | GCGCATCAAGTAACTAACTGCAATGGCGTCACCTGAAGTACCTGTAGATACAACGTATCCCCTGTTGAAACTGTTAACGAATACAACACCCAC | 1637 |
| HPV56 | SD2-SA9 i=18 | 275 | 276 | GCGCATCAAGTAACTAACTGCAATGGCGTCACCTGAAGATGGCGACGTGGCGGCCTAGTGAAAATAAGGTGTATCTACC | 1638 |
| HPV56 | SD2-SA10 i=19 | 277 | 278 | GCGCATCAAGTAACTAACTGCAATGGCGTCACCTGAAGGACAATACCAAAACAAACATTCCCAAAGTTAGTGCATA | 1639 |
| HPV58 | SD3-SA4 i=1 | 279 | 280 | ATGGCAATACTGAAGTGGAAACTGAGCAGATGGCACACCAGATTCACGATGGCCATATTTGCACAGTAGACTAACAGTATT | 1640 |
| HPV58 | SD3-SA5 i=2 | 281 | 282 | ATGGCAATACTGAAGTGGAAACTGAGCAGATGGCACACCAGGACGTGGTGCAAATTAGGCTTAATAGAGGAAGAG | 1641 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV58 | SD3-SA6 i=3 | 283 | 284 | ATGGCAATACTGAAGTGGAAACTGAGCAGATGGCACACCAGTGAT CAAATATCCACTACTGAAACTGCTGACCCAAAGACCACCGAGGCC | 1642 |
| HPV58 | SD1-SA4 i=4 | 285 | 286 | GTGCATGAAATCGAATTGAAATGCGTTGAATGCAAAAAGACTTTGC AGCGATCTGAGATTCACGATGGCCATATTTGCACAGTAGACTAACA GTATT | 1643 |
| HPV58 | SD1-SA5 i=5 | 287 | 288 | GTGCATGAAATCGAATTGAAATGCGTTGAATGCAAAAAGACTTTGC AGCGATCTGAGGACGTGGTGCAAATTAGGCTTAATAGAGGAAGAG | 1644 |
| HPV58 | SD1-SA6 i=6 | 289 | 290 | GTGCATGAAATCGAATTGAAATGCGTTGAATGCAAAAAGACTTTGC AGCGATCTGAGTGATCAAATATCCACTACTGAAACTGCTGACCCAA AGACCACCGAGGCC | 1645 |
| HPV58 | SD1-SA1 i=7 | 291 | 292 | GTGCATGAAATCGAATTGAAATGCGTTGAATGCAAAAAGACTTTGC AGCGATCTGAGATGTATTATTTGTCAAAGACCATTGTGTCCACAAG AAAAAAAAAGGCATGTGGATTTAA | 1646 |
| HPV58 | SD1-SA2 i=8 | 293 | 294 | GTGCATGAAATCGAATTGAAATGCGTTGAATGCAAAAAGACTTTGC AGCGATCTGAGACCCCGACGTAGACAAACACAAGTGTAACCTGTA ACAACGCCA | 1647 |
| HPV58 | SD5-SA9 i=11 | 295 | 296 | GTACATACAAAGGGCGGAACGTGTGTAGTTCTAAAGTTTCACCTAT CGTGCATTTAAAAGATGTCCGTGTGGCGGCCTAGTGAGGCCACTGT GTACCTGCCTCCTG | 1648 |
| HPV58 | SD2-SA4 i=15 | 297 | 298 | GTGTGCCCTAGCTGTGCACAGCAATAAACACCATCTGCAATGGATG ACCCTGAAGATTCACGATGGCCATATTTGCACAGTAGACTAACAGT ATT | 1649 |
| HPV58 | SD2-SA5 i=16 | 299 | 300 | GTGTGCCCTAGCTGTGCACAGCAATAAACACCATCTGCAATGGATG ACCCTGAAGGACGTGGTGCAAATTAGGCTTAATAGAGGAAGAG | 1650 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV58 | SD2-SA6 i=17 | 301 | 302 | GTGTGCCCTAGCTGTGCACAGCAATAAACACCATCTGCAATGGATG ACCCTGAAGTGATCAAATATCCACTACTGAAACTGCTGACCCAAAG ACCACCGAGGCC | 1651 |
| HPV58 | SD2-SA9 i=18 | 303 | 304 | GTGTGCCCTAGCTGTGCACAGCAATAAACACCATCTGCAATGGATG ACCCTGAAGATGTCCGTGTGGCGGCCTAGTGAGGCCACTGTGTACC TGCCTCCTG | 1652 |
| HPV59 | SD3-SA4 i=1 | 305 | 306 | GCGGCTATGGCTATTCTGAAGTGGAAATGCTCGAGACTCAGATAAC AGGTGGCCATATTTAAATAGCAGATTAATGGTATTTAAATT | 1653 |
| HPV59 | SD3-SA5 i=2 | 307 | 308 | GCGGCTATGGCTATTCTGAAGTGGAAATGCTCGAGACTCAGGACGT GGTGCAGATTAGATTTGAACGAGGAAGAGGAAG | 1654 |
| HPV59 | SD3-SA7 i=22 | 309 | 310 | GCGGCTATGGCTATTCTGAAGTGGAAATGCTCGAGACTCAGTGACG AGCAAGTATCCACTGCTGGATCTTCTGAGCAACTATCATACCCCTCC GCAACGCCCC | 1655 |
| HPV59 | SD1-SA4 i=4 | 311 | 312 | GGAACTGCAAGAAAGAGAGATAACAGGTGGCCATATTTAAATAGC AGATTAATGGTATTTAAATT | 1656 |
| HPV59 | SD1-SA5 i=5 | 313 | 314 | GGAACTGCAAGAAAGAGAGGACGTGGTGCAGATTAGATTTGAACG AGGAAGAGGAAG | 1657 |
| HPV59 | SD1-SA7 i=20 | 315 | 316 | GGAACTGCAAGAAAGAGAGTGACGAGCAAGTATCCACTGCTGGAT CTTCTGAGCAACTATCATACCCCTCCGCAACGCCCC | 1658 |
| HPV59 | SD1-SA3 i=9 | 317 | 318 | GGAACTGCAAGAAAGAGAGTGTAATAATCAACTTCAGCTAGTAGT AGAAACCTCGCAAG | 1659 |
| HPV59 | SD5-SA9 i=11 | 319 | 320 | CAACCCGCGACGGCACATCCCTTGCAGTAACACTACGCCTATAATA CACTTAAAAGATGGCTCTATGGCGTTCTAGTGACAACAAGGTGTAT CTACCTCCACCTTCGGTAGCTAAG | 1660 |

(continued)

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV59 | SD5-SA10 i=12 | 321 | 322 | CAACCCGCGACGGCACATCCCTTGCAGTAACACTACGCCTATAATACACTTAAAAGGTGGTAATGGTAGACAGGATGTTCCTAAGGTGTCTGCATATCAATACAGAGTATTTAGGGTT | 1661 |
| HPV59 | SD2-SA4 i=15 | 323 | 324 | GCAGCAAACCAGTAACCTGCAATGGCCGATTCGGAAGATAACAGGTGGCCATATTTAAATAGCAGATTAATGGTATTTAAATT | 1662 |
| HPV59 | SD2-SA5 i=16 | 325 | 326 | GCAGCAAACCAGTAACCTGCAATGGCCGATTCGGAAGGACGTGGTGCAGATTAGATTTGAACGAGGAAGAGGAAG | 1663 |
| HPV59 | SD2-SA7 i=21 | 327 | 328 | GCAGCAAACCAGTAACCTGCAATGGCCGATTCGGAAGTGACGAGCAAGTATCCACTGCTGGATCTTCTGAGCAACTATCATACCCCTCCGCAACGCCCC | 1664 |
| HPV59 | SD2-SA9 i=18 | 329 | 330 | GCAGCAAACCAGTAACCTGCAATGGCCGATTCGGAAGATGGCTCTATGGCGTTCTAGTGACAACAAGGTGTATCTACCTCCACCTTCGGTAGCTAAG | 1665 |
| HPV59 | SD2-SA10 i=19 | 331 | 332 | GCAGCAAACCAGTAACCTGCAATGGCCGATTCGGAAGGTGGTAATGGTAGACAGGATGTTCCTAAGGTGTCTGCATATCAATACAGAGTATTTAGGGTT | 1666 |
| HPV66 | SD3-SA4 i=1 | 333 | 334 | CATTGGAAACATTGGAAACATCACAACAGATGCAAAATTAAGATATTTACACAGTAGAATTTCAGTGTTTAAGTTTGAAAATCCATTCCATTAGATAAC | 1667 |
| HPV66 | SD3-SA5 i=2 | 335 | 336 | CATTGGAAACATTGGAAACATCACAACAGGACATGGTCCAGATTAAAT | 1668 |
| HPV66 | SD3-SA6 i=3 | 337 | 338 | GCAAGTACAAACAGCACATGCAGATGCACAGACGTTGCAAAAACTAAAACGAAAGTATATAGGTAGTCCCTTAAGTGATATTAG | 1669 |

EP 3 715 477 A1

69

(continued)

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV66 | SD3-SA8 i=23 | 339 | 340 | CATTGGAAAACATTGGAAAACATCACAACAGACTGTTAACGAATACAACAAC | 1670 |
| HPV66 | SD1-SA4 i=4 | 341 | 342 | AATACCTTTACTTGATCTTAGAGATTATCATGTGTATACTGCAAAAAGGAACTTACAAGTTTAGAGAGATGCAAAATTAAGATATTTACACAGTAGAATTTCAGTGTGTTTAAGTTTGAAAATCCATTCCATTAGATAAC | 1671 |
| HPV66 | SD1-SA5 i=5 | 343 | 344 | AATACCTTTACTTGATCTTAGAGATTATCATGTGTATACTGCAAAAAGGAACTTACAAGTTTAGAGGACATGTCCAGATTAAAT | 1672 |
| HPV66 | SD1-SA8 i=24 | 345 | 346 | AATACCTTTACTTGATCTTAGAGATTATCATGTGTATACTGCAAAAAGGAACTTACAAGTTTAGAGACTGTTAACGAATACAACAAC | 1673 |
| HPV66 | SD1-SA1 i=7 | 347 | 348 | AATACCTTTACTTGATCTTAGAGATTATCATGTGTATACTGCAAAAAGGAACTTACAAGTTTAGAGGGTGCTACCGATGTCAATGTCCGTTAACACCGGAGGAAAAACAA | 1674 |
| HPV66 | SD1-SA2 i=8 | 349 | 350 | AATACCTTTACTTGATCTTAGAGATTATCATGTGTATACTGCAAAAAGGAACTTACAAGTTTAGAGACATACGAGTAGACAAGCTACAGAATCTACAGTATAACCATGCATGGTAA | 1675 |
| HPV66 | SD1-SA3 i=9 | 351 | 352 | AATACCTTTACTTGATCTTAGAGATTATCATGTGTATACTGCAAAAAGGAACTTACAAGTTTAGAGTGTGAGTTGGTGGGTGCAGTTGGGACATTCAGAGTACCAA | 1676 |
| HPV66 | SD5-SA9 i=11 | 353 | 354 | GGTGATAAAACTACGCCCTGTAATCCATTTAAAAGATGGCGATGTGGCGGCCTAGTGACAATAAGGTGTACCTACC | 1677 |
| HPV66 | SD2-SA4 i=15 | 355 | 356 | GCGCATCATCTAAATAAACTGCAATGGCATCACCTGAAGATGCAAAATTAAGATATTTACACAGTAGAATTTCAGTGTGTTTAAGTTTGAAAATCCATTTCCATTAGATAAC | 1678 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV66 | SD2-SA5 i=16 | 357 | 358 | GCGCATCATCTAAATAACTGCAATGGCATCACCTGAAGGACATGGTCCAGATTAAAT | 1679 |
| HPV66 | SD2-SA8 i=25 | 359 | 360 | GCGCATCATCTAAATAACTGCAATGGCATCACCTGAAGACTGTTAACGAATACAACAAC | 1680 |
| HPV66 | SD2-SA9 i=18 | 361 | 362 | GCGCATCATCTAAATAACTGCAATGGCATCACCTGAAGATGGCGATGTGGCGGCCTAGTGACAATAAGGTGTACCTACC | 1681 |
| HPV68 | SD3-SA4 i=1 | 363 | 364 | CCGGACAGCGGCTATGGCAATATGGAAGTGGAAACTAACTCGGAGACAATAGGTGGCCGTATTTACATAGTAGACTAACCGTGTTTAAATTTC | 1682 |
| HPV68 | SD3-SA6 i=3 | 365 | 366 | CCGGACAGCGGCTATGGCAATATGGAAGTGGAAACTAACTCGGAGTACCACTGACGGAAAAGTATCCACTACTGAATCTGTTGCCGAC | 1683 |
| HPV68 | SD1-SA4 i=4 | 367 | 368 | GTTACAATAGACTGTGTCTATTGCAGAAGGCAACTACAACGGACAGAGACAATAGGTGGCCGTATTTACATAGTAGACTAACCGTGTTTAAATTTC | 1684 |
| HPV68 | SD1-SA1 i=7 | 369 | 370 | GTTACAATAGACTGTGTCTATTGCAGAAGGCAACTACAACGGACAGAGGTGCATGAGTTGCCTGAAACCATTGTGTCCAGCAGAAAAA | 1685 |
| HPV68 | SD1-SA6 i=6 | 371 | 372 | GTTACAATAGACTGTGTCTATTGCAGAAGGCAACTACAACGGACAGAGTACCACTGACGGAAAAGTATCCACTACTGAATCTGTTGCCGAC | 1686 |
| HPV68 | SD1-SA3 i=9 | 373 | 374 | GTTACAATAGACTGTGTCTATTGCAGAAGGCAACTACAACGGACAGAGTGTAACAAGGCACTGCAACTAGTAGTAGAAGCGTCGCGGGAC | 1687 |
| HPV68 | SD5-SA9 i=11 | 375 | 376 | AAGACGGAGCCTTTGTTGTGGTGACACTACACCTATAGTGCATTTAAAAGATGGCATTGTGGCGAGCTAGCGACAACATGGTGTATTTGCCTCCCCCC | 1688 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV68 | SD2-SA4 i=15 | 377 | 378 | ACCCAGTAATCTGCAATGGCCAATTGTGAAGACAATAGGTGGCCGT ATTTACATAGTAGACTAACCGTGTTTAAATTTC | 1689 |
| HPV68 | SD2-SA6 i=17 | 379 | 380 | ACCCAGTAATCTGCAATGGCCAATTGTGAAGTACCACTGACGGAAA AGTATCCACTACTGAATCTGTTGCCGAC | 1690 |
| HPV68 | SD2-SA9 i=18 | 381 | 382 | ACCCAGTAATCTGCAATGGCCAATTGTGAAGATGGCATTGTGGCGA GCTAGCGACAACATGGTGTATTTGCCTCCCCCC | 1691 |
| HPV73 | SD3-SA4 i=1 | 383 | 384 | GGACAGTGGATATGGCAATACTGAAGTGGAAACTTACGAGACAGA GATGATACTTGGAAATATTTACATAGTAGAATTAAGGTGTTTACTTT TTTAAATCCATT | 1692 |
| HPV73 | SD3-SA6 i=3 | 385 | 386 | GGACAGTGGATATGGCAATACTGAAGTGGAAACTTACGAGACAGA GCGCCTGTGAAGTATCCATTCCTGAAATTGTTAACCCACTGCAC | 1693 |
| HPV73 | SD1-SA4 i=4 | 387 | 388 | ATACATGATATAAACCTGGACTGTGTGTTTTGCCAACGTGGACTGT ACAGATCTGAGATGATACTTGGAAATATTTACATAGTAGAATTAAG GTGTTTACTTTTTTAAATCCATT | 1694 |
| HPV73 | SD1-SA6 i=6 | 389 | 390 | ATACATGATATAAACCTGGACTGTGTGTTTTGCCAACGTGGACTGT ACAGATCTGAGCGCCTGTGAAGTATCCATTCCTGAAATTGTTAACC CACTGCAC | 1695 |
| HPV73 | SD1-SA1 i=7 | 391 | 392 | ATACATGATATAAACCTGGACTGTGTGTTTTGCCAACGTGGACTGT ACAGATCTGAGGTGCGGAAAATGCCAAAAACCATTATGTCCACTGG AAAAGCAAAAGCATGTAGATGAAAA | 1696 |
| HPV73 | SD1-SA2 i=8 | 393 | 394 | ATACATGATATAAACCTGGACTGTGTGTTTTGCCAACGTGGACTGT ACAGATCTGAGACCATCTGCAACTGTGGTGTAAGATGCATGGAAAA AAAACAACCTTGCAGGACATTACT | 1697 |

(continued)

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV73 | SD1-SA3 i=9 | 395 | 396 | ATACATGATATAAACCTGGACTGTGTGTTTGCCAACGTGTGGACTGTACAGATCTGAGTGTCAGTGCACAGTATGCCTTGCCATTGAAAGCAACAAAGCTGATTTAAGAGTGA | 1698 |
| HPV73 | SD5-SA9 i=11 | 397 | 398 | CCTGTACCCAGTGTACTACACATAATGTTGCGCCAATAGTGCATTTAAAAGATGTGGCGACCTACTGATGCAAAGTATACCTGCCCCC | 1699 |
| HPV73 | SD5-SA10 i=12 | 399 | 400 | CCTGTACCCAGTGTACTACACATAATGTTGCGCCAATAGTGCATTTAAAAGGATTCTCAAAAACGTAAAACCATAGTTCCTAAAGTTTCAGGTTTG | 1700 |
| HPV73 | SD2-SA4 i=15 | 401 | 402 | GCCCCAACTGTTCCAGAAACCTATAAAAGAAGATGGCTGATTCAGATGATACTTGGAAATATTTACATAGTAGAATTAAGGTGTTTACTTTTTTAAATCCATT | 1701 |
| HPV73 | SD2-SA6 i=17 | 403 | 404 | GCCCCAACTGTTCCAGAAACCTATAAAAGAAGATGGCTGATTCAGCGCCTGTGAAGTATCCATTCCTGAAATTGTTAACCCACTGCAC | 1702 |
| HPV73 | SD2-SA9 i=18 | 405 | 406 | GCCCCAACTGTTCCAGAAACCTATAAAAGAAGATGGCTGATTCAGATGTGGCGACCTACTGATGCAAAGTATACCTGCCCCC | 1703 |
| HPV73 | SD2-SA10 i=19 | 407 | 408 | GCCCCAACTGTTCCAGAAACCTATAAAAGAAGATGGCTGATTCAGGATTCTCAAAAACGTAAAACCATAGTTCCTAAAGTTTCAGGTTTG | 1704 |
| HPV82 | SD3-SA4 i=1 | 409 | 410 | CACAAGTGGAGACTGTGGAAGGACCCTTACAGATCCAAATTTAATGTATTTACATAGTAGAGTGACAGTATTTCAATTTTTAAATGCATTTCCATTTGACCCCCAT | 1705 |
| HPV82 | SD3-SA6 i=3 | 411 | 412 | CACAAGTGGAGACTGTGGAAGGACCCTTACAGTACCTACACAGCACCCCGTCACCCTCTACTACAACTG | 1706 |

| HPV type | Splice junction i= | Forward primer SEQ ID NO | Reverse primer SEQ ID NO | Amplicon nucleic acid sequence | Amplicon SEQ ID NO |
|---|---|---|---|---|---|
| HPV82 | SD1-SA4 i=4 | 413 | 414 | ATTCAGGTATTGTGTGTATATTGTAAAAAGGAGTTGTGTAGAGCAGATCCAAATTTAATGTATTTACATAGTAGAGTGACAGTATTTCAATTTTTAAATGCATTTCCATTTGACCCCCAT | 1707 |
| HPV82 | SD1-SA6 i=6 | 415 | 416 | ATTCAGGTATTGTGTGTATATTGTAAAAAGGAGTTGTGTAGAGCAGTACCTACAGCACCCCGTCACCCTCTACTACAACTG | 1708 |
| HPV82 | SD1-SA1 i=7 | 417 | 418 | ATTCAGGTATTGTGTGTATATTGTAAAAAGGAGTTGTGTAGAGCAGGTGTCATAGATGTCAGAGACCACTTGGGCCTGAAGAAAAG | 1709 |
| HPV82 | SD1-SA2 i=8 | 419 | 420 | ATTCAGGTATTGTGTGTATATTGTAAAAAGGAGTTGTGTAGAGCAGAAAACCACCAAGACAACGTAGTGAAACCCAGGTGTAATAACGCCATGCGTGGTAATGT | 1710 |
| HPV82 | SD1-SA3 i=9 | 421 | 422 | ATTCAGGTATTGTGTGTATATTGTAAAAAGGAGTTGTGTAGAGCAGGTGTTCGAGTGTTGTACAGCTCGCAGTGGAAAGCAGTGGAGACAGCC | 1711 |
| HPV82 | SD5-SA9 i=11 | 423 | 424 | GGAACTGCAGGCCCAAACACCGGAGGGCACCTCAGTGCAACTAAAACTGCGTTTATAGTTCATTTAAAAGATGGCTTTGTGGCGTACTAATGACAGCAAAGTGTATTTACCACCTGCACC | 1712 |
| HPV82 | SD2-SA4 i=15 | 425 | 426 | CATCGGCAATGGACAGTGAAGATCCAAATTTAATGTATTTACATAGTAGAGTGACAGTATTTCAATTTTTAAATGCATTTCCATTTGACCCCCAT | 1713 |
| HPV82 | SD2-SA6 i=17 | 427 | 428 | CATCGGCAATGGACAGTGAAGTACCTACAGCACCCCGTCACCCTCTACTACAACTG | 1714 |
| HPV82 | SD2-SA9 i=18 | 429 | 430 | CATCGGCAATGGACAGTGAAGATGGCTTTGTGGCGTACTAATGACAGCAAAGTGTATTTACCACCTGCACC | 1715 |

Table 2B

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV16 | SD3 | GCGGGTATGGCAATACTGAAGT | 431 | TGGTGTTTGGCATATAGTGTGTCTTT | 432 |
| HPV16 | SD1 | CACAGAGCTGCAAACAACTATACAT | 433 | CACATACAGCATATGGATTCCCATCTC | 434 |
| HPV16 | SA4 | GGATGTAAAGCATAGACCATTGGTACA | 435 | GTTTTCGTCAAATGGAAACTCATTAGGA | 436 |
| HPV16 | SA5 | CGGAAATCCAGTGTATGAGCTTAATGAT | 437 | TGACACACATTTAAACGTTGGCAAAG | 438 |
| HPV16 | SA6 | CATGCGGGTGGTCAGGTAA | 439 | AAGGCGACGGCTTTGGTAT | 440 |
| HPV16 | SD5 | GCTCACACAAAGGACGGATTAAC | 441 | CCAATGCCATGTAGACGACACT | 442 |
| HPV16 | SA1 | GGAACAACATTAGAACAGCAATACAACA | 443 | TGTCCAGATGTCTTTGCTTTTCTTCA | 444 |
| HPV16 | SA2 | CGGTGGACCGGTCGATG | 445 | TCAGTTGTCTCTGGTTGCAAATCT | 446 |
| HPV16 | SA9 | CCTATAGTTCCAGGGTCTCCACAA | 447 | ATCCGTGCTTACAACCTTAGATACTG | 448 |
| HPV16 | SA3 | CTCAGAGGAGGAGGATGAAATAGATG | 449 | CCATTAACAGGTCTTCCAAAGTACGA | 450 |
| HPV16 | SD2 | GGAATTGTGTGCCCCATCTGT | 451 | CATCCATTACATCCCGTACCCT | 452 |
| HPV18 | SD3 | TCAGATAGTGGCTATGGCTGTTCT | 453 | CCGTTGTCTATAGCCTCCGT | 454 |
| HPV18 | SD1 | TTCACTGCAAGACATAGAAATAACCTGT | 455 | CTATACATTTATGGCATGCAGCATGG | 456 |
| HPV18 | SA4 | CTAAAATGTCCTCCAATACTACTAACCACAA | 457 | GTCATTTATTTCATATACTGGATTGCCA | 458 |
| HPV18 | SD4 | GGATTGGACACTGCAAGACACA | 459 | CCCATGCTACATAGGTCATACAATTGTC | 460 |
| HPV18 | SA8 | TGACGACACGGTATCCGCTA | 461 | ACGTCTGGCCGTAGGTCT | 462 |

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV18 | SD5 | CAGCTACACCTACAGGCAACAA | 463 | GTCGCTATGTTTTCGCAATCTGTA | 464 |
| HPV18 | SD6 | CGAAAACATAGCGACCACTATAGAGAT | 465 | TTGTACACTATCTGGAATTGCAACAGT | 466 |
| HPV18 | SA1 | TCAGACTCTGTGTATGGAGACACAT | 467 | CCCAGCTATGTTGTGAAATCGT | 468 |
| HPV18 | SA9 | TCCTAAGAAACGTAAACGTGTTCCC | 469 | GTATTTACAACTCTTGCCACAGAAGGA | 470 |
| HPV18 | SA10 | GCATATTTTATCATGCTGGCAGCTCTA | 471 | TCAGGTAACTGCACCCTAAATACTCTAT | 472 |
| HPV18 | SA3 | CAGAGGAAGAAAACGATGAAATAGATGG | 473 | AGAAACAGCTGCTGGAATGCT | 474 |
| HPV18 | SD2 | TGCATCCCAGCAGTAAGCAA | 475 | CTCGTCATCTGATATTACATCTCCTGTT | 476 |
| HPV31 | SD3 | GCGGGTATGGCAATACTGAAGT | 477 | TGGAGTTTCATTCTCTCGTTCACTATG | 478 |
| HPV31 | SD1 | CGGCATTGGAAATACCCTACGAT | 479 | TCTTAAACATTTTGTACACACTCCGTGT | 480 |
| HPV31 | SA4 | CACTAGATGGCAACCCTGTATCT | 481 | AATGTAAAAACCACCAGTCTGCTATGTA | 482 |
| HPV31 | SA5 | CTGGTGGTTTTTACATTTCCAAATCCAT | 483 | CGTTGAGAAAGAGTCTCCATCGTTTT | 484 |
| HPV31 | SA6 | CATGCGGGTGGTCAGGTAA | 485 | GAATTCGATGTGGTGGTGTTGTTG | 486 |
| HPV31 | SD5 | CAGCTGCATGCACAAACCA | 487 | GCCATGTAGATGACACTTGTTCATACAA | 488 |
| HPV31 | SA1 | GGAACAACATTAGAAAAATTGACAAACAAAGG | 489 | TTTTCTTCTGGACACAACGGTCTT | 490 |

EP 3 715 477 A1

(continued)

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV31 | SA2 | GAAACGATTCCACAACATAGGAGGA | 491 | ACATAGTCTTGCAACGTAGGTGTTT | 492 |
| HPV31 | SA9 | GCCACAAGTGTCTATTTTTGTTGATG | 493 | TTTAGACACTGGGACAGGTGGTA | 494 |
| HPV31 | SA3 | CAGATGAGGAGGATGTCATAGACAGT | 495 | CATTAACAGCTCTTGCAATATGCGAATA | 496 |
| HPV31 | SD2 | AATCGTGTGCCCCAACTGT | 497 | CCCCTGTCTGTCTGTCAATTACTG | 498 |
| HPV33 | SD3 | GATGAGCTAGAAGACAGCGGATATG | 499 | CATCCCCACCCCACTAGAT | 500 |
| HPV33 | SD1 | AGCATTGGAGACAACTATACACAACATT | 501 | CGCAAAACACAGTTACATATTCCAAATG | 502 |
| HPV33 | SA4 | TGTGAAACATAGGGCATTAGTGCAATTA | 503 | CATACACTGGGGTTACCATTTTCATCAAA | 504 |
| HPV33 | SA6 | GGATGCTGCAAAGTATTCTAAAACACAA | 505 | GTGGTGGTCGGTTATCGTTGT | 506 |
| HPV33 | SD5 | ACGTACTGCAACTAACTGCACAA | 507 | GCCAGGTGGATGACATAGAACTATACA | 508 |
| HPV33 | SA1 | ATTCTGTATATGGAAATACATTAGAACAAACAG | 509 | TCGTTTGTTGTTTAAAATCCACATGTCGTTTT | 510 |
| HPV33 | SA2 | CGATTTCATAATATTTCGGGTGTTGG | 511 | CATATTCCTTTAACGTTGGCTTGTGT | 512 |
| HPV33 | SA9 | TTGTTGTGTAGACGGTGTCTGACTTT | 513 | ATCAGTGCTGACAACTTTAGATACAGG | 514 |
| HPV33 | SD2 | GTGCCCTACCTGTGCACAA | 515 | TTCTTCTCTCTATGACTGCTTCTACCT | 516 |
| HPV35 | SD3 | ATTATTTGAACTACCAGACAGCGGTT | 517 | GCTACTAGAGGTTATACTATCCCCACT | 518 |
| HPV35 | SD1 | CGAGGTAGAAGAAAGCATCCATGAAAT | 519 | CATACTCCATATGGCTGGCCTTC | 520 |

(continued)

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV35 | SA4 | CATTAGTGCAATTAAAATGCCCACCTT | 521 | TCATTGTGAAAATGTAAAGACCACCTACCC | 522 |
| HPV35 | SA5 | GGAAACCCAGTGTATGGGCTTAAT | 523 | GGAAAGCGTCTCCATCATTTTCTTTG | 524 |
| HPV35 | SA6 | AAAATATATGGGAAGTGCATGTGGGT | 525 | GCTTTGGTATGGGTCTCGGT | 526 |
| HPV35 | SD5 | TCTACATCTGACTGCACAAAACAAAGA | 527 | CCATCTCCATGTAGATGAAGCATCTTG | 528 |
| HPV35 | SA1 | GGAGAAACGTTAGAAAAACAATGCAACA | 529 | TCCACCGATGTTATGGAATCGTTTT | 530 |
| HPV35 | SA9 | GGGTGACTTTTATTTACACCCCTAGTT | 531 | CATCAGTGCTAACAACCTTAGACACT | 532 |
| HPV35 | SA10 | CATCTACTATCATGCAGGCAGTTCT | 533 | ACTCTGTATTGCAAACCAGATACCTTG | 534 |
| HPV35 | SD2 | CGGCTGTTCACAGAGAGCATAAT | 535 | CCCGTACGTCTACTAACTACTGCTT | 536 |
| HPV39 | SD3 | GGTGTATTCCGTGCCAGACA | 537 | GTACACTGCCGCCATGTTC | 538 |
| HPV39 | SD1 | CACCACCTTGCAGGACATTACAATA | 539 | GATTGGCATGCAGCTAGTGG | 540 |
| HPV39 | SA4 | ATTAGATGGGTATGCAATAAGTTTAGATAGG | 541 | CTGTTTTGGTCAAATGGAAATGCATTAG | 542 |
| HPV39 | SD5 | CACAGTAACAGTACAGGCCACA | 543 | CGTATCCAATGCCAGGTACATGAAA | 544 |
| HPV39 | SA1 | CTCGGACTCGGTGTATGCAA | 545 | CTGTCCTGTATAGCTTCCTGCTATTT | 546 |
| HPV39 | SA9 | GCAATAACCATTCAGGGTTCCAATT | 547 | AGTATTGACAACCTTCGCCACA | 548 |
| HPV39 | SA3 | CATGCAGTTAATCACCAACATCAACT | 549 | TGCTGTAGTTGTCGCAGAGTATC | 550 |
| HPV39 | SD2 | CGTGGTGTGCAACTGCAA | 551 | CACTGTGTCGCCTGTTTGTTTAT | 552 |
| HPV45 | SD3 | TCAGATAGTGGCTATGGCTGTTCT | 553 | ACTATCCCCACCACTACTTTGTGTA | 554 |

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV45 | SD1 | CTACAAGACGTATCTATTGCCTGTGT | 555 | AAGTCTATACATTTATGGCATGCAG CATA | 556 |
| HPV45 | SA4 | CATTATTACAGCTAAAATGTCCTCCAATCC | 557 | GAAATGCATGTGGAAATGTAAATAC CGT | 558 |
| HPV45 | SA8 | TGACGACACGGTATCCGCTA | 559 | CCCACGGATGCGGTTTTG | 560 |
| HPV45 | SD5 | TCCTGTGTTCAAGTACAAGTAACAACAA | 561 | GGTCTGCATATTTGCGTAGCCTATA | 562 |
| HPV45 | SD6 | CGCAAATATGCAGACCATTACTCAGAA | 563 | CCCACCGAGATTTGTACACTGTTA | 564 |
| HPV45 | SA1 | AAACTCTGTATATGGAGAGACACTGGA | 565 | CGTTTGTCCTTAAGGTGTCTACGTTT T | 566 |
| HPV45 | SA9 | GCACACAATATTATTTATGGCCATGGTA | 567 | GCTGACAACTCTGGCCACA | 568 |
| HPV45 | SA3 | GGAGTTAGTCATGCACAACTACCA | 569 | TCAAAAACAGCTGCTGTAGTGTTCT | 570 |
| HPV45 | SD2 | AGCACCTTGTCCTTTGTGTGT | 571 | CAATTGTTTCTACAAAGAACCAGCC ATT | 572 |
| HPV51 | SD3 | CGGACAGCGGATATGGCAATA | 573 | TCTGTTGTTTCCACATCCATAACACT | 574 |
| HPV51 | SD1 | CTGCATGAATTATGTGAAGCTTTGAAC | 575 | GTAAACATTGTTTGCATACTGCATAT GGA | 576 |
| HPV51 | SA4 | AGTATGTCCACCATTACTAATAACGTCAAA C | 577 | TCATTCAATGTATACACAGCATTCCC AT | 578 |
| HPV51 | SA6 | GCACAACAGTGGGAGGTCTATATG | 579 | CCACGCAGGTGGTAAGGG | 580 |
| HPV51 | SD5 | CTAACACTGGAGGGCACCAAA | 581 | ATGCCAGGTTGAGGATACGTTTTTAT | 582 |
| HPV51 | SA1 | GAGAGTATAGACGTTATAGCAGGTCTGT | 583 | TCCCGCTATTTCATGGAACCTTTT | 584 |
| HPV51 | SA9 | GGCCCTATACACATTTACTACGCAAA | 585 | CAATTCGAGACACAGGTGCAG | 586 |
| HPV51 | SA3 | GCGTGACCAGCTACCAGAAA | 587 | CATCTGCTGTACAACGCGAAG | 588 |

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV51 | SD2 | GGGCGAACTAAGCCTGGTTT | 589 | CTCATCATCCGAAACATTATCTCCTGT | 590 |
| HPV52 | SD3 | CAAACCATGTCACGTAGAAGACAG | 591 | CCCCACCCCACTTGATTGA | 592 |
| HPV52 | SD1 | AGAATCGGTGCATGAAATAAGGCT | 593 | CACACGCCATATGGATTATTGTCTCTA | 594 |
| HPV52 | SA4 | CCTTAGTACAAATAAAATGCCCACCAT | 595 | GGGTTTTTGAAATGAAACACAACCAATC | 596 |
| HPV52 | SA6 | GTAACAGGAGTATGGGAAGTACATGTG | 597 | GCGGAGGTCTTGGAGGTTT | 598 |
| HPV52 | SD5 | TCACTGCAACTGAGTGCACAA | 599 | TGCCAGGTAGATGAAATTTGAACATACA | 600 |
| HPV52 | SA1 | GTATGGGAAAACATTAGAAGAGAGGGT | 601 | CGCTTGTTTGCATTAACATGTCTTTCT | 602 |
| HPV52 | SA2 | GACATGTTAATGCAAACAAGCGATTC | 603 | TCAGTTGTTTCAGGTTGCAGATCTAATA | 604 |
| HPV52 | SA9 | TTTTACTACGTCGCAGGCGTAA | 605 | TGCTTACAACCTTAGAGACAGGTACA | 606 |
| HPV52 | SA10 | AAGCATCTATTATTATGCAGGCAGTTCT | 607 | CCTGTATTGCAGGCCAGACA | 608 |
| HPV52 | SA3 | GATGAGGAGGATACAGATGGTGTG | 609 | GCATTTGCTGTAGAGTACGAAGGT | 610 |
| HPV52 | SD2 | GCTGTTGGGCACATTACAAGTT | 611 | TCCTCTGAAATGTTATCTCCTGTTTGTT | 612 |
| HPV56 | SD3 | CAAGACAGCGGGTATGGCAATA | 613 | GGTACTGTTTTGTGAGCCTCCATTT | 614 |
| HPV56 | SD1 | GCACCACTTGAGTGAGGTATTAGAA | 615 | ACAATAAACATACTCTGCACACTGCATA | 616 |

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV56 | SA5 | AGAATGTTAGTGTTTCAGTTTCAAAATCC | 617 | TTTTCTTTGTCCTCGTCGTTATCCAA | 618 |
| HPV56 | SD5 | ACAACAACCACCCTGGTGATAAG | 619 | TATTGTCTGTACTTGTCCAATGATATGT | 620 |
| HPV56 | SA1 | TCAGTGTATGGAGCTACACTAGAAAGT | 621 | CAATTGCTTTTCCTCCGGAGTTAA | 622 |
| HPV56 | SA2 | TGCATTGTGACAGAAAAAGACGATTTC | 623 | ACGTCTTGCAGCGTTGGTA | 624 |
| HPV56 | SA9 | AGGGATCCTCCTTTGCATTATGG | 625 | ACAACCTTTGAAACAGGTGTTGGA | 626 |
| HPV56 | SA10 | ATCATGCAGGCAGTTCACGA | 627 | CAACCGTACCCTAAATACCCTATATTGA | 628 |
| HPV56 | SA3 | ACAGCAAGCTAGACAAGCTAAACAA | 629 | TGTACAACACGCAGGTCCTC | 630 |
| HPV56 | SD2 | GTTAACAGTAACGTGCCCACTCT | 631 | TTCTACAATTGCCTCTACTTCAAACCAT | 632 |
| HPV58 | SD3 | AAAATTATTGAGCTAGAAGACAGCGGAT | 633 | CCCCACTAGACTCCGAGTCATTTAA | 634 |
| HPV58 | SD1 | GTCAGGCGTTGGAGACATCT | 635 | TCGTAAGCACACTTTACATACTGCAAA | 636 |
| HPV58 | SA4 | ATTAGATGGTAACGACATTTCAATAGATGT | 637 | TGCATCAAATGGAAATGGATTGTTAAATTCA | 638 |
| HPV58 | SA6 | ACAATTATGGGAGGTACATGTGGGTA | 639 | CCCTGTGTACTTTCGTTGTTGGT | 640 |
| HPV58 | SD5 | GAGGAGGACTACACAGTACAACTAACT | 641 | CCAATGCCATGTGGATGACATATTACA | 642 |
| HPV58 | SA1 | CGCTATATGGAGACACATTAGAACAAACA | 643 | CGACCCGAAATATTATGAAACCTTTTGT | 644 |
| HPV58 | SA9 | CTGATTTTATGTTGCACCCTAGCTATTT | 645 | GCTTACAACCTTAGACACAGGCA | 646 |

EP 3 715 477 A1

(continued)

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV58 | SD2 | TGCTTATGGGCACATGTACCATT | 647 | CTGTTCTTCGTTCTATTACCGCTTCTA | 648 |
| HPV59 | SD3 | AAAGAAGGTTAATAACAGTGCCAGACA | 649 | GTCTATTTGACTGTCGCTACAAACAC | 650 |
| HPV59 | SD1 | GCATCAATTGTGTGTTTTGCAAAGG | 651 | GCATTTCAGACACGCTGCATAC | 652 |
| HPV59 | SA4 | AGATAGAAAGCATAGGCACCTAGTACAA | 653 | TCTATTTTTGTCAAATGGCAATTTGTTTGGA | 654 |
| HPV59 | SD5 | TCCGTTTGCATCCAGGCAA | 655 | CCAATGCCAGGTAGAGGAAATATTTCA | 656 |
| HPV59 | SA9 | CCTCGTAAACGTGTTCC | 657 | TGACATACTCATCAGTGCTGACAAC | 658 |
| HPV59 | SA10 | GTATGTCACCCGTACCAGTATTTCTAC | 659 | GCCAAATTTATTGGGATCAGGTAACTT | 660 |
| HPV59 | SA3 | CAGATGGGAGTTAATCATCCTTTGCTACT | 661 | TGTAAGGCTCGCAATCCGT | 662 |
| HPV59 | SD2 | ACTATCCTTTGTGTGTCCTTTGTGT | 663 | CGTCATCTGAAATTTTGTCACCTGTTTT | 664 |
| HPV66 | SD3 | GAAGACAGCGGGTATGGCAATA | 665 | GATACCGAGTGCTCACTACAATTACTG | 666 |
| HPV66 | SD1 | CACCCATCTGAGCGAGGTATTACA | 667 | ACAATAAAACATACCCTACATACTGCATATGG | 668 |
| HPV66 | SA6 | GTGGGGTGGTGTAAAGTGTCATCA | 669 | GGACAGTAAATACTCTCGGTTTCCAT | 670 |
| HPV66 | SD5 | GTATCAACACACACAAAGCCACTGT | 671 | TCTGTACTTGTCCAATGATATGTTGTTGT | 672 |

(continued)

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV66 | SA1 | GGGCAACATTAGAAAGTATAACTAAAAAACA | 673 | GAAATCGTCTTTATGTTCACAGTGCAA | 674 |
| HPV66 | SA9 | GCTACATTTGCACTATGGCCTGTA | 675 | ACAACCTTTGAAACAGGTGTTGGA | 676 |
| HPV66 | SA3 | ACAGCAAGCTAGACAAGCTGAA | 677 | TGTACCACACGTAGCTCCTCT | 678 |
| HPV66 | SD2 | GTTAACAGTAACGTGCCCACTCT | 679 | TTCTACAATTGCTTCTACCTGAAACCAT | 680 |
| HPV66 | SD3 | AGACAACCGGCGTATACAGTG | 681 | CACACTACTACAGTGCTCCCCGTAT | 682 |
| HPV68 | SD1 | GACATTGGACACTACATTGCATGAC | 683 | GATTGGCATGCAGCAAATGGTA | 684 |
| HPV68 | SA4 | CCTAATACAAATAAAGTGTCCACCAATGCT | 685 | CTGTTTTGGTCAAATGGAAATGCATTAG | 686 |
| HPV68 | SA1 | GGAATCGGTGTATGCAACTACATTAGAA | 687 | CTTCGTTTTGTTGTTAGGTGCCTTAG | 688 |
| HPV68 | SA6 | CTAGTGGAAAATGGGACGTGCATTATA | 689 | TCGCGGGTGGTTCTGTAG | 690 |
| HPV68 | SD5 | AGTAGAAGTGCAGGCCAAAACAA | 691 | AAGCGTTATGTTTTTGCAACCTATAC C | 692 |
| HPV68 | SA9 | TACAACCTTTGCCATAACTATATATGGT | 693 | ATTGACAACCTTCGCCACTGA | 694 |
| HPV68 | SA3 | CCACCAACATCTACTACTAGCCAGA | 695 | CTGTTGTAGTGTCCGCAGGTT | 696 |
| HPV68 | SD2 | TCCGTGGTGTGCAACTGAA | 697 | GACTGTGTCACCTGTTTGTTTATCTA CT | 698 |
| HPV68 | SD3 | AAACGAAGAGACTGTTTGAGGAGCA | 699 | GACACAATTTGGTTGCCTTCTTCATT AA | 700 |
| HPV73 | SD1 | AGCCGTTATGTGACGAAGTGAATATTTCT | 701 | AAAATTTTAAAACACGGTTGACATAC AC | 702 |
| HPV73 | SA4 | CAAGTT AAA TGCCCTCCA TT ACTGA T AAC | 703 | GGGTTCCCA TT ACTGTCAAA TGGA | 704 |

(continued)

| HPV type | Splice site | Forward primer nucleic acid sequence | Forward primer SEQ ID NO | Reverse primer nucleic acid sequence | Reverse primer SEQ ID NO |
|---|---|---|---|---|---|
| HPV73 | SA6 | GGGTAAAAGGCATATGGGAAGTACAT | 705 | TGGTGTTGGTGGTTGTGGT | 706 |
| HPV73 | SD5 | ACCTACATCCCACCACAGAGT | 707 | GTCCAATGCCATGTTGTTGTTACA | 708 |
| HPV73 | SA1 | AGACAATCAGTATATGGCACTACGTTAGA | 709 | CTGTTCTGCTATTTGATGAAACCGTTTT | 710 |
| HPV73 | SA9 | TGGGTCAGGTTTTTATATTACACCCTAGT | 711 | GCTTACAACCTTAGACACAGACACA | 712 |
| HPV73 | SA10 | TGCAGGTAGCACACGTTTGT | 713 | ACGAAGCCTAAACACCCTGTATTG | 714 |
| HPV73 | SA3 | ACTCAGAGGATGAGGATGAAACAGA | 715 | CCTAGTGTACCCATAAGCAACTCTTCTA | 716 |
| HPV73 | SD2 | TGCTTATGGGTACACTAGGTATTGTGT | 717 | TGGAATTGGATCCCCTGTTTTTCTTT | 718 |
| HPV73 | SD3 | CCGGACAGTGGATATGGCAATA | 719 | GGTCTATCTCTGTACTTCTCTGTCGCT | 720 |
| HPV82 | SD1 | CCTGCAATACGTCTATGCACAAT | 721 | CATGCTGCATATGGCGTATTGTC | 722 |
| HPV82 | SA4 | ACACAGAAGCCTGCTGCAAA | 723 | CATCATTTAGTGCATATACAGGATTCCC | 724 |
| HPV82 | SA6 | GGGCACAACAATGGGAGGTA | 725 | GGGTGTTCGATAGCTGTTCAA | 726 |
| HPV82 | SD5 | TGCGACCACCAAATACACTGT | 727 | CAATGCCAGGTAGATGACACTTCTTTAA | 728 |
| HPV82 | SA1 | GTAGGTCTGTGTATGGTGCTACATT | 729 | TTTTTTGTCGTCCACCACCTTTTG | 730 |
| HPV82 | SA9 | GGGATTACTACTTTGTGGCCGTATA | 731 | GTGTTGACAATGCGTGACACT | 732 |
| HPV82 | SA3 | GGAGGGATGAAGTAGATAATATGGCGTGAC | 733 | CCAGTAACATTTGCTGAAATATGCGAA | 734 |
| HPV82 | SD2 | CGTGGTGTGCGACCAACTAA | 735 | TTGTCAACTACTGCCTCCACATAAA | 736 |

Table 2Bbis

| HPV type | Splice site | Forward primer SEQ ID NO | Reverse primer SEQ ID NO | Amplicon nucleic acid sequence | Amplicon SEQ ID NO |
|---|---|---|---|---|---|
| HPV16 | SD3 | 431 | 432 | GGAAACTCAGCAGATGTTACAGGTAGAAGGGCGCCATGAGACTGAAACACCATGTAGTCAGTATAGTGGTGGAAGTGGGGGTGGTTGCAGTCAGTACAGTAGTGGAAGTGGGGGAGAGGGTGTTAGTG | 1716 |
| HPV16 | SD1 | 433 | 434 | GATATAATATTAGAATGTGTGTACTGCAAGCAACAGTTACTGCGACGTGAGGTATATGACTTTGCTTTTCGGGATTTATGCATAGTATATA | 1717 |
| HPV16 | SA4 | 435 | 436 | ACTAAAATGCCCTCCATTATTAATTACATCTAACATTAATGCTGGTACAGATTCTAGGTGGCCTTATTTACATAATAGATTGGTGGTGTTTACATT | 1718 |
| HPV16 | SA5 | 437 | 438 | AAGAACTGGAAATCCTTTTTCTCAAGGACGTGGTCCAGATTAAGTTTGCACGAGGACGAGGACAAGGAAAACGATGGAGACT | 1719 |
| HPV16 | SA6 | 439 | 440 | TATTATGTCCTACATCTGTGTTTAGCAGCAACGAAGTATCCTCTCCTGAAATTATTAGGCAGCACTTGGCCAACCACCCGCCGCGACCC | 1720 |
| HPV16 | SD5 | 441 | 442 | TGTAATAGTAACACTACACCCATAGTACATTTAAAAGGTGATGCTAATACTTTAAAATGTTTAAGATATAGATTTAAAAAGCATTGTACATTGTATACTGC | 1721 |
| HPV16 | SA1 | 443 | 444 | AACCGTTGTGTGATTTGTTAATTAGGTGTATTAACTGTCAAAAGCCACTGTGTCC | 1722 |
| HPV16 | SA2 | 445 | 446 | TATGTCTTGTTGCAGATCATCAAGAACACGTAGAGAAACCCAGCTGTAATCATGCATGGAGATACACCTACATTGCATGAATATATGTT | 1723 |
| HPV16 | SA9 | 447 | 448 | TATACAATTATTGCTGATGCAGGTGACTTTTATTTACATCCTAGTTATTACATGTTACGAAAACGACGTAAACGTTTACCATATTTTTTTTCAGATGTCTCTTTGGCTGCCTAGTGAGGCCACTGTCTACTTGCCTCCTGTCC | 1724 |
| HPV16 | SA3 | 449 | 450 | GTCCAGCTGGACAAGCAGAACCGGACAGAGCCCATTACAATATTGTAA | 1725 |

| HPV type | Splice site | Forward primer SEQ ID NO | Reverse primer SEQ ID NO | Amplicon nucleic acid sequence | Amplicon SEQ ID NO |
|---|---|---|---|---|---|
| | | | | CCTTTTGTTGCAAGTGTGACTCTACGCTTCGGTTGTGCGTACAAAGCAC ACACGTAGACAT | |
| HPV16 | SD2 | 451 | 452 | TCTCAGAAACCATAATCTACCATGGCTGATCCTGCAGGTACCAATGGGG AAG | 1726 |
| HPV18 | SD3 | 453 | 454 | GAAGTGGAAGCAACACAGATTCAGGTAACTACAAATGGCGAACATGGC GGCAATGTATGTAGTGGCGGCAGT | 1727 |
| HPV18 | SD1 | 455 | 456 | GTATATTGCAAGACAGTATTGGAACTTACAGAGGTATTTGAATTTGCAT TTAAAGATTTATTTGTGGTGTATAGAGACAGTATACC | 1728 |
| HPV18 | SA4 | 457 | 458 | ATATACATCCAGCAAAGGATAATAGATGGCCATATTTAGAAAGTAGAA TAACAGTATTTGAATTTCCAAATGCATTTCCATTTGATAAAAA | 1729 |
| HPV18 | SD4 | 459 | 460 | TGCGAGGAACTATGGAATACAGAACCTACTCACTGCTTTAAAAAAGGT GGCCAAACAGTACAAGTATATTTTGATGGCAACAAA | 1730 |
| HPV18 | SA8 | 461 | 462 | CTCAGCTTGTTAAACAGCTACAGCACACCCCCTCACCGTATTCCAGCAC CGTGTCCGTGGGCACCGCAA | 1731 |
| HPV18 | SD5 | 463 | 464 | CAAAAGACGGAAACTCTGTAGTGGTAACACTACGCCTATAATACATTTA AAAGGTGACAGAAACAGTTTAAAATGTTTACGG | 1732 |
| HPV18 | SD6 | 465 | 466 | ATATCATCCACCTGGCATTGGACAGGTGCAGGCAATGAAAAAACAGGA ATACTGACTGTAACATACCATAGTGAAACACAAGAACAAAATTTTTA AAT | 1733 |
| HPV18 | SA1 | 467 | 468 | TGGAAAAACTAACTAACACTGGGTTATACAATTTATTAATAAGGTGCCT GCGGTGCCAGAAACCGTTGAATCCAGCAGAAAAACTTAGACACCTTAA TGAAAAACG | 1734 |
| HPV18 | SA9 | 469 | 470 | TATTTTTTTGCAGATGGCTTTGTGGCGGCCTAGTGACAATACCGTATATC TTCCACC | 1735 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV18 | SA10 | 471 | 472 | GATTATTAACTGTTGGTAATCCATATTTTAGGGTTCCTGCAGGTGGTGG CAATAAGCAGGATATTCCTAAGGTTTCTGCATACCAAT | 1736 |
| HPV18 | SA3 | 473 | 474 | AGTTAATCATCAACATTTACCAGCCCGACGAGCCGAACCACAACGTCAC ACAATGTTGTGTATGTGTTGTAAGTGTGAAGCCAGAATTGAGCTAGTAG TAGAAAGCTCAGCAGACGACCTTCG | 1737 |
| HPV18 | SD2 | 475 | 476 | CAATGGCTGATCCAGAAGGTACAGACGGGGAGGGCACGGGTTGTAACG GCTGGTTTTATGTACAAGCTATTGTAGACAAAAA | 1738 |
| HPV31 | SD3 | 477 | 478 | GGAAACGCAGCAGATGGTACAGGTAGAGGAGCAACAAACAACATTAA GTTGTAATGGTAGTGACGGGACA | 1739 |
| HPV31 | SD1 | 479 | 480 | GAACTAAGATTGAATTGTGTCTACTGCAAAGGTCAGTTAACAGAAACA GAGGTATTAGATTTTGCATTTACAGATTAACAATAGTATATAGGGACG ACACACC | 1740 |
| HPV31 | SA4 | 481 | 482 | ATAGATGTAAAGCATAAAGCTTTAATGCAGTTAAAATGTCCTCCTTTAT TGATTACATCTAATATAAATGCAGGTAAGGATGACAGATGGCCATACC | 1741 |
| HPV31 | SA5 | 483 | 484 | TTCCATTTGACAAAAACGGAAATCCAGTATATGAATTAAGTGATAAAA ACTGGAAATCCTTTTTCTCAAGGACGTGGTGCAGATTAAATTTGCACGA GGAAGAGGACAAAG | 1742 |
| HPV31 | SA6 | 485 | 486 | TTGTTTTTCCTGAATCTGTATTTAGCAGTGACGAAATATCCTTTGCTGGG ATTGTTACAAAGCTACCAACAGC | 1743 |
| HPV31 | SD5 | 487 | 488 | AACAAGGGCTGTCAGTTGTCCTGCAACTACACCTATAATACACTTAAAA GGTGATGCAAATATATTAAAATGTTTAAGATATAGGCTGTCAAAATATA AACAA | 1744 |
| HPV31 | SA1 | 489 | 490 | TATATGTGATTTGTTAATTAGGTGTATAACGTGTCA | 1745 |

EP 3 715 477 A1

(continued)

| HPV type | Splice site | Forward primer SEQ ID NO | Reverse primer SEQ ID NO | Amplicon nucleic acid sequence | Amplicon SEQ ID NO |
|---|---|---|---|---|---|
| HPV31 | SA2 | 491 | 492 | AGGTGGACAGGACGTTGCATAGCATGTTGGAGAAGAGACCTCGTACTGAAACCCAAGTGTAAACATGCGTGGAG | 1746 |
| HPV31 | SA9 | 493 | 494 | GGGGTGATTTTTATTTGCACCCTAGTTATTATATGTTAAAACGTCGACGTAAACGTGTATCATATTTTTTACAGATGTCTCTGTGGCGGCCTAGCGAGGCTACTGTCTACT | 1747 |
| HPV31 | SA3 | 495 | 496 | CCAGCTGGACAAGCAGGAGAACCGGACACATCCAATTACAATATCGTTACCTTTTGTTGTGTCAGTGTGTAAGTCTACACTTCGTTTGTGTGTACAGAGCACACAAGTAGA | 1748 |
| HPV31 | SD2 | 497 | 498 | TCTACTAGACTGTAACTACAATGGCTGATCCAGCCAGCAGGTACAGATGGGGAGGGGACGGGATGCAATGGTTGGTTTTATGTAGAAG | 1749 |
| HPV33 | SD3 | 499 | 500 | GCAATACTGAAGTGGAAACTCAGCAGATGGTACAACAGGTAGAAAGTCAAAATGGGCGACACAAACTTAAATGACTTAGA | 1750 |
| HPV33 | SD1 | 501 | 502 | GAACTACAGTGCGTGGAATGCAAAAAAACCTTTGCAACGATCTGAGGTATATGATTTTGCATTTGCAGATTTAACAGTTGTATATAGAGAGGGAAATC | 1751 |
| HPV33 | SA4 | 503 | 504 | AAATGTCCACCACTGCTTCTTACCTCAAATACAAATGCAGGCACAGACTCTAGATGGCCATATTTACACATAGTAGATTAACAGTATTTGAATTTAAAAATCCATTCCCA | 1752 |
| HPV33 | SA6 | 505 | 506 | ATGTGGGAAGTACATGTGGGTGGTCAGGTAATTGTTTGTCCTACGTCTATATCTAGCAACCAAATATCCACTACTGAAACTGCTGACATACAGACAG | 1753 |
| HPV33 | SD5 | 507 | 508 | ACAAGCAGCGGACTGTGTGTAGTTCTAACGTTGCACCTATAGTGCATTTAAAAGGTGAATCAAATAGTTTAAAATGTTTAAGATACAGATTAAAAACCTTATAAAGAGT | 1754 |
| HPV33 | SA1 | 509 | 510 | TTAAAAACCTTTAAATGAAATATTAATTAGGTGTATTATATGTCAAAGACCTTTGTGTCCTCAAGAAAA | 1755 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV33 | SA2 | 511 | 512 | GCAGGGCGCTGTGCGGCGTGTTGGAGGTCCCGACGTAGAGAAACTGCACTGTGACGTGTAAAAACGCCATGAGAGG | 1756 |
| HPV33 | SA9 | 513 | 514 | GTTTTACATCCTAGTTATTTTATTTTACGTCGCAGGCGTAAACGTTTTCCATATTTTTTTACAGATGTCCGTGTGGCGGCCTAGTGAGGCCACAGTGTACCTGCCTCCTGTA | 1757 |
| HPV33 | SD2 | 515 | 516 | CAATAAACATCATCTACAATGGCCGATCCTGAAGGTACAAATGGGGCTGGGATGGGGTGTACTGGTTGGTTTG | 1758 |
| HPV35 | SD3 | 517 | 518 | ATGGCAATTCTGAAGTGGAAATACAGCAGATACAACAGGTAGAGGGGCATGATACAGTTGAACAATGTAGTATGGGC | 1759 |
| HPV35 | SD1 | 519 | 520 | TTGTTTGAATTGTGTATACTGCAAACAAGAATTACAGCGGAGTGAGGTATATGACTTTGCATGCTATGATTTGTGTATAGTATATAGA | 1760 |
| HPV35 | SA4 | 521 | 522 | TACTTATTACATCAAATATAAATGCAGGCAAAGATGACAGGTGGCCATACTTACATAGCA | 1761 |
| HPV35 | SA5 | 523 | 524 | GATAAAAACTGGAAATCCTTTTTCTCAAGGACGTGGTGCAGATTAAATTTGCACGAGGAAGAGGA | 1762 |
| HPV35 | SA6 | 525 | 526 | GGTCAGGTAATTGTTTGTCCTGAATCTGTATTTAGCAGCACAGAACTATCCACTGCTGAAATTGCTACACAGCTACACGCCTACAACACC | 1763 |
| HPV35 | SD5 | 527 | 528 | CCGGTGTGGTAGTTGTAGTACAACTACACCTATAGTACATTTAAAAGGTGATGCAAATACATTAAAGTGTTTAAGATATAGATTGGGTAAATATAAAGCATTGTAT | 1764 |
| HPV35 | SA1 | 529 | 530 | AACAGTTATGTCATTTATTAATTAGGTGTATTACATGTCAAAAACCGCTGTGTCCAGTTGAAAAGCAAAGACATTTAGAAGAAAA | 1765 |

(continued)

| HPV type | Splice site | Forward primer SEQ ID NO | Reverse primer SEQ ID NO | Amplicon nucleic acid sequence | Amplicon SEQ ID NO |
|---|---|---|---|---|---|
| HPV35 | SA9 | 531 | 532 | ATTATTTATTAAAAACGACGTTCGTAAAACGTATCCCATATTTTTTGCAGATGTCTCTGTGCGGGTCTAACGAAGCCACTGTCTACCTGCCTCCAGTGTC | 1766 |
| HPV35 | SA10 | 533 | 534 | AGGCTATTAGCTGTGGGTCACCCATACTATGCTATTAAAAAACAAGATTCTAATAAAATAGCAGTACC | 1767 |
| HPV35 | SD2 | 535 | 536 | CTACAATGGCTGATCCTGCCAGGTACAGATGAAGGGGAGGGGACGGGGATGTAATGGATGGTTTTTGTAG | 1768 |
| HPV39 | SD3 | 537 | 538 | GCGGGATATGGCAATATGGAAGTGGAAAACAGCTGAAGTGGGAGGAGGTAACTGTGTAGCAACTAATACAAATGGGGATGCTGAAGGG | 1769 |
| HPV39 | SD1 | 539 | 540 | GCCTGTGTCTATTGCAGACGACCACTACAGCAAACCGAGGTATATGAATTTGCATTTAGTGATTTATATGTAGTATATAGGGACGCGGGAA | 1770 |
| HPV39 | SA4 | 541 | 542 | AAATATAAAAGTTTACTACAAATGAAATGTCCACCATTATTAATAACCTCCAATACCAATCCTGTGGAAGACGATAGGTGGCCCATATTTACGTAGTAGGCTAACAGTGTTTAAATTTC | 1771 |
| HPV39 | SD5 | 543 | 544 | ACACAAGAGACGGTACCTCAGTTGTGGTAACACTACGCCTATAATACATTTAAAAGGTGACAAAAATGTTTAAAATGTTTAAGATATAGACTACAAAAATATGACACATTGTTTGAAAATA | 1772 |
| HPV39 | SA1 | 545 | 546 | CTACATTAGAAAATATAACTAATACAAAGTTATATAATTTATTAATAAGGTGCATGTGTTGTCTGAAACCGCTGTGTCCAGCAGAAAAATTAAGACACCTAAATAGCAAACGAAGATTTCAT | 1773 |
| HPV39 | SA9 | 547 | 548 | ATTATTTGTTGCCATTATTGTATTTTTCCTAAAAAAAACGTATTCCCTATTTTTTTCAGATGGCTATGTGGCGGGTCTAGTGACAGCATGGTGTATTTGCCTCCACCTTC | 1774 |
| HPV39 | SA3 | 549 | 550 | ACTAGCCAGACGGGATGAACCACAGCGTCACACAATACAGTGTTCGTGTTGTAAGTGTAACACACTGCAGCTGGTAGTAGAAGCCTCACGG | 1775 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV39 | SD2 | 551 | 552 | ACCAGTAACCTGCTATGGCCAATCGTGAAGGTACAGACGGGGATGGGT CGGGATGTAACGGATGGTTTCTAGTACAGGCAATAGTAG | 1776 |
| HPV45 | SD3 | 553 | 554 | GAAGTGGAAGCTGCAGAGACTCAGGTAACTGTAAACACTAATGCGGAA AATGGCGGCAGTGTACATAG | 1777 |
| HPV45 | SD1 | 555 | 556 | ATATTGCAAAGCAACATTGGAACGCACAGAGGTATATCAATTTGCTTTT AAAGATTTATGTATAGTGTATAGAGACTGTATAGCA | 1778 |
| HPV45 | SA4 | 557 | 558 | TATTAACATCCAATATTGATCCAGCAAAAGATAATAAATGGCCATATTT AGAAAGTAGGGTG | 1779 |
| HPV45 | SA8 | 559 | 560 | CTCAGATTGTTAGACAGCTACAACACGCCTCCACGTCGACCCC | 1780 |
| HPV45 | SD5 | 561 | 562 | AAGAAGGAAAGTGTGTAGTGGTAACACTACGCCTATAATACACTTAAA AGGTGACAAAAACAGTTTGAAATGTTTAAGA | 1781 |
| HPV45 | SD6 | 563 | 564 | ATATCCTCCACCTGGCATTGGACAGGTTGTAATAAAAACACTGGTATAT TAACTGTAACATATAATAGTGAGGTACAAAGAAATACCTTTTTGGATGT AGTTACTATTCC | 1782 |
| HPV45 | SA1 | 565 | 566 | AAAAATAACTAATACAGAGTTGTATAATTTGTTAATAAGGTGCCTGCGG TGCCAGAAACCATTGAACCCAGCAGA | 1783 |
| HPV45 | SA9 | 567 | 568 | TTATTATTTTCCTAAAAAACGTAAACGTATTCCCTATTTTTTTGCAGATG GCTTTGTGGCGGCCTAGTGACAGTACGGTATATCTTCCACCACCTTC | 1784 |
| HPV45 | SA3 | 569 | 570 | GCCCGACGAGCCGAACCACAGCGTCACAAAATTTTGTGTGTATGTTGTA AGTGTGACGGCAGAATTGAGCTTACAGTAGAGAGCTCGGCAGAGGACC TT | 1785 |
| HPV45 | SD2 | 571 | 572 | CCGTGGTGTGCAACTAACCAATAATCTACAATGGCGGATCCAGAAGGT ACCGACGGGGAGGGAACGGGGTGT | 1786 |

EP 3 715 477 A1

91

| HPV type | Splice site | Forward primer SEQ ID NO | Reverse primer SEQ ID NO | Amplicon nucleic acid sequence | Amplicon SEQ ID NO |
|---|---|---|---|---|---|
| HPV51 | SD3 | 573 | 574 | CACAAGTGGAAACTGTGGAAGCAACGTTGCAGGTAGATGGGCAACATG GCGGTTCACAGAACAGTGTGTGTAGTAGCGGGGGGGGGC | 1787 |
| HPV51 | SD1 | 575 | 576 | GTTTCTATGCACAATATACAGGTAGTGTGTGTGTATTGTAAAAAGGAAT TATGTAGAGCAGATGTATATAATGTAGCATTTACTGAAATTAAGATTGT ATATAGGGATAATAA | 1788 |
| HPV51 | SA4 | 577 | 578 | ATAAATCCACAAGAGGATGCAAACCTAATGTATTTACATACAAGGGTA ACAGTATTAAAGTTTTTAAATACATTTCCATTTGATAACA | 1789 |
| HPV51 | SA6 | 579 | 580 | TATGGTACTGTAATAACATGTCCTGAATATGTATCTAGTACCTGCAGCG ACGCGTTATCCACTACTACAACTGTTGAACAACTATCAAACACCCCAAC GACCAATC | 1790 |
| HPV51 | SD5 | 581 | 582 | GTGCAACTCAGACTGCGTTTATAGTGCATTTAAAAGGTGATACAAATTG TTTAAAATGTTTTAGATACAGATTTACAAAACACAAAGGGTTAT | 1791 |
| HPV51 | SA1 | 583 | 584 | GTATGGTACTACATTAGAGGCAATTACTAAAAAAAGCTTATATGATTTA TCGATAAGGTGTCATAGATGTCAAAGACCACTTGGGCCTGAAGAAAAG CAAAAATTGGTGGACGAAAAA | 1792 |
| HPV51 | SA9 | 585 | 586 | CGCCGTAAACGTATACCCTATTTTTTTACAGATGGCATTGTGGCGCACT AATGACAGCAAGGTGTATTTGCCAC | 1793 |
| HPV51 | SA3 | 587 | 588 | GACGGGCTGGACAGGCTACGTGTTACAGAATTGAAGCTCCGTGTTGCA GGTGTTCAAGTGTAGTACAACTGGCAGTGGAAAGCAGTGGAGACACC | 1794 |
| HPV51 | SD2 | 589 | 590 | GCCCGTGTTGTGCGAACAACTAGCAACGGCGATGGACTGTGAAGGTAC AGAGGATGAGGGGGCGGGGTGTAATGGGTGGTTTTTTGTTGAAGCAAT AGTAGAAAAAAAA | 1795 |
| HPV52 | SD3 | 591 | 592 | CGGCTATGGCAATAGTGAAGTGGAAGCGCAGCAGATGGCAGACCAGGT AGACGGGCAAAATGGCGACTGGCAAAGTAACAGTAG | 1796 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV52 | SD1 | 593 | 594 | GCAGTGTGTGCAGTGCAAAAAAGAGCTACAACGAAGAGAGGTATACAA GTTTCTATTTACAGATTTACGAATAGTATA | 1797 |
| HPV52 | SA4 | 595 | 596 | TAATTTTAACAACAAATACAAATGCAGGAACAGATCCTAGGTGGCCAT ATTTACATAGTA | 1798 |
| HPV52 | SA6 | 597 | 598 | GGTGGTCAGGTAATTGTTTGTCCTGCATCTGTATCTAGTAACGAAGTAT CCACTACTGAAACTGCTGTCCACCTATGCACCG | 1799 |
| HPV52 | SD5 | 599 | 600 | ACAAAGGACGGGTTGCACATACAACTTGTACTGCACCTATAATACACCT AAAAGGTGATCCTAATAGTTTAAAATGTTTAAGATATAGGGTAAAAAC ACATAAAAGTT | 1800 |
| HPV52 | SA1 | 601 | 602 | AAAAAAACCATTAAGTGAAATAACTATTAGATGTATAATTTGTCAAACG CCATTATGTCCTGAAGAAAA | 1801 |
| HPV52 | SA2 | 603 | 604 | ATAATATTATGGGTCGTTGGACAGGGCGCTGTTCAGAGTGTTGGAGACC CCGACCTGTGACCCAAGTGTAACGTCATGCGTGGAGACAAAGCAACTA TAAAAGATTATA | 1802 |
| HPV52 | SA9 | 605 | 606 | ACGTTTTCCATATTTTTTTACAGATGTCCGTGTGGCGGCCTAGTGAGGCC ACTGTGTACCTGCCTCC | 1803 |
| HPV52 | SA10 | 607 | 608 | CGATTACTAACAGTAGGACATCCCTATTTTTCTATTAAAAACACCAGTA GTGGTAATGGTAAAAAGTTTTAGTTCCCAAGG | 1804 |
| HPV52 | SA3 | 609 | 610 | GACCGGCCAGATGGACAAGCAGAACAAGCCACAAGCAATTACTACATT GTGACATATTGTCACAGTTGTGATAGCACACTACGGCTATGCATTCATA GCACTGCGACGG | 1805 |
| HPV52 | SD2 | 611 | 612 | GTGTGCCCCGGCTGTGCACGGCTATAAACAACCCTGCAATGGAGGACC CTGAAGGTACAGAGGGCGAAAGGGAGGGATGTACAGGCTGGTTTGAAG TAGAGGCAATAATAGAAA | 1806 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV56 | SD3 | 613 | 614 | CATTGGAAACTCTGGAAACACCAGAACAGGTAGATGAAGAGGTACAGGGACGTGGGTGCGGGAATACACA | 1807 |
| HPV56 | SD1 | 615 | 616 | ATACCTTTAATTGATCTTAGATTATCATGTGTATATTGCAAAAAAGAACTAACACGTGCTGAGGTATATAATTTTGCATGCACTGAATTAAAATTAGTGTATAGGGATGATTTTCCT | 1808 |
| HPV56 | SA5 | 617 | 618 | ATTTCCATTAGATAATAATGGTAATCCTGTATATGAATTAAGTAATGTAAACTGGAAATGTTTCTTTACAAGGACGTGGTCCAGATTAAAT | 1809 |
| HPV56 | SD5 | 619 | 620 | ACTACGCCTGTAGTACATTTAAAAGGTGAACCTAACAGATTAAAATGTTGTAGATATCGATTTCAAAAATATAAAACATTGTTTGTGGATGTAACATCA | 1810 |
| HPV56 | SA1 | 621 | 622 | ATAACTAAAAAACAGTTATGTGATTTATTAATAAGGTGCTACAGATGTCAAAGTCCG | 1811 |
| HPV56 | SA2 | 623 | 624 | ATCTAATAGCACATGGTTGGACCGGGTCATGTTTGGGGTGCTGGAGACAAACATCTAGAGAACCTAGAGAATCTACAGTATAATCATGCATGGTAAAG | 1812 |
| HPV56 | SA9 | 625 | 626 | CCTGTGTATTTTTTTAGACGTAGGCGCCGTAAACGTATTCCCTATTTTTTGCAGATGGCGACGTGGCGGCCTAGTGAAAATAAGGTGTATCTACC | 1813 |
| HPV56 | SA10 | 627 | 628 | TTGCTTGCCGTAGGACATCCCTATTACTCTGTGACTAAGGACAATACCAAAACAAACATTCCCAAAGTTAGTGCATA | 1814 |
| HPV56 | SA3 | 629 | 630 | CATACGTGTTACCTAATACACGTACCTTGTTGTGAGTGTAAGTTTGTGGTGCAGTTGGACATTCAGAGTACCAAA | 1815 |
| HPV56 | SD2 | 631 | 632 | GCGCATCAAGTAACTAACTGCAATGGCGTCACCTGAAGGTACAGATGGGGAGGGGAAGGGATGTTGTGG | 1816 |

EP 3 715 477 A1

94

(continued)

| HPV type | Splice site | Forward primer SEQ ID NO | Reverse primer SEQ ID NO | Amplicon nucleic acid sequence | Amplicon SEQ ID NO |
|---|---|---|---|---|---|
| HPV58 | SD3 | 633 | 634 | ATGGCAATACTGAAGTGGAAACTGAGCAGATGGCACACCAGGTAGAAA GCCAAAATGGCGACGCAGAC | 1817 |
| HPV58 | SD1 | 635 | 636 | GTGCATGAAATCGAATTGAAATGCGTTGAATGCAAAAAGACTTTGCAG CGATCTGAGGTATATGACTTTGTATTTGCAGATTTAAGAATAGTGTATA GAGATGGAAATCCA | 1818 |
| HPV58 | SA4 | 637 | 638 | AAAACATAGGGCATTAGTACAATTAAAATGTCCACCATTAATAATTACC TCAAATACAAATGCAGGCAAAGATTCACGATGGCCATATTTGCACAGT AGACTAACAGTATT | 1819 |
| HPV58 | SA6 | 639 | 640 | GTCGGGTAATTGTATGTCCTACATCTATACCTAGTGATCAAATATCCAC TACTGAAACTGCTGACCCAAAGACCACCGAGGCC | 1820 |
| HPV58 | SD5 | 641 | 642 | GTACATACAAAGGGCGGAACGTGTGTAGTTCTAAAGTTTCACCTATCGT GCATTTAAAAGGTGACCCAAATAGTTTAAAATGTTTAAGATATAGATTA AAACCATTTAAAGACTTATAC | 1821 |
| HPV58 | SA1 | 643 | 644 | CTAAAAAAGTGTTTAAATGAAATATTAATTAGATGTATTATTTGTCAAA GACCATTGTGTCCACAAGAAAAAAAAGGCATGTGGATTTAA | 1822 |
| HPV58 | SA9 | 645 | 646 | TATTTTGCGTCGCAGACGTAAACGTTTTCCATATTTTTTTGCAGATGTCC GTGTGGCGGCCTAGTGAGGCCACTGTGTACCTGCCTCCTG | 1823 |
| HPV58 | SD2 | 647 | 648 | GTGTGCCCTAGCTGTGCACAGCAATAAACACCATCTGCAATGGATGACC CTGAAGGTACAAACGGGGTAGGGGCGGGCTGTACTGGCTGGTTTGAGG | 1824 |
| HPV59 | SD3 | 649 | 650 | GCGGCTATGGCTATTCTGAAGTGGAAATGCTCGAGACTCAGGTAACCGT GGAGAATACTGGAAATGGGGATAGCAATGGCA | 1825 |
| HPV59 | SD1 | 651 | 652 | GGAACTGCAAGAAAGAGAGGTATTTGAATTTGCTTTTAATGACTTATTT ATAGTGTATAGAGACTGTACACC | 1826 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV59 | SA4 | 653 | 654 | ATTAAATGTCCACCAATGCTTATTACATCAAATACAAATCCAGTTACAG ATAACAGGTGGCCATATTTAAATAGCAGATTAATGGTATTTAAATT | 1827 |
| HPV59 | SD5 | 655 | 656 | CAACCCGCGACGGCACATCCCTTGCAGTAACACTACGCCTATAATACAC TTAAAAGGTGACAAAAATGGCCTTAAGTGTTTAAGGTATAGATTAAGA AAAGTACACTGGTTATT | 1828 |
| HPV59 | SA9 | 657 | 658 | CTATTTTTTTACAGATGGCTCTATGGCGTTCTAGTGACAACAAGGTGTAT CTACCTCCACCTTCGGTAGCTAAG | 1829 |
| HPV59 | SA10 | 659 | 660 | CACGCAGGCAGTTCCAGACTTCTTACAGTTGGACATCCATATTTTAAAG TACCTAAAGGTGGTAATGGTAGACAGGATGTTCCTAAGGTGTCTGCATA TCAATACAGAGTATTTAGGGTT | 1830 |
| HPV59 | SA3 | 661 | 662 | AGCTAGACGAGCTGAACCACAGCGTCACAACATTGTGTGTGTGTGTTGT AAGTGTAATAATCAACTTCAGCTAGTAGTAGAAACCTCGCAAG | 1831 |
| HPV59 | SD2 | 663 | 664 | GCAGCAAACCAGTAACCTGCAATGGCCGATTCGGAAGGTACAGATGGG GAAGGGACGGGGTGCAATGGATGGTTTTTTGTGCAGGCAATAGTAGAT AAA | 1832 |
| HPV66 | SD3 | 665 | 666 | CATTGGAAACATTGGAAACATCACAACAGGTAGAATACGAAAAGGGAA ATGGGTGCGGGAGCTCACAAAATGGAGGCTCGCAAAA | 1833 |
| HPV66 | SD1 | 667 | 668 | AATACCTTTACTTGATCTTAGATTATCATGTGTATACTGCAAAAAGGAA CTTACAAGTTTAGAGCTATATAGGTTTGCATGTATTGAGTTAAAACTAG TATATAGAAACAATTGG | 1834 |
| HPV66 | SA6 | 669 | 670 | GGGGTGGATTACAGAGGCATATATTATATGCATGATGGCCACAAAACA TATTACACAGACTTTGAACAGGAGGCCAAAAAATATGGGTGTACAAAC ATATGGGAAGTACAT | 1835 |

(continued)

| HPV type | Splice site | Forward primer SEQ ID NO | Reverse primer SEQ ID NO | Amplicon nucleic acid sequence | Amplicon SEQ ID NO |
|---|---|---|---|---|---|
| HPV66 | SD5 | 671 | 672 | GGTGATAAAAACTACGCGCCTGTAATCCATTTAAAAAGGTGAAGCTAATAGATTAAAGTGTTGTGTAGATACAGATTTCAAAAATATAAAACATTATTTACAGATGTA | 1836 |
| HPV66 | SA1 | 673 | 674 | GTTATCTGATTTATTCAATAAGGTGCTACCGATGTCAATGTCCGTTAACACCGGAGGAAAAAACAA | 1837 |
| HPV66 | SA9 | 675 | 676 | TATTTTTTTAAACGTAGGCGCCGTAAACGTATTCCCTATTTTTTTGCAGATGGCGATGTGGCGGGCCTAGTGACAATAAGGTGTACCTACC | 1838 |
| HPV66 | SA3 | 677 | 678 | CAACATAAGTGTTACCTAATTCACGTACCTTGTTGTTGTAAGTGTGTGAGTTGGTGGTGCAGTTGGACATTCAGAGTACCAA | 1839 |
| HPV66 | SD2 | 679 | 680 | GCGCATCATCTAAATAAACTGCAATGCATCACCTGAAGTACAGATGGGGAGGGGATGGGGATGTGTGG | 1840 |
| HPV68 | SD3 | 681 | 682 | CCGGACAGGCGGCTATGGCAATATGGAAGTGGAAACTAACTCGGAGGTAACTGTAGCACCTAATATAAATGGGGGAGGATGGGGAAAATGAAGGGGAAAATGGCGACAGT | 1841 |
| HPV68 | SD1 | 683 | 684 | GTTACAATAGACTGTGTCTATTGCAGAAGGCAACTACAACGGACAGAGGTATATGAATTTGCCTTTAGTGACCTATGTGTAGTGTATAGAGACGGGGG | 1842 |
| HPV68 | SA4 | 685 | 686 | AATAACACATCCAATACTAACCCTGTAGAAGACAATAGGTGGCCGTATTTACATAGTAGACTAACCGTGTTTAAATTTC | 1843 |
| HPV68 | SA1 | 687 | 688 | ACCATAACTAATACAAAGTTATATATATTTATTGATAAGGTGCATGAGTTGCCTGAAACCATTGTGTCCAGCAGAAAAA | 1844 |
| HPV68 | SA6 | 689 | 690 | ATGGCAACATAATCCATTGTCCTGACTCTATGTGCAGTACCACTGACGGAAAAGTATCCACTACTGAATCTGTTGCCGAC | 1845 |
| HPV68 | SD5 | 691 | 692 | AAGACGGAGCCTTTGTTGTGGTGACACTACACCTATAGTGCATTTAAAAGGTGACAAAAATGGGATTAAAATGTCTTA | 1846 |

(continued)

| HPV type | Splice site | Forward primer SEQ ID NO | Reverse primer SEQ ID NO | Amplicon nucleic acid sequence | Amplicon SEQ ID NO |
|---|---|---|---|---|---|
| HPV68 | SA9 | 693 | 694 | TCCAATTATTATTTATTACCATTGTTATTCTTTTTATTAAAAAAACGTAA ACACCTTCCTTATTTTTTTACAGATGGCATTGTGGCGAGCTAGCGACAA CATGGTGTATTTGCCTCCCCCC | 1847 |
| HPV68 | SA3 | 695 | 696 | CGGGACGAACAACAGCGTCACAGAATTCAGTGTCTGTGTTGTAAGTGTA ACAAGGCACTGCAACTAGTAGTAGAAGCGTCGCGGGAC | 1848 |
| HPV68 | SD2 | 697 | 698 | ACCCAGTAATCTGCAATGGCCAATTGTGAAGGTACCGATGGGGACGGG ACGGGGTGTAACGGATGGTTTTTTGTAGAAGCAAT | 1849 |
| HPV73 | SD3 | 699 | 700 | GGACAGTGGATATGGCAATACTGAAGTGGAAACTTACGAGACAGAGGT ACCGGGACTTGGGGCAGGGGTAGGGTGTTTACAAAATG | 1850 |
| HPV73 | SD1 | 701 | 702 | ATACATGATATAAACCTGGACTGTGTGTTTTGCCAACGTGGACTGTACA GATCTGAGGTATATGATTTTGCATTTAGTGATTTGTGTATTGTATATAGA AAGGATAAACCATATG | 1851 |
| HPV73 | SA4 | 703 | 704 | ATCAAATACAAATCCTAAAGCAGATGATACTTGGAAATATTTACATAGT AGAATTAAGGTGTTTACTTTTTTAAATCCATT | 1852 |
| HPV73 | SA6 | 705 | 706 | ATGGGTGGTCAGGTAATATGTTGTGCTCCTGTATCTAGCGCCTGTGAAG TATCCATTCCTGAAATTGTTAACCCACTGCAC | 1853 |
| HPV73 | SD5 | 707 | 708 | CCTGTACCCAGTGTACTACACATAATGTTGCGCCAATAGTGCATTTAAA AGGTGACAAAAACAGCTTAAAATGTTTTAGATATAGATTGCATAAAGG CTATTCACATTTATTTAAAAA | 1854 |
| HPV73 | SA1 | 709 | 710 | AAATTTAACTAACAAACAGTTATGTAATATTTTAATAAGGTGCGGAAAA TGCCAAAAACCATTATGTCCACTGGAAAAGCAAAGCATGTAGATGAA AA | 1855 |
| HPV73 | SA9 | 711 | 712 | TATTATTTGTTAAAGCGCAAACGTAAACGTCTGTCATATTCTTTTACAGA TGTGGCGACCTACTGATGCAAAGGTATACCTGCCCCC | 1856 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV73 | SA10 | 713 | 714 | TGGCTGTGGGACACCCATATTTTCCTATCAAGGATTCTCAAAAACGTAA AACCATAGTTCCTAAAGTTTCAGGTTTG | 1857 |
| HPV73 | SA3 | 715 | 716 | CAGCCATCTAGACAGACAAGCTGAACGAGAGTGTTACAGAATAGTTAC TGACTGCACGAAGTGTCAGTGCACAGTATGCCTTGCCATTGAAAGCAAC AAAGCTGATTTAAGAGTGA | 1858 |
| HPV73 | SD2 | 717 | 718 | GCCCCAACTGTTCCAGAAACCTATAAAAGAAGATGGCTGATTCAGGTA ATTGGGAAGGGAGGTGTACGGGATGGTTTAATGTAGAAGCCATTGTAG | 1859 |
| HPV82 | SD3 | 719 | 720 | CACAAGTGGAGACTGTGGAAGGACCCTTACAGGTAGATGGGCAAAATG ACGGGTCACAACATAGTATGTGTAGTGGCGGGGGGAGC | 1860 |
| HPV82 | SD1 | 721 | 722 | ATTCAGGTATTGTGTGTATATTGTAAAAAGGAGTTGTGTAGAGCAGATG TGTATAATGTAGCATTTACAGAACTTAGGATTGTATATAGG | 1861 |
| HPV82 | SA4 | 723 | 724 | TTGTATGCCCACCATTGCTTATTACCTCAAATATCAATCCAAAAGAAGA TCCAAATTTAATGTATTTACATAGTAGAGTGACAGTATTTCAATTTTTAA ATGCATTTCCATTTGACCCCCAT | 1862 |
| HPV82 | SA6 | 725 | 726 | TATATGTGTGGCAATGTAATAACATGTCCTGAATATGTATCTAGTACCT ACAGCACCCCGTCACCCTCTACTACAACTG | 1863 |
| HPV82 | SD5 | 727 | 728 | GGAACTGCAGGCCCAAACACCGGAGGGCACCTCAGTGCAACTAAAACT GCGTTTATAGTTCATTTAAAAGGTGCAACAAATTGTTTAAAATGTTTAA GATACAGATTTGCAAAACATAGAAATTTGT | 1864 |
| HPV82 | SA1 | 729 | 730 | AGAGGCCATTACTAACAAAAGTTTATATGAATTATTAATAAGGTGTCAT AGATGTCAGAGACCACTTGGGCCTGAAGAAAG | 1865 |
| HPV82 | SA9 | 731 | 732 | CATATTTGTTACGCAAACGCCGTAAACGTATACCCTATTTTTTTGCAGAT GGCTTTGTGGCGTACTAATGACAGCAAAGTGTATTTACCACCTGCACC | 1866 |

| HPV type | Splice site | Forward primer SEQ ID NO | Reverse primer SEQ ID NO | Amplicon nucleic acid sequence | Amplicon SEQ ID NO |
|---|---|---|---|---|---|
| HPV82 | SA3 | 733 | 734 | CAGCCAGCCAGACAAGCTGGACAGGATACGTGTTACAGAATTAAAGTG CACTGTTGCAGGTGTTCGAGTGTTGTACAGCTCGCAGTGGAAAGCAGTG GAGACAGCC | 1867 |
| HPV82 | SD2 | 735 | 736 | CATCGGCAATGGACAGTGAAGGTACAGAGGATGAGGGGGCGGGGTGTA CCGGGTGG | 1868 |

Table 2C

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV16 | ctrl1 | AACGTGTTGCGATTGGTGTATTG | 737 | CATTCCCCATGAACATGCTAAACTTTG | 738 |
| HPV16 | ctrl2 | CGTGCTTTTGCTTTGCTTTGT | 739 | GAGGCTGCTGTTATCCACAATAGTAAT | 740 |
| HPV16 | ctrl3 | CCTGTGTAGGTGTTGAGGTAGGT | 741 | TCTATTATCCACACCTGCATTTGCT | 742 |
| HPV16 | ctrl4 | CCAGGCCCATTTTGTAGCTT | 743 | AGGTCAGGAAAACAGGGATTTGG | 744 |
| HPV18 | ctrl1 | TGGAGTAAACCCAACAATAGCAGAAG | 745 | CATTTGTAACGCAACAGGGCTAAT | 746 |
| HPV18 | ctrl2 | CGTATGCATGGGTATTGGTATTTGTG | 747 | CATGTATATGCAATAGTAACATGGGCAA | 748 |
| HPV18 | ctrl3 | GAGGACGTTAGGGACAATGTGT | 749 | CCCTGTGATAAAGGACGCGATTT | 750 |
| HPV18 | ctrl4 | CGCCCTAGTGAGTAACAACTGTATTT | 751 | GGAGGATTGTAGGATAAAATGGATGCT | 752 |
| HPV31 | ctrl1 | GTGAAACACCAGAATGGATAGAAAGAC | 753 | TGCACATGCATTACTATCACTGTCA | 754 |
| HPV31 | ctrl2 | GCATTGTGCTATGCTTTTGCTTTG | 755 | ACAACGTAATGGAGAGGTTGCAATA | 756 |
| HPV31 | ctrl3 | GCTTAGTTTGGGCCTGTGTT | 757 | ACCACCGGCATATCTATTAGAGTTTTC | 758 |
| HPV31 | ctrl4 | TGTGTGTGTTGTGTATGTTGTCCTT | 759 | CAACTTTTACTATGGCGTGACACCTA | 760 |
| HPV33 | ctrl1 | CGGAGCCAAACATGTGCATTG | 761 | CGTTATCATATGCCCACTGTACCATT | 762 |
| HPV33 | ctrl2 | CCATTTCTACCTATGCTTGGTTGCT | 763 | GTTGTGTCATATGCTGTGCATGAAA | 764 |
| HPV33 | ctrl3 | CATGTGTGTAGGCCCTTGAAATAGGTAGAG | 765 | CCTATTATCAGCACCCGGTTGT | 766 |
| HPV33 | ctrl4 | CTTGCCCTACCCTGCATTG | 767 | CGGTTAGGCATACAAAATGGAGGAAAT | 768 |
| HPV35 | ctrl1 | GCTATGTATTTCAGCTGCAAGTATGCT | 769 | CATTCTGGTGTTTCTCCATCAACCT | 770 |
| HPV35 | ctrl2 | CGTTCGCTATTGCTATCTGTGTCATTA | 771 | GCCAAATATTGTGCATGAGCGTTAATC | 772 |
| HPV35 | ctrl3 | GGTACAGATAACACAGGGAATGCATTTCT | 773 | GACATTCTCCTGCTTTTACCTGGTTA | 774 |
| HPV35 | ctrl4 | AACATTCCTACCTCAGCAGAACAC | 775 | TGGGTGGACCACAAGTATGAAAA | 776 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV39 | ctrl1 | AGGGTTACTGTAGGAAAGGGATTAAGT | 777 | CGTATCCCCTGTTACCACACTAATATTG | 778 |
| HPV39 | ctrl2 | TTGGTGTGGTTTGGTGTGTGTATAT | 779 | CTCCAATGGTGTGGTACGTATAAGAA | 780 |
| HPV39 | ctrl3 | CCAGCCATTGGGTGTTGGTA | 781 | GCCTATAATGCACAACTGTGTCTGTT | 782 |
| HPV39 | ctrl4 | CATTTTGTGGCGACCGAAGT | 783 | CCTGGACAGGATGATGAGTAATAAGG | 784 |
| HPV45 | ctrl1 | GCAACGTTATACGCCCATATCCAAT | 785 | GGTACGTGCAACAATGTGCTTAA | 786 |
| HPV45 | ctrl2 | TGCTTTTGCTTGGTTGTTGGT | 787 | CATCACAGGTATGTTACACTGTACTGT | 788 |
| HPV45 | ctrl3 | GGCATGTGTAGGTATGGAAATTGGT | 789 | ACATCCTGCGTAATAACAGCTGTAG | 790 |
| HPV45 | ctrl4 | ATTTCGGTTGCCTGTGGCTTATA | 791 | CAGTTGTGCAAGCCATTGTTTTAGT | 792 |
| HPV51 | ctrl1 | GATGGAGGCAACTGGAGAGAAATT | 793 | GTGTTTGGTGGGCCATATATGACTAT | 794 |
| HPV51 | ctrl2 | AAGCCAATATGTGCTGCTAATTGTA | 795 | AACACGTATTGGGACAGCAGTAG | 796 |
| HPV51 | ctrl3 | ACACCCCTCCACAGGCTAA | 797 | TGTACGCCAACCTGCAACAA | 798 |
| HPV51 | ctrl4 | GGGTATTACATTATCCCCGTAGGTCAA | 799 | GCTGCAGCTGTAACAAAATGGAA | 800 |
| HPV52 | ctrl1 | CACCATCAGTTGCAGAAGGATTAAAAG | 801 | CTGTGACATTAGTTTGGACACTGTT | 802 |
| HPV52 | ctrl2 | CAACACAAGCCAATATTGCTGCTA | 803 | CCTGCGCATACACCGATATAGAT | 804 |
| HPV52 | ctrl3 | GGACTATATGTTTTGGGAGGTGGATTT | 805 | GATGCAGGGCGTTTTAGTTTGG | 806 |
| HPV52 | ctrl4 | TCGGTTGGTCTTGGCACAA | 807 | TTTAGGCGGGACAACAAGTGT | 808 |
| HPV56 | ctrl1 | CAGATGATAGCCAAATTGCGTTTCA | 809 | GCTGTTGTGCCCTTTTATAATGTCTAC | 810 |
| HPV56 | ctrl2 | TGCTACGCATATATATTGCAACCATTGA | 811 | GGATGTGGCTATAACAAACCAAAACAAT | 812 |
| HPV56 | ctrl3 | TGTACTCCCGCTATGGGTGAA | 813 | GTGTCTATCATGTCCCCATCCTCTA | 814 |

EP 3 715 477 A1

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV56 | ctrl4 | AATTCGGTTGCATGGCCTAGT | 815 | GGGTGCGGTACTGTACATAATTCAAG | 816 |
| HPV58 | ctrl1 | CAATGGGACAATGGATACAAAGTAGGT | 817 | GGGCCACACAGTAACATACAACT | 818 |
| HPV58 | ctrl2 | TCTATATATGCTTGGTTGCTGGTGTTG | 819 | CATGTGCAGAACCAGTATACAGTTAGT | 820 |
| HPV58 | ctrl3 | CGTTTGGTCTGGGCATGTGTA | 821 | GCTGTGCGGGATATCTGTTACTG | 822 |
| HPV58 | ctrl4 | TCTATGAGTAAGGTGCTGTCCCTAAAT | 823 | GGAGGTAAAGTAAAATGGAGGCAGTA | 824 |
| HPV59 | ctrl1 | GTGCATGTTAATTGAACCACCCAAA | 825 | TCAAACACGCTATCATCAACTCCAT | 826 |
| HPV59 | ctrl2 | GTTGCAATGTCCCGCTTCTG | 827 | CATGGGCATATAGTAGTAACAGTGGAA | 828 |
| HPV59 | ctrl3 | GCTGTGTACCTGCCATTGGA | 829 | CTGTGTCTACCATATCACCATCTTCA | 830 |
| HPV59 | ctrl4 | GGTTGCACCCAATGAGTAAGGTA | 831 | GCAAAACTGGACATTCAGGACAAAA | 832 |
| HPV66 | ctrl1 | AGACATAGATAGCAATGCACAAGCA | 833 | ATCACCCCCTTCATCTACTTTACTACA | 834 |
| HPV66 | ctrl2 | GTTTGTCTGTGTGTGTGCCATT | 835 | GCATGGCAATATATACACAGTGTAGGT | 836 |
| HPV66 | ctrl3 | GTAGGCCGAGGTCAACCTTTA | 837 | GTGCACATCCCACAATACATAACTG | 838 |
| HPV66 | ctrl4 | GGTTAGGTGGTGTTCCTTACTGTTTA | 839 | CAAAAGGCTAGGCAACCGAATT | 840 |
| HPV68 | ctrl1 | CGACACGCCGGAATGGATAA | 841 | CGCTGCAGCATTACTATTACAATCTG | 842 |
| HPV68 | ctrl2 | GGTGTGGTTTTGTGTATGCATGT | 843 | GGTATACAGCAAACACCTCAAATGGT | 844 |
| HPV68 | ctrl3 | GCCTGTGTTGGTGTTGAAATAGGTA | 845 | TGCAACATTGTCCCTACTGTCTTTAG | 846 |
| HPV68 | ctrl4 | CCCTGTGACTAACATATGTCCTTGT | 847 | CCACACGGTATAGTTTGCAACCAT | 848 |
| HPV73 | ctrl1 | GAACGCATGTTAATTGAACCTCCAA | 849 | GCTGCACTAACGTTTGTCTTTTAATCC | 850 |
| HPV73 | ctrl2 | TCGCTTGCAGTGTCTGTGTATATTT | 851 | CATGGTAATGTACAAGTGCCATAGGA | 852 |
| HPV73 | ctrl3 | TGTATTTTAGGTTGTAGGCCTCCCTTA | 853 | CTCCAAAGCCAACATCTATCATATCAC | 854 |
| HPV73 | ctrl4 | GTCGCCATTTTACATGCATTAAGGT | 855 | AGGAAACAAACCCTGCCAAGTT | 856 |
| HPV82 | ctrl1 | CGTAGTACAGCCGTTGCATTG | 857 | CCCATTGTACCATTTGCGATAGTT | 858 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV82 | ctrl2 | GCTGCTAAGTGTATATAGTTACTCGCA | 859 | CTGCTGCAAACACATATTGGGATT | 860 |
| HPV82 | ctrl3 | GGATGTGTTGGTGTTGAAGTAGGTA | 861 | TCCTGTTGGTCGTTGCCATT | 862 |
| HPV82 | ctrl4 | CCTGTAGGTTAAGGGTGGTGTT | 863 | AAATCGGTCGCCACAAAATGG | 864 |

Table 2Cbis

| HPV type | Region name | Forward primer SEQ ID NO | Reverse primer SEQ ID NO | Amplicon nucleic acid sequence | Amplicon SEQ ID NO |
|---|---|---|---|---|---|
| HPV16 | ctrl1 | 737 | 738 | CTGCATTTGGACTTACACCCAGTATAGCTGACAGTATAAAAACACTATTACAACAATATTGTTTATATTACACATT | 1869 |
| HPV16 | ctrl2 | 739 | 740 | GTGCTTTTGTGTGTCTGCCTATTAATACGTCCGCTGCTTTTGTCTGTGTCTACATACACATCATTAATAATATTGGT | 1870 |
| HPV16 | ctrl3 | 741 | 742 | CGTGGTCAGCCATTAGGTGTGGGCATTAGTGGCCATCCTTTATTAAATAAAATTGGATGACACAGAAAATGCTAGTGCTTATGC | 1871 |
| HPV16 | ctrl4 | 743 | 744 | CAACCGAATTCGGTTGCATGCTTTTTGGCACAAAATGTGTTTTTTAAATAGTTCTATGTCAGCAACTATGGTTAAACTTGTACGTTTCCTGCTTGCCATGCGTG | 1872 |
| HPV18 | ctrl1 | 745 | 746 | GATTTAAAACACTAATACAGCCATTTATATTATATGCCCATATTCAATGTCTAGACTGTAAATGGGGAGTATTAAT | 1873 |
| HPV18 | ctrl2 | 747 | 748 | TATATTGTGGTAATAACGTCCCCTGCCACAGCATTCACAGTATATGTATTTTGTTTTTTA | 1874 |
| HPV18 | ctrl3 | 749 | 750 | CTGTAGATTATAAGCAGACAGACACAGTTATGTATTTGGGCTGTGCCCCTGCTATTGGGGAACACTGGGCTAAAGGCACTGCTTGT | 1875 |
| HPV18 | ctrl4 | 751 | 752 | GTGTTTGTTGGTATGGGTGTTGCTGTTGGGCTATATATTGTCCTGTATTTCAAGTTATAAAAACTGCACACCTTAC | 1876 |
| HPV31 | ctrl1 | 753 | 754 | AAACAGTATTACAGCATAGTTTTAATGACACAACATTTGATTTGTCCCAAATGGTACAATGGGCATATGACAATGATGTTATGGATGATAGTGAAATTGCCTATAAATATGCACAATTAGC | 1877 |
| HPV31 | ctrl2 | 755 | 756 | CTTTTGTGTGCTACTATTTGTGTGTCTTGTCATACGTCCCACTTGTGCTGTCTGTGTCGGTATATGCAACACTACTATTAATTGTGATTTTATGGGT | 1878 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV31 | ctrl3 | 757 | 758 | GGTTTAGAGGTAGGTCGCGGGCAGCCATTAGGTGTAGGTATTAGTG GTCATCCATTATTAAATAAATTTGATGACACT | 1879 |
| HPV31 | ctrl4 | 759 | 760 | ATATACACCCTATTAGTAACATACTATTACTATTTTATAAACTATTG TTCCTACTTGTTCCTACTTGTTCCTGCTCCTCCCAATAGTCATGTACT TATTTCTGCCTATAATT | 1880 |
| HPV33 | ctrl1 | 761 | 762 | TATTGGTTTAGAACAGCAATGTCAAACATTAGTGATGTACAAGGTA CAACACCTGAATGGATAGATAGACTAACTGTTTTACAACATAGCTT TAATGATAATATATTTGATTTAAGTGA | 1881 |
| HPV33 | ctrl2 | 763 | 764 | GGTGTTGGTATTGCTGCTTTGGGTGTTTGTGGGATCTCCTTTAAAAA TTTTTTTTTGCTATTTGTTGTTTTTATATTTACCAATGATGTGTATTA AT | 1882 |
| HPV33 | ctrl3 | 765 | 766 | GGCAGCCATTAGGCGTTGGCATAAGTGGTCATCCTTTATTAAACAA ATTTGATGACACTGAAACCGGTAACAAGTATCCTGG | 1883 |
| HPV33 | ctrl4 | 767 | 768 | CAATGTACCTACCTTTATTTCCCTATATTGTAGTACCTACATGTTTA GTATTGCTTTACCTTTTGACATACTAGTGTCCATATTGTACA | 1884 |
| HPV35 | ctrl1 | 769 | 770 | AATACAACCACCAAAATTACGTAGTACCCCAGCTGCGTTATATTGG TTTAAAACAGCAATGTCAAATATTAGTG | 1885 |
| HPV35 | ctrl2 | 771 | 772 | TACTCAGCATTAATATTACTGGTTTTAATACTGTGGGTTACTGTAGC AACACCACTACGTTGCTTTTGTTGTTTTCTTTGCTTTTTGTATATACC TATGGGAAT | 1886 |
| HPV35 | ctrl3 | 773 | 774 | ATGGATTATAAACAAACACAATTGTGTTTAATAGGTTGTAGGCCTC CTATAGGTGAACATTGGGGAAAAGGCACACCTTGTAATGC | 1887 |
| HPV35 | ctrl4 | 775 | 776 | TTAATCCTTGTGTTCCTGATATATATTGTTTGCCAACTTTATATTGGC TTTTGCCAATCTTTAAACTTGATTCATCTTGCAGTATTAGTCAT | 1888 |

EP 3 715 477 A1

106

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV39 | ctrl1 | 777 | 778 | ACATTGTTACATGTTCCAGAAAGTTGTATGCTTCTGGAGCCTCCTAAACTGCGCGAGCCCCTGTAGCAGCACTATATTGGTATCGCACAGGTATATC | 1889 |
| HPV39 | ctrl2 | 779 | 780 | ATATATGTTGCAAATGTCCCGCTTTGCCGTCTGTGCATGTGTGTGCGTATGTGTGGATAATTGTGTTGTGTTTA | 1890 |
| HPV39 | ctrl3 | 781 | 782 | TTAGTGGACACCCATTATATATAGACAGGATGATGATACTGAAAACTCACCATTTTCATCAACCACCACCAATAAGGACACAGTAGGGATAATGTGTCTGTGGATTATA | 1891 |
| HPV39 | ctrl4 | 783 | 784 | CGGTCGTCGGGTTGAGCATTTTTTTTTAAACTAGTGGAAACCACCTTTCTCAGCAAAAACATGTCTTACCTTAGGTTCACCCTGCATAGTTGGCACTGGTAACAGTTTTACTGGCGCG | 1892 |
| HPV45 | ctrl1 | 785 | 786 | GTTTAGATTGTAAATGGGGAGTATTAATATTAGCTTTATTAAGATATAAATGTGGCAAAAATAGACTAACGTGTTGCAAAAGGC | 1893 |
| HPV45 | ctrl2 | 787 | 788 | GTTTCTTTTTATAGTTGTTATTACATCCCCATTAACAGCATTTGCTGTATACATTTGGTCTATTTGCTATTTACTGTGTTTGTTATTACATATGCATGCTTTACACACCATACCAATAATTACTATAATGT | 1894 |
| HPV45 | ctrl3 | 789 | 790 | CGTGGGCAGCCTTTAGGTATTGGCCTAAGTGGCCATCCATTTTATAATAAATTGGATGATACAGAAAGTGCTCATGCAG | 1895 |
| HPV45 | ctrl4 | 791 | 792 | TGTGACCTTTTAAACATAATACCTAAACTGGCACATTTACAACCCCTACATAGTTTAAACCTACTGGCGGGCGGCCTTCTTGGGCGTACATGTGGCACACCTGGTATTAGTCATTTCCTGTCCAGGTGT | 1896 |
| HPV51 | ctrl1 | 793 | 794 | GCTAAATTTTTAAGATATCAAGGTGTAAACTTTATGTCCTTTATTCAAATGTTTAAACAGTTTTTAAAAGGAACACCAAAAACACAATTGC | 1897 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV51 | ctrl2 | 795 | 796 | TAGACATATTGTAACCATTGCAGTGTTTATTATTTGCTATTTGTGC TTTGCTTGTGTGTGTCTTGTGTCTTGTGTGTTGTTGTTGTTGCCG | 1898 |
| HPV51 | ctrl3 | 797 | 798 | GCCAGATCCTTGGCCAAATATAAATTTTGGGATGTTGATTTAAAG GAACGGATTTTCTTTAGATTTAGACCAATTTGCATTGGGTCGCAAGTT T | 1899 |
| HPV51 | ctrl4 | 799 | 800 | GGGTGGTGGTGTTTCGGGTGGCGGTCCCTATTGCCCTACCCATTTTTTGCAG CACAACAGTTTATATTTGTGCTATTTAGTTATACTTTGTAGC | 1900 |
| HPV52 | ctrl1 | 801 | 802 | TATTAATACAGCCCTATAGCATATATGCCCATTTGCAATGTTTAACA TGTGACAGAGAGGCGTGCTTATACTGCTGCTAATTAGGTTTAAATGTG GAAAAAACAGATT | 1901 |
| HPV52 | ctrl2 | 803 | 804 | TTGTGTATATAACAATGTTAGGATTATTTGTATTTTGTTTTTATTTT GCTTATGGTGTTTTGTTGCAGTGCTTAGGCGCGCTCTTGCT | 1902 |
| HPV52 | ctrl3 | 805 | 806 | AAAAGAAAAGTTTTCTGCAGATTAGATCAGTTTCCTTTAGGTAGG AAGTTTTTGTTACAGGCAGGGCTACAGGCTAGGC | 1903 |
| HPV52 | ctrl4 | 807 | 808 | CTTTGGTTGTGTCCTTGGCACAGTAACAACTATTTTATATAAGTTTCA GCAAACTGCTTAATCCTTTGGTTTCCTGCAGTCCACTGGTCT | 1904 |
| HPV56 | ctrl1 | 809 | 810 | ATATGCACAATTAGCAGATGTAGACAGCAATGCACAAGCCTTTTA AAAAGCAATATGCAGGCAAAATATGTAAAGGATTGTGGAATAATG T | 1905 |
| HPV56 | ctrl2 | 811 | 812 | TTTTTGTGTTATTGGTGTGTTGCGCCTTTGCGCTTTGTGTTTGTTTGCTT GTGTGTCATGTTGTCCCGCTTTTGCTATCTGCCCTCTGTGTTTTCCAGT TGTATATTATTAATAAT | 1906 |
| HPV56 | ctrl3 | 813 | 814 | CATTGGACTAAAGGTGCTGTGTGTAAGTCCACACACAAGTTACCACCAG GGGACTGCGCCCGCCTCTTGCATTAATTAATACACCTA | 1907 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV56 | ctrl4 | 815 | 816 | GCCATTATTTAAACTAAAAGGAATTCGGTTGCATGGCCTAGTGCCATTATTTAAACCAAAAGGCCCTTTTCAGCAGAACAGTTAATCCTTTGGCATATTGCCGTTTCCTGTGTTTTATA | 1908 |
| HPV58 | ctrl1 | 817 | 818 | GTGAAAAAACAAATGATGGAGGTAATTGGAGACCAATAGTACAATTTTTAAGATATCAAAATATTGAATTTACAGCATTTTTAGTTGCATTTAAACAGTTTTTACAAGGTGTACCAAAAAAA | 1909 |
| HPV58 | ctrl2 | 819 | 820 | GTGTTGCTGCTTTGGGTGTCTGTGGGGTCGGCTCTACGAATTTTTTTCTGTTACTTAATATTTTTATATATACCAATGATGTGTATTAATTTTCATGCACAATACTTAACCCAACAAG | 1910 |
| HPV58 | ctrl3 | 821 | 822 | GGCCTTGAAATAGGTAGGGGACAGCCATTGGGTGTTGGCGTAAGTGGTCATCCTTATTTAAATAAATTTGATGACACTGAAAC | 1911 |
| HPV58 | ctrl4 | 823 | 824 | TGCCCTACCCTGCCCTGCCTATTATGCATACCTATGTAATAGTATTTGTATGATATGTATTTTATAGTTTTTAACAG | 1912 |
| HPV59 | ctrl1 | 825 | 826 | TTGCGTAGTGGTGTTGCAGCACTATATTGGTACAGAACAGGAATGTCCAATATTAGTGAAGTTATAGGGGAAACGCCCGAATGGATACAAAGACTAACAATTATACAAC | 1913 |
| HPV59 | ctrl2 | 827 | 828 | CAATCTGTCTATATGTGTGCATATACATGGTTACTAGTATTTGTGTATATTGTGGTTATCACCTCCTCATATGAGTGTTTTTTACTATATATATTGTTTTTTATAA | 1914 |
| HPV59 | ctrl3 | 829 | 830 | GAACACTGGACAAAGGGCACTGCTTGTAAGCCTACTACTGTGGTTCAGGGCGATTGTCCTCCACTAGAATTAATAAATACACCAAT | 1915 |
| HPV59 | ctrl4 | 831 | 832 | CTGTCCCTTTATTGTTTCTTTGTCCTTATTACACATTATTACACATTGCCCTACTTACATAGGTGTGTTTGTTCCTTCA | 1916 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV66 | ctrl1 | 833 | 834 | TTTTTAAAAAGTAATATGCAAGCAAAATATGTAAAGGATTGTGGAATAATGTGTAGACATTACAAAAGGGCACAGCAACAGCAAATGAATATGTGCCAGTGGATAAAGCATATA | 1917 |
| HPV66 | ctrl2 | 835 | 836 | TTGTCCCGCTTTTGCTATCTGCATCTTTATTTACAAGTTGTCTTATACTAATTATTTTATTTTGGTTTGTTGTGGCTACATCATTTTTTGATACTTTTATACTGTTTTTACTATTTTTTTATAT | 1918 |
| HPV66 | ctrl3 | 837 | 838 | GGTGCTGGGTTAAGTGGTCATCCATTATTTAATAGGCTGGATGACACTGAGGTCTCTAATTTAGCAGGTAATAATGTTATAGAAGATAGCCGGGACAATATATCTGTTGATTGTAAACAAACC | 1919 |
| HPV66 | ctrl4 | 839 | 840 | ATGTTATATTAAATAGGTTGTTTGTATGCACTATAGTAACACACCAAACTCCATTTTAGTGCTGTACGCCATTTTATGCATGCAACCG | 1920 |
| HPV68 | ctrl1 | 841 | 842 | AAAGATTAACCATAATACAACATGGAATAGATGATAGTGTATTTGATTTATCAGACATGGTACAATGGGCATTTGATAATGAGTTAACAGATGACAGTGATATAGCATTTCAATATGCTATGTTAG | 1921 |
| HPV68 | ctrl2 | 843 | 844 | ATATATGTTGCACTGTCCCGCTTTTGCAGTCTGTGCATGTGTGTGTGTATGTGTGGATATTTGTGTTTGTGTTTATATTAGTTAGAACTAC | 1922 |
| HPV68 | ctrl3 | 845 | 846 | GGGGGCAGCCATTGGGCGTTGGCCTTAGTGGGCATCCACTATATAATAGGCTGGATGATACTGAAAATTCCCCGTTTTCCTCTAATAAAACTC | 1923 |
| HPV68 | ctrl4 | 847 | 848 | TTTACATATAATAGGACTGCAACATTTCATACATAATTTGTAGCCCTACCCTAAGGTGTGTTACATTATATGCAATATATTT | 1924 |
| HPV73 | ctrl1 | 849 | 850 | GACTACGAAGTACACCATGTGCATTATATTGGTATAGAACTAGTTTATCAAATATTAGTGAAATAGTAGGAGACACACCTGAGT | 1925 |
| HPV73 | ctrl2 | 851 | 852 | ACCCATGGTTATTGGTATTGATTATAATAACCTTTATACATGTATCACAATCATTGTTAAAAGTATTTTTTTTATATGTTTTGGTATTTTATAT | 1926 |

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV73 | ctrl3 | 853 | 854 | GGGGAACATTGGGGTCCAGGCACGCCATGTACTTCACAAACTGTTAATACTGGTGATTGTCCCCCACTGGAATTAAAGAACACCCCTATACAGGATG | 1927 |
| HPV73 | ctrl4 | 855 | 856 | AAAAAGGGCAACCGATTTCGGTTGCACAGTAAAACATGTTTTAATGTGTTTTGCTGTTGTAGCAAAATAGTTGTACTGTTTTTGGCTTCCTGCAGGC | 1928 |
| HPV82 | ctrll | 857 | 858 | TACTTTTATAGAACAGGAATATCAAACATTAGTAGCACATATGGCGAAACACCAGAATGGATTACAAGACAAACACAACTACAGCACAGTTTTGATGATAGCACGTTTG | 1929 |
| HPV82 | ctrl2 | 859 | 860 | ACCATTGCGGTGTTTTTGGTGTGTTTATTTGTGCTTTGCGTGTGTGTGTGTCTTGTGTTGTGTTGTTTGTTGCCGCTATTGC | 1930 |
| HPV82 | ctrl3 | 861 | 862 | GGGGTCAGCCGTTAGGTGTTGGCCTTAGTGGTCATCCTTTATTTAATAAGTATGATGATACTGAAAACTCTAGGTTTGCC | 1931 |
| HPV82 | ctrl4 | 863 | 864 | TAGGTGGCGTCCCTATTGCCCTACCCATATTTGTGGCTTGCAGCACACTTGTATATATATGTTCTTGCTGTATTGCATGTACCACAGGATTCCATTTTGTTTTTTCCTGCAG | 1932 |

EP 3 715 477 A1

111

Table 2D

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV16 | bkpt1-MYC_001_exon1 | AGTACAGACCTACGTGACCATATAGAC | 865 | CTGAGAAGCCCTGCCCTTC | 866 |
| HPV16 | bkpt1-MYC_001_exon2 | AGTACAGACCTACGTGACCATATAGAC | 867 | AAATACGGCTGCACCGAGT | 868 |
| HPV16 | bkpt1-MYC_001_exon3 | AGTACAGACCTACGTGACCATATAGAC | 869 | GGTGATCCAGACTCTGACCTTTTG | 870 |
| HPV16 | bkpt1-PVT1_002_exon3 | AGTACAGACCTACGTGACCATATAGAC | 871 | ATCATGATGGCTGTATGTGCCA | 872 |
| HPV16 | bkpt1-PVT1_004_exon1 | AGTACAGACCTACGTGACCATATAGAC | 873 | CATGGTTCCACCAGCGTTATT | 874 |
| HPV16 | bkpt1-PVT1_005_exon1 | AGTACAGACCTACGTGACCATATAGAC | 875 | TCTTTGCTCGCAGCTCGT | 876 |
| HPV16 | bkpt2-MYC_001_exon1 | GCTCACACAAAGGACGGATTAAC | 877 | CTGAGAAGCCCTGCCCTTC | 878 |
| HPV16 | bkpt2-MYC_001_exon2 | GCTCACACAAAGGACGGATTAAC | 879 | AAATACGGCTGCACCGAGT | 880 |
| HPV16 | bkpt2-MYC_001_exon3 | GCTCACACAAAGGACGGATTAAC | 881 | GGTGATCCAGACTCTGACCTTTTG | 882 |
| HPV16 | bkpt2-PVT1_002_exon3 | GCTCACACAAAGGACGGATTAAC | 883 | ATCATGATGGCTGTATGTGCCA | 884 |
| HPV16 | bkpt2-PVT1_004_exon1 | GCTCACACAAAGGACGGATTAAC | 885 | CATGGTTCCACCAGCGTTATT | 886 |
| HPV16 | bkpt2-PVT1_005_exon1 | GCTCACACAAAGGACGGATTAAC | 887 | TCTTTGCTCGCAGCTCGT | 888 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV16 | SD2-MYC_001_exon1 | GGAATTGTGTGCCCCATCTGT | 889 | CTGAGAAGCCCTGCCCTTC | 890 |
| HPV16 | SD2-MYC_001_exon2 | GGAATTGTGTGCCCCATCTGT | 891 | AAATACGGCTGCACCGAGT | 892 |
| HPV16 | SD2-MYC_001_exon3 | GGAATTGTGTGCCCCATCTGT | 893 | GGTGATCCAGACTCTGACCTTTTG | 894 |
| HPV16 | SD2-PVT1_002_exon3 | GGAATTGTGTGCCCCATCTGT | 895 | ATCATGATGGCTGTATGTGCCA | 896 |
| HPV16 | SD2-PVT1_004_exon1 | GGAATTGTGTGCCCCATCTGT | 897 | CATGGTTCCACCAGCGTTATT | 898 |
| HPV16 | SD2-PVT1_005_exon1 | GGAATTGTGTGCCCCATCTGT | 899 | TCTTTGCTCGCAGCTCGT | 900 |
| HPV18 | bkpt1-MYC_001_exon1 | AATGACAGTAAAGACATAGACAGCC AAA | 901 | CTGAGAAGCCCTGCCCTTC | 902 |
| HPV18 | bkpt1-MYC_001_exon2 | AATGACAGTAAAGACATAGACAGCC AAA | 903 | AAATACGGCTGCACCGAGT | 904 |
| HPV18 | bkpt1-MYC_001_exon3 | AATGACAGTAAAGACATAGACAGCC AAA | 905 | GGTGATCCAGACTCTGACCTTTTG | 906 |
| HPV18 | bkpt1-PVT1_002_exon3 | AATGACAGTAAAGACATAGACAGCC AAA | 907 | ATCATGATGGCTGTATGTGCCA | 908 |
| HPV18 | bkpt1-PVT1_004_exon1 | AATGACAGTAAAGACATAGACAGCC AAA | 909 | CATGGTTCCACCAGCGTTATT | 910 |
| HPV18 | bkpt1-PVT1_005_exon1 | AATGACAGTAAAGACATAGACAGCC AAA | 911 | TCTTTGCTCGCAGCTCGT | 912 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV18 | bkpt2-MYC_001_exon1 | CAGCTACACCTACAGGCAACAA | 913 | CTGAGAAGCCCTGCCCTTC | 914 |
| HPV18 | bkpt2-MYC_001_exon2 | CAGCTACACCTACAGGCAACAA | 915 | AAATACGGCTGCACCGAGT | 916 |
| HPV18 | bkpt2-MYC_001_exon3 | CAGCTACACCTACAGGCAACAA | 917 | GGTGATCCAGACTCTGACCTTTTG | 918 |
| HPV18 | bkpt2-PVT1_002_exon3 | CAGCTACACCTACAGGCAACAA | 919 | ATCATGATGGCTGTATGTGCCA | 920 |
| HPV18 | bkpt2-PVT1_004_exon1 | CAGCTACACCTACAGGCAACAA | 921 | CATGGTTCCACCAGCGTTATT | 922 |
| HPV18 | bkpt2-PVT1_005_exon1 | CAGCTACACCTACAGGCAACAA | 923 | TCTTTGCTCGCAGCTCGT | 924 |
| HPV18 | SD2-MYC_001_exon1 | TGCATCCCAGCAGTAAGCAA | 925 | CTGAGAAGCCCTGCCCTTC | 926 |
| HPV18 | SD2-MYC_001_exon2 | TGCATCCCAGCAGTAAGCAA | 927 | AAATACGGCTGCACCGAGT | 928 |
| HPV18 | SD2-MYC_001_exon3 | TGCATCCCAGCAGTAAGCAA | 929 | GGTGATCCAGACTCTGACCTTTTG | 930 |
| HPV18 | SD2-PVT1_002_exon3 | TGCATCCCAGCAGTAAGCAA | 931 | ATCATGATGGCTGTATGTGCCA | 932 |
| HPV18 | SD2-PVT1_004_exon1 | TGCATCCCAGCAGTAAGCAA | 933 | CATGGTTCCACCAGCGTTATT | 934 |
| HPV18 | SD2-PVT1_005_exon1 | TGCATCCCAGCAGTAAGCAA | 935 | TCTTTGCTCGCAGCTCGT | 936 |

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV31 | bkpt1-MYC_001_exon1 | CAACGTTTAAATGTGTGTCAGGACAAA | 937 | CTGAGAAGCCCTGCCCTTC | 938 |
| HPV31 | bkpt1-MYC_001_exon2 | CAACGTTTAAATGTGTGTCAGGACAAA | 939 | AAATACGGCTGCACCGAGT | 940 |
| HPV31 | bkpt1-MYC_001_exon3 | CAACGTTTAAATGTGTGTCAGGACAAA | 941 | GGTGATCCAGACTCTGACCTTTTG | 942 |
| HPV31 | bkpt1-PVT1_002_exon3 | CAACGTTTAAATGTGTGTCAGGACAAA | 943 | ATCATGATGGCTGTATGTGCCA | 944 |
| HPV31 | bkpt1-PVT1_004_exon1 | CAACGTTTAAATGTGTGTCAGGACAAA | 945 | CATGGTTCCACCAGCGTTATT | 946 |
| HPV31 | bkpt1-PVT1_005_exon1 | CAACGTTTAAATGTGTGTCAGGACAAA | 947 | TCTTTGCTCGCAGCTCGT | 948 |
| HPV31 | bkpt2-MYC_001_exon1 | CAGCTGCATGCACAAACCA | 949 | CTGAGAAGCCCTGCCCTTC | 950 |
| HPV31 | bkpt2-MYC_001_exon2 | CAGCTGCATGCACAAACCA | 951 | AAATACGGCTGCACCGAGT | 952 |
| HPV31 | bkpt2-MYC_001_exon3 | CAGCTGCATGCACAAACCA | 953 | GGTGATCCAGACTCTGACCTTTTG | 954 |
| HPV31 | bkpt2-PVT1_002_exon3 | CAGCTGCATGCACAAACCA | 955 | ATCATGATGGCTGTATGTGCCA | 956 |
| HPV31 | bkpt2-PVT1_004_exon1 | CAGCTGCATGCACAAACCA | 957 | CATGGTTCCACCAGCGTTATT | 958 |
| HPV31 | bkpt2-PVT1_005_exon1 | CAGCTGCATGCACAAACCA | 959 | TCTTTGCTCGCAGCTCGT | 960 |

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV31 | SD2-MYC_001_exon1 | AATCGTGTGCCCCAACTGT | 961 | CTGAGAAGCCCTGCCCTTC | 962 |
| HPV31 | SD2-MYC_001_exon2 | AATCGTGTGCCCCAACTGT | 963 | AAATACGGCTGCACCGAGT | 964 |
| HPV31 | SD2-MYC_001_exon3 | AATCGTGTGCCCCAACTGT | 965 | GGTGATCCAGACTCTGACCTTTTG | 966 |
| HPV31 | SD2-PVT1_002_exon3 | AATCGTGTGCCCCAACTGT | 967 | ATCATGATGGCTGTATGTGCCA | 968 |
| HPV31 | SD2-PVT1_004_exon1 | AATCGTGTGCCCCAACTGT | 969 | CATGGTTCCACCAGCGTTATT | 970 |
| HPV31 | SD2-PVT1_005_exon1 | AATCGTGTGCCCCAACTGT | 971 | TCTTTGCTCGCAGCTCGT | 972 |
| HPV33 | bkpt1-MYC_001_exon1 | GTGCAGGAGAAAATACTAGATCTTTACGA | 973 | CTGAGAAGCCCTGCCCTTC | 974 |
| HPV33 | bkpt1-MYC_001_exon2 | GTGCAGGAGAAAATACTAGATCTTTACGA | 975 | AAATACGGCTGCACCGAGT | 976 |
| HPV33 | bkpt1-MYC_001_exon3 | GTGCAGGAGAAAATACTAGATCTTTACGA | 977 | GGTGATCCAGACTCTGACCTTTTG | 978 |
| HPV33 | bkpt1-PVT1_002_exon3 | GTGCAGGAGAAAATACTAGATCTTTACGA | 979 | ATCATGATGGCTGTATGTGCCA | 980 |
| HPV33 | bkpt1-PVT1_004_exon1 | GTGCAGGAGAAAATACTAGATCTTTACGA | 981 | CATGGTTCCACCAGCGTTATT | 982 |
| HPV33 | bkpt1-PVT1_005_exon1 | GTGCAGGAGAAAATACTAGATCTTTACGA | 983 | TCTTTGCTCGCAGCTCGT | 984 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV33 | bkpt2-MYC_ 001_exon1 | ACGTACTGCAACTAACTGCACAA | 985 | CTGAGAAGCCCTGCCCTTC | 986 |
| HPV33 | bkpt2-MYC_ 001_exon2 | ACGTACTGCAACTAACTGCACAA | 987 | AAATACGGCTGCACCGAGT | 988 |
| HPV33 | bkpt2-MYC_ 001_exon3 | ACGTACTGCAACTAACTGCACAA | 989 | GGTGATCCAGACTCTGACCT TTTG | 990 |
| HPV33 | bkpt2- PVT1_002_exon3 | ACGTACTGCAACTAACTGCACAA | 991 | ATCATGATGGCTGTATGTGC CA | 992 |
| HPV33 | bkpt2- PVT1_004_exon1 | ACGTACTGCAACTAACTGCACAA | 993 | CATGGTTCCACCAGCGTTAT T | 994 |
| HPV33 | bkpt2- PVT1_005_exon1 | ACGTACTGCAACTAACTGCACAA | 995 | TCTTTGCTCGCAGCTCGT | 996 |
| HPV33 | SD2-MYC_ 001_exon1 | GTGCCCTACCTGTGCACAA | 997 | CTGAGAAGCCCTGCCCTTC | 998 |
| HPV33 | SD2-MYC_ 001_exon2 | GTGCCCTACCTGTGCACAA | 999 | AAATACGGCTGCACCGAGT | 1000 |
| HPV33 | SD2-MYC_ 001_exon3 | GTGCCCTACCTGTGCACAA | 1001 | GGTGATCCAGACTCTGACCT TTTG | 1002 |
| HPV33 | SD2- PVT1_002_exon3 | GTGCCCTACCTGTGCACAA | 1003 | ATCATGATGGCTGTATGTGC CA | 1004 |
| HPV33 | SD2- PVT1_004_exon1 | GTGCCCTACCTGTGCACAA | 1005 | CATGGTTCCACCAGCGTTAT T | 1006 |
| HPV33 | SD2- PVT1_005_exon1 | GTGCCCTACCTGTGCACAA | 1007 | TCTTTGCTCGCAGCTCGT | 1008 |

EP 3 715 477 A1

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV35 | bkpt1-MYC_001_exon1 | ATTACGAGACTGATAGCACATGTTTGT | 1009 | CTGAGAAGCCCTGCCCTTC | 1010 |
| HPV35 | bkpt1-MYC_001_exon2 | ATTACGAGACTGATAGCACATGTTTGT | 1011 | AAATACGGCTGCACCGAGT | 1012 |
| HPV35 | bkpt1-MYC_001_exon3 | ATTACGAGACTGATAGCACATGTTTGT | 1013 | GGTGATCCAGACTCTGACCTTTTG | 1014 |
| HPV35 | bkpt1-PVT1_002_exon3 | ATTACGAGACTGATAGCACATGTTTGT | 1015 | ATCATGATGGCTGTATGTGCCA | 1016 |
| HPV35 | bkpt1-PVT1_004_exon1 | ATTACGAGACTGATAGCACATGTTTGT | 1017 | CATGGTTCCACCAGCGTTATT | 1018 |
| HPV35 | bkpt1-PVT1_005_exon1 | ATTACGAGACTGATAGCACATGTTTGT | 1019 | TCTTTGCTCGCAGCTCGT | 1020 |
| HPV35 | bkpt2-MYC_001_exon1 | TCTACATCTGACTGCACAAACAAAGA | 1021 | CTGAGAAGCCCTGCCCTTC | 1022 |
| HPV35 | bkpt2-MYC_001_exon2 | TCTACATCTGACTGCACAAACAAAGA | 1023 | AAATACGGCTGCACCGAGT | 1024 |
| HPV35 | bkpt2-MYC_001_exon3 | TCTACATCTGACTGCACAAACAAAGA | 1025 | GGTGATCCAGACTCTGACCTTTTG | 1026 |
| HPV35 | bkpt2-PVT1_002_exon3 | TCTACATCTGACTGCACAAACAAAGA | 1027 | ATCATGATGGCTGTATGTGCCA | 1028 |
| HPV35 | bkpt2-PVT1_004_exon1 | TCTACATCTGACTGCACAAACAAAGA | 1029 | CATGGTTCCACCAGCGTTATT | 1030 |
| HPV35 | bkpt2-PVT1_005_exon1 | TCTACATCTGACTGCACAAACAAAGA | 1031 | TCTTTGCTCGCAGCTCGT | 1032 |

(continued)

| HPV type | Region name | Forward primer nucleic acid sequence | Forward primer SEQID NO | Reverse primer nucleic acid sequence | Reverse primer SEQID NO |
|---|---|---|---|---|---|
| HPV35 | SD2-MYC_001_exon1 | CGGCTGTTCACAGAGAGCATAAT | 1033 | CTGAGAAGCCCTGCCCTTC | 1034 |
| HPV35 | SD2-MYC_001_exon2 | CGGCTGTTCACAGAGAGCATAAT | 1035 | AAATACGGCTGCACCGAGT | 1036 |
| HPV35 | SD2-MYC_001_exon3 | CGGCTGTTCACAGAGAGCATAAT | 1037 | GGTGATCCAGACTCTGACCTTTTG | 1038 |
| HPV35 | SD2-PVT1_002_exon3 | CGGCTGTTCACAGAGAGCATAAT | 1039 | ATCATGATGGCTGTATGTGCCA | 1040 |
| HPV35 | SD2-PVT1_004_exon1 | CGGCTGTTCACAGAGAGCATAAT | 1041 | CATGGTTCCACCAGCGTTATT | 1042 |
| HPV35 | SD2-PVT1_005_exon1 | CGGCTGTTCACAGAGAGCATAAT | 1043 | TCTTTGCTCGCAGCTCGT | 1044 |
| HPV39 | bkpt1-MYC_001_exon1 | ACAACGTTTAAATGTGTTACAGGACA | 1045 | CTGAGAAGCCCTGCCCTTC | 1046 |
| HPV39 | bkpt1-MYC_001_exon2 | ACAACGTTTAAATGTGTTACAGGACA | 1047 | AAATACGGCTGCACCGAGT | 1048 |
| HPV39 | bkpt1-MYC_001_exon3 | ACAACGTTTAAATGTGTTACAGGACA | 1049 | GGTGATCCAGACTCTGACCTTTTG | 1050 |
| HPV39 | bkpt1-PVT1_002_exon3 | ACAACGTTTAAATGTGTTACAGGACA | 1051 | ATCATGATGGCTGTATGTGCCA | 1052 |
| HPV39 | bkpt1-PVT1_004_exon1 | ACAACGTTTAAATGTGTTACAGGACA | 1053 | CATGGTTCCACCAGCGTTATT | 1054 |
| HPV39 | bkpt1-PVT1_005_exon1 | ACAACGTTTAAATGTGTTACAGGACA | 1055 | TCTTTGCTCGCAGCTCGT | 1056 |
| HPV39 | bkpt2-MYC_001_exon1 | CACAGTAACAGTACAGGCCACA | 1057 | CTGAGAAGCCCTGCCCTTC | 1058 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV39 | bkpt2-MYC_001_exon2 | CACAGTAACAGTACAGGCCACA | 1059 | AAATACGGCTGCACCGAGT | 1060 |
| HPV39 | bkpt2-MYC_001_exon3 | CACAGTAACAGTACAGGCCACA | 1061 | GGTGATCCAGACTCTGACCTTTTG | 1062 |
| HPV39 | bkpt2-PVT1_002_exon3 | CACAGTAACAGTACAGGCCACA | 1063 | ATCATGATGGCTGTATGTGCCA | 1064 |
| HPV39 | bkpt2-PVT1_004_exon1 | CACAGTAACAGTACAGGCCACA | 1065 | CATGGTTCCACCAGCGTTATT | 1066 |
| HPV39 | bkpt2-PVT1_005_exon1 | CACAGTAACAGTACAGGCCACA | 1067 | TCTTTGCTCGCAGCTCGT | 1068 |
| HPV39 | SD2-MYC_001_exon1 | CGTGGTGTGCAACTGCAA | 1069 | CTGAGAAGCCCTGCCCTTC | 1070 |
| HPV39 | SD2-MYC_001_exon2 | CGTGGTGTGCAACTGCAA | 1071 | AAATACGGCTGCACCGAGT | 1072 |
| HPV39 | SD2-MYC_001_exon3 | CGTGGTGTGCAACTGCAA | 1073 | GGTGATCCAGACTCTGACCTTTTG | 1074 |
| HPV39 | SD2-PVT1_002_exon3 | CGTGGTGTGCAACTGCAA | 1075 | ATCATGATGGCTGTATGTGCCA | 1076 |
| HPV39 | SD2-PVT1_004_exon1 | CGTGGTGTGCAACTGCAA | 1077 | CATGGTTCCACCAGCGTTATT | 1078 |
| HPV39 | SD2-PVT1_005_exon1 | CGTGGTGTGCAACTGCAA | 1079 | TCTTTGCTCGCAGCTCGT | 1080 |
| HPV45 | bkpt1-MYC_001_exon1 | CGTTACAGGACAAAATACTAGACCACTA | 1081 | CTGAGAAGCCCTGCCCTTC | 1082 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV45 | bkpt1-MYC_001_exon2 | CGTTACAGGACAAAATACTAGACCACTA | 1083 | AAATACGGCTGCACCGAGT | 1084 |
| HPV45 | bkpt1-MYC_001_exon3 | CGTTACAGGACAAAATACTAGACCACTA | 1085 | GGTGATCCAGACTCTGACCTTTTG | 1086 |
| HPV45 | bkpt1-PVT1_002_exon3 | CGTTACAGGACAAAATACTAGACCACTA | 1087 | ATCATGATGGCTGTATGTGCCA | 1088 |
| HPV45 | bkpt1-PVT1_004_exon1 | CGTTACAGGACAAAATACTAGACCACTA | 1089 | CATGGTTCCACCAGCGTTATT | 1090 |
| HPV45 | bkpt1-PVT1_005_exon1 | CGTTACAGGACAAAATACTAGACCACTA | 1091 | TCTTTGCTCGCAGCTCGT | 1092 |
| HPV45 | bkpt2-MYC_001_exon1 | TCCTGTGTTCAAGTACAAGTAACAACAA | 1093 | CTGAGAAGCCCTGCCCTTC | 1094 |
| HPV45 | bkpt2-MYC_001_exon2 | TCCTGTGTTCAAGTACAAGTAACAACAA | 1095 | AAATACGGCTGCACCGAGT | 1096 |
| HPV45 | bkpt2-MYC_001_exon3 | TCCTGTGTTCAAGTACAAGTAACAACAA | 1097 | GGTGATCCAGACTCTGACCTTTTG | 1098 |
| HPV45 | bkpt2-PVT1_002_exon3 | TCCTGTGTTCAAGTACAAGTAACAACAA | 1099 | ATCATGATGGCTGTATGTGCCA | 1100 |
| HPV45 | bkpt2-PVT1_004_exon1 | TCCTGTGTTCAAGTACAAGTAACAACAA | 1101 | CATGGTTCCACCAGCGTTATT | 1102 |
| HPV45 | bkpt2-PVT1_005_exon1 | TCCTGTGTTCAAGTACAAGTAACAACAA | 1103 | TCTTTGCTCGCAGCTCGT | 1104 |
| HPV45 | SD2-MYC_001_exon1 | AGCACCTTGTCCTTTGTGTGT | 1105 | CTGAGAAGCCCTGCCCTTC | 1106 |

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV45 | SD2-MYC_001_exon2 | AGCACCTTGTCCTTTGTGTGT | 1107 | AAATACGGCTGCACCGAGT | 1108 |
| HPV45 | SD2-MYC_001_exon3 | AGCACCTTGTCCTTTGTGTGT | 1109 | GGTGATCCAGACTCTGACCTTTTG | 1110 |
| HPV45 | SD2-PVT1_002_exon3 | AGCACCTTGTCCTTTGTGTGT | 1111 | ATCATGATGGCTGTATGTGCCA | 1112 |
| HPV45 | SD2-PVT1_004_exon1 | AGCACCTTGTCCTTTGTGTGT | 1113 | CATGGTTCCACCAGCGTTATT | 1114 |
| HPV45 | SD2-PVT1_005_exon1 | AGCACCTTGTCCTTTGTGTGT | 1115 | TCTTTGCTCGCAGCTCGT | 1116 |
| HPV51 | bkpt1-MYC_001_exon1 | GTGCCAGGAGAAAATACTAGACTGTTAT | 1117 | CTGAGAAGCCCTGCCCTTC | 1118 |
| HPV51 | bkpt1-MYC_001_exon2 | GTGCCAGGAGAAAATACTAGACTGTTAT | 1119 | AAATACGGCTGCACCGAGT | 1120 |
| HPV51 | bkpt1-MYC_001_exon3 | GTGCCAGGAGAAAATACTAGACTGTTAT | 1121 | GGTGATCCAGACTCTGACCTTTTG | 1122 |
| HPV51 | bkpt1-PVT1_002_exon3 | GTGCCAGGAGAAAATACTAGACTGTTAT | 1123 | ATCATGATGGCTGTATGTGCCA | 1124 |
| HPV51 | bkpt1-PVT1_004_exon1 | GTGCCAGGAGAAAATACTAGACTGTTAT | 1125 | CATGGTTCCACCAGCGTTATT | 1126 |
| HPV51 | bkpt1-PVT1_005_exon1 | GTGCCAGGAGAAAATACTAGACTGTTAT | 1127 | TCTTTGCTCGCAGCTCGT | 1128 |
| HPV51 | bkpt2-MYC_001_exon1 | CTAACACTGGAGGGCACCAAA | 1129 | CTGAGAAGCCCTGCCCTTC | 1130 |

EP 3 715 477 A1

122

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV51 | bkpt2-MYC_001_exon2 | CTAACACTGGAGGGCACCAAA | 1131 | AAATACGGCTGCACCGAGT | 1132 |
| HPV51 | bkpt2-MYC_001_exon3 | CTAACACTGGAGGGCACCAAA | 1133 | GGTGATCCAGACTCTGACCTTTTG | 1134 |
| HPV51 | bkpt2-PVT1_002_exon3 | CTAACACTGGAGGGCACCAAA | 1135 | ATCATGATGGCTGTATGTGCCA | 1136 |
| HPV51 | bkpt2-PVT1_004_exon1 | CTAACACTGGAGGGCACCAAA | 1137 | CATGGTTCCACCAGCGTTATT | 1138 |
| HPV51 | bkpt2-PVT1_005_exon1 | CTAACACTGGAGGGCACCAAA | 1139 | TCTTTGCTCGCAGCTCGT | 1140 |
| HPV51 | SD2-MYC_001_exon1 | GGGCGAACTAAGCCTGGTTT | 1141 | CTGAGAAGCCCTGCCCTTC | 1142 |
| HPV51 | SD2-MYC_001_exon2 | GGGCGAACTAAGCCTGGTTT | 1143 | AAATACGGCTGCACCGAGT | 1144 |
| HPV51 | SD2-MYC_001_exon3 | GGGCGAACTAAGCCTGGTTT | 1145 | GGTGATCCAGACTCTGACCTTTTG | 1146 |
| HPV51 | SD2-PVT1_002_exon3 | GGGCGAACTAAGCCTGGTTT | 1147 | ATCATGATGGCTGTATGTGCCA | 1148 |
| HPV51 | SD2-PVT1_004_exon1 | GGGCGAACTAAGCCTGGTTT | 1149 | CATGGTTCCACCAGCGTTATT | 1150 |
| HPV51 | SD2-PVT1_005_exon1 | GGGCGAACTAAGCCTGGTTT | 1151 | TCTTTGCTCGCAGCTCGT | 1152 |
| HPV52 | bkpt1-MYC_001_exon1 | GCTGATAGTAATGACCTAAACGCACAAA | 1153 | CTGAGAAGCCCTGCCCTTC | 1154 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV52 | bkpt1-MYC_001_exon2 | GCTGATAGTAATGACCTAAACGCACAAA | 1155 | AAATACGGCTGCACCGAGT | 1156 |
| HPV52 | bkpt1-MYC_001_exon3 | GCTGATAGTAATGACCTAAACGCACAAA | 1157 | GGTGATCCAGACTCTGACCTTTTG | 1158 |
| HPV52 | bkpt1-PVT1_002_exon3 | GCTGATAGTAATGACCTAAACGCACAAA | 1159 | ATCATGATGGCTGTATGTGCCA | 1160 |
| HPV52 | bkpt1-PVT1_004_exon1 | GCTGATAGTAATGACCTAAACGCACAAA | 1161 | CATGGTTCCACCAGCGTTATT | 1162 |
| HPV52 | bkpt1-PVT1_005_exon1 | GCTGATAGTAATGACCTAAACGCACAAA | 1163 | TCTTTGCTCGCAGCTCGT | 1164 |
| HPV52 | bkpt2-MYC_001_exon1 | TCACTGCAACTGAGTGCACAA | 1165 | CTGAGAAGCCCTGCCCTTC | 1166 |
| HPV52 | bkpt2-MYC_001_exon2 | TCACTGCAACTGAGTGCACAA | 1167 | AAATACGGCTGCACCGAGT | 1168 |
| HPV52 | bkpt2-MYC_001_exon3 | TCACTGCAACTGAGTGCACAA | 1169 | GGTGATCCAGACTCTGACCTTTTG | 1170 |
| HPV52 | bkpt2-PVT1_002_exon3 | TCACTGCAACTGAGTGCACAA | 1171 | ATCATGATGGCTGTATGTGCCA | 1172 |
| HPV52 | bkpt2-PVT1_004_exon1 | TCACTGCAACTGAGTGCACAA | 1173 | CATGGTTCCACCAGCGTTATT | 1174 |
| HPV52 | bkpt2-PVT1_005_exon1 | TCACTGCAACTGAGTGCACAA | 1175 | TCTTTGCTCGCAGCTCGT | 1176 |
| HPV52 | SD2-MYC_001_exon1 | GCTGTTGGGCACATTACAAGTT | 1177 | CTGAGAAGCCCTGCCCTTC | 1178 |

124

EP 3 715 477 A1

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV52 | SD2-MYC_001_exon2 | GCTGTTTGGGCACATTACAAGTT | 1179 | AAATACGGCTGCACCGAGT | 1180 |
| HPV52 | SD2-MYC_001_exon3 | GCTGTTTGGGCACATTACAAGTT | 1181 | GGTGATCCAGACTCTGACCTTTTG | 1182 |
| HPV52 | SD2-PVT1_002_exon3 | GCTGTTTGGGCACATTACAAGTT | 1183 | ATCATGATGGCTGTATGTGCCA | 1184 |
| HPV52 | SD2-PVT1_004_exon1 | GCTGTTTGGGCACATTACAAGTT | 1185 | CATGGTTCCACCAGCGTTATT | 1186 |
| HPV52 | SD2-PVT1_005_exon1 | GCTGTTTGGGCACATTACAAGTT | 1187 | TCTTTGCTCGCAGCTCGT | 1188 |
| HPV56 | bkpt1-MYC_001_exon1 | GTGCCAGAACAAAATACTAGACTGTTT | 1189 | CTGAGAAGCCCTGCCCTTC | 1190 |
| HPV56 | bkpt1-MYC_001_exon2 | GTGCCAGAACAAAATACTAGACTGTTT | 1191 | AAATACGGCTGCACCGAGT | 1192 |
| HPV56 | bkpt1-MYC_001_exon3 | GTGCCAGAACAAAATACTAGACTGTTT | 1193 | GGTGATCCAGACTCTGACCTTTTG | 1194 |
| HPV56 | bkpt1-PVT1_002_exon3 | GTGCCAGAACAAAATACTAGACTGTTT | 1195 | ATCATGATGGCTGTATGTGCCA | 1196 |
| HPV56 | bkpt1-PVT1_004_exon1 | GTGCCAGAACAAAATACTAGACTGTTT | 1197 | CATGGTTCCACCAGCGTTATT | 1198 |
| HPV56 | bkpt1-PVT1_005_exon1 | GTGCCAGAACAAAATACTAGACTGTTT | 1199 | TCTTTGCTCGCAGCTCGT | 1200 |
| HPV56 | bkpt2-MYC_001_exon1 | ACAACAACCACCCTGGTGATAAG | 1201 | CTGAGAAGCCCTGCCCTTC | 1202 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV56 | bkpt2-MYC_001_exon2 | ACAACAACCACCCTGGTGATAAG | 1203 | AAATACGGCTGCACCGAGT | 1204 |
| HPV56 | bkpt2-MYC_001_exon3 | ACAACAACCACCCTGGTGATAAG | 1205 | GGTGATCCAGACTCTGACCTTTTG | 1206 |
| HPV56 | bkpt2-PVT1_02_exon3 | ACAACAACCACCCTGGTGATAAG | 1207 | ATCATGATGGCTGTATGTGCCA | 1208 |
| HPV56 | bkpt2-PVT1_004_exon1 | ACAACAACCACCCTGGTGATAAG | 1209 | CATGGTTCCACCAGCGTTATT | 1210 |
| HPV56 | bkpt2-PVT1_005_exon1 | ACAACAACCACCCTGGTGATAAG | 1211 | TCTTTGCTCGCAGCTCGT | 1212 |
| HPV56 | SD2-MYC_001_exon1 | GTTAACAGTAACGTGCCCACTCT | 1213 | CTGAGAAGCCCTGCCCTTC | 1214 |
| HPV56 | SD2-MYC_001_exon2 | GTTAACAGTAACGTGCCCACTCT | 1215 | AAATACGGCTGCACCGAGT | 1216 |
| HPV56 | SD2-MYC_001_exon3 | GTTAACAGTAACGTGCCCACTCT | 1217 | GGTGATCCAGACTCTGACCTTTTG | 1218 |
| HPV56 | SD2-PVT1_002_exon3 | GTTAACAGTAACGTGCCCACTCT | 1219 | ATCATGATGGCTGTATGTGCCA | 1220 |
| HPV56 | SD2-PVT1_004_exon1 | GTTAACAGTAACGTGCCCACTCT | 1221 | CATGGTTCCACCAGCGTTATT | 1222 |
| HPV56 | SD2-PVT1_005_exon1 | GTTAACAGTAACGTGCCCACTCT | 1223 | TCTTTGCTCGCAGCTCGT | 1224 |
| HPV58 | bkpt1-MYC_001_exon1 | GCAGGACAAAATCCTAGACATATACGAA | 1225 | CTGAGAAGCCCTGCCCTTC | 1226 |

126

EP 3 715 477 A1

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV58 | bkpt1-MYC_001_exon2 | GCAGGACAAAATCCTAGACATATACGAA | 1227 | AAATACGGGCTGCACCGAGT | 1228 |
| HPV58 | bkpt1-MYC_001_exon3 | GCAGGACAAAATCCTAGACATATACGAA | 1229 | GGTGATCCAGACTCTGACCTTTTG | 1230 |
| HPV58 | bkpt1-PVT1_002_exon3 | GCAGGACAAAATCCTAGACATATACGAA | 1231 | ATCATGATGGCTGTATGTGCCA | 1232 |
| HPV58 | bkpt1-PVT1_004_exon1 | GCAGGACAAAATCCTAGACATATACGAA | 1233 | CATGGTTCCACCAGCGTTATT | 1234 |
| HPV58 | bkpt1-PVT1_005_exon1 | GCAGGACAAAATCCTAGACATATACGAA | 1235 | TCTTTGCTCGCAGCTCGT | 1236 |
| HPV58 | bkpt2-MYC_001_exon1 | GAGGAGGACTACACAGTACAACTAACT | 1237 | CTGAGAAGCCCTGCCCTTC | 1238 |
| HPV58 | bkpt2-MYC_001_exon2 | GAGGAGGACTACACAGTACAACTAACT | 1239 | AAATACGGCTGCACCGAGT | 1240 |
| HPV58 | bkpt2-MYC_001_exon3 | GAGGAGGACTACACAGTACAACTAACT | 1241 | GGTGATCCAGACTCTGACCTTTTG | 1242 |
| HPV58 | bkpt2-PVT1_002_exon3 | GAGGAGGACTACACAGTACAACTAACT | 1243 | ATCATGATGGCTGTATGTGCCA | 1244 |
| HPV58 | bkpt2-PVT1_004_exon1 | GAGGAGGACTACACAGTACAACTAACT | 1245 | CATGGTTCCACCAGCGTTATT | 1246 |
| HPV58 | bkpt2-PVT1_005_exon1 | GAGGAGGACTACACAGTACAACTAACT | 1247 | TCTTTGCTCGCAGCTCGT | 1248 |
| HPV58 | SD2-MYC_001_exon1 | TGCTTATGGGCACATGTACCATT | 1249 | CTGAGAAGCCCTGCCCTTC | 1250 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV58 | SD2-MYC_001_exon2 | TGCTTATGGGCACATGTACCATT | 1251 | AAATACGGCTGCACCGAGT | 1252 |
| HPV58 | SD2-MYC_001_exon3 | TGCTTATGGGCACATGTACCATT | 1253 | GGTGATCCAGACTCTGACCTTTTG | 1254 |
| HPV58 | SD2-PVT1_002_exon3 | TGCTTATGGGCACATGTACCATT | 1255 | ATCATGATGGCTGTATGTGCCA | 1256 |
| HPV58 | SD2-PVT1_004_exon1 | TGCTTATGGGCACATGTACCATT | 1257 | CATGGTTCCACCAGCGTTATT | 1258 |
| HPV58 | SD2-PVT1 005 exon1 | TGCTTATGGGCACATGTACCATT | 1259 | TCTTTGCTCGCAGCTCGT | 1260 |
| HPV59 | bkpt1-MYC_001_exon1 | GCGTTTAAGTGTGTTACAGGATCAAAT | 1261 | CTGAGAAGCCCTGCCCTTC | 1262 |
| HPV59 | bkpt1-MYC_001_exon2 | GCGTTTAAGTGTGTTACAGGATCAAAT | 1263 | AAATACGGCTGCACCGAGT | 1264 |
| HPV59 | bkpt1-MYC_001_exon3 | GCGTTTAAGTGTGTTACAGGATCAAAT | 1265 | GGTGATCCAGACTCTGACCTTTTG | 1266 |
| HPV59 | bkpt1-PVT1_002_exon3 | GCGTTTAAGTGTGTTACAGGATCAAAT | 1267 | ATCATGATGGCTGTATGTGCCA | 1268 |
| HPV59 | bkpt1-PVT1_004_exon1 | GCGTTTAAGTGTGTTACAGGATCAAAT | 1269 | CATGGTTCCACCAGCGTTATT | 1270 |
| HPV59 | bkpt1-PVT1_005_exon1 | GCGTTTAAGTGTGTTACAGGATCAAAT | 1271 | TCTTTGCTCGCAGCTCGT | 1272 |
| HPV59 | bkpt2-MYC_001_exon1 | TCCGTTTGCATCCAGGCAA | 1273 | CTGAGAAGCCCTGCCCTTC | 1274 |

EP 3 715 477 A1

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV59 | bkpt2-MYC_001_exon2 | TCCGTTTGCATCCAGGCAA | 1275 | AAATACGGCTGCACCGAGT | 1276 |
| HPV59 | bkpt2-MYC_001_exon3 | TCCGTTTGCATCCAGGCAA | 1277 | GGTGATCCAGACTCTGACCT TTTG | 1278 |
| HPV59 | bkpt2-PVT1_002_exon3 | TCCGTTTGCATCCAGGCAA | 1279 | ATCATGATGGCTGTATGTGC CA | 1280 |
| HPV59 | bkpt2-PVT1_004_exon1 | TCCGTTTGCATCCAGGCAA | 1281 | CATGGTTCCACCAGCGTTAT T | 1282 |
| HPV59 | bkpt2-PVT1_005_exon1 | TCCGTTTGCATCCAGGCAA | 1283 | TCTTTGCTCGCAGCTCGT | 1284 |
| HPV59 | SD2-MYC_001_exon1 | ACTATCCTTTGTGTGTCCTTTGTGT | 1285 | CTGAGAAGCCCTGCCCTTC | 1286 |
| HPV59 | SD2-MYC_001_exon2 | ACTATCCTTTGTGTGTCCTTTGTGT | 1287 | AAATACGGCTGCACCGAGT | 1288 |
| HPV59 | SD2-MYC_001_exon3 | ACTATCCTTTGTGTGTCCTTTGTGT | 1289 | GGTGATCCAGACTCTGACCT TTTG | 1290 |
| HPV59 | SD2-PVT1_002_exon3 | ACTATCCTTTGTGTGTCCTTTGTGT | 1291 | ATCATGATGGCTGTATGTGC CA | 1292 |
| HPV59 | SD2-PVT1_004_exon1 | ACTATCCTTTGTGTGTCCTTTGTGT | 1293 | CATGGTTCCACCAGCGTTAT T | 1294 |
| HPV59 | SD2-PVT1_005_exon1 | ACTATCCTTTGTGTGTCCTTTGTGT | 1295 | TCTTTGCTCGCAGCTCGT | 1296 |
| HPV66 | bkpt1-MYC_001_exon1 | CGTGCCAGAACAAAATACTAGACTGT | 1297 | CTGAGAAGCCCTGCCCTTC | 1298 |
| HPV66 | bkpt1-MYC_001_exon2 | CGTGCCAGAACAAAATACTAGACTGT | 1299 | AAATACGGCTGCACCGAGT | 1300 |

129

EP 3 715 477 A1

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV66 | bkpt1-MYC_001_exon3 | CGTGCCAGAACAAAATACTAGACTGT | 1301 | GGTGATCCAGACTCTGACCTTTTG | 1302 |
| HPV66 | bkpt1-PVT_002_exon3 | CGTGCCAGAACAAAATACTAGACTGT | 1303 | ATCATGATGGCTGTATGTGCCA | 1304 |
| HPV66 | bkpt1-PVT1_004_exon1 | CGTGCCAGAACAAAATACTAGACTGT | 1305 | CATGGTTCCACCAGCGTTATT | 1306 |
| HPV66 | bkpt1-PVT1_005_exon1 | CGTGCCAGAACAAAATACTAGACTGT | 1307 | TCTTTGCTCGCAGCTCGT | 1308 |
| HPV66 | bkpt2-MYC_001_exon1 | GTATCAACACACAAAGCCACTGT | 1309 | CTGAGAAGCCCTGCCCTTC | 1310 |
| HPV66 | bkpt2-MYC_001_exon2 | GTATCAACACACAAAGCCACTGT | 1311 | AAATACGGCTGCACCGAGT | 1312 |
| HPV66 | bkpt2-MYC_001_exon3 | GTATCAACACACAAAGCCACTGT | 1313 | GGTGATCCAGACTCTGACCTTTTG | 1314 |
| HPV66 | bkpt2-PVT1_002_exon3 | GTATCAACACACAAAGCCACTGT | 1315 | ATCATGATGGCTGTATGTGCCA | 1316 |
| HPV66 | bkpt2-PVT1_004_exon1 | GTATCAACACACAAAGCCACTGT | 1317 | CATGGTTCCACCAGCGTTATT | 1318 |
| HPV66 | bkpt2-PVT1_005_exon1 | GTATCAACACACAAAGCCACTGT | 1319 | TCTTTGCTCGCAGCTCGT | 1320 |
| HPV66 | SD2-MYC_001_exon1 | GTTAACAGTAACGTGCCCACTCT | 1321 | CTGAGAAGCCCTGCCCTTC | 1322 |
| HPV66 | SD2-MYC_001_exon2 | GTTAACAGTAACGTGCCCACTCT | 1323 | AAATACGGCTGCACCGAGT | 1324 |

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV66 | SD2-MYC_001_exon3 | GTTAACAGTAACGTGCCCACTCT | 1325 | GGTGATCCAGACTCTGACCTTTTG | 1326 |
| HPV66 | SD2-PVT_002_exon3 | GTTAACAGTAACGTGCCCACTCT | 1327 | ATCATGATGGCTGTGTATGTGCCA | 1328 |
| HPV66 | SD2-PVT1_004_exon1 | GTTAACAGTAACGTGCCCACTCT | 1329 | CATGGTTCCACCAGCGTTATT | 1330 |
| HPV66 | SD2-PVT1_005_exon1 | GTTAACAGTAACGTGCCCACTCT | 1331 | TCTTTGCTCGCGAGCTCGT | 1332 |
| HPV68 | bkpt1-MYC_001_exon1 | ACAGGACAGTAAATGTATACAGGACCAT | 1333 | CTGAGAAGCCCTGCCCTTC | 1334 |
| HPV68 | bkpt1-MYC_001_exon2 | ACAGGACAGTAAATGTATACAGGACCAT | 1335 | AAATACGGCTGCACCGAGT | 1336 |
| HPV68 | bkpt1-MYC_001_exon3 | ACAGGACAGTAAATGTATACAGGACCAT | 1337 | GGTGATCCAGACTCTGACCTTTTG | 1338 |
| HPV68 | bkpt1-PVT1_002_exon3 | ACAGGACAGTAAATGTATACAGGACCAT | 1339 | ATCATGATGGCTGTGTATGTGCCA | 1340 |
| HPV68 | bkpt1-PVT1_004_exon1 | ACAGGACAGTAAATGTATACAGGACCAT | 1341 | CATGGTTCCACCAGCGTTATT | 1342 |
| HPV68 | bkpt1-PVT1_005_exon1 | ACAGGACAGTAAATGTATACAGGACCAT | 1343 | TCTTTGCTCGCGAGCTCGT | 1344 |
| HPV68 | bkpt2-MYC_001_exon1 | AGTAGAAGTGCAGGCCAAAACAA | 1345 | CTGAGAAGCCCTGCCCTTC | 1346 |
| HPV68 | bkpt2-MYC_001_exon2 | AGTAGAAGTGCAGGCCAAAACAA | 1347 | AAATACGGCTGCACCGAGT | 1348 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV68 | bkpt2-MYC_001_exon3 | AGTAGAAGTGCAGGCCAAAACAA | 1349 | GGTGATCCAGACTCTGACCTTTTG | 1350 |
| HPV68 | bkpt2-PVT1_002_exon3 | AGTAGAAGTGCAGGCCAAAACAA | 1351 | ATCATGATGGCTGTATGTGCCA | 1352 |
| HPV68 | bkpt2-PVT1_004_exon1 | AGTAGAAGTGCAGGCCAAAACAA | 1353 | CATGGTTCCACCAGCGTTATT | 1354 |
| HPV68 | bkpt2-PVT1_005_exon1 | AGTAGAAGTGCAGGCCAAAACAA | 1355 | TCTTTGCTCGCAGCTCGT | 1356 |
| HPV68 | SD2-MYC_001_exon1 | TCCGTGGTGTGCAACTGAA | 1357 | CTGAGAAGCCCTGCCCTTC | 1358 |
| HPV68 | SD2-MYC_001_exon2 | TCCGTGGTGTGCAACTGAA | 1359 | AAATACGGCTGCACCGAGT | 1360 |
| HPV68 | SD2-MYC_00_exon3 | TCCGTGGTGTGCAACTGAA | 1361 | GGTGATCCAGACTCTGACCTTTTG | 1362 |
| HPV68 | SD2-PVT1_002_exon3 | TCCGTGGTGTGCAACTGAA | 1363 | ATCATGATGGCTGTATGTGCCA | 1364 |
| HPV68 | SD2-PVT1_004_exon1 | TCCGTGGTGTGCAACTGAA | 1365 | CATGGTTCCACCAGCGTTATT | 1366 |
| HPV68 | SD2-PVT1_005_exon1 | TCCGTGGTGTGCAACTGAA | 1367 | TCTTTGCTCGCAGCTCGT | 1368 |
| HPV73 | bkpt1-MYC_001_exon1 | GTATGAACGTGACAGTGTACACCTAA | 1369 | CTGAGAAGCCCTGCCCTTC | 1370 |
| HPV73 | bkpt1-MYC_001_exon2 | GTATGAACGTGACAGTGTACACCTAA | 1371 | AAATACGGCTGCACCGAGT | 1372 |

EP 3 715 477 A1

(continued)

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV73 | bkpt1-MYC_001_exon3 | GTATGAACGTGACAGTGTACACCTAA | 1373 | GGTGATCCAGACTCTGACCTTTTG | 1374 |
| HPV73 | bkpt1-PVT1_002_exon3 | GTATGAACGTGACAGTGTACACCTAA | 1375 | ATCATGATGGCTGTGTATGTGCCA | 1376 |
| HPV73 | bkpt1-PVT1_004_exon1 | GTATGAACGTGACAGTGTACACCTAA | 1377 | CATGGTTCCACCAGCGTTATT | 1378 |
| HPV73 | bkpt1-PVT1_005_exon1 | GTATGAACGTGACAGTGTACACCTAA | 1379 | TCTTTGCTCGCAGCTCGT | 1380 |
| HPV73 | bkpt2-MYC_001_exon1 | ACCTACATCCCACCACAGAGT | 1381 | CTGAGAAGCCCTGCCCTTC | 1382 |
| HPV73 | bkpt2-MYC_001_exon2 | ACCTACATCCCACCACAGAGT | 1383 | AAATACGGGCTGCACCGAGT | 1384 |
| HPV73 | bkpt2-MYC_001_exon3 | ACCTACATCCCACCACAGAGT | 1385 | GGTGATCCAGACTCTGACCTTTTG | 1386 |
| HPV73 | bkpt2-PVT1_002_exon3 | ACCTACATCCCACCACAGAGT | 1387 | ATCATGATGGCTGTGTATGTGCCA | 1388 |
| HPV73 | bkpt2-PVT1_004_exon1 | ACCTACATCCCACCACAGAGT | 1389 | CATGGTTCCACCAGCGTTATT | 1390 |
| HPV73 | bkpt2-PVT1_005_exon1 | ACCTACATCCCACCACAGAGT | 1391 | TCTTTGCTCGCAGCTCGT | 1392 |
| HPV73 | SD2-MYC_001_exon1 | TGCTTATGGGGTACACTAGGTATTGTGT | 1393 | CTGAGAAGCCCTGCCCTTC | 1394 |
| HPV73 | SD2-MYC_001_exon2 | TGCTTATGGGGTACACTAGGTATTGTGT | 1395 | AAATACGGGCTGCACCGAGT | 1396 |

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV73 | SD2-MYC_00_exon3 | TGCTTATGGGTACACTAGGTATTGTGT | 1397 | GGTGATCCAGACTCTGACCTTTTG | 1398 |
| HPV73 | SD2-PVT1_002_exon3 | TGCTTATGGGTACACTAGGTATTGTGT | 1399 | ATCATGATGGCTGTATGTGCCA | 1400 |
| HPV73 | SD2-PVT1_004_exon1 | TGCTTATGGGTACACTAGGTATTGTGT | 1401 | CATGGTTCCACCAGCGTTATT | 1402 |
| HPV73 | SD2-PVT1_005_exon1 | TGCTTATGGGTACACTAGGTATTGTGT | 1403 | TCTTTGCTCGCAGCTCGT | 1404 |
| HPV82 | bkpt1-MYC_001_exon1 | GTGCCAGGAGAAAATACTAGACTGTTAT | 1405 | CTGAGAAGCCCTGCCCTTC | 1406 |
| HPV82 | bkpt1-MYC_001_exon2 | GTGCCAGGAGAAAATACTAGACTGTTAT | 1407 | AAATACGGCTGCACCGAGT | 1408 |
| HPV82 | bkpt1-MYC_001_exon3 | GTGCCAGGAGAAAATACTAGACTGTTAT | 1409 | GGTGATCCAGACTCTGACCTTTTG | 1410 |
| HPV82 | bkpt1-PVT1_002_exon3 | GTGCCAGGAGAAAATACTAGACTGTTAT | 1411 | ATCATGATGGCTGTATGTGCCA | 1412 |
| HPV82 | bkpt1-PVT1_004_exon1 | GTGCCAGGAGAAAATACTAGACTGTTAT | 1413 | CATGGTTCCACCAGCGTTATT | 1414 |
| HPV82 | bkpt1-PVT1_005_exon1 | GTGCCAGGAGAAAATACTAGACTGTTAT | 1415 | TCTTTGCTCGCAGCTCGT | 1416 |
| HPV82 | bkpt2-MYC_001_exon1 | TGCGACCACCAAATACACTGT | 1417 | CTGAGAAGCCCTGCCCTTC | 1418 |
| HPV82 | bkpt2-MYC_001_exon2 | TGCGACCACCAAATACACTGT | 1419 | AAATACGGCTGCACCGAGT | 1420 |

EP 3 715 477 A1

| HPV type | Region name | Forward primer nucleic acid sequence | Forward primer SEQ ID NO | Reverse primer nucleic acid sequence | Reverse primer SEQ ID NO |
|---|---|---|---|---|---|
| HPV82 | bkpt2-MYC_001_exon3 | TGCGACCACCAAATACACTGT | 1421 | GGTGATCCAGACTCTGACCTTTTG | 1422 |
| HPV82 | bkpt2-PVT1_002_exon3 | TGCGACCACCAAATACACTGT | 1423 | ATCATGATGGCTGTATGTGCCA | 1424 |
| HPV82 | bkpt2-PVT1_004_exon1 | TGCGACCACCAAATACACTGT | 1425 | CATGGTTCCACCAGCGTTATT | 1426 |
| HPV82 | bkpt2-PVT1_005_exon1 | TGCGACCACCAAATACACTGT | 1427 | TCTTTGCTCGCAGCTCGT | 1428 |
| HPV82 | SD2-MYC_001_exon1 | CGTGGTGTGCGACCAACTAA | 1429 | CTGAGAAGCCCTGCCCTTC | 1430 |
| HPV82 | SD2-MYC_001_exon2 | CGTGGTGTGCGACCAACTAA | 1431 | AAATACGGCTGCACCGAGT | 1432 |
| HPV82 | SD2-MYC_001_exon3 | CGTGGTGTGCGACCAACTAA | 1433 | GGTGATCCAGACTCTGACCTTTTG | 1434 |
| HPV82 | SD2-PVT1_002_exon3 | CGTGGTGTGCGACCAACTAA | 1435 | ATCATGATGGCTGTATGTGCCA | 1436 |
| HPV82 | SD2-PVT1_004_exon1 | CGTGGTGTGCGACCAACTAA | 1437 | CATGGTTCCACCAGCGTTATT | 1438 |
| HPV82 | SD2-PVT1_005_exon1 | CGTGGTGTGCGACCAACTAA | 1439 | TCTTTGCTCGCAGCTCGT | 1440 |

EP 3 715 477 A1

Table 2Dbis

| HPV type | Region name | Forward primer SEQ ID NO | Reverse primer SEQ ID NO | Amplicon nucleic acid sequence | Amplicon SEQ ID NO |
|---|---|---|---|---|---|
| HPV16 | bkpt1-MYC_ 001_exon1 | 865 | 866 | TATTGGAAACACATGCGCCTAGCTGCTCGCGGCCG CCACCGCCGGGCCCCGGCCGTCCCTGGCTCCCCTC CTGCCTCGA | 1933 |
| HPV16 | bkpt1-MYC_ 001_exo n2 | 867 | 868 | TATTGGAAACACATGCGCCTAGCAGCCTCCCGCG ACGATGCCCCTCAACGTTAGCTTCACCAACAGGA ACTATGACCTCGACTACG | 1934 |
| HPV16 | bkpt1-MYC_ 001_exon3 | 869 | 870 | TATTGGAAACACATGCGCCTAGAGGAGGAACAAG AAGATGAGGAAGAAATCGATGTTGTTTCTGTGGA AAAGAGGCAGGCTCCTGG | 1935 |
| HPV16 | bkpt1- PVT1_002_exon3 | 871 | 872 | TATTGGAAACACATGCGCCTAGCTGACCATACTCC CTGGAGCCTTCTCCCGAGGTGCGCGGGTGACCT | 1936 |
| HPV16 | bkpt1- PVT1_004_exon1 | 873 | 874 | TATTGGAAACACATGCGCCTAGTCTGAGCCTGATG GATTTACAGTGATCTTCAGTGGTCTGGGG | 1937 |
| HPV16 | bkpt1- PVT1_005_exon1 | 875 | 876 | TATTGGAAACACATGCGCCTAGCTCCGGGCAGAG CGCGTGTGGCGGCCGAGCACATGGGCCCGCGGGC CGGGCGGGCTCGGGGCGGCCGGGACGAGGAGGG GCGACG | 1938 |
| HPV16 | bkpt2-MYC_ 001_exon1 | 877 | 878 | TGTAATAGTAACACTACACCCATAGCTGCTCGCGG CCGCCACCGCCGGGCCCCGGCCGTCCCTGGCTCCC CTCCTGCCTCGA | 1939 |
| HPV16 | bkpt2-MYC_ 001_exon2 | 879 | 880 | TGTAATAGTAACACTACACCCATAGCAGCCTCCCG CGACGATGCCCCTCAACGTTAGCTTCACCAACAGG AACTATGACCTCGACTACG | 1940 |

EP 3 715 477 A1

136

| HPV type | Region name | Forward primer SEQ ID NO | Reverse primer SEQ ID NO | Amplicon nucleic acid sequence | Amplicon SEQ ID NO |
|---|---|---|---|---|---|
| HPV16 | bkpt2-MYC_001_exon3 | 881 | 882 | TGTAATAGTAACACTACACCCATAGAGGAGGAAC AAGAAGATGAGGAAGAAATCGATGTTGTTTCTGT GGAAAAGAGGCAGGCTCCTGG | 1941 |
| HPV16 | bkpt2-PVT1_002_exon3 | 883 | 884 | TGTAATAGTAACACTACACCCATAGCTGACCATAC TCCCTGGAGCCTTCTCCCGAGGTGCGCGGGTGACC T | 1942 |
| HPV16 | bkpt2-PVT1_004_exon1 | 885 | 886 | TGTAATAGTAACACTACACCCATAGTCTGAGCCTG ATGGATTTACAGTGATCTTCAGTGGTCTGGGG | 1943 |
| HPV16 | bkpt2-PVT1_005_exon1 | 887 | 888 | TGTAATAGTAACACTACACCCATAGCTCCGGGCA GAGCGCGTGTGGCGGCCGAGCACATGGGCCCGCG GGCCGGGCGGGCTCGGGGCGGCCGGGACGAGGA GGGGCGACG | 1944 |
| HPV16 | SD2-MYC_001_exon1 | 889 | 890 | TCTCAGAAACCATAATCTACCATGGCTGATCCTGC AGCTGCTCGCGGCCGCCACCGCCGGGCCCCGGCC GTCCCTGGCTCCCCTCCTGCCTCGA | 1945 |
| HPV16 | SD2-MYC_001_exon2 | 891 | 892 | TCTCAGAAACCATAATCTACCATGGCTGATCCTGC AGCAGCCTCCCGCGACGATGCCCCTCAACGTTAGC TTCACCAACAGGAACTATGACCTCGACTACG | 1946 |
| HPV16 | SD2-MYC_001_exon3 | 893 | 894 | TCTCAGAAACCATAATCTACCATGGCTGATCCTGC AGAGGAGGAACAAGAAGATGAGGAAGAAATCGA TGTTGTTTCTGTGGAAAAGAGGCAGGCTCCTGG | 1947 |
| HPV16 | SD2-PVT1_002_exon3 | 895 | 896 | TCTCAGAAACCATAATCTACCATGGCTGATCCTGC AGCTGACCATACTCCCTGGAGCCTTCTCCCGAGGT GCGCGGGTGACCT | 1948 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV16 | SD2-PVT1_004_exon1 | 897 | 898 | TCTCAGAAACCATAATCTACCATGGCTGATCCTGC AGTCTGAGCCTGATGGATTTACAGTGATCTTCAGT GGTCTGGGG | 1949 |
| HPV16 | SD2-PVT1_005_exon1 | 899 | 900 | TCTCAGAAACCATAATCTACCATGGCTGATCCTGC AGCTCCGGGCAGAGCGCGTGTGGCGGCCGAGCAC ATGGGCCCGCGGGCCGGGCGGGCTCGGGGCGGCC GGGACGAGGAGGGGCGACG | 1950 |
| HPV18 | bkpt1-MYC_001_exon1 | 901 | 902 | TACAGTATTGGCAACTAATACGTTGGGCTGCTCGC GGCCGCCACCGCCGGGCCCCGGCCGTCCCTGGCTC CCCTCCTGCCTCGA | 1951 |
| HPV18 | bkpt1-MYC_001_exon2 | 903 | 904 | TACAGTATTGGCAACTAATACGTTGGGCAGCCTCC CGCGACGATGCCCCTCAACGTTAGCTTCACCAACA GGAACTATGACCTCGACTACG | 1952 |
| HPV18 | bkpt1-MYC_001_exon3 | 905 | 906 | TACAGTATTGGCAACTAATACGTTGGGAGGAGGA ACAAGAAGATGAGGAAGAAATCGATGTTGTTTCT GTGGAAAAGAGGCAGGCTCCTGG | 1953 |
| HPV18 | bkpt1-PVT1_002_exon3 | 907 | 908 | TACAGTATTGGCAACTAATACGTTGGGCTGACCAT ACTCCCTGGAGCCTTCTCCCGAGGTGCGCGGGTGA CCT | 1954 |
| HPV18 | bkpt1-PVT1_004_exon1 | 909 | 910 | TACAGTATTGGCAACTAATACGTTGGGTCTGAGCC TGATGGATTTACAGTGATCTTCAGTGGTCTGGGG | 1955 |
| HPV18 | bkpt1-PVT1_005_exon1 | 911 | 912 | TACAGTATTGGCAACTAATACGTTGGGCTCCGGGC AGAGCGCGTGTGGCGGCCGAGCACATGGGCCCGC GGGCCGGGCGGGCTCGGGGCGGCCGGGACGAGG AGGGGCGACG | 1956 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV18 | bkpt2-MYC_001_exon1 | 913 | 914 | CAAAAGACGGAAACTCTGTAGTGGTAACACTACGCCTATAACTGCTCGCGGCCGCCACCGCCGGGCCCCGGCCGTCCCTGGCTCCCCTCCCTCGA | 1957 |
| HPV18 | bkpt2-MYC_001_exon2 | 915 | 916 | CAAAAGACGGAAACTCTGTAGTGGTAACACTACGCCTATAAACAGCCTCCCGGCAGCCCCCTCAACGTTAGCTTCACCAACAGGAACTATGACCTCGACTACG | 1958 |
| HPV18 | bkpt2-MYC_001_exon3 | 917 | 918 | CAAAAGACGGAAACTCTGTAGTGGTAACACTACGCCTATAAAGGAGGAACAAGAAGGAGAAGAAATCGATGTTGTTTCTGTGGAAAAGAGGCAGGCTCCTGG | 1959 |
| HPV18 | bkpt2-PVT1_002_exon3 | 919 | 920 | CAAAAGACGGAAACTCTGTAGTGGTAACACTACGCCTATAACTGACCATACTCCCCTGGAGCCTTCTCCCGAGGTGCCGCGGGTGACCT | 1960 |
| HPV18 | bkpt2-PVT1_004_exon1 | 921 | 922 | CAAAAGACGGAAACTCTGTAGTGGTAACACTACGCCTATAATCTGAGCCTGATGGATTTACAGTGATCTTCAGTGGTCTGGGG | 1961 |
| HPV18 | bkpt2-PVT1_005_exon1 | 923 | 924 | CAAAAGACGGAAACTCTGTAGTGGTAACACTACGCCTATAACTCCGGGCAGAGCGCGTGTGGCGGCCGAGCACATGGGCCCCGGCCCGGGGCCGGGCTCGGGGCGGCCGGGACGAGGAGGGGCGACG | 1962 |
| HPV18 | SD2-MYC_001_exon1 | 925 | 926 | CAATGGCTGATCCAGAAGCTGCTCGCGGCCGCCACCGCCGGGCCCCGGCCGTCCCTGGCTCCCCTCCCTGCCTCGA | 1963 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV18 | SD2-MYC_001_exon2 | 927 | 928 | CAATGGCTGATCCAGAAGCAGCCTCCCGCGACGATGCCCCTCAACGTTAGCTTCACCAACAGGAACTATGACCTCGACTACG | 1964 |
| HPV18 | SD2-MYC_001_exon3 | 929 | 930 | CAATGGCTGATCCAGAAGAGGAGGAACAAGAAGATGAGGAAGAAATCGATGTTGTTTCTGTGGAAAAGAGGCAGGCTCCTGG | 1965 |
| HPV18 | SD2-PVT1_002_exon3 | 931 | 932 | CAATGGCTGATCCAGAAGCTGACCATACTCCCTGGAGCCTTCTCCCGAGGTGCGCGGGTGACCT | 1966 |
| HPV18 | SD2-PVT1_004_exon1 | 933 | 934 | CAATGGCTGATCCAGAAGTCTGAGCCTGATGGATTTACAGTGATCTTCAGTGGTCTGGGG | 1967 |
| HPV18 | SD2-PVT1_005_exon1 | 935 | 936 | CAATGGCTGATCCAGAAGCTCCGGGCAGAGCGCGTGTGGCGGCCGAGCACATGGGCCCGCGGGCCGGGCGGGCTCGGGGCGGCCGGGACGAGGAGGGGCGACG | 1968 |
| HPV31 | bkpt1-MYC_001_exon1 | 937 | 938 | ATATTAGAACATTATGAAAATGATAGTAAACGACTTTGTGATCATATAGACTATTGGAAACATATTCGACTTGCTGCTCGCGGCCGCCACCGCCGGGCCCCGGCCGTCCCTGGCTCCCCTCCTGCCTCGA | 1969 |
| HPV31 | bkpt1-MYC_001_exon2 | 939 | 940 | ATATTAGAACATTATGAAAATGATAGTAAACGACTTTGTGATCATATAGACTATTGGAAACATATTCGACTTGCAGCCTCCCGCGACGATGCCCCTCAACGTTAGCTTCACCAACAGGAACTATGACCTCGACTACG | 1970 |
| HPV31 | bkpt1-MYC_001_exon3 | 941 | 942 | ATATTAGAACATTATGAAAATGATAGTAAACGACTTTGTGATCATATAGACTATTGGAAACATATTCGACTTGAGGAGGAACAAGAAGATGAGGAAGAAATCGATGTTGTTTCTGTGGAAAAGAGGCAGGCTCCTGG | 1971 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV31 | bkpt1-PVT1_002_exon3 | 943 | 944 | ATATTAGAACACATTATTGAAAAATGATAGTAAACGACTTTGTGTGATCATATAGACTATTGGAAAACATATTCGACTTGCTGACCATACTCCCTGGAGCCTTCTCCCGAGGTGCGCGGGGTGACCT | 1972 |
| HPV31 | bkpt1-PVT1_004_exon1 | 945 | 946 | ATATTAGAACACATTATTGAAAAATGATAGTAAACGACTTTGTGTGATCATATAGACTATTGGAAAACATATTCGACTTGTCTGAGCCTGATGGGATTTACAGTGATCTTCAGTGGTCTGGGG | 1973 |
| HPV31 | bkpt1-PVT1_005_exon1 | 947 | 948 | ATATTAGAACACATTATTGAAAAATGATAGTAAACGACTTTGTGTGATCATATAGACTATTGGAAAACATATTCGACTTGCTCCGGCAGAGCGCGTGTGGCGGCCGAGCACATGGGCGCCCGGCGCCGGGGCGGGGCTCGGGGCGGCCGGGACGAGGAGGGGCGACG | 1974 |
| HPV31 | bkpt2-MYC_001_exon1 | 949 | 950 | AACAAGGGCTGTCAGTTGTCCTGCAACTACACCTATAACTGCTCGCGGCCGCCACCGCCGGCCCCCGGCCGTCCCTGGCTCCCCTCCTGCCTCGA | 1975 |
| HPV31 | bkpt2-MYC_001_exon2 | 951 | 952 | AACAAGGGCTGTCAGTTGTCCTGCAACTACACCTATAACAGCCTCCCGCGACGATGCCCCTCAACGTTAGCTTCACCAACAGGAACTATGACCTCGACTACG | 1976 |
| HPV31 | bkpt2-MYC_001_exon3 | 953 | 954 | AACAAGGGCTGTCAGTTGTCCTGCAACTACACCTATAAAGGAGGAACAAGAAGATGAGGAAGAAATCGATGTTGTTTCTGTGGAAAAGAGGCAGGCTCCTGG | 1977 |
| HPV31 | bkpt2-PVT1_002_exon3 | 955 | 956 | AACAAGGGCTGTCAGTTGTCCTGCAACTACACCTATAACTGACCATACTCCCTGGAGCCTTCTCCCGAGGTGCGCGGGGTGACCT | 1978 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV31 | bkpt2-PVT1_004_exon1 | 957 | 958 | AACAAGGGCTGTCAGTTGTCCTGCAACTACACCTATAATCTGAGCCTGATGGATTTACAGTGATCTTCAGTGGTCTGGGG | 1979 |
| HPV31 | bkpt2-PVT1_005_exon1 | 959 | 960 | AACAAGGGCTGTCAGTTGTCCTGCAACTACACCTATAACTCCGGGCAGAGCGCGTGTGGCGGCCGAGCACATGGGCCCGCGGGCCGGGCGGGCTCGGGGCGGCCGGGACGAGGAGGGGCGACG | 1980 |
| HPV31 | SD2-MYC_001_exon1 | 961 | 962 | TCTACTAGACTGTAACTACAATGGCTGATCCAGCAGCTGCTCGCGGCCGCCACCGCCGGGCCCCGGCCGTCCCTGGCTCCCCTCCTGCCTCGA | 1981 |
| HPV31 | SD2-MYC_001_exon2 | 963 | 964 | TCTACTAGACTGTAACTACAATGGCTGATCCAGCAGCAGCCTCCCGCGACGATGCCCCTCAACGTTAGCTTCACCAACAGGAACTATGACCTCGACTACG | 1982 |
| HPV31 | SD2-MYC_001_exon3 | 965 | 966 | TCTACTAGACTGTAACTACAATGGCTGATCCAGCAGAGGAGGAACAAGAAGATGAGGAAGAAATCGATGTTGTTTCTGTGGAAAAGAGGCAGGCTCCTGG | 1983 |
| HPV31 | SD2-PVT1_002_exon3 | 967 | 968 | TCTACTAGACTGTAACTACAATGGCTGATCCAGCAGCTGACCATACTCCCTGGAGCCTTCTCCCGAGGTGCGCGGGTGACCT | 1984 |
| HPV31 | SD2-PVT1_004_exon1 | 969 | 970 | TCTACTAGACTGTAACTACAATGGCTGATCCAGCAGTCTGAGCCTGATGGATTTACAGTGATCTTCAGTGGTCTGGGG | 1985 |

142

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV31 | SD2-PVT1_005_exon1 | 971 | 972 | TCTACTAGACTGTAACTACAATGGCTGATCCAGCAGCTCCGGGCAGAGCGCGTGTGGCGGCCGAGCACATGGGCCCGCGGGCCGGGCGGGCTCGGGGCGGCCGGGACGAGGAGGGGCGACG | 1986 |
| HPV33 | bkpt1-MYC_001_exon1 | 973 | 974 | AGCTGATAAAACTGATTTACCATCACAAATTGAACATTGGAAACTGATACGCATGGCTGCTCGCGGCCGCCACCGCCGGGCCCCGGCCGTCCCTGGCTCCCCTCCTGCCTCGA | 1987 |
| HPV33 | bkpt1-MYC_001_exon2 | 975 | 976 | AGCTGATAAAACTGATTTACCATCACAAATTGAACATTGGAAACTGATACGCATGGCAGCCTCCCGCGACGATGCCCCTCAACGTTAGCTTCACCAACAGGAACTATGACCTCGACTACG | 1988 |
| HPV33 | bkpt1-MYC_001_exon3 | 977 | 978 | AGCTGATAAAACTGATTTACCATCACAAATTGAACATTGGAAACTGATACGCATGGAGGAGGAACAAGAAGATGAGGAAGAAATCGATGTTGTTTCTGTGGAAAAGAGGCAGGCTCCTGG | 1989 |
| HPV33 | bkpt1-PVT1_002_exon3 | 979 | 980 | AGCTGATAAAACTGATTTACCATCACAAATTGAACATTGGAAACTGATACGCATGGCTGACCATACTCCCTGGAGCCTTCTCCCGAGGTGCGCGGGTGACCT | 1990 |
| HPV33 | bkpt1-PVT1_004_exon1 | 981 | 982 | AGCTGATAAAACTGATTTACCATCACAAATTGAACATTGGAAACTGATACGCATGGTCTGAGCCTGATGGATTTACAGTGATCTTCAGTGGTCTGGGG | 1991 |
| HPV33 | bkpt1-PVT1_005_exon1 | 983 | 984 | AGCTGATAAAACTGATTTACCATCACAAATTGAAC | 1992 |

| HPV type | Region name | Forward primer SEQ ID NO | Reverse primer SEQ ID NO | Amplicon nucleic acid sequence | Amplicon SEQ ID NO |
|---|---|---|---|---|---|
| | | | | ATTGGAAACTGATACGCATGGCTCCGGGCAGAGC GCGTGTGGCGGCCGAGCACATGGGCCCGCGGGCC GGGCGGGCTCGGGGCGGCCGGGACGAGGAGGGG CGACG | |
| HPV33 | bkpt2-MYC_ 001_exon1 | 985 | 986 | ACAAGCAGCGGACTGTGTGTAGTTCTAACGTTGCA CCTATAGCTGCTCGCGGCCGCCACCGCCGGGCCCC GGCCGTCCCTGGCTCCCTCCTGCCTCGA | 1993 |
| HPV33 | bkpt2-MYC_ 001_exon2 | 987 | 988 | ACAAGCAGCGGACTGTGTGTAGTTCTAACGTTGCA CCTATAGCAGCCTCCCGCGACGATGCCCCTCAACG TTAGCTTCACCAACAGGAACTATGACCTCGACTAC G | 1994 |
| HPV33 | bkpt2-MYC_ 001_exon3 | 989 | 990 | ACAAGCAGCGGACTGTGTGTAGTTCTAACGTTGCA CCTATAGAGGAGGAACAAGAAGATGAGGAAGAA ATCGATGTTGTTTCTGTGGAAAAGAGGCAGGCTCC TGG | 1995 |
| HPV33 | bkpt2- PVT1_002_exon3 | 991 | 992 | ACAAGCAGCGGACTGTGTGTAGTTCTAACGTTGCA CCTATAGCTGACCATACTCCCTGGAGCCTTCTCCC GAGGTGCGCGGGTGACCT | 1996 |
| HPV33 | bkpt2- PVT1_004_exon1 | 993 | 994 | ACAAGCAGCGGACTGTGTGTAGTTCTAACGTTGCA CCTATAGTCTGAGCCTGATGGATTTACAGTGATCT TCAGTGGTCTGGGG | 1997 |
| HPV33 | bkpt2- PVT1_005_exon1 | 995 | 996 | ACAAGCAGCGGACTGTGTGTAGTTCTAACGTTGCA CCTATAGCTCCGGGCAGAGCGCGTGTGGCGGCCG AGCACATGGGCCCGCGGGCCGGGCGGGCTCGGGG CGGCCGGGACGAGGAGGGGCGACG | 1998 |

144

EP 3 715 477 A1

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV33 | SD2-MYC_001_exon1 | 997 | 998 | CAATAAAACATCATCTACAATGGCCGATCCTGAAGCTGCTCGCGGCGGCCGCCACCGGCCGCGGGCCCGGCCGTCCCTGGCTCCCCTCCCTGCCTCGA | 1999 |
| HPV33 | SD2-MYC_001_exon2 | 999 | 1000 | CAATAAAACATCATCTACAATGGCCGATCCTGAAGCAGCCTCCCGCGACGATGCCCCTCAACGTTAGCTTCACCCAACAGGAACTATGACCTCGACTACG | 2000 |
| HPV33 | SD2-MYC_001_exon3 | 1001 | 1002 | CAATAAAACATCATCTACAATGGCCGATCCTGAAGAGGAGGAACAAGAAGATGAGGAAGAAATCGATGTTGTTTCTGTGGAAAAGAGGCAGGCTCCTGG | 2001 |
| HPV33 | SD2-PVT1_002_exon3 | 1003 | 1004 | CAATAAAACATCATCTACAATGGCCGATCCTGAAGCTGACCCATACTCCCTGGAGCCTTCTCCCGAGGTGCGCGGGTGACCT | 2002 |
| HPV33 | SD2-PVT1_004_exon1 | 1005 | 1006 | CAATAAAACATCATCTACAATGGCCGATCCTGAAGTCTGAGCCTGATGGATTTACAGTGATCTTCAGTGGTCTGGGG | 2003 |
| HPV33 | SD2-PVT1_005_exon1 | 1007 | 1008 | CAATAAAACATCATCTACAATGGCCGATCCTGAAGCTCCGGGCAGAGCGGCGTGTGGCGGCCGAGCACATGGGCCCGCCGCGGGCGGGCTCGGGGCGGCCGGGACGAGGAGGGGGCGACG | 2004 |
| HPV35 | bkpt1-MYC_001_exon1 | 1009 | 1010 | CTGATCACATACAGTATTGGAAACTGATTCGTCTTGCTGCTCGCGGCGCCGCCACCGCCGCGGCCCCGGGCCGTCCCTGGCTCCCCTCCCTGCCTCGA | 2005 |
| HPV35 | bkpt1-MYC_001_exon2 | 1011 | 1012 | CTGATCACATACACAGTATTGGAAACTGATTCGTCTTGCAGCCCTCCCGCGACGATGCCCCTCAACGTTAGCTTCACCAACAGGAACTATGACCTCGACTACG | 2006 |

145

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV35 | bkpt1-MYC_001_exon3 | 1013 | 1014 | CTGATCACATACAGTATTGGAAAACTGATTCGTCTTGAGGAGGAACAAGAAGAGATGAGGAAGAAATCGATGTTGTTTCTGTGGAAAAGAGGCAGGCTCCTGG | 2007 |
| HPV35 | bkpt1-PVT1_002_exon3 | 1015 | 1016 | CTGATCACATACAGTATTGGAAAACTGATTCGTCTTGCTGACCATACTCCCTGGAGCCTTCTCCCGAGGTGCGCGGGTGACCT | 2008 |
| HPV35 | bkpt1-PVT1_004_exon1 | 1017 | 1018 | CTGATCACATACAGTATTGGAAAACTGATTCGTCTTGTCTGAGCCTGATGGATTTACAGTGATCTTCAGTGGTCTGGGG | 2009 |
| HPV35 | bkpt1-PVT1_005_exon1 | 1019 | 1020 | CTGATCACATACAGTATTGGAAAACTGATTCGTCTTGCTCCGGGCAGAGCGGCGTGTGGCGGCCGAGCACATGGGCCGCCGGGCCGCCGGGGCCTCGGGGGCCGGGACGAGGAGGGGCGACG | 2010 |
| HPV35 | bkpt2-MYC_001_exon1 | 1021 | 1022 | CCGGTGTGGTAGTTGTAGTACAACTACACCTATAGCTGCTCCGGCGGCCGCCACCGCCGGCCCCGGCCGTCCCTGGCTCCCCTCCTGCCTCGA | 2011 |
| HPV35 | bkpt2-MYC_001_exon2 | 1023 | 1024 | CCGGTGTGGTAGTTGTAGTACAACTACACCTATAGCAGCCCTCCCGCGACGATGCCCCTCAACGTTAGCTTCACCAACAGGAACTATGACCTCGACTACG | 2012 |
| HPV35 | bkpt2-MYC_001_exon3 | 1025 | 1026 | CCGGTGTGGTAGTTGTAGTACAACTACACCTATAGAGGAGGAACAAGAAGAGATGAGGAAGAAATCGATGTTGTTTCTGTGGAAAAGAGGCAGGCTCCTGG | 2013 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV35 | bkpt2-PVT1_002_exon3 | 1027 | 1028 | CCGGTGTGGTAGTTGTGTAGTACAACTACACCTATAGCTGACCATACTCCCTGGAGCCTTCTCCCGAGGTGCGCGGGTGACCT | 2014 |
| HPV35 | bkpt2-PVT1_004_exon1 | 1029 | 1030 | CCGGTGTGGTAGTTGTGTAGTACAACTACACCTATAGTCTGAGCCTGATGGATTTACAGTGATCTTCAGTGGTCTGGGG | 2015 |
| HPV35 | bkpt2-PVT1_005_exon1 | 1031 | 1032 | CCGGTGTGGTAGTTGTGTAGTACAACTACACCTATAGCTCCGGGCAGAGCGCGTGTGGCGGCCGAGCACATGGGCCCGCGCGGGCCGGGGCGGGGGCTCGGGGGCGGCCGGGACGAGGAGGGGCGACG | 2016 |
| HPV35 | SD2-MYC_001_exon1 | 1033 | 1034 | CTACAATGGCTGATCCTGCAGCTGCTCGCGGCCGCCACCGCGCGGGCCCCGCGCCGTCCCCTGGCCTCCCCTGCCTCGA | 2017 |
| HPV35 | SD2-MYC_001_exon2 | 1035 | 1036 | CTACAATGGCTGATCCTGCAGCAGCCTCCCGCGACGATGCCCCTCAACGTTAGCTTCACCAACAGGAACTATGACCTCGACTACG | 2018 |
| HPV35 | SD2-MYC_001_exon3 | 1037 | 1038 | CTACAATGGCTGATCCTGCAGAGGAGGAACAAGAAGATGAAGAAGAAATCGATGTTGTTTCTGTGGAAAAGAGGCAGGCTCCTGG | 2019 |
| HPV35 | SD2-PVT1_002_exon3 | 1039 | 1040 | CTACAATGGCTGATCCTGCAGCTGACCATACTCCCTGGAGCCTTCTCCCGAGGTGCGCGGGTGACCT | 2020 |
| HPV35 | SD2-PVT1_004_exon1 | 1041 | 1042 | CTACAATGGCTGATCCTGCAGTCTGAGCCTGATGGATTTACAGTGATCTTCAGTGGTCTGGGG | 2021 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV35 | SD2-PVT1_005_exon1 | 1043 | 1044 | CTACAATGGCTGATCCTGCAGCTCCGGGCAGAGC GCGTGTGGCGGCCGAGCACATGGGCCCGCGGGCC GGGCGGGCTCGGGGCGGCCGGGACGAGGAGGGG CGACG | 2022 |
| HPV39 | bkpt1-MYC_001_exon1 | 1045 | 1046 | AAATACTAGAATACTATGAACAAGACAGTAAATC AATATATGATCAAATTAATTATTGGAAATGTGTGC GAATGGCTGCTCGCGGCCGCCACCGCCGGGCCCC GGCCGTCCCTGGCTCCCCTCCTGCCTCGA | 2023 |
| HPV39 | bkpt1-MYC_001_exon2 | 1047 | 1048 | AAATACTAGAATACTATGAACAAGACAGTAAATC AATATATGATCAAATTAATTATTGGAAATGTGTGC GAATGGCAGCCTCCCGCGACGATGCCCCTCAACG TTAGCTTCACCAACAGGAACTATGACCTCGACTAC G | 2024 |
| HPV39 | bkpt1-MYC_001_exon3 | 1049 | 1050 | AAATACTAGAATACTATGAACAAGACAGTAAATC AATATATGATCAAATTAATTATTGGAAATGTGTGC GAATGGAGGAGGAACAAGAAGATGAGGAAGAAA TCGATGTTGTTTCTGTGGAAAAGAGGCAGGCTCCT GG | 2025 |
| HPV39 | bkpt1-PVT1_002_exon3 | 1051 | 1052 | AAATACTAGAATACTATGAACAAGACAGTAAATC AATATATGATCAAATTAATTATTGGAAATGTGTGC GAATGGCTGACCATACTCCCTGGAGCCTTCTCCCG AGGTGCGCGGGTGACCT | 2026 |
| HPV39 | bkpt1-PVT1_004_exon1 | 1053 | 1054 | AAATACTAGAATACTATGAACAAGACAGTAAATC AATATATGATCAAATTAATTATTGGAAATGTGTGC GAATGGTCTGAGCCTGATGGATTTACAGTGATCTT CAGTGGTCTGGGG | 2027 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV39 | bkpt1-PVT1_005_exon1 | 1055 | 1056 | AAATACTAGAATACTATGAACAAGACAGTAAATCAATATATGATCAAATTAATTATTGGAAATGTGTGCGAATGGCTCCGGCAGAGCGCGTGTGGCGCCGAGCACATGGGCCCGCGGCCGCGGGCGCGGGGCGGCCGGGGACGAGGAGGGGCGACG | 2028 |
| HPV39 | bkpt2-MYC_001_exon1 | 1057 | 1058 | ACACAAGACGGTACCTCAGTTGTGGTAACACTACGCCTATAAACTGCTGCGGCGCCACGCGCCGGGCCCCGGCCCGTCCCTGGCTCCCCTCGA | 2029 |
| HPV39 | bkpt2-MYC_001_exon2 | 1059 | 1060 | ACACAAGACGGTACCTCAGTTGTGGTAACACTACGCCTATAAACAGCCTCCCGCGACGATGCCCCTCAACGTTAGCTTCACCAACAGGAACTATGACCTCGACTACG | 2030 |
| HPV39 | bkpt2-MYC_001_exon3 | 1061 | 1062 | ACACAAGACGGTACCTCAGTTGTGGTAACACTACGCCTATAAAGGAGGAACAAGAAGATGAGGAAGAAATCGATGTTGTTTCTGTGGAAAAGAGGCAGGCTCCTGG | 2031 |
| HPV39 | bkpt2-PVT1_002_exon3 | 1063 | 1064 | ACACAAGACGGTACCTCAGTTGTGGTAACACTACGCCTATAAACTGACCATACTCCCCTGGAGCCTTCTCCCGAGGTGCGCGGGGTGACCT | 2032 |
| HPV39 | bkpt2-PVT1_004_exon1 | 1065 | 1066 | ACACAAGACGGTACCTCAGTTGTGGTAACACTACGCCTATAAATCTGAGCCTGATGGATTTTACAGTGATCTTCAGTGGTCTGGGG | 2033 |
| HPV39 | bkpt2-PVT1_005_exon1 | 1067 | 1068 | ACACAAGACGGTACCTCAGTTGTGGTAACACTACGCCTATAAACTCCGGGCAGAGCGCGTGTGGCGGCCGAGCACATGGGCCCGCGGCCCGCGGGGCGCGGGCTCGGGGCGGGCCGCGGAGGACGAGGAGGGGGCGACG | 2034 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV39 | SD2-MYC_001_exon1 | 1069 | 1070 | ACCAGTAACCTGCTATGGCCAATCGTGAAGCTGCT CGCGGCCGCCACCGCCGGGCCCCGGCCGTCCCTG GCTCCCCTCCTGCCTCGA | 2035 |
| HPV39 | SD2-MYC_001_exon2 | 1071 | 1072 | ACCAGTAACCTGCTATGGCCAATCGTGAAGCAGC CTCCCGCGACGATGCCCCTCAACGTTAGCTTCACC AACAGGAACTATGACCTCGACTACG | 2036 |
| HPV39 | SD2-MYC_001_exon3 | 1073 | 1074 | ACCAGTAACCTGCTATGGCCAATCGTGAAGAGGA GGAACAAGAAGATGAGGAAGAAATCGATGTTGTT TCTGTGGAAAGAGGCAGGCTCCTGG | 2037 |
| HPV39 | SD2-PVT1_002_exon3 | 1075 | 1076 | ACCAGTAACCTGCTATGGCCAATCGTGAAGCTGA CCATACTCCCTGGAGCCTTCTCCCGAGGTGCGCGG GTGACCT | 2038 |
| HPV39 | SD2-PVT1_004_exon1 | 1077 | 1078 | ACCAGTAACCTGCTATGGCCAATCGTGAAGTCTGA GCCTGATGGATTTACAGTGATCTTCAGTGGTCTGG GG | 2039 |
| HPV39 | SD2-PVT1_005_exon1 | 1079 | 1080 | ACCAGTAACCTGCTATGGCCAATCGTGAAGCTCCG GGCAGAGCGCGTGTGGCGGCCGAGCACATGGGCC CGCGGGCCGGGCGGGCTCGGGGCGGCCGGGACGA GGAGGGGCGACG | 2040 |
| HPV45 | bkpt1-MYC_001_exon1 | 1081 | 1082 | TGAAAATGACAGTAAAGACATAAACAGCCAAATA AGTTATTGGCAACTTATACGTTTGGCTGCTCGCGG CCGCCACCGCCGGGCCCCGGCCGTCCCTGGCTCCC CTCCTGCCTCGA | 2041 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV45 | bkpt1-MYC_001_exon2 | 1083 | 1084 | TGAAAATGACAGTAAAGACATAAACAGCCAAATA AGTTATTGGCAACTTATACGTTTGGCAGCCTCCCG CGACGATGCCCCTCAACGTTAGCTTCACCAACAGG AACTATGACCTCGACTACG | 2042 |
| HPV45 | bkpt1-MYC_001_exon3 | 1085 | 1086 | TGAAAATGACAGTAAAGACATAAACAGCCAAATA AGTTATTGGCAACTTATACGTTTGGAGGAGGAAC AAGAAGATGAGGAAGAAATCGATGTTGTTTCTGT GGAAAAGAGGCAGGCTCCTGG | 2043 |
| HPV45 | bkpt1-PVT1_002_exon3 | 1087 | 1088 | TGAAAATGACAGTAAAGACATAAACAGCCAAATA AGTTATTGGCAACTTATACGTTTGGCTGACCATAC TCCCTGGAGCCTTCTCCCGAGGTGCGCGGGTGACC T | 2044 |
| HPV45 | bkpt1-PVT1_004_exon1 | 1089 | 1090 | TGAAAATGACAGTAAAGACATAAACAGCCAAATA AGTTATTGGCAACTTATACGTTTGGTCTGAGCCTG ATGGATTTACAGTGATCTTCAGTGGTCTGGGG | 2045 |
| HPV45 | bkpt1-PVT1_005_exon1 | 1091 | 1092 | TGAAAATGACAGTAAAGACATAAACAGCCAAATA AGTTATTGGCAACTTATACGTTTGGCTCCGGGCAG AGCGCGTGTGGCGGCCGAGCACATGGGCCCGCGG GCCGGGCGGGCTCGGGGCGGCCGGGACGAGGAG GGGCGACG | 2046 |
| HPV45 | bkpt2-MYC_001_exon1 | 1093 | 1094 | AAGAAGGAAAGTGTGTAGTGGTAACACTACGCCT ATAACTGCTCGCGGCCGCCACCGCCGGGCCCCGG CCGTCCCTGGCTCCCCTCCTGCCTCGA | 2047 |
| HPV45 | bkpt2-MYC_001_exon2 | 1095 | 1096 | AAGAAGGAAAGTGTGTAGTGGTAACACTACGCCT ATAACAGCCTCCCGCGACGATGCCCCTCAACGTTA GCTTCACCAACAGGAACTATGACCTCGACTACG | 2048 |

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV45 | bkpt2-MYC_ 001_exon3 | 1097 | 1098 | AAGAAGGAAAGTGTGTAGTGGTAACACTACGCCT ATAAAGGAGGAACAAGAAGATGAGGAAGAAATC GATGTTGTTTCTGTGGAAAAGAGGCAGGCTCCTGG | 2049 |
| HPV45 | bkpt2- PVT1_002_exon3 | 1099 | 1100 | AAGAAGGAAAGTGTGTAGTGGTAACACTACGCCT ATAACTGACCATACTCCCTGGAGCCTTCTCCCGAG GTGCGCGGGTGACCT | 2050 |
| HPV45 | bkpt2- PVT1_004_exon1 | 1101 | 1102 | AAGAAGGAAAGTGTGTAGTGGTAACACTACGCCT ATAATCTGAGCCTGATGGATTTACAGTGATCTTCA GTGGTCTGGGG | 2051 |
| HPV45 | bkpt2- PVT1_005_exon1 | 1103 | 1104 | AAGAAGGAAAGTGTGTAGTGGTAACACTACGCCT ATAACTCCGGGCAGAGCGCGTGTGGCGGCCGAGC ACATGGGCCCGCGGGCCGGGCGGGCTCGGGGCGG CCGGGACGAGGAGGGGCGACG | 2052 |
| HPV45 | SD2-MYC_ 001_exon1 | 1105 | 1106 | CCGTGGTGTGCAACTAACCAATAATCTACAATGGC GGATCCAGAAGCTGCTCGCGGCCGCCACCGCCGG GCCCCGGCCGTCCCTGGCTCCCCTCCTGCCTCGA | 2053 |
| HPV45 | SD2-MYC_ 001_exon2 | 1107 | 1108 | CCGTGGTGTGCAACTAACCAATAATCTACAATGGC GGATCCAGAAGCAGCCTCCCGCGACGATGCCCCT CAACGTTAGCTTCACCAACAGGAACTATGACCTCG ACTACG | 2054 |
| HPV45 | SD2-MYC_ 001_exon3 | 1109 | 1110 | CCGTGGTGTGCAACTAACCAATAATCTACAATGGC GGATCCAGAAGAGGAGGAACAAGAAGATGAGGA AGAAATCGATGTTGTTTCTGTGGAAAAGAGGCAG GCTCCTGG | 2055 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV45 | SD2-PVT1_002_exon3 | 1111 | 1112 | CCGTGGTGTGCAACTAACCAATAATCTACAATGGC GGATCCAGAAGCTGACCATACTCCCTGGAGCCTTC TCCCGAGGTGCGCGGGTGACCT | 2056 |
| HPV45 | SD2-PVT1_004_exon1 | 1113 | 1114 | CCGTGGTGTGCAACTAACCAATAATCTACAATGGC GGATCCAGAAGTCTGAGCCTGATGGATTTACAGT GATCTTCAGTGGTCTGGGG | 2057 |
| HPV45 | SD2-PVT1_005_exon1 | 1115 | 1116 | CCGTGGTGTGCAACTAACCAATAATCTACAATGGC GGATCCAGAAGCTCCGGGCAGAGCGCGTGTGGCG GCCGAGCACATGGGCCCGCGGGCCGGGCGGGCTC GGGGCGGCCGGGACGAGGAGGGGCGACG | 2058 |
| HPV51 | bkpt1-MYC_001_exon1 | 1117 | 1118 | GAACTGGACAGTGATAAATTAGTAGATCAAATTA ACTATTGGACATTGTTACGATATGCTGCTCGCGGC CGCCACCGCCGGGCCCCGGCCGTCCCTGGCTCCCC TCCTGCCTCGA | 2059 |
| HPV51 | bkpt1-MYC_001_exon2 | 1119 | 1120 | GAACTGGACAGTGATAAATTAGTAGATCAAATTA ACTATTGGACATTGTTACGATATGCAGCCTCCCGC GACGATGCCCCTCAACGTTAGCTTCACCAACAGG AACTATGACCTCGACTACG | 2060 |
| HPV51 | bkpt1-MYC_001_exon3 | 1121 | 1122 | GAACTGGACAGTGATAAATTAGTAGATCAAATTA ACTATTGGACATTGTTACGATATGAGGAGGAACA AGAAGATGAGGAAGAAATCGATGTTGTTTCTGTG GAAAAGAGGCAGGCTCCTGG | 2061 |
| HPV51 | bkpt1-PVT1_002_exon3 | 1123 | 1124 | GAACTGGACAGTGATAAATTAGTAGATCAAATTA ACTATTGGACATTGTTACGATATGCTGACCATACT CCCTGGAGCCTTCTCCCGAGGTGCGCGGGTGACCT | 2062 |

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV51 | bkpt1-PVT1_004_exon1 | 1125 | 1126 | GAACTGGACAGTGATAAATTAGTAGATCAAATTAACTATTGGACATTGTTACGATATGTCTGAGCCTGATGGATTTACAGTGATCTTCAGTGGTCTGGGG | 2063 |
| HPV51 | bkpt1-PVT1_005_exon1 | 1127 | 1128 | GAACTGGACAGTGATAAATTAGTAGATCAAATTAACTATTGGACATTGTTACGATATGCTCCGGGCAGAGCGCGTGTGGCGGCCGAGCACATGGGCCCGCGGGCCGGGCGGGCTCGGGGCGGCCGGGACGAGGAGGGGCGACG | 2064 |
| HPV51 | bkpt2-MYC_001_exon1 | 1129 | 1130 | GTGCAACTCAGACTGCGTTTATAGCTGCTCGCGGCCGCCACCGCCGGGCCCCGGCCGTCCCTGGCTCCCCTCCTGCCTCGA | 2065 |
| HPV51 | bkpt2-MYC_001_exon2 | 1131 | 1132 | GTGCAACTCAGACTGCGTTTATAGCAGCCTCCCGCGACGATGCCCCTCAACGTTAGCTTCACCAACAGGAACTATGACCTCGACTACG | 2066 |
| HPV51 | bkpt2-MYC_001_exon3 | 1133 | 1134 | GTGCAACTCAGACTGCGTTTATAGAGGAGGAACAAGAAGATGAGGAAGAAATCGATGTTGTTTCTGTGGAAAAGAGGCAGGCTCCTGG | 2067 |
| HPV51 | bkpt2-PVT1_002_exon3 | 1135 | 1136 | GTGCAACTCAGACTGCGTTTATAGCTGACCATACTCCCTGGAGCCTTCTCCCGAGGTGCGCGGGTGACCT | 2068 |
| HPV51 | bkpt2-PVT1_004_exon1 | 1137 | 1138 | GTGCAACTCAGACTGCGTTTATAGTCTGAGCCTGATGGATTTACAGTGATCTTCAGTGGTCTGGGG | 2069 |
| HPV51 | bkpt2-PVT1_005_exon1 | 1139 | 1140 | GTGCAACTCAGACTGCGTTTATAGCTCCGGGCAGAGCGCGTGTGGCGGCCGAGCACATGGGCCCGCGGGCCGGGCGGGCTCGGGGCGGCCGGGACGAGGAGGGGCGACG | 2070 |

154

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV51 | SD2-MYC_001_exon1 | 1141 | 1142 | GCCCGTGTTGTGCGAACAACTAGCAACGGCGATG GACTGTGAAGCTGCTCGCGGCCGCCACCGCCGGG CCCCGGCCGTCCCTGGCTCCCCTCCTGCCTCGA | 2071 |
| HPV51 | SD2-MYC_001_exon2 | 1143 | 1144 | GCCCGTGTTGTGCGAACAACTAGCAACGGCGATG GACTGTGAAGCAGCCTCCCGCGACGATGCCCCTC AACGTTAGCTTCACCAACAGGAACTATGACCTCG ACTACG | 2072 |
| HPV51 | SD2-MYC_001_exon3 | 1145 | 1146 | GCCCGTGTTGTGCGAACAACTAGCAACGGCGATG GACTGTGAAGAGGAGGAACAAGAAGATGAGGAA GAAATCGATGTTGTTTCTGTGGAAAAGAGGCAGG CTCCTGG | 2073 |
| HPV51 | SD2-PVT1_002_exon3 | 1147 | 1148 | GCCCGTGTTGTGCGAACAACTAGCAACGGCGATG GACTGTGAAGCTGACCATACTCCCTGGAGCCTTCT CCCGAGGTGCGCGGGTGACCT | 2074 |
| HPV51 | SD2-PVT1_004_exon1 | 1149 | 1150 | GCCCGTGTTGTGCGAACAACTAGCAACGGCGATG GACTGTGAAGTCTGAGCCTGATGGATTTACAGTGA TCTTCAGTGGTCTGGGG | 2075 |
| HPV51 | SD2-PVT1_005_exon1 | 1151 | 1152 | GCCCGTGTTGTGCGAACAACTAGCAACGGCGATG GACTGTGAAGCTCCGGGCAGAGCGCGTGTGGCGG CCGAGCACATGGGCCCGCGGGCCGGGCGGGCTCG GGGCGGCCGGGACGAGGAGGGGCGACG | 2076 |
| HPV52 | bkpt1-MYC_001_exon1 | 1153 | 1154 | TTGAACATTGGAAATTGACTCGAATGGCTGCTCGC GGCCGCCACCGCCGGGCCCCGGCCGTCCCTGGCTC CCCTCCTGCCTCGA | 2077 |
| HPV52 | bkpt1-MYC_001_exon2 | 1155 | 1156 | TTGAACATTGGAAATTGACTCGAATGGCAGCCTCC CGCGACGATGCCCCTCAACGTTAGCTTCACCAACA | 2078 |

| HPV type | Region name | Forward primer SEQ ID NO | Reverse primer SEQ ID NO | Amplicon nucleic acid sequence | Amplicon SEQ ID NO |
|---|---|---|---|---|---|
| | | | | GGAACTATGACCTCGACTACG | |
| HPV52 | bkpt1-MYC_ 001_exon3 | 1157 | 1158 | TTGAACATTGGAAATTGACTCGAATGGAGGAGGA ACAAGAAGATGAGGAAGAAATCGATGTTGTTTCT GTGGAAAAGAGGCAGGCTCCTGG | 2079 |
| HPV52 | bkpt1- PVT1_002_exon3 | 1159 | 1160 | TTGAACATTGGAAATTGACTCGAATGGCTGACCAT ACTCCCTGGAGCCTTCTCCCGAGGTGCGCGGGTGA CCT | 2080 |
| HPV52 | bkpt1- PVT1_004_exon1 | 1161 | 1162 | TTGAACATTGGAAATTGACTCGAATGGTCTGAGCC TGATGGATTTACAGTGATCTTCAGTGGTCTGGGG | 2081 |
| HPV52 | bkpt1- PVT1_005_exon1 | 1163 | 1164 | TTGAACATTGGAAATTGACTCGAATGGCTCCGGGC AGAGCGCGTGTGGCGGCCGAGCACATGGGCCCGC GGGCCGGGCGGGCTCGGGGCGGCCGGGACGAGG AGGGGCGACG | 2082 |
| HPV52 | bkpt2-MYC_ 001_exon1 | 1165 | 1166 | ACAAAGGACGGGTTGCACATACAACTTGTACTGC ACCTATAACTGCTCGCGGCCGCCACCGCCGGGCCC CGGCCGTCCCTGGCTCCCCTCCTGCCTCGA | 2083 |
| HPV52 | bkpt2-MYC_ 001_exon2 | 1167 | 1168 | ACAAAGGACGGGTTGCACATACAACTTGTACTGC ACCTATAACAGCCTCCCGCGACGATGCCCCTCAAC GTTAGCTTCACCAACAGGAACTATGACCTCGACTA CG | 2084 |
| HPV52 | bkpt2-MYC_ 001_exon3 | 1169 | 1170 | ACAAAGGACGGGTTGCACATACAACTTGTACTGC ACCTATAAAGGAGGAACAAGAAGATGAGGAAGA AATCGATGTTGTTTCTGTGGAAAAGAGGCAGGCTC CTGG | 2085 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV52 | bkpt2-PVT1_002_exon3 | 1171 | 1172 | ACAAAGGACGGGTTGCACATACAACTTGTACTGCACCTATAACTGACCATACTCCCTGGAGCCTTCTCCCGAGGTGCGCGGGTGACCT | 2086 |
| HPV52 | bkpt2-PVT1_004_exon1 | 1173 | 1174 | ACAAAGGACGGGTTGCACATACAACTTGTACTGCACCTATAATCTGAGCCTGATGGATTTACAGTGATCTTCAGTGGTCTGGGG | 2087 |
| HPV52 | bkpt2-PVT1_005_exon1 | 1175 | 1176 | ACAAAGGACGGGTTGCACATACAACTTGTACTGCACCTATAACTCCGGGCAGAGCGCGTGTGGCGGCCGAGCACATGGGCCCGCGGGCCGGGCGGGCTCGGGGCGGCCGGGACGAGGAGGGGCGACG | 2088 |
| HPV52 | SD2-MYC_001_exon1 | 1177 | 1178 | GTGTGCCCCGGCTGTGCACGGCTATAAACAACCCTGCAATGGAGGACCCTGAAGCTGCTCGCGGCCGCCACCGCCGGGCCCCGGCCGTCCCTGGCTCCCCTCCTGCCTCGA | 2089 |
| HPV52 | SD2-MYC_001_exon2 | 1179 | 1180 | GTGTGCCCCGGCTGTGCACGGCTATAAACAACCCTGCAATGGAGGACCCTGAAGCAGCCTCCCGCGACGATGCCCCTCAACGTTAGCTTCACCAACAGGAACTATGACCTCGACTACG | 2090 |
| HPV52 | SD2-MYC_001_exon3 | 1181 | 1182 | GTGTGCCCCGGCTGTGCACGGCTATAAACAACCCTGCAATGGAGGACCCTGAAGAGGAGGAACAAGAAGATGAGGAAGAAATCGATGTTGTTTCTGTGGAAAAGAGGCAGGCTCCTGG | 2091 |
| HPV52 | SD2-PVT1_002_exon3 | 1183 | 1184 | GTGTGCCCCGGCTGTGCACGGCTATAAACAACCCTGCAATGGAGGACCCTGAAGCTGACCATACTCCCTGGAGCCTTCTCCCGAGGTGCGCGGGTGACCT | 2092 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV52 | SD2-PVT1_004_exon1 | 1185 | 1186 | GTGTGCCCCGGCTGTGCACGGCTATAAACAACCCTGCAATGGAGGACCCTGAAGTCTGAGCCTGATGGATTTACAGTGATCTTCAGTGGTCTGGGG | 2093 |
| HPV52 | SD2-PVT1_005_exon1 | 1187 | 1188 | GTGTGCCCCGGCTGTGCACGGCTATAAACAACCCTGCAATGGAGGACCCTGAAGCTCCGGGCAGAGCGCGTGTGGCGGCCGAGCACATGGGCCCGCGGGCCGGGCGGGCTCGGGGCGGCCGGGACGAGGAGGGGCGACG | 2094 |
| HPV56 | bkpt1-MYC_001_exon1 | 1189 | 1190 | TGAAAAAGATAGTAGATGTATTGCAGATCATATAGAATATTGGAAAGCTGTGCGACATGCTGCTCGCGGCCGCCACCGCCGGGCCCCGGCCGTCCCTGGCTCCCCTCCTGCCTCGA | 2095 |
| HPV56 | bkpt1-MYC_001_exon2 | 1191 | 1192 | TGAAAAAGATAGTAGATGTATTGCAGATCATATAGAATATTGGAAAGCTGTGCGACATGCAGCCTCCCGCGACGATGCCCCTCAACGTTAGCTTCACCAACAGGAACTATGACCTCGACTACG | 2096 |
| HPV56 | bkpt1-MYC_001_exon3 | 1193 | 1194 | TGAAAAAGATAGTAGATGTATTGCAGATCATATAGAATATTGGAAAGCTGTGCGACATGAGGAGGAACAAGAAGATGAGGAAGAAATCGATGTTGTTTCTGTGGAAAGAGGCAGGCTCCTGG | 2097 |
| HPV56 | bkpt1-PVT1_002_exon3 | 1195 | 1196 | TGAAAAAGATAGTAGATGTATTGCAGATCATATAGAATATTGGAAAGCTGTGCGACATGCTGACCATACTCCCTGGAGCCTTCTCCCGAGGTGCGCGGGTGACCT | 2098 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV56 | bkpt1-PVT1_004_exon1 | 1197 | 1198 | TGAAAAAAGATAGTAGATGTATTGCAGATCATAT AGAATATTGGAAAGCTGTGCGACATGTCTGAGCC TGATGGATTTACAGTGATCTTCAGTGGTCTGGGG | 2099 |
| HPV56 | bkpt1-PVTI_005_exon1 | 1199 | 1200 | TGAAAAAAGATAGTAGATGTATTGCAGATCATAT AGAATATTGGAAAGCTGTGCGACATGCTCCGGGC AGAGCGCGTGTGGCGGCCGAGCACATGGGCCCGC GGGCCGGGCGGGCTCGGGGCGGCCGGGACGAGG AGGGGCGACG | 2100 |
| HPV56 | bkpt2-MYC_001_exon1 | 1201 | 1202 | ACTACGCCTGTAGCTGCTCGCGGCCGCCACCGCCG GGCCCCGGCCGTCCCTGGCTCCCCTCCTGCCTCGA | 2101 |
| HPV56 | bkpt2-MYC_001_exon2 | 1203 | 1204 | ACTACGCCTGTAGCAGCCTCCCGCGACGATGCCCC TCAACGTTAGCTTCACCAACAGGAACTATGACCTC GACTACG | 2102 |
| HPV56 | bkpt2-MYC_001_exon3 | 1205 | 1206 | ACTACGCCTGTAGAGGAGGAACAAGAAGATGAGG AAGAAATCGATGTTGTTTCTGTGGAAAAGAGGCA GGCTCCTGG | 2103 |
| HPV56 | bkpt2-PVT1_002_exon3 | 1207 | 1208 | ACTACGCCTGTAGCTGACCATACTCCCTGGAGCCT TCTCCCGAGGTGCGCGGGTGACCT | 2104 |
| HPV56 | bkpt2-PVT1_004_exon1 | 1209 | 1210 | ACTACGCCTGTAGTCTGAGCCTGATGGATTTACAG TGATCTTCAGTGGTCTGGGG | 2105 |
| HPV56 | bkpt2-PVT1_005_exon1 | 1211 | 1212 | ACTACGCCTGTAGCTCCGGGCAGAGCGCGTGTGG CGGCCGAGCACATGGGCCCGCGGGCCGGGCGGGC TCGGGGCGGCCGGGACGAGGAGGGGCGACG | 2106 |

159

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV56 | SD2-MYC_001_exon1 | 1213 | 1214 | GCGCATCAAGTAACTAACTGCAATGGCGTCACCT GAAGCTGCTCGCGGCCGCCACCGCCGGGCCCCGG CCGTCCCTGGCTCCCCTCCTGCCTCGA | 2107 |
| HPV56 | SD2-MYC_001_exon2 | 1215 | 1216 | GCGCATCAAGTAACTAACTGCAATGGCGTCACCT GAAGCAGCCTCCCGCGACGATGCCCCTCAACGTT AGCTTCACCAACAGGAACTATGACCTCGACTACG | 2108 |
| HPV56 | SD2-MYC_001_exon3 | 1217 | 1218 | GCGCATCAAGTAACTAACTGCAATGGCGTCACCT GAAGAGGAGGAACAAGAAGATGAGGAAGAAATC GATGTTGTTTCTGTGGAAAAGAGGCAGGCTCCTGG | 2109 |
| HPV56 | SD2-PVT1_002_exon3 | 1219 | 1220 | GCGCATCAAGTAACTAACTGCAATGGCGTCACCT GAAGCTGACCATACTCCCTGGAGCCTTCTCCCGAG GTGCGCGGGTGACCT | 2110 |
| HPV56 | SD2-PVT1_004_exon1 | 1221 | 1222 | GCGCATCAAGTAACTAACTGCAATGGCGTCACCT GAAGTCTGAGCCTGATGGATTTACAGTGATCTTCA GTGGTCTGGGG | 2111 |
| HPV56 | SD2-PVT1_005_exon1 | 1223 | 1224 | GCGCATCAAGTAACTAACTGCAATGGCGTCACCT GAAGCTCCGGGCAGAGCGCGTGTGGCGGCCGAGC ACATGGGCCCGCGGGCCGGGCGGGCTCGGGGCGG CCGGGACGAGGAGGGGCGACG | 2112 |
| HPV58 | bkpt1-MYC_001_exon1 | 1225 | 1226 | GCTGATAAAAATGATTTAACATCACAAATTGAAC<br><br>ATTGGAAACTAATACGCATGGCTGCTCGCGGCCG CCACCGCCGGGCCCCGGCCGTCCCTGGCTCCCCTC CTGCCTCGA | 2113 |

EP 3 715 477 A1

160

| HPV type | Region name | Forward primer SEQ ID NO | Reverse primer SEQ ID NO | Amplicon nucleic acid sequence | Amplicon SEQ ID NO |
|---|---|---|---|---|---|
| HPV58 | bkpt1-MYC_ 001_exon2 | 1227 | 1228 | GCTGATAAAAATGATTTAACATCACAAATTGAAC ATTGGAAACTAATACGCATGGCAGCCTCCCGCGA CGATGCCCCTCAACGTTAGCTTCACCAACAGGAAC TATGACCTCGACTACG | 2114 |
| HPV58 | bkpt1-MYC_ 001_exon3 | 1229 | 1230 | GCTGATAAAAATGATTTAACATCACAAATTGAAC ATTGGAAACTAATACGCATGGAGGAGGAACAAGA AGATGAGGAAGAAATCGATGTTGTTTCTGTGGAA AAGAGGCAGGCTCCTGG | 2115 |
| HPV58 | bkpt1- PVT1_002_exon3 | 1231 | 1232 | GCTGATAAAAATGATTTAACATCACAAATTGAAC ATTGGAAACTAATACGCATGGCTGACCATACTCCC TGGAGCCTTCTCCCGAGGTGCGCGGGTGACCT | 2116 |
| HPV58 | bkpt1- PVT1_004_exon1 | 1233 | 1234 | GCTGATAAAAATGATTTAACATCACAAATTGAAC ATTGGAAACTAATACGCATGGTCTGAGCCTGATG GATTTACAGTGATCTTCAGTGGTCTGGGG | 2117 |
| HPV58 | bkpt1- PVT1_005_exon1 | 1235 | 1236 | GCTGATAAAAATGATTTAACATCACAAATTGAAC ATTGGAAACTAATACGCATGGCTCCGGGCAGAGC GCGTGTGGCGGCCGAGCACATGGGCCCGCGGCC GGGCGGGCTCGGGGCGGCCGGGACGAGGAGGGG CGACG | 2118 |
| HPV58 | bkpt2-MYC_ 001_exon1 | 1237 | 1238 | GTACATACAAAGGGCGGAACGTGTGTAGTTCTAA AGTTTCACCTATCGCTGCTCGCGGCCGCCACCGCC GGGCCCCGGCCGTCCCTGGCTCCCCTCCTGCCTCG A | 2119 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV58 | bkpt2-MYC_001_exon2 | 1239 | 1240 | GTACATACAAAGGGCGGAACGTGTGTAGTTCTAA AGTTTCACCTATCGCAGCCTCCCGCGACGATGCCC CTCAACGTTAGCTTCACCAACAGGAACTATGACCT CGACTACG | 2120 |
| HPV58 | bkpt2-MYC_001_exon3 | 1241 | 1242 | GTACATACAAAGGGCGGAACGTGTGTAGTTCTAA AGTTTCACCTATCGAGGAGGAACAAGAAGATGAG GAAGAAATCGATGTTGTTTCTGTGGAAAAGAGGC AGGCTCCTGG | 2121 |
| HPV58 | bkpt2-PVT1_002_exon3 | 1243 | 1244 | GTACATACAAAGGGCGGAACGTGTGTAGTTCTAA AGTTTCACCTATCGCTGACCATACTCCCTGGAGCC TTCTCCCGAGGTGCGCGGGTGACCT | 2122 |
| HPV58 | bkpt2-PVT1_004_exon1 | 1245 | 1246 | GTACATACAAAGGGCGGAACGTGTGTAGTTCTAA AGTTTCACCTATCGTCTGAGCCTGATGGATTTACA GTGATCTTCAGTGGTCTGGGG | 2123 |
| HPV58 | bkpt2-PVTI_005_exon1 | 1247 | 1248 | GTACATACAAAGGGCGGAACGTGTGTAGTTCTAA AGTTTCACCTATCGCTCCGGGCAGAGCGCGTGTGG CGGCCGAGCACATGGGCCCGCGGGCCGGGCGGGC TCGGGGCGGCCGGGACGAGGAGGGGCGACG | 2124 |
| HPV58 | SD2-MYC_001_exon1 | 1249 | 1250 | GTGTGCCCTAGCTGTGCACAGCAATAAACACCATC TGCAATGGATGACCCTGAAGCTGCTCGCGGCCGC CACCGCCGGGCCCCGGCCGTCCCTGGCTCCCCTCC TGCCTCGA | 2125 |
| HPV58 | SD2-MYC_001_exon2 | 1251 | 1252 | GTGTGCCCTAGCTGTGCACAGCAATAAACACCATC TGCAATGGATGACCCTGAAGCAGCCTCCCGCGAC GATGCCCTCAACGTTAGCTTCACCAACAGGAACT ATGACCTCGACTACG | 2126 |

162

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV58 | SD2-MYC_001_exon3 | 1253 | 1254 | GTGTGCCCTAGCTGTGCACAGCAATAAACACCATCTGCAATGGATGACCCTGAAGAGGAGGAACAAGAAGATGAGGAAGAAATCGATGTTGTTTCTGTGGAAAAGAGGCAGGCTCCTGG | 2127 |
| HPV58 | SD2-PVT1_002_exon3 | 1255 | 1256 | GTGTGCCCTAGCTGTGCACAGCAATAAACACCATCTGCAATGGATGACCCTGAAGCTGACCATACTCCCTGGAGCCTTCTCCCGAGGTGCGCGGGTGACCT | 2128 |
| HPV58 | SD2-PVT1_004_exon1 | 1257 | 1258 | GTGTGCCCTAGCTGTGCACAGCAATAAACACCATC<br><br>TGCAATGGATGACCCTGAAGTCTGAGCCTGATGGATTTACAGTGATCTTCAGTGGTCTGGGG | 2129 |
| HPV58 | SD2-PVT1_005_exon1 | 1259 | 1260 | GTGTGCCCTAGCTGTGCACAGCAATAAACACCATCTGCAATGGATGACCCTGAAGCTCCGGGCAGAGCGCGTGTGGCGGCCGAGCACATGGGCCCGCGGGCCGGGCGGGCTCGGGGCGGCCGGGACGAGGAGGGGCGACG | 2130 |
| HPV59 | bkpt1-MYC_001_exon1 | 1261 | 1262 | ATTAGAACATTATGAAAACGATAGTAAAGACATTAATGAACACATAAACTATTGGAAACTGGTGCGTATGGCTGCTCGCGGCCGCCACCGCCGGGCCCCGGCCGTCCCTGGCTCCCCTCCTGCCTCGA | 2131 |
| HPV59 | bkpt1-MYC_001_exon2 | 1263 | 1264 | ATTAGAACATTATGAAAACGATAGTAAAGACATTAATGAACACATAAACTATTGGAAACTGGTGCGTATGGCAGCCTCCCGCGACGATGCCCCTCAACGTTAGCTTCACCAACAGGAACTATGACCTCGACTACG | 2132 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV59 | bkpt1-MYC_001_exon3 | 1265 | 1266 | ATTAGAACATTATGAAAACGATAGTAAAGACATT AATGAACACATAAACTATTGGAAACTGGTGCGTA TGGAGGAGGAACAAGAAGATGAGGAAGAAATCG ATGTTGTTTCTGTGGAAAAGAGGCAGGCTCCTGG | 2133 |
| HPV59 | bkpt1-PVT1_002_exon3 | 1267 | 1268 | ATTAGAACATTATGAAAACGATAGTAAAGACATT AATGAACACATAAACTATTGGAAACTGGTGCGTA TGGCTGACCATACTCCCTGGAGCCTTCTCCCGAGG TGCGCGGGTGACCT | 2134 |
| HPV59 | bkpt1-PVT1_004_exon1 | 1269 | 1270 | ATTAGAACATTATGAAAACGATAGTAAAGACATT AATGAACACATAAACTATTGGAAACTGGTGCGTA TGGTCTGAGCCTGATGGATTTACAGTGATCTTCAG TGGTCTGGGG | 2135 |
| HPV59 | bkpt1-PVT1_005_exon1 | 1271 | 1272 | ATTAGAACATTATGAAAACGATAGTAAAGACATT AATGAACACATAAACTATTGGAAACTGGTGCGTA TGGCTCCGGGCAGAGCGCGTGTGGCGGCCGAGCA CATGGGCCCGCGGGCCGGGCGGGCTCGGGGCGGC CGGGACGAGGAGGGGCGACG | 2136 |
| HPV59 | bkpt2-MYC_001_exon1 | 1273 | 1274 | CAACCCGCGACGGCACATCCCTTGCAGTAACACT ACGCCTATAACTGCTCGCGGCCGCCACCGCCGGG CCCCGGCCGTCCCTGGCTCCCCTCCTGCCTCGA | 2137 |
| HPV59 | bkpt2-MYC_001_exon2 | 1275 | 1276 | CAACCCGCGACGGCACATCCCTTGCAGTAACACT ACGCCTATAACAGCCTCCCGCGACGATGCCCCTCA ACGTTAGCTTCACCAACAGGAACTATGACCTCGAC TACG | 2138 |

EP 3 715 477 A1

164

| HPV type | Region name | Forward primer SEQ ID NO | Reverse primer SEQ ID NO | Amplicon nucleic acid sequence | Amplicon SEQ ID NO |
|---|---|---|---|---|---|
| HPV59 | bkpt2-MYC_ 001_exon3 | 1277 | 1278 | CAACCCGCGACGGCACATCCCTTGCAGTAACACT ACGCCTATAAAGGAGGAACAAGAAGATGAGGAA GAAATCGATGTTGTTTCTGTGGAAAAGAGGCAGG CTCCTGG | 2139 |
| HPV59 | bkpt2- PVT1_002_exon3 | 1279 | 1280 | CAACCCGCGACGGCACATCCCTTGCAGTAACACT ACGCCTATAACTGACCATACTCCCTGGAGCCTTCT CCCGAGGTGCGCGGGTGACCT | 2140 |
| HPV59 | bkpt2- PVT1_004_exon1 | 1281 | 1282 | CAACCCGCGACGGCACATCCCTTGCAGTAACACT ACGCCTATAATCTGAGCCTGATGGATTTACAGTGA TCTTCAGTGGTCTGGGG | 2141 |
| HPV59 | bkpt2- PVT1_005_exon1 | 1283 | 1284 | CAACCCGCGACGGCACATCCCTTGCAGTAACACT ACGCCTATAACTCCGGGCAGAGCGCGTGTGGCGG CCGAGCACATGGGCCCGCGGGCCGGGCGGGCTCG GGGCGGCCGGGACGAGGAGGGGCGACG | 2142 |
| HPV59 | SD2-MYC_ 001_exon1 | 1285 | 1286 | GCAGCAAACCAGTAACCTGCAATGGCCGATTCGG AAGCTGCTCGCGGCCGCCACCGCCGGGCCCCGGC CGTCCCTGGCTCCCCTCCTGCCTCGA | 2143 |
| HPV59 | SD2-MYC_ 001_exon2 | 1287 | 1288 | GCAGCAAACCAGTAACCTGCAATGGCCGATTCGG AAGCAGCCTCCCGCGACGATGCCCCTCAACGTTA GCTTCACCAACAGGAACTATGACCTCGACTACG | 2144 |
| HPV59 | SD2-MYC_ 001_exon3 | 1289 | 1290 | GCAGCAAACCAGTAACCTGCAATGGCCGATTCGG<br><br>AAGAGGAGGAACAAGAAGATGAGGAAGAAATCG ATGTTGTTTCTGTGGAAAAGAGGCAGGCTCCTGG | 2145 |

165

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV59 | SD2-PVT1_002_exon3 | 1291 | 1292 | GCAGCAAACCAGTAACCTGCAATGGCCGATTCGG AAGCTGACCATACTCCCTGGAGCCTTCTCCCGAGG TGCGCGGGTGACCT | 2146 |
| HPV59 | SD2-PVT1_004_exon1 | 1293 | 1294 | GCAGCAAACCAGTAACCTGCAATGGCCGATTCGG AAGTCTGAGCCTGATGGATTTACAGTGATCTTCAG TGGTCTGGGG | 2147 |
| HPV59 | SD2-PVT1_005_exon1 | 1295 | 1296 | GCAGCAAACCAGTAACCTGCAATGGCCGATTCGG AAGCTCCGGGCAGAGCGCGTGTGGCGGCCGAGCA CATGGGCCCGCGGGCCGGGCGGGCTCGGGGCGGC CGGGACGAGGAGGGGCGACG | 2148 |
| HPV66 | bkpt1-MYC_001_exon1 | 1297 | 1298 | TATGAAAAGATAGTAAATGCATTATAGATCACA TAGACTATTGGAAAGCTGTACGACATGCTGCTCGC GGCCGCCACCGCCGGGCCCCGGCCGTCCCTGGCTC CCCTCCTGCCTCGA | 2149 |
| HPV66 | bkpt1-MYC_001_exon2 | 1299 | 1300 | TATGAAAAGATAGTAAATGCATTATAGATCACA TAGACTATTGGAAAGCTGTACGACATGCAGCCTCC CGCGACGATGCCCCTCAACGTTAGCTTCACCAACA GGAACTATGACCTCGACTACG | 2150 |
| HPV66 | bkpt1-MYC_001_exon3 | 1301 | 1302 | TATGAAAAGATAGTAAATGCATTATAGATCACA TAGACTATTGGAAAGCTGTACGACATGAGGAGGA ACAAGAAGATGAGGAAGAAATCGATGTTGTTTCT GTGGAAAGAGGCAGGCTCCTGG | 2151 |
| HPV66 | bkpt1-PVT1_002_exon3 | 1303 | 1304 | TATGAAAAGATAGTAAATGCATTATAGATCACA TAGACTATTGGAAAGCTGTACGACATGCTGACCAT ACTCCCTGGAGCCTTCTCCCGAGGTGCGCGGGTGA CCT | 2152 |

EP 3 715 477 A1

166

| HPV type | Region name | Forward primer SEQ ID NO | Reverse primer SEQ ID NO | Amplicon nucleic acid sequence | Amplicon SEQ ID NO |
|---|---|---|---|---|---|
| HPV66 | bkpt1-PVT1_004_exon1 | 1305 | 1306 | TATGAAAAAGATAGTAAATGCATTATAGATCACA TAGACTATTGGAAAGCTGTACGACATGTCTGAGCC TGATGGATTTACAGTGATCTTCAGTGGTCTGGGG | 2153 |
| HPV66 | bkpt1-PVT1_005_exon1 | 1307 | 1308 | TATGAAAAAGATAGTAAATGCATTATAGATCACA TAGACTATTGGAAAGCTGTACGACATGCTCCGGG CAGAGCGCGTGTGGCGGCCGAGCACATGGGCCCG CGGGCCGGGCGGGCTCGGGGCGGCCGGGACGAGG AGGGGCGACG                     r | 2154 |
| HPV66 | bkpt2-MYC_001_exon1 | 1309 | 1310 | GGTGATAAAACTACGCCTGTAACTGCTCGCGGCC GCCACCGCCGGGCCCCGGCCGTCCCTGGCTCCCCT CCTGCCTCGA | 2155 |
| HPV66 | bkpt2-MYC_001_exon2 | 1311 | 1312 | GGTGATAAAACTACGCCTGTAACAGCCTCCCGCG ACGATGCCCCTCAACGTTAGCTTCACCAACAGGA ACTATGACCTCGACTACG | 2156 |
| HPV66 | bkpt2-MYC_001_exon3 | 1313 | 1314 | GGTGATAAAACTACGCCTGTAAAGGAGGAACAAG AAGATGAGGAAGAAATCGATGTTGTTTCTGTGGA AAAGAGGCAGGCTCCTGG | 2157 |
| HPV66 | bkpt2-PVT1_002_exon3 | 1315 | 1316 | GGTGATAAAACTACGCCTGTAACTGACCATACTCC CTGGAGCCTTCTCCCGAGGTGCGCGGGTGACCT | 2158 |
| HPV66 | bkpt2-PVT1_004_exon1 | 1317 | 1318 | GGTGATAAAACTACGCCTGTAATCTGAGCCTGATG GATTTACAGTGATCTTCAGTGGTCTGGGG          r | 2159 |
| HPV66 | bkpt2-PVT1_005_exo n1 | 1319 | 1320 | GGTGATAAAACTACGCCTGTAACTCCGGGCAGAG CGCGTGTGGCGGCCGAGCACATGGGCCCGCGGGC CGGGCGGGCTCGGGGCGGCCGGGACGAGGAGGG GCGACG | 2160 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV66 | SD2-MYC_001_exon1 | 1321 | 1322 | GCGCATCATCTAAATAACTGCAATGGCATCACCTGAAGCTGCTCGGGCGCCGCCACCGCCGCGGGCCCCGGCCGTCCCTGGCTCCCCTCCCTGCCTCGA | 2161 |
| HPV66 | SD2-MYC_001_exon2 | 1323 | 1324 | GCGCATCATCTAAATAACTGCAATGGCATCACCTGAAGCAGCCTCCCGCGACGATGCCCCTCAACGTTAGCTTCACCAACAGGAACTATGACCTGACTACG | 2162 |
| HPV66 | SD2-MYC_001_exon3 | 1325 | 1326 | GCGCATCATCTAAATAACTGCAATGGCATCACCTGAAGAGGAGGAACAAGAAGATGAGGAAGAAATCGATGTTGTTTCTGTGGAAAAGAGGCAGGCTCCTGG | 2163 |
| HPV66 | SD2-PVT1_002_exon3 | 1327 | 1328 | GCGCATCATCTAAATAACTGCAATGGCATCACCTGAAGCTGACCATACTCCCCTGGAGCCTTCTCCCGAGGTGCGCGGGGTGACCT | 2164 |
| HPV66 | SD2-PVT1_004_exon1 | 1329 | 1330 | GCGCATCATCTAAATAACTGCAATGGCATCACCTGAAGTCTGAGCCTGATGGATTTACAGTGATCTTCAGTGGTCTGGGG | 2165 |
| HPV66 | SD2-PVT1_005_exon1 | 1331 | 1332 | GCGCATCATCTAAATAACTGCAATGGCATCACCTGAAGCTCCGGGCAGAGCGGTGTGCGGCCGAGCACATGGGCCCGCCGCGGGGCTCGGGGGGGCCGGGACGAGGAGGGGGACG | 2166 |
| HPV68 | bkpt1-MYC_001_exon1 | 1333 | 1334 | ATTAACTATTGGAATTGTGTGCGACTGGCTGCTCGCGGCCGCCACCGGCCGCGGGCCCCGCCGTCCCCTGGCTCCCCTCCCTGCCTCGA | 2167 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV68 | bkpt1-MYC_001_exon2 | 1335 | 1336 | ATTAACTATTGGAATTGTGTGCGACTGGCAGCCTCCCGCGACGATGCCCCTCAACGTTAGCTTCACCAACAGGAACTATGACCTCGACTACG | 2168 |
| HPV68 | bkpt1-MYC_001_exon3 | 1337 | 1338 | ATTAACTATTGGAATTGTGTGCGACTGGAGGAGGAACAAGAAGATGAGGAAGAAATCGATGTTGTTTCTGTGGAAAAGAGGCAGGCTCCTGG | 2169 |
| HPV68 | bkpt1-PVT1_002_exon3 | 1339 | 1340 | ATTAACTATTGGAATTGTGTGCGACTGGCTGACCATACTCCCTGGAGCCTTCTCCCGAGGTGCGCGGGTGACCT | 2170 |
| HPV68 | bkpt1-PVT1_004_exon1 | 1341 | 1342 | ATTAACTATTGGAATTGTGTGCGACTGGTCTGAGCCTGATGGATTTACAGTGATCTTCAGTGGTCTGGGG | 2171 |
| HPV68 | bkpt1-PVT1_005_exon1 | 1343 | 1344 | ATTAACTATTGGAATTGTGTGCGACTGGCTCCGGG CAGAGCGCGTGTGGCGGCCGAGCACATGGGCCCGCGGGCCGGGCGGGCTCGGGGCGGCCGGGACGAGGAGGGGCGACG | 2172 |
| HPV68 | bkpt2-MYC_001_exon1 | 1345 | 1346 | AAGACGGAGCCTTTGTTGTGGTGACACTACACCTATAGCTGCTCGCGGCCGCCACCGCCGGGCCCCGGCCGTCCCTGGCTCCCCTCCTGCCTCGA | 2173 |
| HPV68 | bkpt2-MYC_001_exon2 | 1347 | 1348 | AAGACGGAGCCTTTGTTGTGGTGACACTACACCTATAGCAGCCTCCCGCGACGATGCCCCTCAACGTTAGCTTCACCAACAGGAACTATGACCTCGACTACG | 2174 |
| HPV68 | bkpt2-MYC_001_exon3 | 1349 | 1350 | AAGACGGAGCCTTTGTTGTGGTGACACTACACCTATAGAGGAGGAACAAGAAGATGAGGAAGAAATCGATGTTGTTTCTGTGGAAAAGAGGCAGGCTCCTGG | 2175 |

EP 3 715 477 A1

169

| HPV type | Region name | Forward primer SEQ ID NO | Reverse primer SEQ ID NO | Amplicon nucleic acid sequence | Amplicon SEQ ID NO |
|---|---|---|---|---|---|
| HPV68 | bkpt2-PVT1_002_exon3 | 1351 | 1352 | AAGACGGAGCCTTTGTTGTGGTGACACTACACCTATAGCTGACCATACTCCCTGGAGCCTTCTCCCGAGGTGCGCGGGTGACCT | 2176 |
| HPV68 | bkpt2-PVT1_004_exon1 | 1353 | 1354 | AAGACGGAGCCTTTGTTGTGGTGACACTACACCTATAGTCTGAGCCTGATGGATTTACAGTGATCTTCAGTGGTCTGGGG | 2177 |
| HPV68 | bkpt2-PVTI_005_exon1 | 1355 | 1356 | AAGACGGAGCCTTTGTTGTGGTGACACTACACCTATAGCTCCGGGCAGAGCGCGTGTGGCGGCCGAGCACATGGGCCCGCGGGCCGGGCGGGCTCGGGGCGGCCGGGACGAGGAGGGGCGACG | 2178 |
| HPV68 | SD2-MYC_001_exon1 | 1357 | 1358 | ACCCAGTAATCTGCAATGGCCAATTGTGAAGCTGCTCGCGGCCGCCACCGCCGGGCCCCGGCCGTCCCTGGCTCCCCTCCTGCCTCGA | 2179 |
| HPV68 | SD2-MYC_001_exon2 | 1359 | 1360 | ACCCAGTAATCTGCAATGGCCAATTGTGAAGCAGCCTCCCGCGACGATGCCCCTCAACGTTAGCTTCACCAACAGGAACTATGACCTCGACTACG | 2180 |
| HPV68 | SD2-MYC_001_exon3 | 1361 | 1362 | ACCCAGTAATCTGCAATGGCCAATTGTGAAGAGGAGGAACAAGAAGATGAGGAAGAAATCGATGTTGTTTCTGTGGAAAAGAGGCAGGCTCCTGG | 2181 |
| HPV68 | SD2-PVT1_002_exon3 | 1363 | 1364 | ACCCAGTAATCTGCAATGGCCAATTGTGAAGCTGACCATACTCCCTGGAGCCTTCTCCCGAGGTGCGCGGGTGACCT | 2182 |
| HPV68 | SD2-PVT1_004_exon1 | 1365 | 1366 | ACCCAGTAATCTGCAATGGCCAATTGTGAAGTCTGAGCCTGATGGATTTACAGTGATCTTCAGTGGTCTGGGG | 2183 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV68 | SD2-PVT1_005_exon1 | 1367 | 1368 | ACCCAGTAATCTGCAATGGCCAATTGTGAAGCTCCGGGCAGAGCGCGTGTGGCGGCCGAGCACATGGGCCCGCGGGCCGGGCGGGCTCGGGGCGGCCGGGACGAGGAGGGGCGACG | 2184 |
| HPV73 | bkpt1-MYC_001_exon1 | 1369 | 1370 | GTGATCATATTGATCATTGGAAACACGTGCGACATGCTGCTCGCGGCCGCCACCGCCGGGCCCCGGCCGTCCCTGGCTCCCTCCTGCCTCGA | 2185 |
| HPV73 | bkpt1-MYC_001_exon2 | 1371 | 1372 | GTGATCATATTGATCATTGGAAACACGTGCGACATGCAGCCTCCCGCGACGATGCCCCTCAACGTTAGCTTCACCAACAGGAACTATGACCTCGACTACG | 2186 |
| HPV73 | bkpt1-MYC_001_exon3 | 1373 | 1374 | GTGATCATATTGATCATTGGAAACACGTGCGACATGAGGAGGAACAAGAAGATGAGGAAGAAATCGATGTTGTTTCTGTGGAAAAGAGGCAGGCTCCTGG | 2187 |
| HPV73 | bkpt1-PVT1_002_exon3 | 1375 | 1376 | GTGATCATATTGATCATTGGAAACACGTGCGACATGCTGACCATACTCCCTGGAGCCTTCTCCCGAGGTGCGCGGGTGACCT | 2188 |
| HPV73 | bkpt1-PVT1_004_exon1 | 1377 | 1378 | GTGATCATATTGATCATTGGAAACACGTGCGACATGTCTGAGCCTGATGGATTTACAGTGATCTTCAGTGGTCTGGGG | 2189 |
| HPV73 | bkpt1-PVT1_005_exon1 | 1379 | 1380 | GTGATCATATTGATCATTGGAAACACGTGCGACATGCTCCGGGCAGAGCGCGTGTGGCGGCCGAGCACATGGGCCCGCGGGCCGGGCGGGCTCGGGGCGGCCGGGACGAGGAGGGGCGACG | 2190 |

(continued)

| HPV type | Region name | Forward primer SEQ ID NO | Reverse primer SEQ ID NO | Amplicon nucleic acid sequence | Amplicon SEQ ID NO |
|---|---|---|---|---|---|
| HPV73 | bkpt2-MYC_001_exon1 | 1381 | 1382 | CCTGTACCCAGTGTACTACACATAATGTTGCGCCAATAGCTGCTCGCGGCCGCCACCGCCGGGCCCCGGCCGTCCCTGGCTCCCCTCCTGCCTCGA | 2191 |
| HPV73 | bkpt2-MYC_001_exon2 | 1383 | 1384 | CCTGTACCCAGTGTACTACACATAATGTTGCGCCAATAGCAGCCTCCCGCGACGATGCCCCTCAACGTTAGCTTCACCAACAGGAACTATGACCTCGACTACG | 2192 |
| HPV73 | bkpt2-MYC_001_exon3 | 1385 | 1386 | CCTGTACCCAGTGTACTACACATAATGTTGCGCCAATAGAGGAGGAACAAGAAGATGAGGAAGAAATCGATGTTGTTTCTGTGGAAAAGAGGCAGGCTCCTGG | 2193 |
| HPV73 | bkpt2-PVT1_002_exon3 | 1387 | 1388 | CCTGTACCCAGTGTACTACACATAATGTTGCGCCAATAGCTGACCATACTCCCTGGAGCCTTCTCCCGAGGTGCGCGGGTGACCT | 2194 |
| HPV73 | bkpt2-PVT1_004_exon1 | 1389 | 1390 | CCTGTACCCAGTGTACTACACATAATGTTGCGCCAATAGTCTGAGCCTGATGGATTTACAGTGATCTTCAGTGGTCTGGGG | 2195 |
| HPV73 | bkpt2-PVT1_005_exon1 | 1391 | 1392 | CCTGTACCCAGTGTACTACACATAATGTTGCGCCAATAGCTCCGGGCAGAGCGCGTGTGGCGGCCGAGCACATGGGCCCGCGGGCCGGGCGGGCTCGGGGCGGCCGGGACGAGGAGGGGCGACG | 2196 |
| HPV73 | SD2-MYC_001_exon1 | 1393 | 1394 | GCCCCAACTGTTCCAGAAACCTATAAAAGAAGATGGCTGATTCAGCTGCTCGCGGCCGCCACCGCCGGGCCCCGGCCGTCCCTGGCTCCCCTCCTGCCTCGA | 2197 |

172

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV73 | SD2-MYC_001_exon2 | 1395 | 1396 | GCCCCAACTGTTCCAGAAACCTATAAAAGAAGAT GGCTGATTCAGCAGCCTCCCGCGACGATGCCCCTC AACGTTAGCTTCACCAACAGGAACTATGACCTCG ACTACG | 2198 |
| HPV73 | SD2-MYC_001_exon3 | 1397 | 1398 | GCCCCAACTGTTCCAGAAACCTATAAAAGAAGAT GGCTGATTCAGAGGAGGAACAAGAAGATGAGGA AGAAATCGATGTTGTTTCTGTGGAAAAGAGGCAG GCTCCTGG | 2199 |
| HPV73 | SD2-PVT1_002_exon3 | 1399 | 1400 | GCCCCAACTGTTCCAGAAACCTATAAAAGAAGAT GGCTGATTCAGCTGACCATACTCCCTGGAGCCTTC TCCCGAGGTGCGCGGGTGACCT | 2200 |
| HPV73 | SD2-PVT1_004_exon1 | 1401 | 1402 | GCCCCAACTGTTCCAGAAACCTATAAAAGAAGAT GGCTGATTCAGTCTGAGCCTGATGGATTTACAGTG ATCTTCAGTGGTCTGGGG | 2201 |
| HPV73 | SD2-PVT1_005_exon1 | 1403 | 1404 | GCCCCAACTGTTCCAGAAACCTATAAAAGAAGAT GGCTGATTCAGCTCCGGGCAGAGCGCGTGTGGCG GCCGAGCACATGGGCCCGCGGGCCGGGCGGGCTC GGGGCGGCCGGGACGAGGAGGGGCGACG | 2202 |
| HPV82 | bkpt1-MYC_001_exon1 | 1405 | 1406 | GAACTGGACAGTGATAAATTAGTAGATCAAATTA ATTATTGGACGTTGGTACGATATGCTGCTCGCGGC CGCCACCGCCGGGCCCCGGCCGTCCCTGGCTCCCC TCCTGCCTCGA | 2203 |
| HPV82 | bkpt1-MYC_001_exon2 | 1407 | 1408 | GAACTGGACAGTGATAAATTAGTAGATCAAATTA ATTATTGGACGTTGGTACGATATGCAGCCTCCCGC GACGATGCCCCTCAACGTTAGCTTCACCAACAGG AACTATGACCTCGACTACG | 2204 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV82 | bkpt1-MYC_001_exon3 | 1409 | 1410 | GAACTGGACAGTGATAAATTAGTAGATCAAATTA ATTATTGGACGTTGGTACGATATGAGGAGGAACA AGAAGATGAGGAAGAAATCGATGTTGTTTCTGTG GAAAAGAGGCAGGCTCCTGG | 2205 |
| HPV82 | bkpt1-PVT1_002_exon3 | 1411 | 1412 | GAACTGGACAGTGATAAATTAGTAGATCAAATTA ATTATTGGACGTTGGTACGATATGCTGACCATACT CCCTGGAGCCTTCTCCCGAGGTGCGCGGGTGACCT | 2206 |
| HPV82 | bkpt1-PVT1_004_exon1 | 1413 | 1414 | GAACTGGACAGTGATAAATTAGTAGATCAAATTA ATTATTGGACGTTGGTACGATATGTCTGAGCCTGA TGGATTTACAGTGATCTTCAGTGGTCTGGGG | 2207 |
| HPV82 | bkpt1-PVT1_005_exon1 | 1415 | 1416 | GAACTGGACAGTGATAAATTAGTAGATCAAATTA ATTATTGGACGTTGGTACGATATGCTCCGGGCAGA GCGCGTGTGGCGGCCGAGCACATGGGCCCGCGGG CCGGGCGGGCTCGGGGCGGCCGGGACGAGGAGG GGCGACG | 2208 |
| HPV82 | bkpt2-MYC_001_exon1 | 1417 | 1418 | GGAACTGCAGGCCCAAACACCGGAGGGCACCTCA GTGCAACTAAAACTGCGTTTATAGCTGCTCGCGGC CGCCACCGCCGGGCCCCGGCCGTCCCTGGCTCCCC TCCTGCCTCGA | 2209 |
| HPV82 | bkpt2-MYC_001_exon2 | 1419 | 1420 | GGAACTGCAGGCCCAAACACCGGAGGGCACCTCA GTGCAACTAAAACTGCGTTTATAGCAGCCTCCCGC GACGATGCCCCTCAACGTTAGCTTCACCAACAGG AACTATGACCTCGACTACG | 2210 |

| HPV type | Region name | Forward primer SEQ ID NO | Reverse primer SEQ ID NO | Amplicon nucleic acid sequence | Amplicon SEQ ID NO |
|---|---|---|---|---|---|
| HPV82 | bkpt2-MYC_001_exon3 | 1421 | 1422 | GGAACTGCAGGCCCAAACACCGGAGGGCACCTCAGTGCAACTAAAACTGCGTTTATAGAGGAGGAACAAGAAGATGAGGAAGAAATCGATGTTGTTTCTGTGGAAAAGAGGCAGGCTCCTGG | 2211 |
| HPV82 | bkpt2-PVT1_002_exon3 | 1423 | 1424 | GGAACTGCAGGCCCAAACACCGGAGGGCACCTCAGTGCAACTAAAACTGCGTTTATAGCTGACCATACTCCCTGGAGCCTTCTCCCGAGGTGCGCGGGTGACCT | 2212 |
| HPV82 | bkpt2-PVT1_004_exon1 | 1425 | 1426 | GGAACTGCAGGCCCAAACACCGGAGGGCACCTCAGTGCAACTAAAACTGCGTTTATAGTCTGAGCCTGATGGATTTACAGTGATCTTCAGTGGTCTGGGG | 2213 |
| HPV82 | bkpt2-PVT1_005_exon1 | 1427 | 1428 | GGAACTGCAGGCCCAAACACCGGAGGGCACCTCAGTGCAACTAAAACTGCGTTTATAGCTCCGGGCAGAGCGCGTGTGGCGGCCGAGCACATGGGCCCGCGGGCCGGGCGGGCTCGGGGCGGCCGGGACGAGGAGGGGCGACG | 2214 |
| HPV82 | SD2-MYC_001_exon1 | 1429 | 1430 | CATCGGCAATGGACAGTGAAGCTGCTCGCGGCCGCCACCGCCGGGCCCCGGCCGTCCCTGGCTCCCCTCCTGCCTCGA | 2215 |
| HPV82 | SD2-MYC_001_exon2 | 1431 | 1432 | CATCGGCAATGGACAGTGAAGCAGCCTCCCGCGACGATGCCCCTCAACGTTAGCTTCACCAACAGGAACTATGACCTCGACTACG | 2216 |
| HPV82 | SD2-MYC_001_exon3 | 1433 | 1434 | CATCGGCAATGGACAGTGAAGAGGAGGAACAAGAAGATGAGGAAGAAATCGATGTTGTTTCTGTGGAAAAGAGGCAGGCTCCTGG | 2217 |

EP 3 715 477 A1

| HPV type | Region name | Forward primer SEQ ID NO | Reverse primer SEQ ID NO | Amplicon nucleic acid sequence | Amplicon SEQ ID NO |
|---|---|---|---|---|---|
| HPV82 | SD2-PVT1_002_exon3 | 1435 | 1436 | CATCGGCAATGGACAGTGAAGCTGACCATACTCCCTGGAGCCTTCTCCCGAGGTGCGCGGGTGACCT | 2218 |
| HPV82 | SD2-PVT1_004_exon1 | 1437 | 1438 | CATCGGCAATGGACAGTGAAGTCTGAGCCTGATGGATTTACAGTGATCTTCAGTGGTCTGGGG | 2219 |
| HPV82 | SD2-PVT1_005_exon1 | 1439 | 1440 | CATCGGCAATGGACAGTGAAGCTCCGGGCAGAGCGCGTGTGGCGGCCGAGCACATGGGCCCGCGGGCCGGGCGGGCTCGGGGCGGCCGGGACGAGGAGGGGCGACG | 2220 |

Table 2E

| Human gene name | Forward primer Nucleic acid sequence | Forward primer SEQ ID NO | Reverse primer Nucleic acid sequence | Reverse primer SEQ ID NO |
|---|---|---|---|---|
| ACTB | CCAGGTCATCACCATTGGCAAT | 1441 | CGTACAGGTCTTTGCGGATGT | 1442 |
| AKT1 | CCATGAGCGACGTGGCTATT | 1443 | CTCACGTTGGTCCACATCCT | 1444 |
| B2M | CTGTGCTCGCGCTACTCT | 1445 | CAACTTCAATGTCGGATGGATGAAAC | 1446 |
| BCL2 | GTGGATGACTGAGTACCTGAACC | 1447 | GGCCAAACTGAGCAGAGTCTT | 1448 |
| BRAF | CGGGACTCGAGTGATGATTGG | 1449 | CTGAGGTGTAGGTGCTGTCA | 1450 |
| CDH1 | CTCCTGAAAAGAGAGTGGAAGTGT | 1451 | CCGGATTAATCTCCAGCCAGTT | 1452 |
| CDKN2A | AACGCACCGAATAGTTACGGT | 1453 | ACGGGTCGGGTGAGAGT | 1454 |
| CDKN2B | CGGATCCCAACGGAGTCAA | 1455 | ACCGGTCGGGTGAGAGT | 1456 |
| ERBB2 | TCTTCCAGAACCTGCAAGTAATCC | 1457 | GGTGGGTGTTATGGTGGATGA | 1458 |
| FOS | AGGAGAATCCGAAGGGAAAGGAATA | 1459 | TCCTTCAGCAGGTTGGCAAT | 1460 |
| GAPDH | AGTCCACTGGCGTCTTCAC | 1461 | TGATCTTGAGGCTGTTGTCATACTTC | 1462 |
| GUSB | GCGAGTATGGAGCAGAAACGA | 1463 | AATTCCAAATGAGCTCTCCAACCA | 1464 |
| HRAS | CGGAATATAAGCTGGTGGTGGT | 1465 | GCACGTCTCCCCATCAATGA | 1466 |
| KRAS | GTGCAATGAGGGACCAGTACA | 1467 | CTACTAGGACCATAGGTACATCTTCAGA | 1468 |
| KRT10 | GATGAGCTGACCCTGACCAA | 1469 | GGCAGCATTCATTTCCACATTCAC | 1470 |
| KRT14 | AGGAGCTGGCCTACCTGAA | 1471 | CTTCTCATACTGGTCACGCATCT | 1472 |
| KRT17 | AACACTGAGCTGGAGGTGAAG | 1473 | CTGTAGCAGGATGTTGGCATTG | 1474 |
| MET | TGTGTGCATTCCCTATCAAATATGTCAA | 1475 | GCGCTTCACAGCCTGATGA | 1476 |
| MKI67 | CGTCGTGTCTCAAGATCTAGCTT | 1477 | TGAGTCATCTGCGGTACTGTCT | 1478 |
| MYC | GCTTCTCTGAAAGGCTCTCCTT | 1479 | AAATACGGCTGCACCGAGT | 1480 |
| NOTCH1 | CCGACGCACAAGGTGTCTT | 1481 | GTCGGCGTGTGAGTTGATGA | 1482 |
| PCNA | GACGGAGTGAAATTTTCTGCAAGT | 1483 | GAAGTTCAGGTACCTCAGTGCAAA | 1484 |
| PTEN | AGCGTGCAGATAATGACAAGGAA | 1485 | GATTTGACGGCTCCTCTACTGT | 1486 |
| RB1 | CGGTCTTCATGCAGAGACTGA | 1487 | GTGAAATATAGATGTTCCCTCCAGGAAT | 1488 |
| RPLP0 | GACGGATTACACCTTCCCACTT | 1489 | GACTCTTCCTTGGCTTCAACCTTA | 1490 |

(continued)

| Human gene name | Forward primer Nucleic acid sequence | Forward primer SEQ ID NO | Reverse primer Nucleic acid sequence | Reverse primer SEQ ID NO |
|---|---|---|---|---|
| STAT1 | CGATGGGCTCAGCTTTCAGA | 1491 | ACAAAACCTCGTCCACGGAAT | 1492 |
| TERT | TCCTGCGTTTGGTGGATGAT | 1493 | CCTCGTCTTCTACAGGGAAGTTCA | 1494 |
| TOP2A | TGGGTGGTCCTGCAAAATCC | 1495 | ACATATTGATTTGGAGCCAGTTCTTCA | 1496 |
| TP53 | CTGGCCCCTGTCATCTTCTG | 1497 | CTTGGCCAGTTGGCAAAACAT | 1498 |
| WNT1 | CTGGAACTGTCCCACTGCT | 1499 | CAGGATTCGATGGAACCTTCTGA | 1500 |

Table 2Ebis

| Human gene name | Forward primer SEQ ID NO | Reverse primer SEQ ID NO | Amplicon nucleic acid sequence | Amplicon SEQ ID NO |
|---|---|---|---|---|
| ACTB | 1441 | 1442 | GAGCGGTTCCGCTGCCCTGAGGCACTCTTCCAGCCTTCCTTCCTGGGCATGGAGTCCTGTGGCATCCACGAAACTACCTTCAACTCCATCATGAAGTGTGACGTGG | 2221 |
| AKT1 | 1443 | 1444 | GTGAAGGAGGGTTGGCTGCACAAACGAGGGGAGTACATCAAGACCTGGCGGCCACGCTACTTCCTCCTCAAGAATGATGGCACCTTCATTGGCTACAAGGAGCGGCCGC | 2222 |
| B2M | 1445 | 1446 | CTCTTTCTGGCCTGGAGGCTATCCAGCGTACTCCAAAGATTCAGGTTTACTCACGTCATCCAGCAGAGAATGGAAAGTCAAATTTCCTGAATTGCTATGTGTCTGG | 2223 |
| BCL2 | 1447 | 1448 | GGCACCTGCACACCTGGATCCAGGATAACGGAGGCTGGGATGCCTTTGTGGAACTGTACGGCCCCAGCATGCGGCCTCTGTTTGATTTCTCCTGGCTGTCTCTG | 2224 |
| BRAF | 1449 | 1450 | GAGATTCCTGATGGGCAGATTACAGTGGGACAAAGAATTGGATCTGGATCATTTGGAACAGTCTACAAGGGAAAGTGGCATGGTGATGTGGCAGTGAAAATGTTGAATG | 2225 |
| CDH1 | 1451 | 1452 | CCGAGGACTTTGGCGTGGGCCAGGAAATCACATCCTACACTGCCCAGGAGCCAGACACATTTATGGAACAGAAAATAACATATCGGATTTGGAGAGACACTGCC | 2226 |
| CDKN2A | 1453 | 1454 | CGGAGGCCGATCCAGGTCATGATGATGGGCAGCGCCCGAGTGGCGGAGCTGCTGCTGCTCCACGGCGCGGAGCCCAACTGCGCCGACCCCGCC | 2227 |
| CDKN2B | 1455 | 1456 | CCGTTTCGGGAGGCGCGCGATCCAGGTCATGATGATGGGCAGCGCCCGCGTGGCGGAGCTGCTGCTGCTCCACGGCGCGGAGCCCAACTGCGCAGACCCTGCC | 2228 |
| ERBB2 | 1457 | 1458 | GGGGACGAATTCTGCACAATGGCGCCTACTCGCTGACCCTGCAAGGGCTGGGCATCAGCTGGCTGGGGCTGCGCTCACTGAGGGAACTGGGCAGTGGACTGGCCC | 2229 |

| Human gene name | Forward primer SEQ ID NO | Reverse primer SEQ ID NO | Amplicon nucleic acid sequence | Amplicon SEQ ID NO |
|---|---|---|---|---|
| FOS | 1459 | 1460 | AGATGGCTGCAGCCAAATGCCGCAACCGGAGGAGGGAGCTGACTGATACAC TCCAAGCGGAGACAGACCAACTAGAAGATGAGAAGTCTGCTTTGCAGACCG AG | 2230 |
| GAPDH | 1461 | 1462 | CACCATGGAGAAGGCTGGGGCTCATTTGCAGGGGGGAGCCAAAAGGGTCAT CATCTCTGCCCCCTCTGCTGATGCCCCCATGTTCGTCATGGGTGTGAACCATG A | 2231 |
| GUSB | 1463 | 1464 | TTGCAGGGTTTCACCAGGATCCACCTCTGATGTTCACTGAAGAGTACCAGAA AAGTCTGCTAGAGCAGTACCATCTGGGTCTGGATCAAAAACGCAGAAAATAC G | 2232 |
| HRAS | 1465 | 1466 | GGGCGCCGGCGGTGTGGGCAAGAGTGCGCTGACCATCCAGCTGATCCAGAA CCATTTTGTGGACGAATACGACCCCACTATAGAGGATTCCTACCGGAAGCAG GTGG | 2233 |
| KRAS | 1467 | 1468 | TGAGGACTGGGGAGGGCTTTCTTTGTGTATTTGCCATAAATAATACTAAATCA TTTGAAGATATTCACCATTATAGAGAACAAATTAAAAGAGTTAAGGAC | 2234 |
| KRT10 | 1469 | 1470 | GGCTGACCTGGAGATGCAAATTGAGAGCCTGACTGAAGAGCTGGCCTATCTG AAGAAGAACCACGAGGAGGAAATGAAAGACCTTCGAAATGTGTCCACTGGT GAT | 2235 |
| KRT14 | 1471 | 1472 | GAAGAACCACGAGGAGGAGATGAACGCCCTGCGAGGCCAGGTGGGTGGTGA GATCAATGTGGAGATGGACGCTGCCCCAGGCGTGGACCTGAGCCGCATCCTC AACG | 2236 |
| KRT17 | 1473 | 1474 | ATCCGTGACTGGTACCAGAGGCAGGCCCCGGGGCCCGCCCGTGACTACAGCC AGTACTACAGGACAATTGAGGAGCTGCAGAACAAGATCCTCACAGCCACCGT GGA | 2237 |
| MET | 1475 | 1476 | CGACTTCTTCAACAAGATCGTCAACAAAAACAATGTGAGATGTCTCCAGCAT TTTTACGGACCCAATCATGAGCACTGCTTTAATAGGACACTTCTGAGAAAT | 2238 |

EP 3 715 477 A1

(continued)

| Human gene name | Forward primer SEQ ID NO | Reverse primer SEQ ID NO | Amplicon nucleic acid sequence | Amplicon SEQ ID NO |
|---|---|---|---|---|
| MKI67 | 1477 | 1478 | CTCTTCTGACCCCTGATGAGAAAGCTCAAGATTCCAAGGCCTATTCAAAAATCACTGAAGGAAAAGTTTCAGGAAATCCTTCAGGTACATATCAAGAAATGTCAAAGA | 2239 |
| MYC | 1479 | 1480 | GCAGCTGCTTAGACGCTGGATTTTTTCGGGTAGTGGAAAAACCAGCAGCCTCCCGCGACGATGCCCCTCAACGTTAGCTTCACCAACAGGAACTATGACCTCGACTACG | 2240 |
| NOTCH1 | 1481 | 1482 | CCAGATCCTGATCCGGAACCGAGCCACAGACCTGGATGCCCGCATGCATGATGGCACGACGACGCCACTGATCCTGCCTGGCCCGGTGGGAGGGCATGCTGGAGGACC | 2241 |
| PCNA | 1483 | 1484 | GGAGAACTTGGAAATGGAAACATTAAATTGTCACAGACAAGTAATGTCGATAAAGAGGAGGAGCTGTTACCATAGAGATGAATGAACCAGTTCAACTAACT | 2242 |
| PTEN | 1485 | 1486 | TATCTAGTACTTACTTTAACAAAAAATGATCTTGACAAAGCAAATAAAGACAAAGCCAACCGATACTTTCTCCAAATTTAAGGTGAAGCTGTACTTCACAAAA | 2243 |
| RB1 | 1487 | 1488 | AAACAAATATTTTGCAGTATGCTTCCACCAGGCCCCCTACCTTGTCACCAATACCTCACATTCCTCGAAGCCCTTACAAGTTCCTAGTTCACCCTTACGG | 2244 |
| RPLP0 | 1489 | 1490 | GCTGAAAAGGTCAAGGCCTTCTGGCTGATCCATCTGCCTTTGTGGCTGCTGCCCTGTGGCTGCTGCCACCAGCTGCTCCTGCTGCTGCTGCAGCCCCAGC | 2245 |
| STAT1 | 1491 | 1492 | AGTGCTGCTGAGTTGGCAGTTTCTTCTGTCTGTCACCAAAAGAGAGTTCTCAATGTGGACCAGCTGAACATGTTGGGAGAGAAGCTTCTTGGTCCTAACGCCCAGCCCGATGGTCTC | 2246 |
| TERT | 1493 | 1494 | TTCTTGTTGGTGACACCTCACCTCACCCACGCGAAAACCTTCCTCAGGACCCTGGTCCGAGGTGTCCCTGAGTATGGCTGCGTGGTGAACTTGCGGAAGACAGTGG | 2247 |

(continued)

| Human gene name | Forward primer SEQ ID NO | Reverse primer SEQ ID NO | Amplicon nucleic acid sequence | Amplicon SEQ ID NO |
|---|---|---|---|---|
| TOP2A | 1495 | 1496 | CCAACTTTGATGTGCGTGAAATTGTAAATAACATCAGGCGTTTGATGGATGGAGAAGAACCTTTGCCAATGCTTCCAAGTTACAAGAACTTCAAGGGTACTAT | 2248 |
| TP53 | 1497 | 1498 | TCCCTTCCCAGAAAACCTACCAGGGCAGCTACGGTTTCCGTCTGGGCTTCTTGCATTCTGGGACAGCCAAGTCTGTGACTTGCACGTACTCCCCTGCCCTCAACAAG | 2249 |
| WNT1 | 1499 | 1500 | CCAGGGCCCCACCTCTTCGGCAAGATCGTCAACCGAGGCTGTCGAGAAACGGCGTTTATCTTCGCTATCACCTCCGCCGGGGTCACCCATTCGGTGGCGCGCTCCTGC | 2250 |

Study participants

Study participants were women aged from 25 to 65 years old referred for colposcopy consultation in French hospitals. The patients were referred for colposcopy in the context of a LSIL or a HSIL result at their cytology test performed in accordance with French recommendations regarding the cervical cancer screening program. Patients provided written informed consent according to French legislation.

Specimen collection

Genital samples were collected just before performing colposcopy using a cervical sampling device, immersed and rinsed in a vial filled with 20mL of PreservCyt Solution (Hologic, USA), and sent at room temperature to the HPV National Reference Center (CNR) at Institut Pasteur, Paris, France. From July 2014 to April 2015, 84 patients were enrolled in the study, coming from 3 different French centers: CHU Angers (n=66); CHU Kremlin-Bicêtre (n=10); CHU Tours (n=6). Samples were removed of the study because of technical reasons (sample leakage, n=1) or legal issues (n=7) or because they were used for initial technical tests (RNA conservation, RNA extraction and amplification, n=4). The remaining 72 samples (HSIL=37; LSIL=35) were processed.

Data collection

The following bio-clinical data were collected: date and results of the cytology test, age at the time of the cytology test, date and results of all available histological results posterior to colposcopy. As colposcopy was performed in the context of routine healthcare, biopsies were not performed in case of normal colposcopy.

HPV DNA detection using the PapilloCheck Test Kit (HPV DNA)

Upon reception at CNR, 16 mL of cytological sample were transferred into a 50 mL Falcon tube and centrifuged at 4,500 g for 10 minutes. The supernatant was removed and the pellet washed with 1 mL of PBS. Sample was then centrifuged again at 5000 g for 10 minutes and the supernatant removed. The pellet was frozen at -80°C before DNA extraction. Following DNA extraction (Macherey Nagel, Germany), HPV detection was done using the PapilloCheck Test Kit (Greiner Bio-One GmbH, Germany) according to manufacturer instructions.

RNA extraction and characterization

In parallel to the HPV DNA procedure, 3 x 1 mL aliquots of cytological specimen were centrifuged at 14,000 rpm for 7 minutes, the supernatant was removed and the pellet was washed with 1 mL of PBS. Sample was then centrifuged again at 14,000 rpm for 7 minutes and the supernatant removed. The pellet was frozen at -80°C before RNA extraction. RNA extractions were done using the PicoPure RNA Isolation kit (Thermo Fisher Scientific,), including on-column DNAse treatment, with a final elution volume of 30µl. Total RNA was quantified on a Nanodrop (Life Technologies) and RNA integrity was evaluated on a Bioanalyzer RNA 6000 pico chip (Agilent) using the RIN (RNA Integrity Number), a quality score ranging from 1 (strongly degraded RNA) to 10 (intact RNA). For each sample, RT-qPCR targeting mRNA from on housekeeping genes ACTB (forward primer: CATCGAGCACGGCATCGTCA (SEQ ID NO: 2258); reverse primer: TAGCACAGCCTGGATAGCAAC (SEQ ID NO: 2259); amplicon size = 210bp) and GAPDH (forward primer: GAAGGTGAAGGTCGGAGTC (SEQ ID NO: 2260); reverse primer: GAAGATGGTGATGGGATTTC (SEQ ID NO: 2261); amplicon size = 226bp) were done in a SYBR Green format with 45 cycles of amplification. RT-negative (RT-) PCR were also run to evaluate the presence of residual DNA after RNA extraction.

Amplification and sequencing

Starting from RNA, cDNA were generated using the SuperScript III (n=17 samples) or Superscript IV (n=55 samples) (Thermo Fisher Scientific) with random hexamers and a final RNAse H treatment. Libraries were prepared using the Ion AmpliSeq Library Kit 2.0 and AmpliSeq custom panel WG_WG00141, with 21 cycles of amplification before adapter's ligation. Each sample was barcoded individually. Only positive libraries were sequenced. In total, 55 clinical samples plus 1 cellular model (SiHa) were sequenced on 4 Ion Proton runs.

Sequencing data processing

Reads were aligned to the reference sequences of the amplicons using STAR23 v2.5.3a in local alignment mode (parameter --alignEndsType EndToEnd), by only reporting uniquely mapped reads (--outFilterMultimapNmax 1) and turning off splicing alignment (-alignIntronMax 1). The expression of each amplicon was evaluated by the number of sequencing reads uniquely mapping to their respective sequence (read counts). For reference sequences containing a splice junction, only reads mapping at the junction site and encompassing at least 10 bases before and 10 bases after the junction were kept.

HSIL prediction model

Selection of amplicons

Read counts were normalized by the size of the library (each read count was divided by a ratio of the library size for a given sample to that of the average library size across samples) and the 215 amplicons capturing splice junctions (sp) of the 16 high-risk or putative high risk HPV were selected. These amplicons have been annotated with generic names with respect to the type of transcripts they capture, which are shared across HPV species (e.g. "SD1-SA1", see Figure 2). Amplicons capturing homolog generic splice junctions conserved across the 16 HPV species were summed up, leading to the definition of 18 variables used as predictors in the model. 33 out of the 55 clinical samples have been selected as presenting enough coverage of these specific amplicons (20 mono-infected and 13 multi-infected samples). The remaining 22 samples of the dataset were not used in the logistic regression analysis because they had missing or too low expression signal at splice junctions for the prediction, reflecting for example HPV-negative samples.

Logistic regression model

Calling high grade cytology $Y$ as taking the value 1 for high grade (HSIL) and 0 for low grade (LSIL), and a set of amplicons x, a logistic regression model was used to predict the probability that a given observation belongs to the "1" class versus the probability that it belongs to the "0" class. Logistic regression models the log odds of the event (here the grade of the cytology) as a function of the predictor variables (here the amplicon expression estimated by its read count). Formally, the logistic regression model assumes that the log odds is a linear function of the predictors:

$$\text{logit}(\pi) = \ln\left(\frac{\pi}{1-\pi}\right) = \beta_0 + \beta^t x$$

where indicates the probability of the event (being of high-grade), βi are the regression coefficients, and xi the explanatory variables, in our case the log2 number of reads mapping to the amplicons.

Solving for $\pi$, this gives:

$$\pi = \frac{1}{1 + e^{-(\beta_0 + \beta x)}}$$

Implementation of the logistic regression model

To limit overfitting, the inventors used L2-norm (ridge) regularization, which allows shrinking the magnitudes of the regression coefficients such that they will better fit future data. The inventors estimated the logistic model using the R (http://www.r-project.org/) package glmnet (Friedman J, Hastie T, Tibshirani R. Regularization Paths for Generalized Linear Models via Coordinate Descent. J Stat Softw, 2010, 33:1-22). Leave-one-out (LOO) cross-validation was used to pick the regularization parameter λ, the one that gives minimum mean cross-validated misclassification error was used. Using λ as the regularization parameter, the model output consisted in an estimate of a coefficient value β for each variable in the logistic regression model. This model was then used to predict the grade of the multi-infected observations, by treating each HPV species separately.

Training set and test set

The model was built upon the clinical outcome LSIL or HSIL obtained from the cytological analysis, and estimated on a training set consisting of 20 mono-infected samples (5 LSIL and 15 HSIL) in order to avoid a confusion bias. It is indeed anticipated that, in the case of multi-infected samples, several HPV could contribute differently to the progression of the lesion or to a mix of several grades within the same sample, because they are engaged in different stages of their cycle. The performance of the model was then evaluated on a test set consisting of 13 multi-infected samples. In this case, the set of amplicons of each HPV species was used separately to classify the multi-infected samples, to get one prediction per HPV, as done for the mono-infected samples. For example if a sample had expression of amplicons from both HPV16 and HPV32, two predictions were given: one using only sequencing reads mapping to HPV16, and one using only sequencing reads mapping to HPV32. Like this it became possible to interpret the results finely from a virological point of view, as the inventors could discriminate which HPV was responsible of the lesion.

## Results:

### Evaluation of transport medium for RNA conservation

The stability of total RNA from cervical cells at room temperature was evaluated in four solutions: PreservCyt (Hologic), the most widely used solution for gynecological specimen collection; NovaPrep HQ+ Solution (Novaprep), a competitor product used for cells and DNA recovery but never evaluated for RNA conservation; RNA Protect Cell Reagent (Qiagen), a popular solution for RNA stability; and NucliSens Lysis Buffer (BioMérieux), a lysis buffer part of the NucliSens automated acid nucleic procedure which has been described as a RNA stabilizer. The amount of spiked HPV16-positive cervical squamous cell carcinoma cells (SiHa) was calibrated to be representative of a cervical smear. After 48h at room temperature, RT-qPCR measurement of cellular and viral transcripts showed no or little RNA loss in PreservCyt, only limited RNA degradation (<1 log) in RNA Protect and NucliSens Lysis Buffer, and a marked RNA loss in NovaPrep HQ+ Solution (>2 log). After 7 days and up to 21 days, only the PreservCyt solution provided RNA quality with a limited RNA degradation pattern as indicated by the detection of 18S and 28S rRNA. The inventors therefore decided to use the PreservCyt solution to collect the gynecological specimen of the study.

### HPV RNA-Seq AmpliSeq custom panel

Transcriptomic maps known for HPV1620 and HPV1821 were used to predict unknown but likely splice donor and splice acceptor sites for HPV31, 33, 35, 39, 45, 51, 52, 56, 58, 59, 66, 68, 73, and 82 (Figures 1 and 2). The resulting reconstructed transcripts, as well as HPV genomic sequences, were used as a template for the design of an Ion AmpliSeq panel targeting 16 high-risk or putative high-risk HPV and named HPV RNA-Seq. Putative breakpoints in HPV genomes, and 30 human cellular genes used as internal controls, were also added to the design. In total, 750 sequences are targeted by a single mix made of 525 unique primers (Table 1 and Tableau 2A-2E).

### Samples, RNA & sequencing

72 gynecological samples (HSIL=37; LSIL=35) coming from 3 different French centers (Angers, Kremlin-Bicêtre and Tours) and collected in PreservCyt solution were processed with RNA extraction using a method designed to recover total RNA from as little as a single cell (PicoPure RNA Isolation kit, Thermo Fisher Scientific, USA). In most of the cases total RNA was measurable using a Nanodrop (70/72 positive, average on positive RNA eluates = 18 ng/$\mu$L) and was detectable on a Bioanalyzer pico RNA chip with a pattern indicating RNA degradation (63/72 positive, average RNA Integrity Number on positive = 2.2). RT-qPCR performed for all samples on ACTB mRNA (amplicon size = 210bp) and GAPDH mRNA (amplicon size = 226bp) indicated that RNA quality was compatible with amplification of 200-250bp size fragments (ACTB mRNA average Ct=27.8; GAPDH mRNA average Ct=30.1). Samples that failed passing this initial RT-PCR quality control were not sequenced. qPCR performed after omitting the reverse transcription step (RT-) were also run and showed in general no or little traces of residual genomic DNA (ACTB DNA average Ct=38.4 ; GAPDH DNA average Ct=35.6). Note, the presence of residual cellular DNA or HPV DNA in RNA preparation is not a major concern since the AmpliSeq assay can differentiate between HPV transcripts and genomic sequences. AmpliSeq libraries were initiated from total RNA and were positive after 21 cycles of amplification for 55 samples (i.e. detectable on a Bioanalyzer HS DNA chip). Attempts to add one or two amplification cycles did not bring any significant improvement to the results (data not shown). In total, 55 patients (HSIL=27; LSIL=28), plus SiHa HPV16-positive cells as a control, had been sequenced on Ion Proton. The sequencing reads were aligned to the target sequences and read counts were generated. An average of 2.4 million usable reads per sample was reached (min=0.02M; max=8.3M), among which an average of 2.1 million reads mapped to the human sequences (hg) used as internal controls (min=0.01M; max=8.06M). The detection of highly expressed human sequences in all samples, even though inter-sample variations were observed, contributed to validate the sequencing procedure, which is important especially for the interpretation of HPV-negative samples. Rare non-zero values were also observed for some of the numerous HPV-human fusion sequences (fus) that were hypothesized but were all false positives, identified as such because only half of the reference sequences were covered by reads.

HPV RNA-Seq used for HPV detection and genotyping

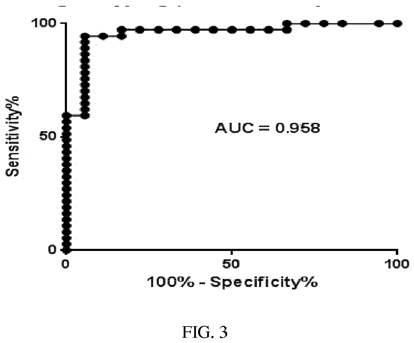

The first application of HPV RNA-Seq is to detect the presence in a given sample of any of the 16 high-risk or putative high-risk HPV targeted by the panel. The number of reads mapping to HPV-specific amplicons (i.e. the sum of categories "sp", "unsp" and "gen") was used to detect the presence of a given HPV genotype. To help determining a threshold for detection, we took as a reference a HPV DNA test validated for clinical use (PapilloCheck, Greiner Bio-One GmbH). The best sensitivity and specificity values between the two tests were obtained for threshold of 100-200 reads (Figure 3). For example, a threshold value of 150 reads resulted in a Sensitivity (Se(HPV-DNA)) of 97.3%, a Specificity (Sp(HPV-DNA)) of 83.3%, leading to a Positive Predictive Value (PPV(HPV-DNA)) of 92.3% and a Negative Predictive Value (NPV(HPV-DNA)) of 93.8% for detecting high-risk HPV in this population composed of around 50% of HSIL and 50% of LSIL (Table 3). Table 3 shows the performances of HPV RNA-Seq for HPV detection vs HPV DNA (PapilloCheck) at threshold value of 150 reads. Sensitivity (Se), Specificity (Sp), Positive Predictive Value (PPV) and Negative Predictive Value (NPV) are given. HPV+ means that at least one HPV genotype is identified in a patient..

Table 3

|  |  | HPV DNA | |  |  |
|---|---|---|---|---|---|
|  |  | HPV+ | HPV- | **Se**(HPV-DNA) | 97,3% |
| HPV RNA-Seq | HPV+ | 36 | 3 | **Sp**(HPV-DNA) | 83,3% |
|  | HPV - | 1 | 15 | **PPV**(HPV-DNA) | 92,3% |
|  |  |  |  | **NPV**(HPV-DNA) | 93,8% |

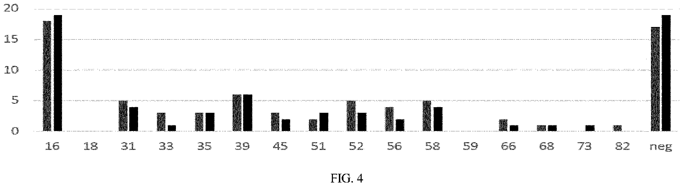

A more detailed view of the genotypes identified by both techniques is given in Figure 4. The number of mono-infected, multi-infected, or HPV-negative samples identified by the two tests is summarized in Table 4. Note that, because the HPV DNA test can detect the 16 high-risk or putative high-risk HPV captured by HPV RNA-Seq (HPV16, 18, 31, 33, 35, 39, 45, 51, 52, 56, 58, 59, 66, 68, 73, and 82) plus 8 additional low-risk HPV (HPV6, 11, 40, 42, 43, 44/45, 53 and 70), the comparison was based only on the 16 HPV common to both tests.

Table 4

|  | HPV RNA-Seq | HPV DNA |
|---|---|---|
| Mono-infected samples | 26 | 27 |
| Multi-infected samples | 13 | 10 |
| HPV-negative samples | 16 | 18 |

Using a threshold value of 150 reads, HPV RNA-Seq detected two more positive patients than the HPV DNA test (n=39 vs n=37, Table 3). HPV RNA-Seq identified the presence of more than one HPV for three more patients than the HPV DNA test (n=13 vs n=10 multi-infected samples, Table 4). Globally, HPV16 was found at a slightly weaker occurrence by HPV RNA-Seq (n=18 vs n=19) in favor of other genotypes such as HPV31, 33, 45, 52, 56, 58 or 66 which were less commonly found by the HPV DNA test (HPV31 n=5 vs n=4; HPV33 n=3 vs n=1; HPV45 n=3 vs n=2; HPV52 n=5 vs n=3; HPV56 n=4 vs n=2; HPV58 n=5 vs n=4; HPV66 n=2 vs n=1, Figure 4). Apart from HPV16, only HPV51 was less frequently found by HPV RNA-Seq than by HPV DNA (n=2 vs n=3). The cellular model (SiHa) gave only HPV16 signal in both tests, as expected.

HPV RNA-Seq used as a marker of high-grade cytology

The inventors conducted an exploratory analysis on 20 of the mono-infected samples in which they showed that HPV RNA splice junctions could be used to predict high-grade cytology. They focused the analysis on amplicons capturing splice junctions (category "sp") to be sure to detect HPV transcripts. However, the number of mono-infected samples (n=20) used as training set was small, in particular the number of samples of LSIL (n=5). LOO cross-validation was used to pick the lambda giving the minimum cross-validated error using ridge regularization. Lambda = 0.08 gave a mean cross-validated error of 15%. The inventors also computed a 20% prediction error using nested cross-validation. This error rate can be seen as an indicator of how the model could fit future datasets. The inventors used the corresponding parameter to fit a regularized logistic regression model, assigning a coefficient to each amplicon (Table 5) and a probability of being of high-grade to each sample (Table 6). In table 5, the first and fourth columns give the id of the splice junction captured by the amplicon, the second column gives the coefficient assigned by the logistic regression, the third column indicate whether the splice junction comes from a "late" or "early" transcript.

Table 5

| junction | Coefficient | name_transcript_category | name_transcript_contents |
|---|---|---|---|
| (Intercept) | 0,468298365 | | |
| SD2_SA10 | -0,693322203 | Late | L1 |
| SD3_SA4 | 0,545728771 | Early | (E1) E4 E5 |
| SD1_SA4 | 0,387642812 | Early | (E6) E2 E5 |
| SD2_SA4 | -0,262522618 | Early | (E7) E2 E5 |
| SD1_SA2 | 0,146954179 | Early | E6 E7 |
| SD2_SA5 | 0,12050536 | Early | (E7) E2 E5 |
| SD1_SA6 | 0,107204358 | Early | (E6) E4 E5 |
| SD5_SA10 | 0,096088118 | Late | L1 |
| SD3_SA6 | 0,093052957 | Early | (E1) E4 E5 |
| SD1_SA5 | 0,092877361 | Early | (E6) E2 E5 |
| SD2_SA6 | -0,088655106 | Early | (E7) E4 E5 |
| SD1_SA1 | 0,07669912 | Early | E6 E7 |
| SD1_SA3 | 0,069688722 | Early | E6 E7 |
| SD2_SA8 | 0,061867993 | Early | (E7) E4 E5 |
| SD3_SA5 | 0,051702326 | Early | (E1) E4 E5 |
| SD2_SA9 | -0,040972141 | Late | L1 |
| SD5_SA9 | -0,026083777 | Late | L1 |
| SD3_SA8 | 0 | Early | (E1) E4 E5 |

Table 6

| sample | prediction_score | prediction_class | prediction_class | prediction_accuracy |
|---|---|---|---|---|
| IonXpress_039_115 | 0,115 | -1 | LSIL | TRUE |
| IonXpress_033_730 | 0,204 | -1 | LSIL | TRUE |
| IonXpress_038_114 | 0,259 | -1 | LSIL | TRUE |
| 1492 | 0,425 | -1 | LSIL | TRUE |
| IonXpress_019_2613 | 0,562 | 1 | LSIL | FALSE |
| IonXpress_027_598 | 0,653 | 1 | HSIL | TRUE |
| 729 | 0,716 | 1 | HSIL | TRUE |
| 567 | 0,718 | 1 | HSIL | TRUE |
| IonXpress_018_2439 | 0,902 | 1 | HSIL | TRUE |
| 610 | 0,904 | 1 | HSIL | TRUE |
| 1066 | 0,911 | 1 | HSIL | TRUE |
| IonXpress_034_758 | 0,919 | 1 | HSIL | TRUE |
| 1122 | 0,934 | 1 | HSIL | TRUE |
| 25 | 0,944 | 1 | HSIL | TRUE |

(continued)

| sample | prediction_score | prediction_class | prediction_class | prediction_accuracy |
|---|---|---|---|---|
| IonXpress_037_1267 | 0,947 | 1 | HSIL | TRUE |
| IonXpress_024_26 | 0,965 | 1 | HSIL | TRUE |
| IonXpress_025_538 | 0,97 | 1 | HSIL | TRUE |
| 752 | 0,976 | 1 | HSIL | TRUE |
| IonXpress_021_443 | 0,984 | 1 | HSIL | TRUE |
| 2612 | 0,993 | 1 | HSIL | TRUE |

Table 6 shows the classification results of the (ridge) logistic regression. The first column gives the sample id, the second column gives the probability estimate that the sample is HSIL, the third and fourth columns gives the corresponding prediction, the fifth column contains TRUE if the prediction is consistent with the grade evaluated by cytology.

The grade of the 20 mono-infected samples was classified correctly, except for one observation (Table 5). It is interesting to note that this unique misclassified sample (IonXpress_019_2613), which was classified LSIL by the cytological analysis, was further found as containing a mixture of LSIL and HSIL lesions after histological examination performed more than one year after the sampling done for HPV RNA-Seq/cytology sampling.

The estimated model was then used to classify the 13 multi-infected samples, with each HPV species present within one sample being classified individually for its implication in HSIL development. If at least one HPV species gave a HSIL prediction, the sample was considered to be HSIL. We calculated performances for HSIL prediction for all samples, considering as not being of high-grade both the six samples without sufficient coverage of the splice junctions and the 16 HPV-negative samples not exceeding the threshold of HPV detection. The calculated performances for HSIL prediction in comparison to cytology for the 55 patients (mono-infected, multi-infected and HPV-negative) were Se(cyto)=66.7%, Sp(cyto)=85.7%, PPV(cyto)=81.8% and NPV(cyto)=72.7% (Table 7A). The performances were also calculated for the subset of 39 samples having at least one HPV identified by HPV RNA-Seq, giving in this case Se(cyto/HR+)=94.7%, Sp(cyto/HR+)=80.0%, PPV(cyto/HR+)=81.8% and NPV(cyto/HR+)=94.1% (Table 7B). In table 7, Sensitivity (Se), Specificity (Sp), Positive Predictive Value (PPV) and Negative Predictive Value (NPV) are given. "Not HSIL" means that either no HPV was detected in the sample by HPV RNA-Seq or that none of the HPV genotypes detected were given HSIL prediction.

Table 7

| A | | Cytology | | | |
|---|---|---|---|---|---|
| | | HSIL | LSIL | **Se**(cyto) | 66,7% |
| HPV RNA-Seq | HSIL | 18 | 4 | **Sp**(cyto) | 85,7% |
| | Not HSIL | 9 | 24 | **PPV**(cyto) | 81,8% |
| | | | | **NPV**(cyto) | 72,7% |
| | | | | | |
| | | | | | |
| B | | Cytology | | | |
| | | HSIL | LSIL | **Se**(cyto/HR+) | 94,7% |
| HPVRNA-Seq HR+ | HSIL | 18 | 4 | **Sp**(cyto/HR+) | 80,0% |
| | Not HSIL | 1 | 16 | **PPV**(cyto/HR+) | 81,8% |
| | | | | **NPV**(cyto/HR+) | 94,1% |

Note that the ratio HSIL to LSIL remained similar between these two populations (around 1:1), making the comparison of the PPV and the NPV possible. Finally a summary of the results for HPV detection and genotyping (HPV RNA-Seq vs HPV DNA) and high-grade cytology prediction (HPV RNA-Seq vs cytology), including posterior histological data of cervix biopsies when available, is presented in Table 8.

Table 8

| Sample name | HPV DNA | Genotyping | Marker of HSIL | | | | | Cytology | Histology | Time (days) cyto-histo |
|---|---|---|---|---|---|---|---|---|---|---|
| | | HPV RNA-Seq | | | | | | | | |
| | | Per patient | Per HPV | | | Per patient | | | | |
| | | Detection | Not enough coverage on splice junctions | Not HSIL | HSIL | Prediction | | | | |
| D-15-0041_1066_BC13 | 16 | 16 | | | 16 | HSIL | | HSIL | HSIL | 55 |
| D-15-0041_1122_BC14 | 16 | 16 | | | 16 | HSIL | | HSIL | HSIL | 130 |
| D-15-0041_1124_BC5 | 16,39 | 16,39 | 39 | 16 | | Not HSIL | | LSIL | HSIL | [70-434] |
| D-15-0041_1490_BC6 | 16,39 | 16,35,39 | | 39 | 16,35 | HSIL | | LSIL | HSIL | 67 |
| D-15-0041_1492_BC7 | 16 | 16 | | 16 | | Not HSIL | | LSIL | LSIL | 81 |
| D-15-0041_151_BC15 | 16,(53) | 16 | | | 16 | HSIL | | LSIL | HSIL | 130 |
| D-15-0041_152_BC16 | 16,(42) | 16,52,82 | 16,52,82 | | | Not HSIL | | LSIL | LSIL | 41 |
| D-15-0041-2209-BC11 | 16,(42),52 | 16,39,52 | 39 | 16,52 | | Not HSIL | | LSIL | HSIL | n.d. |
| D-15-0041_250_BC12 | 16,39,(42) | 16,39 | | 16,39 | | Not HSIL | | LSIL | LSIL | 55 |
| D-15-0041_25_BC4 | 16 | 16 | | | 16 | HSIL | | HSIL | HSIL | 75 |
| D-15-0041_2612_BC8 | 16 | 16 | | | 16 | HSIL | | HSIL | n.d. | n.d. |
| D-15-0041_567_BC9 | 16 | 16 | | | 16 | HSIL | | HSIL | HSIL | n.d. |
| D-15-0041_610_BC2 | 16 | 16 | | | 16 | HSIL | | HSIL | HSIL | 113 |
| D-15-0041_729_BC3 | 16 | 16 | | | 16 | HSIL | | HSIL | HSIL | 59 |
| D-15-0041_752_BC10 | 16 | 16 | | | 16 | HSIL | | HSIL | HSIL | 444 |
| IonXpress_017_2437 | (43),51 | 51 | | 51 | | Not HSIL | | LSIL | LSIL | 195 |
| IonXpress_017_251 | neg | neg | | | | Not HSIL | | HSIL | LSIL | 85 |
| IonXpress_018_2439 | 58 | 58 | | | 58 | HSIL | | HSIL | LSIL | 164 |
| IonXpress_018_440 | neg | neg | | | | Not HSIL | | LSIL | LSIL | 38 |
| IonXpress_019_2613 | 16 | 16 | | | 16 | HSIL | | LSIL | HSIL | [416-780] |
| IonXpress_020_3137 | (53) | 56 | 56 | | | Not HSIL | | HSIL | HSIL | 350 |
| IonXpress_021_10 | 56,(44/55) | 56 | | 56 | | Not HSIL | | LSIL | n.d. | 130 |

(continued)

| Sample name | HPV DNA | HPV RNA-Seq | | | | | | Cytology | Histology | Time (days) cyto-histo |
| | | Genotyping | Marker of HSIL | | | | | | | |
| | | Per patient | Per HPV | | | Per patient | | | | |
| | | Detection | Not enough coverage on splice junctions | Not HSIL | HSIL | Prediction | | | | |
|---|---|---|---|---|---|---|---|---|---|---|
| IonXpress_021_443 | 58 | 33,58 | 33 | | 58 | HSIL | HSIL | LSIL | 99 | |
| IonXpress_022_23 | neg | neg | | | | Not HSIL | HSIL | HSIL | n.d. | |
| IonXpress_022_444 | 16,33 | 16,33 | | 33 | 16 | HSIL | HSIL | HSIL | 69 | |
| IonXpress_023_24 | (6),(11),(53) | neg | | | | Not HSIL | HSIL | HSIL | [0-13] | |
| IonXpress_023_536 | neg | neg | | | | Not HSIL | LSIL | LSIL | 101 | |
| IonXpress_024_26 | 45 | 45 | | | 45 | HSIL | HSIL | HSIL | 106 | |
| IonXpress_024_537 | neg | neg | | | | Not HSIL | LSIL | LSIL | 71 | |
| IonXpress_025_457 | neg | neg | | | | Not HSIL | LSIL | LSIL | 278 | |
| IonXpress_025_538 | 35 | 31,35 | 31 | | 35 | HSIL | HSIL | HSIL | 191 | |
| IonXpress_026_539 | neg | neg | | | | Not HSIL | LSIL | n.d. | n.d. | |
| IonXpress_026_565 | 16 | neg | | | | Not HSIL | HSIL | HSIL | 65 | |
| IonXpress_027_598 | 31 | 31 | | | 31 | HSIL | HSIL | HSIL | 52 | |
| IonXpress_028_609 | 35,52 | 52 | | | 52 | HSIL | LSIL | HSIL | 83 | |
| IonXpress_029_611 | neg | neg | | | | Not HSIL | HSIL | n.d. | n.d. | |
| IonXpress_030_612 | neg | neg | | | | Not HSIL | LSIL | LSIL | 113 | |
| IonXpress_031_613 | 35,39,(44/55) | 35,39 | | 35,39 | | Not HSIL | LSIL | LSIL | 83 | |
| IonXpress_032_728 | neg | neg | | | | Not HSIL | HSIL | HSIL | 59 | |
| IonXpress_033_730 | 31 | 31 | | 31 | | Not HSIL | LSIL | HSIL | [211-575] | |
| IonXpress_034_758 | 58 | 58 | | | 58 | HSIL | HSIL | HSIL | 43 | |
| IonXpress_035_1150 | 16,39,52 | 16,39,52 | | 52 | 16,39 | HSIL | HSIL | HSIL | 125 | |
| IonXpress_036_1151 | (11),31 | 31 | | | 31 | HSIL | HSIL | HSIL | 125 | |

| | | HPV RNA-Seq | | | | | | | |
| | | Genotyping | Marker of HSIL | | | | | | |
| | | Per patient | Per HPV | | | Per patient | | | |
| Sample name | HPV DNA | Detection | Not enough coverage on splice junctions | Not HSIL | HSIL | Prediction | Cytology | Histology | Time (days) cyto-histo |
|---|---|---|---|---|---|---|---|---|---|
| IonXpress_036_98 | (42) | neg | | | | Not HSIL | LSIL | n.d. | 20 |
| IonXpress_037_100 | neg | neg | | | | Not HSIL | LSIL | LSIL | 57 |
| IonXpress_037_1267 | 45 | 45 | | | 45 | HSIL | HSIL | LSIL | 71 |
| IonXpress_038_114 | 31 | 31 | | 31 | | Not HSIL | LSIL | HSIL | 154 |
| IonXpress_038_1597 | neg | neg | | | | Not HSIL | HSIL | HSIL | 85 |
| IonXpress_039_115 | 56 | 56 | | 56 | | Not HSIL | LSIL | LSIL | 34 |
| IonXpress_039_1598 | neg | neg | | | | Not HSIL | HSIL | LSIL | 115 |
| IonXpress_041_1650 | 66,(70) | 56,66 | 56,66 | | | Not HSIL | LSIL | LSIL | 115 |
| IonXpress_043_1871 | 51,58,68,73 | 33,51,58,68 | 33 | 51,58,68 | | Not HSIL | LSIL | LSIL | 101 |
| IonXpress_044_2064 | 39,51 | 45 | 45 | | | Not HSIL | LSIL | HSIL | 129 |
| IonXpress_045_2065 | neg | 52,58 | 52,58 | | | Not HSIL | LSIL | LSIL | 160 |
| IonXpress_046_2066 | (6) | 66 | 66 | | | Not HSIL | LSIL | HSIL | 99 |

**EP 3 715 477 A1**

**HPV RNA-Seq used as a triage test**

The performances of HPV RNA-Seq as a triage test were evaluated using histology as gold standard. Results from histological examination were, however, not available for all patients. The time interval separating HPV RNA-Seq/cytology tests from histological analysis, varying between 0 and 780 days, was another limitation in this study. To try to overcome these drawbacks, we compared the performances of HPV RNA-Seq vs histology to the performances of cytology vs histology, considering either all available samples or only samples for which histology was done less than 3 months after HPV RNA-Seq/cytology or only samples for which histology was done less than 6 months after HPV RNA-Seq/cytology. In addition and for each category, we made the distinction between the performances obtained when HPV RNA-Seq HPV-positive and HPV-negative patients were grouped together or when only HPV-positive patients were considered. Calculation of the PPV as a function of HSIL prevalence in the population was also done.

**REFERENCES:**

Throughout this application, various references describe the state of the art to which this invention pertains. The disclosures of these references are hereby incorporated by reference into the present application.

**Claims**

1.  A composition of primers for detecting high grade squamous intraepithelial lesion (HSIL) comprising a first set of primers, called splice junctions set of primers, which comprises:

   - at least 2 pairs of primers of a first subset of HPV16 specific pairs of primers having the nucleic acid sequence selected from the group consisting of SEQ ID NO: 1-2 to SEQ ID NO: 27-28;
   and
   - at least 2 pairs of primers of a second subset of HPV18 specific pairs of primers having the nucleic acid sequence selected from the group consisting of SEQ ID NO: 29-30 to SEQ ID NO: 63-64;
   and
   - at least 2 pairs of primers of a third subset of HPV31 specific pairs of primers having the nucleic acid sequence selected from the group consisting of SEQ ID NO: 65-66 to SEQ ID NO: 91-92;
   and
   - at least 2 pairs of primers of a fourth subset HPV33 specific of pairs of primers having the nucleic acid sequence selected from the group consisting of SEQ ID NO: 93-94 to SEQ ID NO: 117-118;
   and
   - at least 2 pairs of primers of a fifth subset of HPV35 specific pairs of primers having the nucleic acid sequence selected from the group consisting of SEQ ID NO: 119-120 to SEQ ID NO: 145-146;
   and
   - at least 2 pairs of primers of a sixth subset of HPV39 specific pairs of primers having the nucleic acid sequence selected from the group consisting of SEQ ID NO: 147-148 to SEQ ID NO: 165-166;
   and
   - at least 2 pairs of primers of a seventh subset of HPV45 specific pairs of primers having the nucleic acid sequence selected from the group consisting of SEQ ID NO: 167-168 to SEQ ID NO: 193-194;
   and
   - at least 2 pairs of primers of a eighth subset of HPV51 specific pairs of primers having the nucleic acid sequence selected from the group consisting of SEQ ID NO: 195-196 to SEQ ID NO: 213-214;
   and
   - at least 2 pairs of primers of a ninth subset of HPV52 specific pairs of primers having the nucleic acid sequence selected from the group consisting of SEQ ID NO: 215-216 to SEQ ID NO: 245-246;
   and
   - at least 2 pairs of primers of a tenth subset of HPV56 specific pairs of primers having the nucleic acid sequence selected from the group consisting of SEQ ID NO: 247-248 to SEQ ID NO: 277-278;
   and
   - at least 2 pairs of primers of an eleventh subset of HPV58 specific pairs of primers having the nucleic acid sequence selected from the group consisting of SEQ ID NO: 279-280 to SEQ ID NO: 303-304;
   and
   - at least 2 pairs of primers of a twelfth subset of HPV59 specific pairs of primers having the nucleic acid sequence selected from the group consisting of SEQ ID NO: 305-306 to SEQ ID NO: 331-332;

and
- at least 2 pairs of primers of a thirteenth subset of HPV66 specific pairs of primers having the nucleic acid sequence selected from the group consisting of SEQ ID NO: 333-334 to SEQ ID NO: 361-362.

2. The composition of primers for HSIL according to claim 1, wherein the splice junctions set of primers further comprises:

- at least 2 pairs of primers of a fourteenth subset of HPV68 specific pairs of primers having the nucleic acid sequence selected from the group consisting of SEQ ID NO: 363-364 to SEQ ID NO: 381-382; and/or
- at least 2 pairs of primers of a fifteenth subset of HPV73 specific pairs of primers having the nucleic acid sequence selected from the group consisting of SEQ ID NO: 383-384 to SEQ ID NO: 407-408; and/or
- at least 2 pairs of primers of a sixteenth subset of HPV82 specific pairs of primers having the nucleic acid sequence selected from the group consisting of SEQ ID NO: 409-410 to SEQ ID NO: 429-430.

3. The composition of primers for detecting HSIL according to claim 1 or 2, wherein the splice junctions set of primers comprises at least 10, preferably 10 pairs of primers of the first to the thirteenth subsets of pairs of primers as defined in claim 1 and optionally at least 10, preferably 10 pairs of primers of the fourteenth and/or the fifteenth and/or the sixteenth subsets of pairs of primers as defined in claim 2.

4. A composition of primers for detecting HSIL comprising a first set of pairs of primers, called splice junctions set of primers, which comprises:

- at least 2 pairs of primers of a first subset of HPV16 specific pairs of primers able to produce the amplicons having the nucleic acid sequence selected from the group consisting of SEQ ID NO: 1501 to SEQ ID NO: 1514; and
- at least 2 pairs of primers of a second subset of HPV18 specific pairs of primers able to produce the amplicons having the nucleic acid sequence selected from the group consisting of SEQ ID NO: 1515 to SEQ ID NO: 1532; and
- at least 2 pairs of primers of a third subset of HPV31 specific pairs of primers able to produce the amplicons having the nucleic acid sequence selected from the group consisting of SEQ ID NO: 1533 to SEQ ID NO: 1546; and
- at least 2 pairs of primers of a fourth subset of HPV33 specific pairs of primers able to produce the amplicons having the nucleic acid sequence selected from the group consisting of SEQ ID NO:1547 to SEQ ID NO:1559; and
- at least 2 pairs of primers of a fifth subset of HPV35 specific pairs of primers able to produce the amplicons having the nucleic acid sequence selected from the group consisting of SEQ ID NO: 1560 to SEQ ID NO: 1573; and
- at least 2 pairs of primers of a sixth subset of HPV39 specific pairs of primers able to produce the amplicons having the nucleic acid sequence selected from the group consisting of SEQ ID NO: 1574 to SEQ ID NO: 1583; and
- at least 2 pairs of primers of a seventh subset of HPV45 specific pairs of primers able to produce the amplicons having the nucleic acid sequence selected from the group consisting of SEQ ID NO: 1584 to SEQ ID NO: 1597; and
- at least 2 pairs of primers of a eighth subset HPV51 specific of pairs of primers able to produce the amplicons having the nucleic acid sequence selected from the group consisting of SEQ ID NO: 1598 to SEQ ID NO: 1607; and
- at least 2 pairs of primers of a ninth subset of HPV52 specific pairs of primers able to produce the amplicons having the nucleic acid sequence selected from the group consisting of SEQ ID NO: 1608 to SEQ ID NO: 1623; and
- at least 2 pairs of primers of a tenth subset HPV56 specific of pairs of primers able to produce the amplicons having the nucleic acid sequence selected from the group consisting of SEQ ID NO: 1624 to SEQ ID NO: 1639; and
- at least 2 pairs of primers of an eleventh subset HPV58 specific of pairs of primers able to produce the amplicons having the nucleic acid sequence selected from the group consisting of SEQ ID NO:1640 to SEQ ID NO:1652; and
- at least 2 pairs of primers of a twelfth subset of HPV59 specific pairs of primers able to produce the amplicons having the nucleic acid sequence selected from the group consisting of SEQ ID NO: 1653 to SEQ ID NO: 1666;

and
- at least 2 pairs of primers of a thirteenth subset of HPV66 specific pairs of primers able to produce the amplicons having the nucleic acid sequence selected from the group consisting of SEQ ID NO: 1667 to SEQ ID NO: 1681.

5. The composition of primers for detecting HSIL according to claim 4, wherein the splice junctions set of primers further comprises:

- at least 2 pairs of primers of a fourteenth subset of HPV68 specific pairs of primers able to produce the amplicons having the nucleic acid sequence selected from the group consisting of SEQ ID NO: 1682 to SEQ ID NO: 1691;
and/or
- at least 2 pairs of primers of a fifteenth subset of HPV73 specific pairs of primers able to produce the amplicons having the nucleic acid sequence selected from the group consisting of SEQ ID NO: 1692 to SEQ ID NO: 1704;
and/or
- at least 2 pairs of primers of a sixteenth subset of HPV82 specific pairs of primers able to produce the amplicons having the nucleic acid sequence selected from the group consisting of SEQ ID NO: 1705 to SEQ ID NO: 1715.

6. The composition of primers for detecting HSIL according to claim 4 or 5, wherein the splice junctions set of primers comprises at least 10, preferably 10 pairs of primers of the first to the thirteenth subsets of pairs of primers as defined in claim 4 and optionally at least at least 10, preferably 10 pairs of primers of the fourteenth and/or the fifteenth and/or the sixteenth subsets of pairs of primers as defined in claim 5.

7. A kit for detecting HSIL comprising:

- the composition of primers for detecting HSIL as defined in claims 1 to 6;
and
- optionally reagents for a cDNA amplification.

8. Use of the composition of primers as defined in claims 1 to 6 or of the kit as defined in claim 7 for detecting HSIL.

9. An *in vitro* method for detecting HSIL in a biological sample comprising the steps of:

- (a) extraction of RNA from the biological sample,
- (b) reverse transcription of the RNA so as to generate cDNA,
- (c) amplification of the cDNA generated at step (b) with the composition of primers as defined in any of claims 1-6 so as to produce amplicon(s),
- (d) quantifying the expression level of each amplicon produced at step (c),
- (e) determining if the biological sample comprises HSIL based on the expression level of the amplicons quantified at step (d).

10. The *in vitro* method for detecting HSIL according to claim 9, wherein the step of determining if the biological sample comprises HSIL comprises:

- for each type of HPV selected from the group consisting of HPV16, HPV18, HPV31, HPV33, HPV35, HPV39, HPV45, HPV51, HPV52, HPV56, HPV58, HPV59, and HPV66 and optionally HPV68 and/or HPV73 and/or HPV82, a step calculating a probability $p_{HPVj}$ that the biological sample comprises an HSIL of $HPV_j$ type wherein $j$ =16, 18, 31, 33, 35, 39, 45, 51, 52, 56, 58, 59, 66, 68, 73 and/or 82 using the following formula:

$$logit(p_{HPVj}) = \beta_0 + \sum_{i=1}^{25}(\beta_i X_{ij})$$

with:

$\beta_0$ is the intercept,
$\beta_i$ is a coefficient corresponding to a given splice junction, called splice junction i,
$X_{ij}$ is a variable depending on the quantified level of expression of the amplicon corresponding to the splice junction i for the HPV$_j$,
wherein if one p$_{HPVj}$ is higher than 0.5, it is indicative of the presence of an HPV$_j$ HSIL in the biological sample.

11. The *in vitro* method for detecting HSIL according to claim 10, wherein:

the amplicons corresponding to the splice junction i=1 are respectively SEQ ID NO: 1501 for HPV16, SEQ ID NO: 1515 for HPV18, SEQ ID NO: 1533 for HPV31, SEQ ID NO: 1547 for HPV33, SEQ ID NO: 1560 for HPV35, SEQ ID NO: 1574 for HPV39, SEQ ID NO: 1584 for HPV45, SEQ ID NO: 1598 for HPV51, SEQ ID NO: 1608 for HPV52, SEQ ID NO: 1624 for HPV56, SEQ ID NO: 1640 for HPV58, SEQ ID NO: 1653 for HPV59, SEQ ID NO: 1667 for HPV66, SEQ ID NO: 1682 for HPV68, SEQ ID NO: 1692 for HPV73 and SEQ ID NO: 1705 for HPV82,
the amplicons corresponding to the splice junction i=2 are respectively SEQ ID NO: 1502 for HPV16, SEQ ID NO: 1516 for HPV18, SEQ ID NO: 1534 for HPV31, SEQ ID NO: 1548 for HPV33, SEQ ID NO: 1561 for HPV35, absent for HPV39, SEQ ID NO: 1585 for HPV45, absent for HPV51, SEQ ID NO: 1609 for HPV52, SEQ ID NO: 1625 for HPV56, SEQ ID NO: 1641 for HPV58, SEQ ID NO: 1654 for HPV59, SEQ ID NO: 1668 for HPV66, absent for HPV68, absent for HPV73 and absent for HPV82,
the amplicons corresponding to the splice junction i=3 are respectively SEQ ID NO: 1503 for HPV16, SEQ ID NO: 1517 for HPV18, SEQ ID NO: 1535 for HPV31, SEQ ID NO: 1549 for HPV33, SEQ ID NO: 1562 for HPV35, absent for HPV39, absent for HPV45, SEQ ID NO: 1599 for HPV51, SEQ ID NO: 1610 for HPV52, SEQ ID NO: 1626 for HPV56, SEQ ID NO: 1642 for HPV58, absent for HPV59, SEQ ID NO: 1669 for HPV66, SEQ ID NO: 1683 for HPV68, SEQ ID NO: 1693 for HPV73 and SEQ ID NO: 1706 for HPV82,
the amplicons corresponding to the splice junction i=4 are respectively SEQ ID NO: 1504 for HPV16, SEQ ID NO: 1518 for HPV18, SEQ ID NO: 1536 for HPV31, SEQ ID NO: 1550 for HPV33, SEQ ID NO: 1563 for HPV35, SEQ ID NO: 1576 for HPV39, SEQ ID NO: 1587 for HPV45, SEQ ID NO: 1600 for HPV51, SEQ ID NO: 1611for HPV52, SEQ ID NO: 1627 for HPV56, SEQ ID NO: 1643 for HPV58, SEQ ID NO: 1656 for HPV59, SEQ ID NO: 1671 for HPV66, SEQ ID NO: 1684 for HPV68, SEQ ID NO: 1694 for HPV7 and SEQ ID NO: 1707 for HPV82,
the amplicons corresponding to the splice junction i=5 are respectively SEQ ID NO: 1505 for HPV16, SEQ ID NO: 1519 for HPV18, SEQ ID NO: 1537 for HPV31, SEQ ID NO: 1551 for HPV33, SEQ ID NO: 1564 for HPV35, absent for HPV39, SEQ ID NO: 1588 for HPV45, absent for HPV51, SEQ ID NO: 1612 for HPV52, SEQ ID NO: 1628 for HPV56, SEQ ID NO: 1644 for HPV58, SEQ ID NO: 1657 for HPV59, SEQ ID NO: 1672 for HPV66, absent for HPV68, absent for HPV73 and absent for HPV82,
the amplicons corresponding to the splice junction i=6 are respectively SEQ ID NO: 1506 for HPV16, SEQ ID NO: 1520 for HPV18, SEQ ID NO: 1538 for HPV31, SEQ ID NO: 1552 for HPV33, SEQ ID NO: 1565 for HPV35, absent for HPV39, absent for HPV45, SEQ ID NO: 1601 for HPV51, SEQ ID NO: 1613 for HPV52, SEQ ID NO: 1629 for HPV56, SEQ ID NO: 1645 for HPV58, absent for HPV59, absent for HPV66, SEQ ID NO: 1686 for HPV68, SEQ ID NO: 1695 for HPV73 and SEQ ID NO: 1708 for HPV82,
the amplicons corresponding to the splice junction i=7 are respectively SEQ ID NO: 1507 for HPV16, SEQ ID NO: 1521 for HPV18, SEQ ID NO: 1539 for HPV31, SEQ ID NO: 1553 for HPV33, SEQ ID NO: 1566 for HPV35, SEQ ID NO: 1578 for HPV39, SEQ ID NO: 1590 for HPV45, SEQ ID NO: 1602 for HPV51, SEQ ID NO: 1614 for HPV52, SEQ ID NO: 1630 for HPV56, SEQ ID NO: 1646 for HPV58, absent for HPV59, SEQ ID NO: 1674 for HPV66, SEQ ID NO: 1685 for HPV68, SEQ ID NO: 1696 for HPV73 and SEQ ID NO: 1709 for HPV82
the amplicons corresponding to the splice junction i=8 are respectively SEQ ID NO: 1508 for HPV16, absent for HPV18, SEQ ID NO: 1540 for HPV31, SEQ ID NO: 1554 for HPV33, absent for HPV35, absent for HPV39, absent for HPV45, absent for HPV51, SEQ ID NO: 1615 for HPV52, SEQ ID NO: 1631 for HPV56, SEQ ID NO: 1647 for HPV58, absent for HPV59, SEQ ID NO: 1675 for HPV66, absent for HPV68, SEQ ID NO: 1697 for HPV73 and SEQ ID NO: 1710 for HPV82,
the amplicons corresponding to the splice junction i=9 are respectively SEQ ID NO: 1509 for HPV16, SEQ ID NO: 1522 for HPV18, SEQ ID NO: 1541 for HPV31, absent for HPV33, absent for HPV35, SEQ ID NO: 1579 for HPV39, SEQ ID NO: 1591 for HPV45, SEQ ID NO: 1603 for HPV51, SEQ ID NO: 1616 for HPV52, SEQ ID NO: 1632 for HPV56, absent HPV58, SEQ ID NO: 1659 for HPV59, SEQ ID NO: 1676 for HPV66, SEQ ID NO: 1687 for HPV68, SEQ ID NO: 1698 for HPV73 and SEQ ID NO: 1711 for HPV82
the amplicons corresponding to the splice junction i=10 are respectively absent for HPV16, SEQ ID NO: 1523 for HPV18, absent for HPV31, absent for HPV33, absent for HPV35, absent for HPV39, absent for HPV45, absent for HPV51, absent for HPV52, absent for HPV56, absent for HPV58, absent for HPV59, absent for HPV66, absent for HPV68, absent for HPV73 and absent for HPV82,
the amplicons corresponding to the splice junction i=11 are respectively SEQ ID NO: 1510 for HPV16, SEQ ID NO: 1524 for HPV18, SEQ ID NO: 1542 for HPV31, SEQ ID NO: 1555 for HPV33, SEQ ID NO: 1567 for HPV35, SEQ ID NO: 1580 for HPV39, SEQ ID NO: 1592 for HPV45, SEQ ID NO: 1604 for HPV51, SEQ ID NO: 1617 for HPV52, SEQ ID NO: 1633 for HPV56, SEQ ID NO: 1648 for HPV58, SEQ ID NO: 1660 for HPV59, SEQ ID NO: 1677 for HPV66, SEQ ID NO: 1688 for HPV68, SEQ ID NO: 1699 for HPV73 and SEQ ID NO: 1712 for HPV82,

the amplicons corresponding to the splice junction i=12 are respectively absent for HPV16, SEQ ID NO: 1525 for HPV18, absent for HPV31, absent for HPV33, SEQ ID NO: 1568 or HPV35, absent for HPV39, absent for HPV45, absent for HPV51, SEQ ID NO: 1618 for HPV52, SEQ ID NO: 1634 for HPV56, absent for HPV58, SEQ ID NO: 1661 for HPV59, absent for HPV66, absent for HPV68, SEQ ID NO: 1700 for HPV73, absent for HPV82,

the amplicons corresponding to the splice junction i=13 are respectively absent for HPV16, SEQ ID NO: 1526 for HPV18, absent for HPV31, absent for HPV33, absent for HPV35, absent for HPV39, SEQ ID NO: 1593 for HPV45, absent for HPV51, absent for HPV52, absent for HPV56, absent for HPV58, absent for HPV59, absent for HPV66, absent for HPV68, absent for HPV73 and absent for HPV82,

the amplicons corresponding to the splice junction i=14 are respectively absent for HPV16, SEQ ID NO: 1527 for HPV18, absent for HPV31, absent for HPV33, absent for HPV35, absent for HPV39, absent for HPV45, absent for HPV51, absent for HPV52, absent for HPV56, absent for HPV58, absent for HPV59, absent for HPV66, absent for HPV68, absent for HPV73 and absent for HPV82,

the amplicons corresponding to the splice junction i=15 are respectively SEQ ID NO: 1511 for HPV16, SEQ ID NO: 1528 for HPV18, SEQ ID NO: 1543 for HPV31, SEQ ID NO: 1556 for HPV33, SEQ ID NO: 1569 for HPV35, SEQ ID NO: 1581 for HPV39, SEQ ID NO: 1594 for HPV45, SEQ ID NO: 1605 for HPV51, SEQ ID NO: 1619 for HPV52, SEQ ID NO: 1635 for HPV56, SEQ ID NO: 1649 for HPV58, SEQ ID NO: 1662 for HPV59, SEQ ID NO: 1678 for HPV66, SEQ ID NO: 1689 for HPV68, SEQ ID NO: 1701 for HPV73 and SEQ ID NO: 1713 for HPV82,

the amplicons corresponding to the splice junction i=16 are respectively SEQ ID NO: 1512 for HPV16, SEQ ID NO: 1529 for HPV18, SEQ ID NO: 1544 for HPV31, SEQ ID NO: 1557 for HPV33, SEQ ID NO: 1570 for HPV35, absent for HPV39, SEQ ID NO: 1595 for HPV45, absent for HPV51, SEQ ID NO: 1620 for HPV52, SEQ ID NO: 1636 for HPV56, SEQ ID NO: 1650 for HPV58, SEQ ID NO: 1663 for HPV59, SEQ ID NO: 1679 for HPV66, absent for HPV68, absent for HPV73 and absent for HPV82,

the amplicons corresponding to the splice junction i=17 are respectively SEQ ID NO: 1513 for HPV16, absent for HPV18, SEQ ID NO: 1545 for HPV31, SEQ ID NO: 1558 for HPV33, SEQ ID NO: 1571 for HPV35, absent for HPV39, absent for HPV45, SEQ ID NO: 1606 for HPV51, SEQ ID NO: 1621 for HPV52, SEQ ID NO: 1637 for HPV56, SEQ ID NO: 1651 for HPV58, absent for HPV59, absent for HPV66, SEQ ID NO: 1690 for HPV68, SEQ ID NO: 1702 for HPV73 and SEQ ID NO: 1714 for HPV82,

the amplicons corresponding to the splice junction i=18 are respectively SEQ ID NO:1514 for HPV16, SEQ ID NO: 1531 for HPV18, SEQ ID NO: 1546 for HPV31, SEQ ID NO: 1559 for HPV33, SEQ ID NO: 1572 for HPV35, SEQ ID NO: 1583 for HPV39, SEQ ID NO: 1597 for HPV45, SEQ ID NO: 1607 for HPV51, SEQ ID NO: 1622 for HPV52, SEQ ID NO: 1638 for HPV56, SEQ ID NO: 1652 for HPV58, SEQ ID NO: 1665 for HPV59, SEQ ID NO: 1681 for HPV66, SEQ ID NO: 1691 for HPV68, SEQ ID NO: 1703 for HPV73 and SEQ ID NO: 1715 for HPV82,

the amplicons corresponding to the splice junction i=19 are respectively absent for HPV16, SEQ ID NO: 1532 for HPV18, absent for HPV31, absent for HPV33, SEQ ID NO: 1573 for HPV35, absent for HPV39, absent for HPV45, absent for HPV51, SEQ ID NO: 1623 for HPV52, SEQ ID NO: 1639 for HPV56, absent for HPV58, SEQ ID NO: 1666 for HPV59, absent for HPV66, absent for HPV68, SEQ ID NO: 1704 for HPV73 and absent for HPV82

the amplicons corresponding to the splice junction i=20 are respectively absent for HPV16, absent for HPV18, absent for HPV31, absent for HPV33, absent for HPV35, SEQ ID NO: 1577 for HPV39, absent for HPV45, absent for HPV51, absent for HPV52, absent for HPV56, absent for HPV58, SEQ ID NO: 1658 for HPV59, absent for HPV66, absent for HPV68, absent for HPV73 and absent for HPV82,

the amplicons corresponding to the splice junction i=21 are respectively absent for HPV16, absent for HPV18, absent for HPV31, absent for HPV33, absent for HPV35, SEQ ID NO: 1582 for HPV39, absent for HPV45, absent for HPV51, absent for HPV52, absent for HPV56, absent for HPV58, SEQ ID NO: 1664 for HPV59, absent for HPV66, absent for HPV68, absent for HPV73 and absent for HPV82,

the amplicons corresponding to the splice junction i=22 are respectively absent for HPV16, absent for HPV18, absent for HPV31, absent for HPV33, absent for HPV35, SEQ ID NO: 1575 for HPV39, absent for HPV45, absent for HPV51, absent for HPV52, absent for HPV56, absent for HPV58, SEQ ID NO: 1655 for HPV59, absent for HPV66, absent for HPV68, absent for HPV73 and absent for HPV82,

the amplicons corresponding to the splice junction i=23 are respectively absent for HPV16, absent for HPV18, absent for HPV31, absent for HPV33, absent for HPV35, absent for HPV39, SEQ ID NO: 1586 for HPV45, absent for HPV51, absent for HPV52, absent for HPV56, absent for HPV58, absent for HPV59, SEQ ID NO: 1670 for HPV66, absent for HPV68, absent for HPV73 and absent for HPV82,

the amplicons corresponding to the splice junction i=24 are respectively absent for HPV16, absent for HPV18, absent for HPV31, absent for HPV33, absent for HPV35, absent for HPV39, SEQ ID NO: 1589 for HPV45, absent for HPV51, absent for HPV52, absent for HPV56, absent for HPV58, absent for HPV59, SEQ ID NO:

1673 for HPV66, absent for HPV68, absent for HPV73 and absent for HPV82,
the amplicons corresponding to the splice junction i=25 are respectively absent for HPV16, SEQ ID NO: 1530 for HPV18, absent for HPV31, absent for HPV33, absent for HPV35, absent for HPV39, SEQ ID NO: 1596 HPV45, absent for HPV51, absent for HPV52, absent for HPV56, absent for HPV58, absent for HPV59, SEQ ID NO: 1680 for HPV66, absent for HPV68, absent for HPV73 and absent for HPV82.

12. A composition of primers for typing HPV comprising the splice junctions set of primers as defined in claims 1-6 and an additional set of primers selected from the group consisting of:

- a second set of primers, called unsplice junctions set of primers wherein the primers target high risk and optionally of putative high risk HPV genomic regions spanning either splice donor or splice acceptor sites in the absence of any splice event,
- a third set of primers, called genomic set of primers wherein the primers target high risk and optionally of putative high risk HPV genomic regions away from any splice donor or splice acceptor sites, and
- a fourth set of primers, called fusion set of primers wherein the primers target high risk and optionally of putative high risk HPV fusion transcripts.

13. A kit for HPV typing comprising:

- the composition of primers for HPV typing according to claim 12 and optionally reagents for cDNA amplification.

14. Use of the composition of the primers according to claim 12 or of the kit according to claim 13 for HPV typing.

15. An *in vitro* method for HPV typing in a biological sample comprising the steps of:

- (a) extraction of RNA from the biological sample,
- (b) reverse transcription of the RNA so as to generate cDNA,
- (c) amplification of the cDNA generated at step (b) with the primers as defined in claim 12 so as to produce amplicon(s),
- (d) quantification of the expression level of each amplicon and
- (e) for each HPV type, a step of:

- comparing the expression level of all amplicons specific of said HPV type with a reference value,

wherein if the expression level of all the amplicons specific of said HPV type is higher than the reference value, it is indicative of the presence of said HPV type in the biological sample.

FIG. 1

| | 5'- | SD1 | SA1 | SA2 | SA3 | SD2 | SD3 | SA4 | SA5 | *put bkpt* | SD4 | SA6 | SA7 | SA8 | *put bkpt* | SD5 | pA early | SD6 | SA9 | SA10 | pA late | -3' |
|---|---|---|---|---|---|---|---|---|---|---|---|---|---|---|---|---|---|---|---|---|---|---|
| HPV16 | | 226 | 409 | 526 | 742 | 880 | 1301 | 2581 | 2708 | 2869 | | 3357 | | | 3619 | 3631 | 4218 | | 5637 | | 7265 | |
| HPV18 | | 233 | 416 | | 791 | 929 | 1357 | 2651 | 2779 | 2943 | 3165 | 3434 | 3440 | 3465 | 3684 | 3696 | 4240 | 3786 | 5613 | 5776 | 7283 | |
| HPV31 | | 230 | 413 | 530 | 740 | 877 | 1296 | 2518 | 2646 | 2807 | | 3285 | | | 3578 | 3590 | 4143 | | 5552 | | 7232 | |
| HPV33 | | 231 | 414 | 531 | | 894 | 1316 | 2575 | 2702 | 2863 | | 3351 | | | 3577 | 3589 | 4181 | | 5594 | | 7291 | |
| HPV35 | | 232 | 415 | | | 883 | 1305 | 2543 | 2670 | 2831 | | 3319 | | | 3584 | 3596 | 4191 | | 5601 | 5767 | 7292 | |
| HPV39 | | 235 | 418 | | 802 | 943 | 1368 | 2636 | | 2927 | | 3418 | 3424 | | 3677 | 3689 | 4248 | | 5643 | | 7266 | |
| HPV45 | | 230 | 413 | | 791 | 929 | 1357 | 2610 | 2737 | 2901 | | 3392 | 3398 | 3423 | 3648 | 3660 | 4233 | 3750 | 5608 | | 7315 | |
| HPV51 | | 217 | 402 | | 751 | 886 | 1302 | 2548 | | 2834 | | 3319 | | | 3572 | 3584 | 4109 | | 5521 | | 7174 | |
| HPV52 | | 224 | 407 | 524 | 738 | 879 | 1301 | 2569 | 2696 | 2857 | | 3345 | | | 3613 | 3625 | 4231 | | 5643 | 5810 | 7344 | |
| HPV56 | | 233 | 416 | 533 | 773 | 910 | 1295 | 2572 | 2699 | 2861 | | 3349 | | | 3629 | 3641 | 4189 | | 5597 | 5759 | 7282 | |
| HPV58 | | 232 | 415 | 532 | | 898 | 1320 | 2579 | 2706 | 2867 | | 3355 | | | 3596 | 3608 | 4214 | | 5643 | | 7301 | |
| HPV59 | | 183 | | | 749 | 887 | 1306 | 2571 | 2698 | 2862 | | 3353 | 3359 | | 3615 | 3627 | 4228 | | 5606 | 5769 | 7260 | |
| HPV66 | | 233 | 416 | 533 | 773 | 910 | 1290 | 2555 | 2682 | 2843 | | 3331 | | 3362 | 3605 | 3617 | 4241 | | 5647 | | 7300 | |
| HPV68 | | 129 | 312 | | 697 | 838 | 1233 | 2510 | | 2801 | | 3292 | | | 3551 | 3563 | 4096 | | 5488 | | 7153 | |
| HPV73 | | 227 | 410 | 527 | 727 | 862 | 1287 | 2570 | | 2858 | | 3346 | | | 3560 | 3572 | 4059 | | 5494 | 5647 | 7204 | |
| HPV82 | | 222 | 407 | 521 | 753 | 888 | 1316 | 2575 | | 2860 | | 3345 | | | 3601 | 3613 | 4140 | | 5571 | | 7219 | |

FIG. 2

FIG. 3

FIG. 4

Europäisches
Patentamt

European
Patent Office

Office européen
des brevets

# EUROPEAN SEARCH REPORT

Application Number

EP 19 30 5394

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2016/005905 A2 (PASTEUR INSTITUT [FR] ET AL.) 14 January 2016 (2016-01-14) <br> * claims 6,32,36 * <br> * page 50, paragraph 3 - page 54, paragraph 2 * <br> * page 74 - page 76; table 10 * <br> * page 77, paragraph 17.4 * <br> * page 82, paragraph 17.11-17.12 * <br> * page 83 - page 107; table 14 * | 1-15 | INV. <br> C12Q1/70 <br> C12Q1/6886 |
| X | WO 2010/052317 A1 (DKFZ KREBSFORSCHUNGSZENTRUM [DE]; SCHMITT MARKUS [DE] ET AL.) 14 May 2010 (2010-05-14) <br> * claims 8-9 * <br> * page 4, last paragraph * <br> * page 8, paragraph 3 * <br> * page 17, last paragraph * <br> * page 19, paragraph 2 * <br> * page 29, paragraph 3 - page 30, paragraph 2 * <br> * page 34, paragraph 2 - page 35, paragraph 2 * <br> * page 44, paragraph 4 * <br> * example 3; table 3 * <br> * page 69 - page 70; example 5 * <br> * figure 1 * | 1-15 | |

-----

-----

-/--

TECHNICAL FIELDS
SEARCHED (IPC)

C12Q

The present search report has been drawn up for all claims

| Place of search | Date of completion of the search | Examiner |
|---|---|---|
| Munich | 7 August 2019 | Eveleigh, Anna |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

**Application Number**

EP 19 30 5394

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | WO 2011/138234 A2 (DEUTSCHES KREBSFORSCH [DE]; SCHMITT MARKUS [DE] ET AL.) 10 November 2011 (2011-11-10) * claims 14-17 * * page 5, last paragraph * * page 6, last paragraph * * page 8, paragraph 3 * * page 9 - page 10 * * page 16, paragraph 2 * * page 18 - page 19 * * page 29 - page 30; example 1 * ----- | 1-15 | |
| X | HÖFLER DANIELA ET AL: "HPV16 RNA patterns defined by novel high-throughput RT-qPCR as triage marker in HPV-based cervical cancer precursor screening", GYNECOLOGIC ONCOLOGY, vol. 138, no. 3, 3 July 2015 (2015-07-03), pages 676-682, XP029262016, ISSN: 0090-8258, DOI: 10.1016/J.YGYNO.2015.06.039 * abstract * * page 677, left-hand column * * page 678, right-hand column, last paragraph * * page 681, right-hand column, last paragraph * * figure 1 * ----- | 1-15 | |

TECHNICAL FIELDS
SEARCHED       (IPC)

-/--

The present search report has been drawn up for all claims

| Place of search | Date of completion of the search | Examiner |
|---|---|---|
| Munich | 7 August 2019 | Eveleigh, Anna |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

Europäisches
Patentamt

European
Patent Office

Office européen
des brevets

# EUROPEAN SEARCH REPORT

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ROOSMARIJN LUTTMER ET AL: "Comparing triage algorithms using HPV DNA genotyping, HPV E7 mRNA detection and cytology in high-risk HPV DNA-positive women", JOURNAL OF CLINICAL VIROLOGY, vol. 67, June 2015 (2015-06), pages 59-66, XP055611223, NL ISSN: 1386-6532, DOI: 10.1016/j.jcv.2015.04.004 * the whole document * | 1-15 | |
| A | SHULING LIU ET AL: "Separate analysis of human papillomavirus E6 and E7 messenger RNAs to predict cervical neoplasia progression", PLOS ONE, vol. 13, no. 2, 21 February 2018 (2018-02-21), page e0193061, XP55611245, DOI: 10.1371/journal.pone.0193061 * the whole document * | 1-15 | TECHNICAL FIELDS SEARCHED (IPC) |
| A | WO 2012/047897 A2 (SEQUENOM INC [US]; STOERKER JAY [US]) 12 April 2012 (2012-04-12) * claims 3-6,26 * * page 3, paragraph 2-3 * * page 4, paragraph 2 * * page 5, paragraph 6; figure 5 * * page 6, paragraph 3 * | 1-15 | |

The present search report has been drawn up for all claims

| Place of search | Date of completion of the search | Examiner |
|---|---|---|
| Munich | 7 August 2019 | Eveleigh, Anna |

EPO FORM 1503 03.82 (P04C01)

## ANNEX TO THE EUROPEAN SEARCH REPORT
## ON EUROPEAN PATENT APPLICATION NO.

EP 19 30 5394

This annex lists the patent family members relating to the patent documents cited in the above-mentioned European search report.
The members are as contained in the European Patent Office EDP file on
The European Patent Office is in no way liable for these particulars which are merely given for the purpose of information.

07-08-2019

| Patent document cited in search report | | Publication date | Patent family member(s) | | Publication date |
|---|---|---|---|---|---|
| WO 2016005905 | A2 | 14-01-2016 | EP | 3167079 A2 | 17-05-2017 |
| | | | US | 2017107582 A1 | 20-04-2017 |
| | | | WO | 2016005789 A1 | 14-01-2016 |
| | | | WO | 2016005905 A2 | 14-01-2016 |
| WO 2010052317 | A1 | 14-05-2010 | CA | 2742917 A1 | 14-05-2010 |
| | | | CN | 102272329 A | 07-12-2011 |
| | | | EP | 2184368 A1 | 12-05-2010 |
| | | | EP | 2352845 A1 | 10-08-2011 |
| | | | JP | 5823868 B2 | 25-11-2015 |
| | | | JP | 2012507999 A | 05-04-2012 |
| | | | JP | 2016013139 A | 28-01-2016 |
| | | | US | 2012040334 A1 | 16-02-2012 |
| | | | WO | 2010052317 A1 | 14-05-2010 |
| WO 2011138234 | A2 | 10-11-2011 | CA | 2798224 A1 | 10-11-2011 |
| | | | CN | 103314117 A | 18-09-2013 |
| | | | CN | 106191270 A | 07-12-2016 |
| | | | EP | 2566986 A2 | 13-03-2013 |
| | | | JP | 6189748 B2 | 30-08-2017 |
| | | | JP | 2013534806 A | 09-09-2013 |
| | | | JP | 2016182139 A | 20-10-2016 |
| | | | US | 2011275059 A1 | 10-11-2011 |
| | | | US | 2018094326 A1 | 05-04-2018 |
| | | | WO | 2011138234 A2 | 10-11-2011 |
| WO 2012047897 | A2 | 12-04-2012 | US | 2014220554 A1 | 07-08-2014 |
| | | | WO | 2012047897 A2 | 12-04-2012 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- **LIN C et al.** Human papillomavirus types from infection to cancer in the anus, according to sex and HIV status: a systematic review and meta-analysis. *Lancet Infect Dis,* 2018, vol. 18, 198-206
- **CHATURVEDI AK et al.** Human papillomavirus and rising oropharyngeal cancer incidence in the United States. *J Clin Oncol Off J Am Soc Clin Oncol,* 2011, vol. 29, 4294-301
- **WALBOOMERS JM et al.** Human papillomavirus is a necessary cause of invasive cervical cancer worldwide. *J Pathol,* 1999, vol. 189, 12-9
- **SCHIFFMAN M et al.** S. Carcinogenic human papillomavirus infection. *Nat Rev Dis Primer,* 2016, vol. 2, 16086
- **WOODMAN CBJ et al.** The natural history of cervical HPV infection: unresolved issues. *Nat Rev Cancer,* 2007, vol. 7, 11-22
- **DOORBAR J et al.** The biology and life-cycle of human papillomaviruses. *Vaccine,* 2012, vol. 5, F55-70
- **SHULZHENKO N et al.** Ménage à trois: an evolutionary interplay between human papillomavirus, a tumor, and a woman. *Trends Microbiol,* 2014, vol. 22, 345-53
- **TORNESELLO ML et al.** Viral and cellular biomarkers in the diagnosis of cervical intraepithelial neoplasia and cancer. *BioMed Res Int,* 2013, vol. 2013, 519619
- **OGILVIE GS et al.** Effect of Screening With Primary Cervical HPV Testing vs Cytology Testing on High-grade Cervical Intraepithelial Neoplasia at 48 Months: The HPV FOCAL Randomized Clinical Trial. *JAMA,* 2018, vol. 320, 43-52
- **CUZICK J et al.** Comparing the performance of six human papillomavirus tests in a screening population. *Br J Cancer,* 2013, vol. 108, 908-13
- **VIRTANEN E et al.** Performance of mRNA- and DNA-based high-risk human papillomavirus assays in detection of high-grade cervical lesions. *Acta Obstet Gynecol Scand,* 2017, vol. 96, 61-8
- **COOK DA et al.** Aptima HPV Assay versus Hybrid Capture® 2 HPV test for primary cervical cancer screening in the HPV FOCAL trial. *J Clin Virol Off Publ Pan Am Soc Clin Virol,* 2017, vol. 87, 23-9
- **GE Y et al.** Aptima Human Papillomavirus E6/E7 mRNA Test Results Strongly Associated With Risk for High-Grade Cervical Lesions in Follow-Up Biopsies. *J Low Genit Tract Dis,* 2018, vol. 22, 195-200

- **DE THURAH L et al.** Concordant testing results between various human papillomavirus assays in primary cervical cancer screening: systematic review. *Clin Microbiol Infect Off Publ Eur Soc Clin Microbiol Infect Dis,* 2018, vol. 24, 29-36
- **HAWKES D et al.** Not all HPV nucleic acid tests are equal: only those calibrated to detect high grade lesions matter for cervical screening. *Clin Microbiol Infect Off Publ Eur Soc Clin Microbiol Infect Dis,* 2018, vol. 24, 436-7
- **DE THURAH L et al.** Not all HPV nucleic acid tests are equal: only those calibrated to detect high grade lesions matter for cervical screening: Response to "Concordant testing results between various human papillomavirus assays in primary cervical cancer screening: systematic review" Published 27 May, 2017. *Clin Microbiol Infect Off Publ Eur Soc Clin Microbiol Infect Dis,* 2018, vol. 24, 438-9
- **J. SAMBROOK ; D.W. RUSSELL.** Molecular Cloning: A Laboratory Manual. Cold Spring Harbor Laboratory Press, 2001
- Short Protocols in Molecular Biology. Current Protocols, 2002
- **CHEN et al.** Evolution and Taxonomic Classification of Alphapapillomavirus 7 Complete Genomes: HPV18, HPV39, HPV45, HPV59, HPV68 and HPV70. *PLOS ONE,* 2013, vol. 8, e72565
- **KEARSE M. et al.** Geneious Basic: an integrated and extendable desktop software platform for the organization and analysis of sequence data. *Bioinforma Oxf Engl,* 2012, vol. 28, 1647-9
- **ZHENG Z-M et al.** Papillomavirus genome structure, expression, and post-transcriptional regulation. *Front Biosci J Virtual Libr,* 2006, vol. 11, 2286-302
- **WANG X et al.** Construction of a full transcription map of human papillomavirus type 18 during productive viral infection. *J Virol,* 2011, vol. 85, 8080-92
- **WENTZENSEN N et al.** Characterization of viral-cellular fusion transcripts in a large series of HPV16 and 18 positive anogenital lesions. *Oncogene,* 2002, vol. 21, 419-2622, 26
- **TANG K-W et al.** The landscape of viral expression and host gene fusion and adaptation in human cancer. *Nat Commun,* 2013, vol. 4, 2513
- **PETER M et al.** MYC activation associated with the integration of HPV DNA at the MYC locus in genital tumors. *Oncogene,* 2006, vol. 25, 5985-93

- **LU X et al.** Multiple-integrations of HPV16 genome and altered transcription of viral oncogenes and cellular genes are associated with the development of cervical cancer. *PloS One,* 2014, vol. 9, e97588

- **KRAUS I et al.** The majority of viral-cellular fusion transcripts in cervical carcinomas cotranscribe cellular sequences of known or predicted genes. *Cancer Res,* 2008, vol. 68, 2514-22
- **FRIEDMAN J ; HASTIE T ; TIBSHIRANI R.** Regularization Paths for Generalized Linear Models via Coordinate Descent. *J Stat Softw,* 2010, vol. 33, 1-22